# EUROPEAN PATENT APPLICATION

(11) **EP 1 847 541 A1**
(43) Date of publication of application: **24.10.2007**
(21) Application number: 06713347.0
(22) Date of filing: 02.02.2006
(51) Int. Cl.: C07D 487/04, A61K 31/519, A61K 31/5377, A61K 31/55, A61P 1/06, A61P 5/00, A61P 13/08, A61P 15/00, A61P 15/08, A61P 15/18, A61P 17/10, A61P 17/14, A61P 25/28, A61P 35/00, A61P 43/00, C07D 495/04, C07D 495/14, C07D 513/04, C07D 519/00

(54) **FUSED PYRIMIDINE DERIVATIVE AND USES THEREOF**

(30) Priority: 03.02.2005 JP 2005027806
(71) Applicant: Takeda Pharmaceutical Company Limited, Osaka-shi, Osaka 541-0045 (JP)
(72) Inventor: HAMAMURA, Kazumasa, TAKEDA PHARMACEUTICAL CO. LTD., Osaka-shi, Osaka, 532-8686 (JP); ODA, Tsuneo, TAKEDA PHARMACEUTICAL CO. LTD., Osaka-shi, Osaka 532-8686 (JP); KAKU, Tomohiro, TAKEDA PHARMACEUTICAL CO. LTD., Osaka-shi, Osaka, 532-8686 (JP); SUZAKI, Tomohiko, TAKEDA PHARMACEUTICAL CO. LTD., Osaka-shi, Osaka 532-8686 (JP)
(74) Representative: Lewin, John Harvey
(86) International application number: PCT/JP2006/302204
(87) International publication number: WO 2006/083005

(57) **Abstract**

A compound represented by the formula (I) 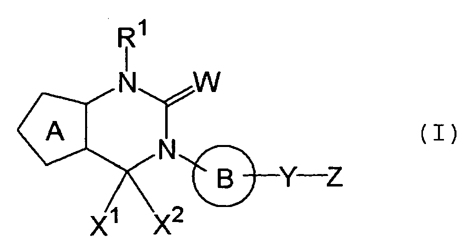 wherein ring A is a 5-membered aromatic heterocycle optionally having substituent(s),
R¹ is a hydrogen atom or a hydrocarbon group optionally having substituent(s),
W is an oxygen atom or a sulfur atom,
X¹ and X² may be the same or different and each is a hydrogen atom, a hydrocarbon group optionally having substituent(s) or a heterocyclic group, or, X¹ and X² in combination optionally form an oxygen atom, a sulfur atom or =NR²,
ring B is an aromatic ring optionally further having substituent(s),
Y is a bond, C₁₋₆ alkylene C₂₋₆ alkenylene or C₂₋₆ alkynylene, optionally having substituent(s), and
Z is a group represented by formula: -SOₙR³, or a group represented by formula: -COR⁴, or a salt thereof, is useful as a pharmaceutical agent having GnRH antagonistic action.

## Description

### Technical Field

The present invention relates to a fused pyrimidine derivative having excellent antagonistic activity against gonadotropin releasing hormone.

### Background Art

Secretion of anterior pituitary hormones is regulated by peripheral hormones secreted from target organs of the respective hormones and by secretion-promoting or secretion-inhibiting hormones secreted from the hypothalamus, which is the upper central organ of the anterior lobe of the pituitary (hereinafter, these hormones are collectively called "hypothalamic hormones" in the present specification). So far, the existence of nine species of hypothalamic hormones has been confirmed, including, for example, thyrotropin releasing hormone (TRH), gonadotropin releasing hormone [GnRH, sometimes called LH-RH (luteinizing hormone releasing hormone)] and the like. These hypothalamic hormones are believed to show their actions via the receptors that are considered to exist in the anterior lobe of the pituitary, and analysis of the receptor gene which is specific to these hormones, including for humans, is being made. Accordingly, antagonists or agonists specifically and selectively acting on these receptors should control the action of the hypothalamic hormones and the secretion of anterior pituitary hormones. As a result, such antagonists or agonists are expected to be useful in preventing or treating anterior pituitary hormone-dependent diseases.

As the compounds having GnRH-antagonizing activity, there are known GnRH-derived linear peptides (USP 5,140,009 and USP 5,171,835), a cyclic hexapeptide derivative (JP-A-61-191698), a bicyclic peptide derivative (Journal of Medicinal Chemistry, vol. 36, p. 3265-3273, 1993) and the like. Non-peptidic compounds having GnRH-antagonizing activity include the compounds described in JP-A-8-295693 (WO 95/28405), JP-A-9-169768 (WO 96/24597), JP-A-9-169735 (WO 97/14682), JP-A-9-169767 (WO 97/14697), JP-A-11-315079 (WO 99/33831), JP-A-2000-219691 (WO 00/00493), JP-A-2001-278884 (WO 00/56739) and JP-A-2002-30087.

Further, the fused pyrimidine rings include the compounds described in WO 03/64429, WO 04/67535, JP-A-10-259176, DE 19650975A, JP-A-9-221476 (WO 95/34540), WO 91/14430, GB 753171A, WO 2001/032621, DE 3420799B, WO 2003/101985, WO 97/40846, WO 2002/064598, Journal of Heterocyclic Chemistry, vol. 32(4), p. 1181-1183 (1995), Journal of the Chemical Society, Perkin Transactions 1, vol. 11, p. 2884-2889 (1981), Acta Chimica Academiae Scientiarum Hungaricae, vol. 107(1), p. 57-66 (1981), Journal of Organic Chemistry, vol. 41(16), p. 2728-2731 (1976), Pharmazie, vol. 30(4), p. 254-255 (1975), Acta Chimica Academiae Scientiarum Hungaricae, vol. 48(1), p. 77-87 (1966), Chemische Berichte, vol. 99(5), p. 1532-1540 (1966), LaboTest Stock catalog, January 2, 2002, Tetrahedron, vol. 59(29), p. 5603-5608 (2003), Journal of Heterocyclic Chemistry, vol. 27(5), p. 1341-4 (1990), Journal of the Chinese Chemical Society, vol. 49(3), p. 407-414 (2002), ChemDiv, Inc. Product Library catalog, and Scientific Exchange Product List.

Particularly, as a pyrimidine compound fused with 5-membered aromatic heterocycle, the following compounds (1) - (34) are known. 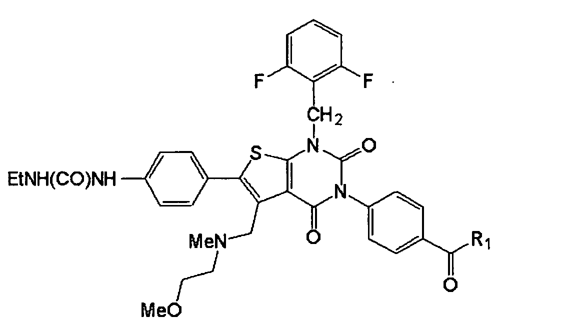 wherein R₁=(1) dimethylamino, (2) methylamino, (3) cyclopropylamino, (4) isopropylamino, (5) amino 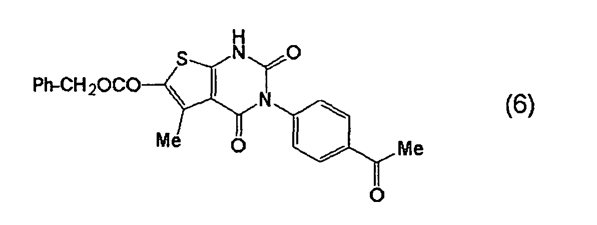 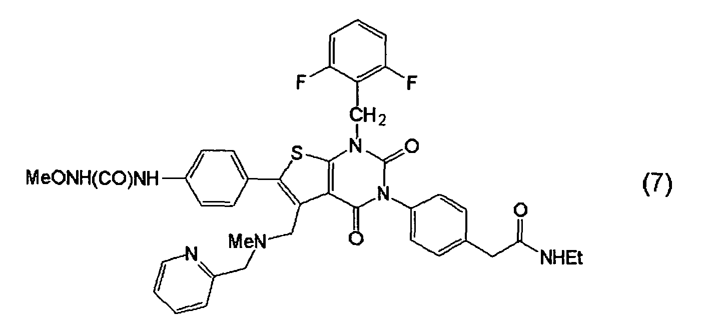 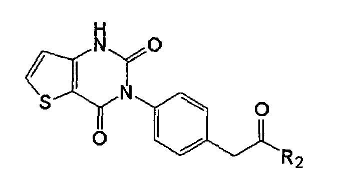 wherein R₂=(8) 1,2,3,4-tetrahydronaphthalen-1-ylamino, (9) cyclohexylamino, (10) benzylamino, (11) 2-phenylethylamino, (12) 4-methoxybenzylamino, (13) 2-chlorobenzylamino, (14) 2-(4-sulfamoylphenyl)ethylamino, (15) cycloheptylamino, (16) 4-(ethoxycarbonyl)piperazin-1-yl, (17) 2-(3,4-dimethoxyphenyl)ethylamino 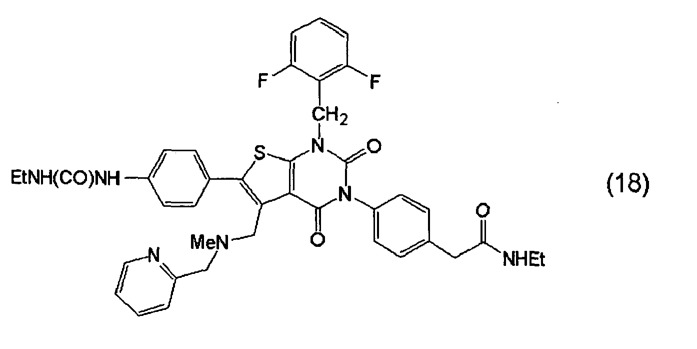 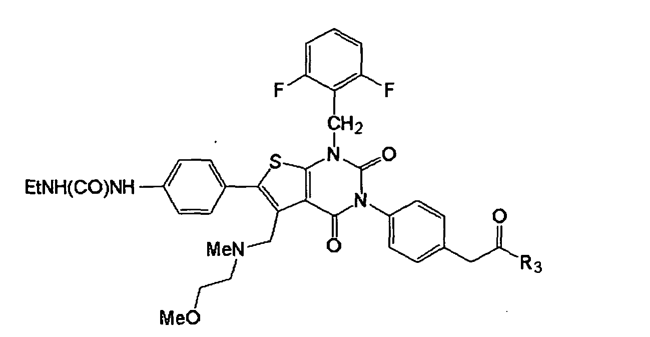 wherein R₃=(19) methylamino, (20) ethylamino, (21) dimethylamino 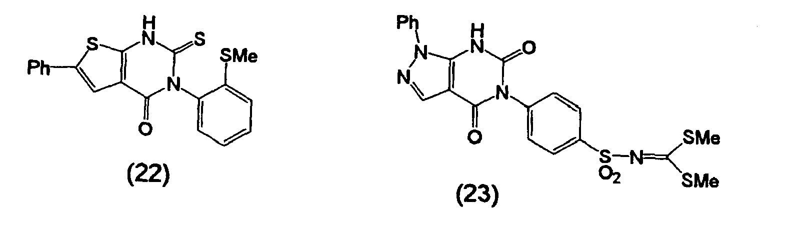 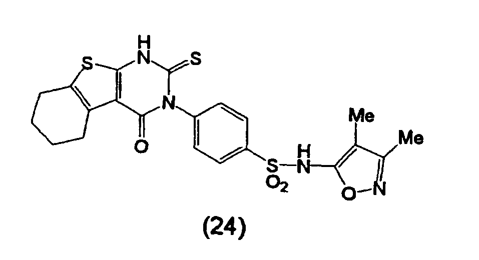 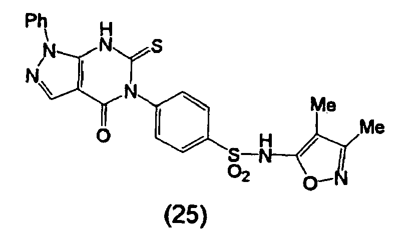 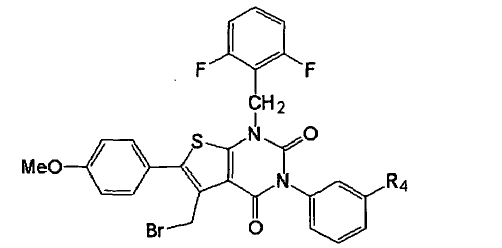 wherein R₄=(26) CH₃-SO₂-, (27) CH₃-SO-, (28) CH₃-S- 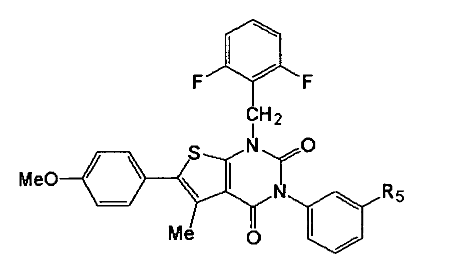 wherein R₅=(29) CH₃-SO₂-, (30) CH₃-SO-, (31) CH₃-S- 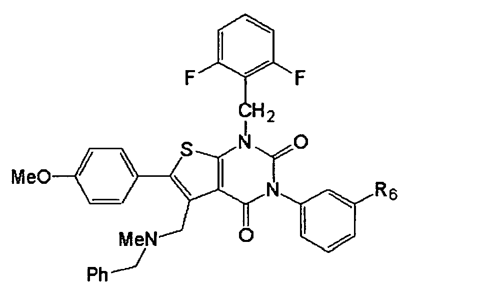 wherein R₆=(32) CH₃-SO₂-, (33) CH₃-SO-, (34) CH₃-S-.

### Disclosure of the Invention

Peptidic compounds have many problems to be resolved in the aspects of oral absorption, administration mode, dosage, drug stability, sustained action, metabolic stability and the like. There is a strong demand for a GnRH antagonist having excellent oral absorption, especially a non-peptidic antagonist, which has excellent therapeutic effect on hormone-dependent diseases such as hormone-dependent cancers such as prostate cancer, endometriosis, precocious puberty and the like, and which does not show transient pituitary-gonadotropic action (acute action).

The present inventors have conducted intensive studies and first synthesized a fused pyrimidine compound represented by the below-mentioned formula (I) [hereinafter sometimes to be abbreviated as compound (I). Compound (I) encompasses a compound represented by the below-mentioned formula (I-a) (hereinafter sometimes to be abbreviated as compound (I-a))] or a salt thereof. In addition, the present inventors have first found that the compound has unexpectedly excellent GnRH antagonizing activity, particularly, strong antagonist activity, based on its specific chemical structure. Moreover, they have also found for the first time that these compounds have low toxicity and are sufficiently satisfactory as medicines having GnRH antagonizing activity, thus completing the invention based on these findings.

Thus, the invention relates to the following [1] -[23] and the like.
[1] A compound represented by the formula (I) 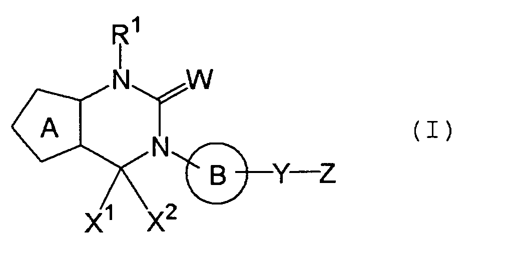 wherein
   ring A is a 5-membered aromatic heterocycle optionally having substituent(s),
   R¹ is a hydrogen atom or a hydrocarbon group optionally having substituent(s),
   W is an oxygen atom or a sulfur atom,
   X¹ and X² may be the same or different and each is a hydrogen atom, a hydrocarbon group optionally having substituent(s) or a heterocyclic group optionally having substituent(s), or, X¹ and
   X² optionally form an oxygen atom, a sulfur atom or =NR² wherein
   R² is a hydrogen atom or a hydrocarbon group optionally having substituent(s) in combination,
   ring B is an aromatic ring optionally further having substituent(s),
   Y is a bond, a C₁₋₆ alkylene optionally having substituent(s), a C₂₋₆ alkenylene optionally having substituent(s) or a C₂₋₆ alkynylene optionally having substituent(s), and
   Z is a group represented by the formula: -SOₙR³ wherein n is 0, 1 or 2, and R³ is an amino optionally having substituent(s), a hydrocarbon group optionally having substituent(s) or a heterocyclic group optionally having substituent(s) (provided that R³ is not a methyl group), or
   a group represented by the formula: -COR⁴ wherein R⁴ is an amino optionally having substituent(s), a hydrocarbon group optionally having substituent(s) or a heterocyclic group optionally having substituent(s);
   provided that when ring B is a ring represented by the formula: 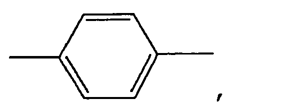
   then the amino optionally having substituent(s) for R³ is a disubstituted amino or a cyclic amino optionally having substituent(s),
   the amino optionally having substituent(s) for R⁴ is a disubstituted amino (except dimethylamino) or a cyclic amino optionally having substituent(s) (except monocyclic piperazin-1-yl optionally having substituent(s)), and
   the hydrocarbon group optionally having substituent(s) for R⁴ is not a methyl group,
      or a salt thereof.
[2] The compound of the above-mentioned [1], wherein ring A is a thiophene ring optionally having substituent(s) or a furan ring optionally having substituent(s).
[3] The compound of the above-mentioned [1], wherein ring A is a 5-membered aromatic heterocycle optionally substituted with substituent(s) selected from the group consisting of (a) a hydrocarbon group optionally having substituent(s), (b) a heterocyclic group optionally having substituent(s), (c) a halogen, (d) a hydroxy, (e) a cyano, (f) a carboxyl, (g) an alkoxycarbonyl, (h) an amino optionally having substituent(s) and (i) a carbamoyl optionally having substituent(s), or optionally substituted with a hydrocarbon group optionally having the above-mentioned substituent(s) (a) - (i).
[4] The compound of the above-mentioned [3], wherein the hydrocarbon group is an alkyl group, a cycloalkyl group, an alkenyl group, a cycloalkenyl group, an alkynyl group, an aralkyl group or a C₁₀₋₁₄ aryl group.
[5] The compound of the above-mentioned [1], wherein X¹ and X² form an oxygen atom, a sulfur atom or =NR² wherein R² is a hydrogen atom or a hydrocarbon group optionally having substituent(s) in combination.
[6] The compound of the above-mentioned [1], wherein n is 2.
[7] The compound of the above-mentioned [1], wherein Y is a bond.
[8] The compound of the above-mentioned [1], wherein ring B is a C₆₋₁₄ aryl ring optionally further having substituent(s) or an aromatic heterocycle optionally further having substituent(s).
[9] The compound of the above-mentioned [1], wherein ring B is a ring represented by the formula: 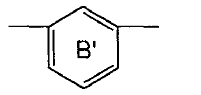 wherein ring B' is a benzene ring optionally further having substituent(s),
   or an aromatic heterocycle optionally further having substituent(s).
[10] The compound of the above-mentioned [1], wherein R¹ is a hydrogen atom, Y is a bond, and X¹ and X² form an oxygen atom, a sulfur atom or =NR² wherein R² is a hydrogen atom or a hydrocarbon group optionally having substituent(s) in combination.
[11] The compound of the above-mentioned [1], wherein ring A is a 5-membered aromatic heterocycle containing, as ring-constituting atom besides carbon atom, 1 to 4 hetero atoms selected from the group consisting of an oxygen atom, a sulfur atom and a nitrogen atom and optionally having substituent(s) selected from the group consisting of (i) - (xi),
   (i) a C₁₋₆ alkyl group optionally having substituent(s) selected from the group consisting of (a) - (d),
      (a) a hydroxy,
      (b) a carboxyl,
      (c) a C₁₋₆ alkoxycarbonyl, and
      (d) an amino optionally mono- or di-substituted with a C₁₋₆ alkyl optionally having substituent(s) selected from the group consisting of a C₁₋₆ alkoxy and a 5- to 16-membered heterocyclic group containing, as ring-constituting atom besides carbon atom, 1 to 4 hetero atoms selected from the group consisting of an oxygen atom, a sulfur atom and a nitrogen atom,
   (ii) a C₂₋₆ alkenyl group optionally having a C₁₋₆ alkoxycarbonyl
   (iii) a C₆₋₁₄ aryl group optionally having a halogen atom,
   (iv) a 5- to 16-membered heterocyclic group containing, as ring-constituting atom besides carbon atom, 1 to 4 hetero atoms selected from the group consisting of an oxygen atom, a sulfur atom and a nitrogen atom and optionally having a C₁₋₆ alkyl,
   (v) a cyano,
   (vi) a carboxyl,
   (vii) a formyl,
   (viii) a C₁₋₆ alkoxycarbonyl,
   (ix) HO-N=CH-,
   (x) a carbamoyl optionally mono- or di-substituted with substituent(s) selected from the group consisting of (a) - (b),
      (a) a C₁₋₆ alkyl group optionally having substituent(s) selected from the group consisting of a hydroxy, a C₁₋₆ alkoxycarbonyl, a carboxyl and a 5- to 16-membered heterocyclic group containing, as ring-constituting atom besides carbon atom, 1 to 4 hetero atoms selected from the group consisting of an oxygen atom, a sulfur atom and a nitrogen atom, and
      (b) a 5- to 16-membered heterocyclic group containing, as ring-constituting atom besides carbon atom, 1 to 4 hetero atoms selected from the group consisting of an oxygen atom, a sulfur atom and a nitrogen atom, and
   (xi) a 5- to 16-membered heterocycle containing, as ring-constituting atom besides carbon atom, 1 to 4 hetero atoms selected from the group consisting of an oxygen atom, a sulfur atom and a nitrogen atom, which is formed by two substituents bonded to each other and optionally has a C₁₋₆ alkoxy group,
      R¹ is a hydrogen atom,
      W is an oxygen atom,
      X¹ and X² are hydrogen atoms, or X¹ and X² form an oxygen atom in combination,
      ring B is a C₆₋₁₄ aryl ring optionally further having a halogen atom, or a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom or a 8- to 16-membered bicyclic or tricyclic fused aromatic heterocycle containing, as ring-constituting atom besides carbon atom, 1 to 12 hetero atoms selected from the group consisting of an oxygen atom, a sulfur atom and a nitrogen atom, each of which optionally further has a halogen atom,
      Y is a bond, and
      Z is
         (i) a group represented by formula: -SOₙR³
            wherein n is 2 and R³ is a 5- to 16-membered cyclic amino, or
         (ii) a group represented by formula: -CONR⁸(R⁹)
            wherein R⁸ is a hydrogen atom, a C₁₋₆ alkyl group or a C₃₋₈ cycloalkyl group, and R⁹ is a hydrogen atom or a C₆₋₁₄ aryl group optionally having substituent(s) selected from the group consisting of a halogen atom and a cyano,
   when ring B is a ring represented by formula: 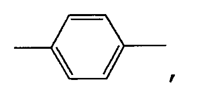 then R⁸ and R⁹ are not hydrogen atoms.
[12] A compound represented by the formula (I-a): 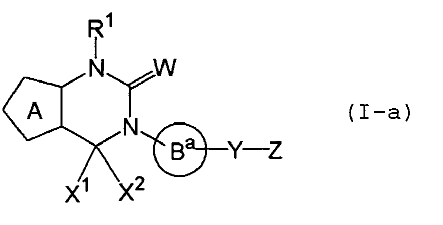 wherein
   ring A is a 5-membered aromatic heterocycle optionally having substituent(s),
   R¹ is a hydrogen atom or a hydrocarbon group optionally having substituent(s),
   W is an oxygen atom or a sulfur atom,
   X¹ and X² may be the same or different and each is a hydrogen atom, a hydrocarbon group optionally having substituent(s) or a heterocyclic group optionally having substituent(s), or X¹ and X² optionally form an oxygen atom, a sulfur atom or =NR² wherein R² is a hydrogen atom or a hydrocarbon group optionally having substituent(s) in combination,
   ring B^{a} is a ring represented by formula: 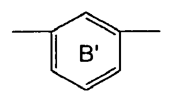
   wherein ring B' is a benzene ring optionally further having substituent(s),
   or an aromatic heterocycle optionally further having substituent(s),
   Y is a bond, a C₁₋₆ alkylene optionally having substituent(s), a C₂₋₆ alkenylene optionally having substituent(s) or a C₂₋₆ alkynylene optionally having substituent(s), and
   Z is a group represented by formula: -SOₙR^{3a} wherein n is 0, 1 or 2, and R^{3a} is an amino optionally having substituent (s), a C₂₋₂₀ hydrocarbon group optionally having substituent(s) or a heterocyclic group optionally having substituent(s), or
   a group represented by formula: -COR⁴ wherein R⁴ is an amino optionally having substituent(s), a hydrocarbon group optionally having substituent(s) or a heterocyclic group optionally having substituent(s),
   or a salt thereof.
[13] The compound of the above-mentioned [12], wherein ring A is a 5-membered aromatic heterocycle containing, as ring-constituting atom besides carbon atom, 1 to 4 hetero atoms selected from the group consisting of an oxygen atom, a sulfur atom and a nitrogen atom and optionally having substituent(s) selected from the group consisting of (i) - (xi),
   (i) a C₁₋₆ alkyl group optionally having substituent(s) selected from the group consisting of (a) - (d),
      (a) a hydroxy,
      (b) a carboxyl,
      (c) a C₁₋₆ alkoxycarbonyl, and
      (d) an amino optionally mono- or di-substituted with a C₁₋₆ alkyl optionally having substituent(s) selected from the group consisting of a C₁₋₆ alkoxy and a 5- to 16-membered heterocyclic group containing, as ring-constituting atom besides carbon atom, 1 to 4 hetero atoms selected from the group consisting of an oxygen atom, a sulfur atom and a nitrogen atom,
   (ii) a C₂₋₆ alkenyl group optionally having a C₁₋₆ alkoxycarbonyl,
   (iii) a C₆₋₁₄ aryl group optionally having a halogen atom,
   (iv) a 5- to 16-membered heterocyclic group containing, as ring-constituting atom besides carbon atom, 1 to 4 hetero atoms selected from the group consisting of an oxygen atom, a sulfur atom and a nitrogen atom and optionally having a C₁₋₆ alkyl (heterocyclic group includes, for example, a 5- to 16-membered aromatic heterocyclic group containing, as ring-constituting atom besides carbon atom, 1 to 4 hetero atoms selected from the group consisting of an oxygen atom, a sulfur atom and a nitrogen atom such as oxadiazole, triazole, tetrazole, oxazole and the like, a 5- to 16-membered nonaromatic heterocyclic group containing, as ring-constituting atom besides carbon atom and nitrogen atom, 1 to 4 hetero atoms selected from the group consisting of an oxygen atom and a sulfur atom such as morpholinyl and the like, and the like, preferably a 5- or 6-membered heterocyclic group),
   (v) a cyano,
   (vi) a carboxyl,
   (vii) a formyl,
   (viii) a C₁₋₆ alkoxycarbonyl,
   (ix) HO-N=CH-,
   (x) a carbamoyl optionally mono- or di-substituted with substituent(s) selected from the group consisting of (a) - (b),
      (a) a C₁₋₆ alkyl group optionally having substituent(s) selected from the group consisting of a hydroxy, a C₁₋₆ alkoxycarbonyl and a 5- to 16-membered heterocyclic group containing, as ring-constituting atom besides carbon atom, 1 to 4 hetero atoms selected from the group consisting of an oxygen atom, a sulfur atom and a nitrogen atom, and
      (b) a 5- to 16-membered heterocyclic group containing, as ring-constituting atom besides carbon atom, 1 to 4 hetero atoms selected from the group consisting of an oxygen atom, a sulfur atom and a nitrogen atom, and
   (xi) a 5- to 16-membered heterocycle containing, as ring-constituting atom besides carbon atom, 1 to 4 hetero atoms selected from the group consisting of an oxygen atom, a sulfur atom and a nitrogen atom, which is formed by two substituents bonded to each other and optionally has a C₁₋₆ alkoxy group,
      R¹ is a hydrogen atom,
      W is an oxygen atom,
      X¹ and X² are hydrogen atoms, or X¹ and X² form an oxygen atom in combination,
      ring B^{a} is a ring represented by formula: 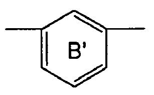 wherein ring B' is a benzene ring optionally further having a halogen atom, or a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom or a 8- to 16-membered bicyclic or tricyclic fused aromatic heterocycle containing, as ring-constituting atom besides carbon atom, 1 to 12 hetero atoms selected from the group consisting of an oxygen atom, a sulfur atom and a nitrogen atom, each of which optionally further has a halogen atom,
      Y is a bond, and
      Z is,
         (i) a group represented by formula: -SOₙR³
            wherein n is 2, and R³ is a 5- to 16-membered cyclic amino, or
         (ii) a group represented by formula: -CONR⁸(R⁹)
            wherein R⁸ is a hydrogen atom, a C₁₋₆ alkyl group or a C₃₋₈ cycloalkyl group, and R⁹ is a hydrogen atom, or a C₆₋₁₄ aryl group optionally having substituent(s) selected from the group consisting of a halogen atom and a cyano.
[14] The compound of the above-mentioned [1], which is selected from the group consisting of methyl 3-[4-(3,4-dihydroquinolin-1(2H)-ylsulfonyl)-2-thienyl]-2,4-dioxo-1,2,3,4-tetrahydrothieno[3,4-d]pyrimidine-5-carboxylate, 3-[4-(3,4-dihydroquinolin-1(2H)-ylsulfonyl)-2-thienyl]-5-(hydroxymethyl)thieno[3,4-d]pyrimidin-2,4(1H,3H)-dione, and 3-(4-{[ethyl(phenyl)amino]carbonyl}-2-thienyl)-2,4-dioxo-1,2,3,4-tetrahydrothieno[3,4-d]pyrimidine-5-carboxylic acid, and a salt thereof.
[15] A prodrug of the compound of the above-mentioned [1].
[16] A pharmaceutical agent comprising the compound of the above-mentioned [1] or a prodrug thereof.
[17] A gonadotropin releasing hormone antagonist comprising the compound of the above-mentioned [1] or a prodrug thereof.
[18] An agent for the prophylaxis or treatment of a sex hormone-dependent disease, comprising the compound of the above-mentioned [1] or a prodrug thereof.
[19] An agent for the prophylaxis or treatment of prostatomegaly, hysteromyoma, endometriosis, metrofibroma, precocious puberty, amenorrhea, premenstrual syndrome, dysmenorrhea, polycystic ovary syndrome, acne, baldness, Alzheimer's disease, infertility, irritable bowel syndrome, prostate cancer, uterine cancer, breast cancer or pituitary tumor, a reproduction regulator, a contraceptive, an ovulation inducing agent, or a preventive agent for post-operative recurrence of sex hormone-dependent cancers, a preventive of premature ovulation during external fertilization or embryo transplantation, or a preventive of ovulation due to endogenous LH during external fertilization, which comprises the compound of the above-mentioned [1] or a prodrug thereof.
[20] A method for the prevention and/or treatment of prostatomegaly, hysteromyoma, endometriosis, metrofibroma, precocious puberty, amenorrhea, premenstrual syndrome, dysmenorrhea, polycystic ovary syndrome, acne, baldness, Alzheimer's disease, infertility or irritable bowel syndrome, prostate cancer, uterine cancer, breast cancer or pituitary tumor, a method for reproduction regulation, a method for contraception, a method for ovulation induction, a method for prevention of post-operative recurrence of sex hormone-dependent cancers, a method for prevention of premature ovulation during external fertilization or embryo transplantation, or a method for prevention of ovulation due to endogenous LH during external fertilization, which comprises administering an effective amount of the compound of the above-mentioned [1] or a prodrug thereof to a mammal.
[21] Use of the compound of the above-mentioned [1] or a prodrug thereof for the production of an agent for the prophylaxis or treatment of prostatomegaly, hysteromyoma, endometriosis, metrofibroma, precocious puberty, amenorrhea, premenstrual syndrome, dysmenorrhea, polycystic ovary syndrome, acne, baldness, Alzheimer's disease, infertility, irritable bowel syndrome, prostate cancer, uterine cancer, breast cancer or pituitary tumor, a reproduction regulator, a contraceptive, an ovulation inducing agent, a preventive agent for post-operative recurrence of sex hormone-dependent cancers, a preventive of premature ovulation during external fertilization or embryo transplantation, or a preventive of ovulation due to endogenous LH during external fertilization.
[22] The compound of the above-mentioned [1], wherein ring A is a 5-membered aromatic heterocycle containing, besides carbon atoms, 1 to 4 hetero atoms selected from the group consisting of a nitrogen atom, an oxygen atom and a sulfur atom (e.g., thiophene ring, furan ring, diazole ring (imidazole ring, pyrazole ring), triazole ring, thiazole ring, oxazole ring, thiadiazole ring, oxadiazole ring), which optionally has substituent(s) selected from substituent group A' consisting of the following (a) - (ff);
   (a) a linear or branched C₁₋₁₅ alkyl group, a C₃₋₈ cycloalkyl group, a C₃₋₈ cycloalkyl group fused with a C₆₋₁₄ aryl ring, a C₂₋₁₈ alkenyl group, a C₃₋₈ cycloalkenyl group, a C₂₋₁₈ alkynyl group, an aromatic monocyclic, bicyclic or tricyclic C₆₋₁₄ aryl group, a C₆₋₁₄ aryl group fused with C₃₋₈ cycloalkane or a C₇₋₂₀ aralkyl group, each of which optionally has substituent(s) selected from substituent group B', (hereinafter sometimes to be abbreviated as "hydrocarbon group optionally having substituent(s)" of substituent group A');
   (b) a 5- to 7-membered monocyclic aromatic heterocyclic group containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocyclic group containing, besides carbon atoms, 1 to 12 hetero atoms selected from the group consisting of an oxygen atom, a sulfur atom and a nitrogen atom (e.g., a bicyclic or tricyclic fused ring group wherein the 5- to 7-membered monocyclic aromatic heterocyclic group and a benzene ring are fused, or a bicyclic or tricyclic fused ring group wherein the 5- to 7-membered monocyclic aromatic heterocycles are fused), each of which optionally has substituent(s) selected from substituent group B', where the above-mentioned monocyclic aromatic heterocycle or fused aromatic heterocycle is optionally fused with a 5- to 7-membered monocyclic non-aromatic heterocycle or 8- to 15-membered polycyclic non-aromatic heterocycle (e.g., a bicyclic or tricyclic fused ring wherein the 5- to 7-membered monocyclic non-aromatic heterocycle and a C₃₋₈ cycloalkane are fused, a bicyclic or tricyclic fused ring wherein the 5- to 7-membered monocyclic non-aromatic heterocycles are fused) containing, as ring-constituting atom besides carbon atom, 1 to 4 hetero atoms selected from the group consisting of an oxygen atom, a sulfur atom and a nitrogen atom (hereinafter sometimes to be abbreviated as "heterocyclic group optionally having substituent(s)" of substituent group A');
   (c) a halogen;
   (d) a hydroxy optionally protected by a tert-butyl(dimethyl)silyl [TBDMS];
   (e) a cyano;
   (f) a carboxyl optionally protected by a trimethylsilylethyl [TMS-CH₂CH₂-];
   (g) a C₁₋₆ alkoxycarbonyl;
   (h) an amino optionally having 1 or 2 substituents selected from the group consisting of "hydrocarbon group optionally having substituent(s)" of substituent group A', "heterocyclic group optionally having substituent(s)" of substituent group A' and an acyl group, or a 5- to 16-membered cyclic amino optionally having substituent(s) selected from the group consisting of substituent group B' and an acyl group; preferably, an amino optionally having substituent(s), which is selected from the following (1) - (8);
      (1) an amino,
      (2) an amino monosubstituted with "hydrocarbon group optionally having substituent(s)" of substituent group A',
      (3) an amino monosubstituted with "heterocyclic group optionally having substituent(s)" of substituent group A',
      (4) an amino monosubstituted with an acyl group represented by R⁶CO-, R⁶OCO-, R⁶SO₂-, R⁶SO- or R⁶(R⁷)CO- wherein R⁶ and R⁷ may be the same or different and each is a hydrogen atom, "hydrocarbon group optionally having substituent(s)" of substituent group A' or "heterocyclic group optionally having substituent(s)" of substituent group A',
      (5) an amino disubstituted with "hydrocarbon group optionally having substituent(s)" of substituent group A',
      (6) an amino disubstituted with "heterocyclic group optionally having substituent(s)" of substituent group A',
      (7) an amino disubstituted with substituents selected from the above-mentioned acyl group, and
      (8) a 5- to 8-membered cyclic amino optionally having substituent(s) selected from the group consisting of substituent group B' and the above-mentioned acyl group;
   (i) a carbamoyl optionally having 1 or 2 substituents selected from the group consisting of "hydrocarbon group optionally having substituent(s)" of substituent group A', "heterocyclic group optionally having substituent(s)" of substituent group A' and an acyl group; preferably, a carbamoyl optionally having substituent(s), which is selected from the following (1) - (8),;
      (1) a carbamoyl,
      (2) an N-mono-C₁₋₆ alkylcarbamoyl (wherein the C₁₋₆ alkyl moiety of the N-mono-C₁₋₆ alkylcarbamoyl optionally has, at its substitutable position(s), 1 - 5 substituents selected from the group consisting of (i) a C₁₋₆ alkoxy, (ii) a phenyl, (iii) an amino, (iv) a di-C₁₋₆ alkylamino, (v) a hydroxy, (vi) a carboxyl, (vii) a C₁₋₆ alkoxycarbonyl and (viii) a heterocyclic group optionally having substituent(s) selected from substituent group B'),
      (3) an N,N-di-C₁₋₆ alkylcarbamoyl (wherein the two C₁₋₆ alkyl moieties of the N,N-di-C₁₋₆ alkylcarbamoyl may be the same or different and each optionally has, at its substitutable position(s), 1 - 5 substituents selected from the group consisting of (i) a C₁₋₆ alkoxy, (ii) a phenyl, (iii) an amino, (iv) a di-C₁₋₆ alkylamino, (v) a hydroxy, (vi) a carboxyl, (vii) a C₁₋₆ alkoxycarbonyl and (viii) a heterocyclic group optionally having substituent(s) selected from substituent group B'),
      (4) an N-mono-C₆₋₁₄ aryl-carbamoyl,
      (5) an N-mono-heterocyclyl-carbamoyl (wherein the heterocycle of the N-mono-heterocyclyl-carbamoyl is a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocycle containing, besides carbon atoms, 1 to 12 hetero atoms selected from the group consisting of an oxygen atom, a sulfur atom and a nitrogen atom (e.g., a bicyclic or tricyclic fused ring wherein the 5- to 7-membered monocyclic aromatic heterocycle and a benzene ring are fused, or a bicyclic or tricyclic fused ring wherein the 5- to 7-membered monocyclic aromatic heterocycles are fused)),
      (6) an N,N-di-C₆₋₁₄ aryl-carbamoyl,
      (7) N,N-diheterocyclyl-carbamoyl (wherein the heterocycles of the N,N-diheterocyclyl-carbamoyl may be the same or different and each is a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocycle containing, besides carbon atoms, 1 to 12 hetero atoms selected from the group consisting of an oxygen atom, a sulfur atom and a nitrogen atom (e.g., a bicyclic or tricyclic fused ring wherein the 5- to 7-membered monocyclic aromatic heterocycle and a benzene ring are fused, or a bicyclic or tricyclic fused ring wherein the 5- to 7-membered monocyclic aromatic heterocycles are fused)), and
      (8) an N-C₆₋₁₄ aryl-N-heterocyclyl-carbamoyl (wherein the heterocycle of the N-C₆₋₁₄ aryl-N-heterocyclyl-carbamoyl is a 5-to 7-membered monocyclic aromatic heterocycle containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocycle containing, besides carbon atoms, 1 to 12 hetero atoms selected from the group consisting of an oxygen atom, a sulfur atom and a nitrogen atom (e.g., a bicyclic or tricyclic fused ring wherein the 5- to 7-membered monocyclic aromatic heterocycle and a benzene ring are fused, or a bicyclic or tricyclic fused ring
      wherein the 5- to 7-membered monocyclic aromatic heterocycles are fused));
   (j) a C₁₋₆ alkoxy;
   (k) a C₆₋₁₄ aryloxy;
   (l) a heterocyclyloxy (wherein the heterocycle of the heterocyclyloxy is a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocycle containing, besides carbon atoms, 1 to 12 hetero atoms selected from the group consisting of an oxygen atom, a sulfur atom and a nitrogen atom (e.g., a bicyclic or tricyclic fused ring wherein the 5- to 7-membered monocyclic aromatic heterocycle and a benzene ring are fused, or a bicyclic or tricyclic fused ring wherein the 5- to 7-membered monocyclic aromatic heterocycles are fused));
   (m) a C₁₋₆ alkyl-carbonyloxy;
   (n) a C₁₋₆ alkylthio;
   (o) a C₆₋₁₄ arylthio;
   (p) a heterocyclylthio (wherein the "heterocycle" of the heterocyclylthio is a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocycle containing, besides carbon atoms, 1 to 12 hetero atoms selected from the group consisting of an oxygen atom, a sulfur atom and a nitrogen atom (e.g., a bicyclic or tricyclic fused ring wherein the 5- to 7-membered monocyclic aromatic heterocycle and a benzene ring are fused, or a bicyclic or tricyclic fused ring wherein the 5- to 7-membered monocyclic aromatic heterocycles are fused));
   (q) a C₁₋₆ alkylsulfinyl;
   (r) a C₆₋₁₄ arylsulfinyl;
   (s) a heterocyclesulfinyl (wherein the heterocycle of the heterocyclesulfinyl is a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocycle containing, besides carbon atoms, 1 to 12 hetero atoms selected from the group consisting of an oxygen atom, a sulfur atom and a nitrogen atom (e.g., a bicyclic or tricyclic fused ring wherein the 5- to 7-membered monocyclic aromatic heterocycle and a benzene ring are fused, or a bicyclic or tricyclic fused ring wherein the 5- to 7-membered monocyclic aromatic heterocycles are fused));
   (t) a C₁₋₆ alkylsulfonyl;
   (u) a C₆₋₁₄ arylsulfonyl;
   (v) a heterocyclesulfonyl (wherein the heterocycle of the heterocyclesulfonyl is a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocycle containing, besides carbon atoms, 1 to 12 hetero atoms selected from the group consisting of an oxygen atom, a sulfur atom and a nitrogen atom (e.g., a bicyclic or tricyclic fused ring wherein the 5- to 7-membered monocyclic aromatic heterocycle and a benzene ring are fused, or a bicyclic or tricyclic fused ring wherein the 5- to 7-membered monocyclic aromatic heterocycles are fused));
   (w) a formyl;
   (x) a C₁₋₆ alkyl-carbonyl;
   (y) a C₆₋₁₄ arylcarbonyl;
   (z) a heterocyclecarbonyl (wherein the heterocycle of the heterocyclecarbonyl is a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocycle containing, besides carbon atoms, 1 to 12 hetero atoms selected from the group consisting of an oxygen atom, a sulfur atom and a nitrogen atom (e.g., a bicyclic or tricyclic fused ring wherein the 5- to 7-membered monocyclic aromatic heterocycle and a benzene ring are fused, or a bicyclic or tricyclic fused ring wherein the 5- to 7-membered monocyclic aromatic heterocycles are fused));
      (aa) a heterocyclyl-C₁₋₆ alkyl (wherein the heterocycle of the heterocyclyl-C₁₋₆ alkyl is a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocycle containing, besides carbon atoms, 1 to 12 hetero atoms selected from the group consisting of an oxygen atom, a sulfur atom and a nitrogen atom (e.g., a bicyclic or tricyclic fused ring wherein the 5- to 7-membered monocyclic aromatic heterocycle and a benzene ring are fused, or a bicyclic or tricyclic fused ring wherein the 5- to 7-membered monocyclic aromatic heterocycles are fused));
      (bb) a C₁₋₆ alkoxy-C₁₋₆ alkyl;
      (cc) a nitro;
      (dd) an oxo;
      (ee) a C₁₋₂ alkylenedioxy; and
      (ff) R⁵O-N=CH-(R⁵ is a hydrogen atom, or a linear or branched C₁-15 alkyl group, a C₃₋₈ cycloalkyl group, a C₃₋₈ cycloalkyl group fused with a C₆-₁₄ aryl ring, a C₂₋₁₈ alkenyl group, a C₃-₈ cycloalkenyl group, a C₂₋₁₈ alkynyl group, an aromatic monocyclic, bicyclic or tricyclic C₆-₁₄ aryl group, a C₆-₁₄ aryl group fused with a C₃-₈ cycloalkane or a C₇-₂₀ aralkyl group, each of which optionally has substituent(s) selected from substituent group B'),
         the substituent group B' consists of the following (a) - (ff);

      (a) a linear or branched C₁₋₁₅ alkyl group, a C₃₋₈ cycloalkyl group, a C₃₋₈ cycloalkyl group fused with a C₆₋₁₄ aryl ring, a C₂₋₁₈ alkenyl group, a C₃₋₈ cycloalkenyl group, a C₂₋₁₈ alkynyl group, an aromatic monocyclic, bicyclic or tricyclic C₆₋₁₄ aryl group, a C₆-14 aryl group fused with a C₃₋₈ cycloalkane or a C₇₋₂₀ aralkyl group, each of which optionally has substituent(s) selected from substituent group C';
      (b) a 5- to 7-membered monocyclic aromatic heterocyclic group containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocyclic group containing, besides carbon atoms, 1 to 12 hetero atoms selected from the group consisting of an oxygen atom, a sulfur atom and a nitrogen atom (e.g., a bicyclic or tricyclic fused ring group wherein the 5- to 7-membered monocyclic aromatic heterocyclic group and a benzene ring are fused, or a bicyclic or tricyclic fused ring group wherein the 5- to 7-membered monocyclic aromatic heterocycles are fused), each of which optionally has substituent(s) selected from substituent group C', wherein the above-mentioned monocyclic aromatic heterocycle or fused aromatic heterocycle is optionally fused with a 5- to 7-membered monocyclic non-aromatic heterocycle or an 8- to 15-membered polycyclic non-aromatic heterocycle (e.g., a bicyclic or tricyclic fused ring wherein the 5- to 7-membered monocyclic non-aromatic heterocycle and a C₃₋₈ cycloalkane are fused, a bicyclic or tricyclic fused ring wherein the 5- to 7-membered monocyclic non-aromatic heterocycles are fused) containing, as ring-constituting atom besides carbon atom, 1 to 4 hetero atoms selected from the group consisting of an oxygen atom, a sulfur atom and a nitrogen atom;
      (c) a halogen;
      (d) a hydroxy optionally protected by a tert-butyl(dimethyl)silyl [TBDMS];
      (e) a cyano;
      (f) a carboxyl optionally protected by a trimethylsilylethyl [TMS-CH₂CH₂-];
      (g) a C₁₋₆ alkoxycarbonyl;
      (h) an amino optionally having substituent(s) (amino, N-mono-C₁₋₆ alkylamino; N,N-di-C₁₋₆ alkylamino; N-mono-C₆₋₁₄ aryl-amino; N-mono-heterocyclyl-amino; N,N-di-C₆₋₁₄ aryl-amino; N,N-diheterocyclyl-amino; N-C₆₋₁₄ aryl-N-heterocyclyl-amino; or 5- to 8-membered cyclic amino);
      (i) a carbamoyl optionally having substituent(s), which is selected from the following (1) - (8);
         (1) a carbamoyl,
         (2) an N-mono-C₁₋₆ alkylcarbamoyl (wherein the C₁₋₆ alkyl moiety of the N-mono-C₁₋₆ alkylcarbamoyl optionally has, at its substitutable position(s), 1 - 5 substituents selected from the group consisting of (i) a C₁₋₆ alkoxy, (ii) a phenyl, (iii) an amino, (iv) a di-C₁₋₆ alkylamino, (v) a hydroxy, (vi) a carboxyl, (vii) a C₁₋₆ alkoxycarbonyl and (viii) a heterocyclic group optionally having substituent(s) selected from substituent group C'),
         (3) N,N-di-C₁₋₆ alkylcarbamoyls (wherein the two C₁₋₆ alkyl moieties of the N,N-di-C₁₋₆ alkylcarbamoyl may be the same or different and each optionally has, at its substitutable position(s), 1 - 5 substituents selected from the group consisting of (i) a C₁₋₆ alkoxy, (ii) a phenyl, (iii) an amino, (iv) a di-C₁₋₆ alkylamino, (v) a hydroxy, (vi) a carboxyl, (vii) a C₁₋₆ alkoxycarbonyl and (viii) a heterocyclic group optionally having substituent(s) selected from substituent group C'),
         (4) an N-mono-C₆₋₁₄ aryl-carbamoyl,
         (5) an N-mono-heterocyclyl-carbamoyl (wherein the heterocycle of the N-mono-heterocyclyl-carbamoyl is a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocycle containing, besides carbon atoms, 1 to 12 hetero atoms selected from the group consisting of an oxygen atom, a sulfur atom and a nitrogen atom (e.g., a bicyclic or tricyclic fused ring wherein the 5- to 7-membered monocyclic aromatic heterocycle and a benzene ring are fused, or a bicyclic or tricyclic fused ring wherein the 5- to 7-membered monocyclic aromatic heterocycles are fused)),
         (6) an N,N-di-C₆₋₁₄ aryl-carbamoyl,
         (7) an N,N-diheterocyclyl-carbamoyl (wherein the heterocycles of the N,N-diheterocyclyl-carbamoyl may be the same or different and each is a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocycle containing, besides carbon atoms, 1 to 12 hetero atoms selected from the group consisting of an oxygen atom, a sulfur atom and a nitrogen atom (e.g., a bicyclic or tricyclic fused ring wherein the 5- to 7-membered monocyclic aromatic heterocycle and a benzene ring are fused, or a bicyclic or tricyclic fused ring wherein the 5- to 7-membered monocyclic aromatic heterocycles are fused)), and
         (8) an N-C₆₋₁₄ aryl-N-heterocyclyl-carbamoyl (wherein the heterocycle of the N-C₆₋₁₄ aryl-N-heterocyclyl-carbamoyl is a 5-to 7-membered monocyclic aromatic heterocycle containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocycle containing, besides carbon atoms, 1 to 12 hetero atoms selected from the group consisting of an oxygen atom, a sulfur atom and a nitrogen atom (e.g., a bicyclic or tricyclic fused ring wherein the 5- to 7-membered monocyclic aromatic heterocycle and a benzene ring are fused, or a bicyclic or tricyclic fused ring
         wherein the 5- to 7-membered monocyclic aromatic heterocycles are fused));
      (j) a C₁₋₆ alkoxy;
      (k) a C₆₋₁₄ aryloxy;
      (l) a heterocyclyloxy (wherein the heterocycle of the heterocyclyloxy is a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocycle containing, besides carbon atoms, 1 to 12 hetero atoms selected from the group consisting of an oxygen atom, a sulfur atom and a nitrogen atom (e.g., a bicyclic or tricyclic fused ring wherein the 5- to 7-membered monocyclic aromatic heterocycle and a benzene ring are fused, or a bicyclic or tricyclic fused ring wherein the 5- to 7-membered monocyclic aromatic heterocycles are fused));
      (m) a C₁₋₆ alkyl-carbonyloxy;
      (n) a C₁₋₆ alkylthio;
      (o) a C₆₋₁₄ arylthio;
      (p) a heterocyclylthio (wherein the "heterocycle" of the heterocyclylthio is a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocycle containing, besides carbon atoms, 1 to 12 hetero atoms selected from the group consisting of an oxygen atom, a sulfur atom and a nitrogen atom (e.g., a bicyclic or tricyclic fused ring wherein the 5- to 7-membered monocyclic aromatic heterocycle and a benzene ring are fused, or a bicyclic or tricyclic fused ring wherein the 5- to 7-membered monocyclic aromatic heterocycles are fused));
      (q) a C₁₋₆ alkylsulfinyl;
      (r) a C₆₋₁₄ arylsulfinyl;
      (s) a heterocyclesulfinyl (wherein the heterocycle of the heterocyclesulfinyl is a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocycle containing, besides carbon atoms, 1 to 12 hetero atoms selected from the group consisting of an oxygen atom, a sulfur atom and a nitrogen atom (e.g., a bicyclic or tricyclic fused ring wherein the 5- to 7-membered monocyclic aromatic heterocycle and a benzene ring are fused, or a bicyclic or tricyclic fused ring wherein the 5- to 7-membered monocyclic aromatic heterocycles are fused));
      (t) a C₁₋₆ alkylsulfonyl;
      (u) a C₆₋₁₄ arylsulfonyl;
      (v) a heterocyclesulfonyl (wherein the heterocycle of the heterocyclesulfonyl is a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocycle containing, besides carbon atoms, 1 to 12 hetero atoms selected from the group consisting of an oxygen atom, a sulfur atom and a nitrogen atom (e.g., a bicyclic or tricyclic fused ring wherein the 5- to 7-membered monocyclic aromatic heterocycle and a benzene ring are fused, or a bicyclic or tricyclic fused ring wherein the 5- to 7-membered monocyclic aromatic heterocycles are fused));
      (w) a formyl;
      (x) a C₁₋₆ alkyl-carbonyl;
      (y) a C₆₋₁₄ arylcarbonyl;
      (z) a heterocyclecarbonyl (wherein the heterocycle of the heterocyclecarbonyl is a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocycle containing, besides carbon atoms, 1 to 12 hetero atoms selected from the group consisting of an oxygen atom, a sulfur atom and a nitrogen atom (e.g., a bicyclic or tricyclic fused ring wherein the 5- to 7-membered monocyclic aromatic heterocycle and a benzene ring are fused, or a bicyclic or tricyclic fused ring wherein the 5- to 7-membered monocyclic aromatic heterocycles are fused));

      (aa) a heterocyclyl-C₁₋₆ alkyl (wherein the heterocycle of the heterocyclyl-C₁₋₆ alkyl is a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocycle containing, besides carbon atoms, 1 to 12 hetero atoms selected from the group consisting of an oxygen atom, a sulfur atom and a nitrogen atom (e.g., a bicyclic or tricyclic fused ring wherein the 5- to 7-membered monocyclic aromatic heterocycle and a benzene ring are fused, or a bicyclic or tricyclic fused ring wherein the 5- to 7-membered monocyclic aromatic heterocycles are fused)),
      (bb) a C₁₋₆ alkoxy-C₁₋₆ alkyl;
      (cc) a nitro;
      (dd) an oxo;
      (ee) a C₁₋₂ alkylenedioxy; and
      (ff) R⁵O-N=CH- (R⁵ is a hydrogen atom or a linear or branched C₁₋₁₅ alkyl group, a C₃₋₈ cycloalkyl group, a C₃₋₈ cycloalkyl group fused with a C₆₋₁₄ aryl ring, a C₂₋₁₈ alkenyl group, a C₃₋₈ cycloalkenyl group, a C₂₋₁₈ alkynyl group, an aromatic monocyclic, bicyclic or tricyclic C₆₋₁₄ aryl group, a C₆₋₁₄ aryl group fused with a C₃₋₈ cycloalkane or a C₇₋₂₀ aralkyl group, each of which optionally has substituent(s) selected from substituent group C', the substituent group C' consists of a halogen; a hydroxy optionally protected by a tert-butyl(dimethyl)silyl [TBDMS]; a cyano; a carboxyl optionally protected by a trimethylsilylethyl [TMS-CH₂CH₂-] ; a C₁₋₆ alkoxycarbonyl; a C₁₋₆ alkyl; a C₆₋₁₄ aryl; a aralkyl; a heterocyclic group; an amino; an N-mono-C₁₋₆ alkylamino; an N,N-di-C₁₋₆ alkylamino; an N-mono-C₆₋₁₄ aryl-amino; an N-mono-heterocyclyl-amino; an N,N-di-C₆₋₁₄ aryl-amino; an N,N-diheterocyclyl-amino; an N-C₆₋₁₄ aryl-N-heterocyclyl-amino; a carbamoyl; an N-mono-C₁₋₆ alkylcarbamoyl; an N,N-di-C₁₋₆ alkylcarbamoyl; an N-mono-C₆₋₁₄ aryl-carbamoyl; an N-mono-heterocyclyl-carbamoyl; an N,N-di-C₆₋₁₄ aryl-carbamoyl; an N,N-diheterocyclyl-carbamoyl; an N-C₆₋₁₄ aryl-N-heterocyclyl-carbamoyl; a C₁₋₆ alkoxy; a C₆₋₁₄ aryloxy; a heterocyclyloxy; a C₁-6 alkyl-carbonyloxy; a C₁₋₆ alkylthio; a C₆₋₁₄ arylthio; a heterocyclylthio ; a C₁₋₆ alkylsulfinyl; a C₆₋₁₄ arylsulfinyl; a heterocyclylsulfinyl; a C₁₋₆ alkylsulfonyl; a C₆₋₁₄ arylsulfonyl; a heterocyclesulfonyl; a formyl; a C₁₋₆ alkyl-carbonyl; a C₆₋₁₄ arylcarbonyl; a heterocyclecarbonyl; a heterocyclyl-C₁₋₆ alkyl; a C₁₋₆ alkoxy-C₁₋₆ alkyl; a nitro; an oxo; and a C₁₋₂ alkylenedioxy (wherein the heterocycle is a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocycle containing, besides carbon atoms, 1 to 12 hetero atoms selected from the group consisting of an oxygen atom, a sulfur atom and a nitrogen atom (e.g., a bicyclic or tricyclic fused ring wherein the 5- to 7-membered monocyclic aromatic heterocycle and a benzene ring are fused, or a bicyclic or tricyclic fused ring wherein the 5- to 7-membered monocyclic aromatic heterocycles are fused), the heterocyclic group is a 5- to 7-membered monocyclic aromatic heterocyclic group containing, as ring-constituting atom besides carbon atom, 1 to 4 hetero atoms selected from the group consisting of an oxygen atom, a sulfur atom and a nitrogen atom, or a 8-to 16-membered bicyclic or tricyclic fused aromatic heterocyclic group containing, besides carbon atoms, 1 to 12 hetero atoms selected from the group consisting of an oxygen atom, a sulfur atom and a nitrogen atom (e.g., a bicyclic or tricyclic fused ring group wherein the 5- to 7-membered monocyclic aromatic heterocycle and a benzene ring are fused, or a bicyclic or tricyclic fused ring group wherein the 5- to 7-membered monocyclic aromatic heterocycles are fused),
         R¹ is

      (i) a hydrogen atom or
      (ii) a linear or branched C₁₋₁₅ alkyl group, a C₃₋₈ cycloalkyl group, a C₃₋₈ cycloalkyl group fused with a C₆₋₁₄ aryl ring, a C₂₋₁₈ alkenyl group, a C₃₋₈ cycloalkenyl group, a C₂₋₁₈ alkynyl group, an aromatic monocyclic, bicyclic or tricyclic C₆₋₁₄ aryl group, a C₆₋₁₄ aryl group fused with a C₃₋₈ cycloalkane or a C₇₋₂₀ aralkyl group, each of which optionally has substituent(s) selected from substituent group B',
         W is an oxygen atom or a sulfur atom,
         X¹ and X² may be the same or different and each is

      (i) a hydrogen atom,
      (ii) a linear or branched C₁₋₁₅ alkyl group, a C₃₋₈ cycloalkyl group, a C₃₋₈ cycloalkyl group fused with a C₆₋₁₄ aryl ring, a C₂₋₁₈ alkenyl group, a C₃₋₈ cycloalkenyl group, a C₂₋₁₈ alkynyl group, an aromatic monocyclic, bicyclic or tricyclic C₆₋₁₄ aryl group, a C₆₋₁₄ aryl group fused with a C₃₋₈ cycloalkane or a C₇₋₂₀ aralkyl group, each of which optionally has substituent(s) selected from substituent group B' or
      (iii) a 5- to 7-membered monocyclic aromatic heterocyclic group containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocyclic group containing, besides carbon atoms, 1 to 12 hetero atoms selected from the group consisting of an oxygen atom, a sulfur atom and a nitrogen atom (e.g., a bicyclic or tricyclic fused ring group wherein the 5- to 7-membered monocyclic aromatic heterocyclic group and a benzene ring are fused, or a bicyclic or tricyclic fused ring group wherein the 5- to 7-membered monocyclic aromatic heterocycles are fused), each of which optionally has substituent(s) selected from substituent group B',
         or
         X¹ and X² optionally form, in combination, an oxygen atom, a sulfur atom or =NR²
   wherein R² is
   (i) a hydrogen atom or
   (ii) a linear or branched C₁₋₁₅ alkyl group, a C₃₋₈ cycloalkyl group, a C₃₋₈ cycloalkyl group fused with a C₆₋₁₄ aryl ring, a C₂-18 alkenyl group, a C₃₋₈ cycloalkenyl group, a C₂₋₁₈ alkynyl group, an aromatic monocyclic, bicyclic or tricyclic C₆₋₁₄ aryl group, a C₆₋₁₄ aryl group fused with a C₃₋₈ cycloalkane or a C₇₋₂₀ aralkyl group, each of which optionally has substituent(s) selected from substituent group B',
   ring B is a C₆₋₁₄ aryl ring, a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom or a 8- to 16-membered bicyclic or tricyclic fused aromatic heterocycle containing, besides carbon atoms, 1 to 12 hetero atoms selected from the group consisting of an oxygen atom, a sulfur atom and a nitrogen atom (e.g., a bicyclic or tricyclic fused ring wherein the 5- to 7-membered monocyclic aromatic heterocycle and a benzene ring are fused, or a bicyclic or tricyclic fused ring wherein the 5- to 7-membered monocyclic aromatic heterocycles are fused), each of which optionally further has substituent(s) selected from substituent group A',
   Y is
   (i) a bond,
   (ii) a C₁₋₆ alkylene optionally having substituent(s) selected from substituent group B'
   (iii) a C₂₋₆ alkenylene optionally having substituent(s) selected from substituent group B' or
   (iv) a C₂₋₆ alkynylene optionally having substituent(s) selected from substituent group B', and
   Z is a group represented by formula: -SOₙR³ wherein n is 0, 1 or 2,
   R³ is
   (i) an amino optionally having 1 or 2 substituents selected from the group consisting of "hydrocarbon group optionally having substituent(s)" of substituent group A', "heterocyclic group optionally having substituent(s)" of substituent group A' and an acyl group,
   (ii) a 5- to 16-membered cyclic amino optionally having substituent(s) selected from the group consisting of substituent group B' and an acyl group,
   (iii) a linear or branched C₁₋₁₅ alkyl group, a C₃₋₈ cycloalkyl group, a C₃₋₈ cycloalkyl group fused with a C₆₋₁₄ aryl ring, a C₂₋₁₈ alkenyl group, a C₃₋₈ cycloalkenyl group, a C₂₋₁₈ alkynyl group, an aromatic monocyclic, bicyclic or tricyclic C₆₋₁₄ aryl group, a C₆₋₁₄ aryl group fused with a C₃₋₈ cycloalkane or a C₇₋₂₀ aralkyl group, each of which optionally has substituent(s) selected from substituent group B', or
   (iv) a 5- to 7-membered monocyclic aromatic heterocyclic group containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocyclic group containing, besides carbon atoms, 1 to 12 hetero atoms selected from the group consisting of an oxygen atom, a sulfur atom and a nitrogen atom (e.g., a bicyclic or tricyclic fused ring group wherein the 5- to 7-membered monocyclic aromatic heterocyclic group and a benzene ring are fused, or a bicyclic or tricyclic fused ring group wherein the 5- to 7-membered monocyclic aromatic heterocycles are fused), each of which optionally has substituent(s) selected from substituent group B' (provided that R³ is not a methyl group), or
   a group represented by formula: -COR⁴ wherein R⁴ is
   (i) an amino optionally having 1 or 2 substituents the group consisting of "hydrocarbon group optionally having substituent(s)" of substituent group A', "heterocyclic group optionally having substituent(s)" of substituent group A' and an acyl group,
   (ii) a 5- to 16-membered cyclic amino optionally having substituent(s) selected from the group consisting of substituent group B' and an acyl group,
   (iii) a linear or branched C₁₋₁₅ alkyl group, a C₃₋₈ cycloalkyl group, a C₃₋₈ cycloalkyl group fused with a C₆₋₁₄ aryl ring, a C₂₋₁₈ alkenyl group, a C₃₋₈ cycloalkenyl group, a C₂₋₁₈ alkynyl group, an aromatic monocyclic, bicyclic or tricyclic C₆₋₁₄ aryl group, a C₆₋₁₄ aryl group fused with a C₃₋₈ cycloalkane or a C₇₋₂₀ aralkyl group, each of which optionally has substituent(s) selected from substituent group B', or
   (iv) a 5- to 7-membered monocyclic aromatic heterocyclic group containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocyclic group containing, besides carbon atoms, 1 to 12 hetero atoms selected from the group consisting of an oxygen atom, a sulfur atom and a nitrogen atom (e.g., a bicyclic or tricyclic fused ring group wherein the 5- to 7-membered monocyclic aromatic heterocyclic group and a benzene ring are fused, or a bicyclic or tricyclic fused ring group wherein the 5- to 7-membered monocyclic aromatic heterocycles are fused), each of which optionally has substituent(s) selected from substituent group B'; provided that when ring B is a ring represented by formula: 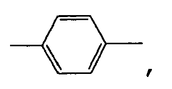 then the amino optionally having substituent(s) for R³ is

   (i) an amino disubstituted with substituents selected from the group consisting of "hydrocarbon group optionally having substituent(s)" of substituent group A', "heterocyclic group optionally having substituent(s)" of substituent group A' and an acyl group or
   (ii) a 5- to 16-membered cyclic amino optionally having substituent(s) selected from the group consisting of substituent group B' and an acyl group, and
      the amino optionally having substituent(s) for R⁴ is

   (i) an amino disubstituted with substituents selected from the group consisting of "hydrocarbon group optionally having substituent(s)" of substituent group A', "heterocyclic group optionally having substituent(s)" of substituent group A' and an acyl group (except dimethylamino) or
   (ii) a 5- to 16-membered cyclic amino optionally having substituent(s) selected from the group consisting of substituent group B' and an acyl group (except monocyclic piperazin-1-yl optionally having substituent(s)), and a linear or branched C₁₋₁₅ alkyl group, a C₃₋₈ cycloalkyl group, a C₃₋₈ cycloalkyl group fused with a C₆₋₁₄ aryl ring, a C₂₋₁₈ alkenyl group, a C₃₋₈ cycloalkenyl group, a C₂₋₁₈ alkynyl group, an aromatic monocyclic, bicyclic or tricyclic C₆₋₁₄ aryl group, a C₆₋₁₄ aryl group fused with a C₃₋₈ cycloalkane or a C₇₋₂₀ aralkyl group, each of which optionally has substituent(s) selected from substituent group B' for R⁴, does not contain a methyl group.
[23] The compound of the above-mentioned [12], wherein ring A is a 5-membered aromatic heterocycle containing, besides carbon atoms, 1 to 4 hetero atoms selected from the group consisting of a nitrogen atom, an oxygen atom and a sulfur atom (e.g., thiophene ring, furan ring, diazole ring (imidazole ring, pyrazole ring), triazole ring, thiazole ring, oxazole ring, thiadiazole ring, oxadiazole ring), which optionally has substituent(s) selected from substituent group A' consisting of the following (a) - (ff);
   (a) a linear or branched C₁₋₁₅ alkyl group, a C₃₋₈ cycloalkyl group, a C₃₋₈ cycloalkyl group fused with a C₆₋₁₄ aryl ring, a C₂₋₁₈ alkenyl group, a C₃₋₈ cycloalkenyl group, a C₂₋₁₈ alkynyl group, an aromatic monocyclic, bicyclic or tricyclic C₆₋₁₄ aryl group, a C₆₋₁₄ aryl group fused with C₃₋₈ cycloalkane or a C₇₋₂₀ aralkyl group, each of which optionally has substituent(s) selected from substituent group B';
   (b) a 5- to 7-membered monocyclic aromatic heterocyclic group containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocyclic group containing, besides carbon atoms, 1 to 12 hetero atoms selected from the group consisting of an oxygen atom, a sulfur atom and a nitrogen atom (e.g., a bicyclic or tricyclic fused ring group wherein the 5- to 7-membered monocyclic aromatic heterocyclic group and a benzene ring are fused, or a bicyclic or tricyclic fused ring group wherein the 5- to 7-membered monocyclic aromatic heterocycles are fused), each of which optionally has substituent(s) selected from substituent group B', where the above-mentioned monocyclic aromatic heterocycle or fused aromatic heterocycle is optionally fused with a 5- to 7-membered monocyclic non-aromatic heterocycle or 8- to 15-membered polycyclic non-aromatic heterocycle (e.g., a bicyclic or tricyclic fused ring wherein the 5- to 7-membered monocyclic non-aromatic heterocycle and a C₃₋₈ cycloalkane are fused, a bicyclic or tricyclic fused ring wherein the 5- to 7-membered monocyclic non-aromatic heterocycles are fused) containing, as ring-constituting atom besides carbon atom, 1 to 4 hetero atoms selected from the group consisting of an oxygen atom, a sulfur atom and a nitrogen atom;
   (c) a halogen;
   (d) a hydroxy optionally protected by a tert-butyl(dimethyl)silyl [TBDMS];
   (e) a cyano;
   (f) a carboxyl optionally protected by a trimethylsilylethyl [TMS-CH₂CH₂-];
   (g) a C₁₋₆ alkoxycarbonyl;
   (h) an amino optionally having 1 or 2 substituents selected from the group consisting of "hydrocarbon group optionally having substituent(s)" of substituent group A', "heterocyclic group optionally having substituent(s)" of substituent group A' and an acyl group, or a 5- to 16-membered cyclic amino optionally having substituent(s) selected from the group consisting of substituent group B' and an acyl group; preferably, an amino optionally having substituent(s), which is selected from the following (1) - (8);
      (1) an amino,
      (2) an amino monosubstituted with "hydrocarbon group optionally having substituent(s)" of substituent group A',
      (3) an amino monosubstituted with "heterocyclic group optionally having substituent(s)" of substituent group A',
      (4) an amino monosubstituted with an acyl group represented by R⁶CO-, R⁶OCO-, R⁶SO₂-, R⁶SO- or R⁶N(R⁷)CO- wherein R⁶ and R⁷ may be the same or different and each is a hydrogen atom, "hydrocarbon group optionally having substituent(s)" of substituent group A' or "heterocyclic group optionally having substituent(s)" of substituent group A',
      (5) an amino disubstituted with "hydrocarbon group optionally having substituent(s)" of substituent group A',
      (6) an amino disubstituted with "heterocyclic group optionally having substituent(s)" of substituent group A',
      (7) an amino disubstituted with substituents selected from the above-mentioned acyl group, and
      (8) a 5- to 8-membered cyclic amino optionally having substituent(s) selected from the group consisting of substituent group B' and the above-mentioned acyl group;
   (i) a carbamoyl optionally having 1 or 2 substituents selected from the group consisting of "hydrocarbon group optionally having substituent(s)" of substituent group A', "heterocyclic group optionally having substituent(s)" of substituent group A' and the above-mentioned acyl group;
      preferably, a carbamoyl optionally having substituent(s), which is selected from the following (1) - (8),;
      (1) a carbamoyl,
      (2) an N-mono-C₁₋₆ alkylcarbamoyl (wherein the C₁₋₆ alkyl moiety of the N-mono-C₁₋₆ alkylcarbamoyl optionally has, at its substitutable position(s), 1 - 5 substituents selected from the group consisting of (i) a C₁₋₆ alkoxy, (ii) a phenyl, (iii) an amino, (iv) a di-C₁₋₆ alkylamino, (v) a hydroxy, (vi) a carboxyl, (vii) a C₁₋₆ alkoxycarbonyl and (viii) a heterocyclic group optionally having substituent(s) selected from substituent group B'),
      (3) an N,N-di-C₁₋₆ alkylcarbamoyl (wherein the two C₁₋₆ alkyl moieties of the N,N-di-C₁₋₆ alkylcarbamoyl may be the same or different and each optionally has, at its substitutable position(s), 1 - 5 substituents selected from the group consisting of (i) a C₁₋₆ alkoxy, (ii) a phenyl, (iii) an amino, (iv) a di-C₁₋₆ alkylamino, (v) a hydroxy, (vi) a carboxyl, (vii) a C₁₋₆ alkoxycarbonyl and (viii) a heterocyclic group optionally having substituent(s) selected from substituent group B'),
      (4) an N-mono-C₆₋₁₄ aryl-carbamoyl,
      (5) an N-mono-heterocyclyl-carbamoyl (wherein the heterocycle of the N-mono-heterocyclyl-carbamoyl is a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocycle containing, besides carbon atoms, 1 to 12 hetero atoms selected from the group consisting of an oxygen atom, a sulfur atom and a nitrogen atom (e.g., a bicyclic or tricyclic fused ring wherein the 5- to 7-membered monocyclic aromatic heterocycle and a benzene ring are fused, or a bicyclic or tricyclic fused ring wherein the 5- to 7-membered monocyclic aromatic heterocycles are fused)),
      (6) an N,N-di-C₆₋₁₄ aryl-carbamoyl,
      (7) N,N-diheterocyclyl-carbamoyl (wherein the heterocycles of the N,N-diheterocyclyl-carbamoyl may be the same or different and each is a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocycle containing, besides carbon atoms, 1 to 12 hetero atoms selected from the group consisting of an oxygen atom, a sulfur atom and a nitrogen atom (e.g., a bicyclic or tricyclic fused ring wherein the 5- to 7-membered monocyclic aromatic heterocycle and a benzene ring are fused, or a bicyclic or tricyclic fused ring wherein the 5- to 7-membered monocyclic aromatic heterocycles are fused)), and
      (8) an N-C₆₋₁₄ aryl-N-heterocyclyl-carbamoyl (wherein the heterocycle of the N-C₆₋₁₄ aryl-N-heterocyclyl-carbamoyl is a 5-to 7-membered monocyclic aromatic heterocycle containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocycle containing, besides carbon atoms, 1 to 12 hetero atoms selected from the group consisting of an oxygen atom, a sulfur atom and a nitrogen atom (e.g., a bicyclic or tricyclic fused ring wherein the 5- to 7-membered monocyclic aromatic heterocycle and a benzene ring are fused, or a bicyclic or tricyclic fused ring
      wherein the 5- to 7-membered monocyclic aromatic heterocycles are fused));
   (j) a C₁₋₆ alkoxy;
   (k) a C₆₋₁₄ aryloxy;
   (l) a heterocyclyloxy (wherein a heterocycle of the heterocyclyloxy is a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocycle containing, besides carbon atoms, 1 to 12 hetero atoms selected from the group consisting of an oxygen atom, a sulfur atom and a nitrogen atom (e.g., a bicyclic or tricyclic fused ring wherein the 5- to 7-membered monocyclic aromatic heterocycle and a benzene ring are fused, or a bicyclic or tricyclic fused ring wherein the 5- to 7-membered monocyclic aromatic heterocycles are fused));
   (m) a C₁₋₆ alkyl-carbonyloxy;
   (n) a C₁₋₆ alkylthio;
   (o) a C₆₋₁₄ arylthio;
   (p) a heterocyclylthio (wherein the "heterocycle" of the heterocyclylthio is a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocycle containing, besides carbon atoms, 1 to 12 hetero atoms selected from the group consisting of an oxygen atom, a sulfur atom and a nitrogen atom (e.g., a bicyclic or tricyclic fused ring wherein the 5- to 7-membered monocyclic aromatic heterocycle and a benzene ring are fused, or a bicyclic or tricyclic fused ring wherein the 5- to 7-membered monocyclic aromatic heterocycles are fused));
   (q) a C₁₋₆ alkylsulfinyl;
   (r) a C₆₋₁₄ arylsulfinyl;
   (s) a heterocyclesulfinyl (wherein the heterocycle of the heterocyclesulfinyl is a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocycle containing, besides carbon atoms, 1 to 12 hetero atoms selected from the group consisting of an oxygen atom, a sulfur atom and a nitrogen atom (e.g., a bicyclic or tricyclic fused ring wherein the 5- to 7-membered monocyclic aromatic heterocycle and a benzene ring are fused, or a bicyclic or tricyclic fused ring wherein the 5- to 7-membered monocyclic aromatic heterocycles are fused));
   (t) a C₁₋₆ alkylsulfonyl;
   (u) a C₆₋₁₄ arylsulfonyl;
   (v) a heterocyclesulfonyl (wherein the heterocycle of the heterocyclesulfonyl is a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocycle containing, besides carbon atoms, 1 to 12 hetero atoms selected from the group consisting of an oxygen atom, a sulfur atom and a nitrogen atom (e.g., a bicyclic or tricyclic fused ring wherein the 5- to 7-membered monocyclic aromatic heterocycle and a benzene ring are fused, or a bicyclic or tricyclic fused ring wherein the 5- to 7-membered monocyclic aromatic heterocycles are fused));
   (w) a formyl;
   (x) a C₁₋₆ alkyl-carbonyl;
   (y) a C₆₋₁₄ arylcarbonyl;
   (z) a heterocyclecarbonyl (wherein the heterocycle of the heterocyclecarbonyl is a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocycle containing, besides carbon atoms, 1 to 12 hetero atoms selected from the group consisting of an oxygen atom, a sulfur atom and a nitrogen atom (e.g., a bicyclic or tricyclic fused ring wherein the 5- to 7-membered monocyclic aromatic heterocycle and a benzene ring are fused, or a bicyclic or tricyclic fused ring wherein the 5- to 7-membered monocyclic aromatic heterocycles are fused));
      (aa) a heterocyclyl-C₁₋₆ alkyl (wherein the heterocycle of the heterocyclyl-C₁₋₆ alkyl is a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocycle containing, besides carbon atoms, 1 to 12 hetero atoms selected from the group consisting of an oxygen atom, a sulfur atom and a nitrogen atom (e.g., a bicyclic or tricyclic fused ring wherein the 5- to 7-membered monocyclic aromatic heterocycle and a benzene ring are fused, or a bicyclic or tricyclic fused ring wherein the 5- to 7-membered monocyclic aromatic heterocycles are fused));
      (bb) a C₁₋₆ alkoxy-C₁₋₆ alkyl;
      (cc) a nitro;
      (dd) an oxo;
      (ee) a C₁₋₂ alkylenedioxy; and
      (ff) R⁵O-N=CH-(R⁵ is a hydrogen atom, or a linear or branched C₁₋₁₅ alkyl group, a C₃₋₈ cycloalkyl group, a C₃₋₈ cycloalkyl group fused with a C₆₋₁₄ aryl ring, a C₂₋₁₈ alkenyl group, a C₃₋₈ cycloalkenyl group, a C₂₋₁₈ alkynyl group, an aromatic monocyclic, bicyclic or tricyclic C₆₋₁₄ aryl group, a C₆₋₁₄ aryl group fused with a C₃₋₈ cycloalkane or a C₇₋₂₀ aralkyl group, each of which optionally has substituent(s) selected from substituent group B', the substituent group B' consists of the following (a) - (ff);

   (a) a linear or branched C₁₋₁₅ alkyl group, a C₃₋₈ cycloalkyl group, a C₃₋₈ cycloalkyl group fused with a C₆₋₁₄ aryl ring, a C₂₋₁₈ alkenyl group, a C₃₋₈ cycloalkenyl group, a C₂₋₁₈ alkynyl group, an aromatic monocyclic, bicyclic or tricyclic C₆₋₁₄ aryl group, a C₆₋₁₄ aryl group fused with a C₃₋₈ cycloalkane or a C₇₋₂₀ aralkyl group, each of which optionally has substituent(s) selected from substituent group C';
   (b) a 5- to 7-membered monocyclic aromatic heterocyclic group containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocyclic group containing, besides carbon atoms, 1 to 12 hetero atoms selected from the group consisting of an oxygen atom, a sulfur atom and a nitrogen atom (e.g., a bicyclic or tricyclic fused ring group wherein the 5- to 7-membered monocyclic aromatic heterocyclic group and a benzene ring are fused, or a bicyclic or tricyclic fused ring group wherein the 5- to 7-membered monocyclic aromatic heterocycles are fused), each of which optionally has substituent(s) selected from substituent group C', wherein the above-mentioned monocyclic aromatic heterocycle or fused aromatic heterocycle is optionally fused with a 5- to 7-membered monocyclic non-aromatic heterocycle or an 8- to 15-membered polycyclic non-aromatic heterocycle (e.g., a bicyclic or tricyclic fused ring wherein the 5- to 7-membered monocyclic non-aromatic heterocycle and a C₃₋₈ cycloalkane are fused, a bicyclic or tricyclic fused ring wherein the 5- to 7-membered monocyclic non-aromatic heterocycles are fused) containing, as ring-constituting atom besides carbon atom, 1 to 4 hetero atoms selected from the group consisting of an oxygen atom, a sulfur atom and a nitrogen atom;
   (c) a halogen;
   (d) a hydroxy optionally protected by a tert-butyl(dimethyl)silyl [TBDMS] ;
   (e) a cyano;
   (f) a carboxyl optionally protected by a trimethylsilylethyl [TMS-CH₂CH₂-];
   (g) a C₁₋₆ alkoxycarbonyl;
   (h) an amino optionally having substituent(s) (amino, N-mono-C₁₋₆ alkylamino; N,N-di-C₁₋₆ alkylamino; N-mono-C₆₋₁₄ aryl-amino; N-mono-heterocyclyl-amino; N,N-di-C₆₋₁₄ aryl-amino; N,N-diheterocyclyl-amino; N-C₆₋₁₄ aryl-N-heterocyclyl-amino; or 5- to 8-membered cyclic amino);
   (i) a carbamoyl optionally having substituent(s), which is selected from the following (1) - (8);
      (1) a carbamoyl,
      (2) an N-mono-C₁₋₆ alkylcarbamoyl (wherein the C₁₋₆ alkyl moiety of the N-mono-C₁₋₆ alkylcarbamoyl optionally has, at its substitutable position(s), 1 - 5 substituents selected from the group consisting of (i) a C₁₋₆ alkoxy, (ii) a phenyl, (iii) an amino, (iv) a di-C₁₋₆ alkylamino, (v) a hydroxy, (vi) a carboxyl, (vii) a C₁₋₆ alkoxycarbonyl and (viii) a heterocyclic group optionally having substituent(s) selected from substituent group C'),
      (3) N,N-di-C₁₋₆ alkylcarbamoyls (wherein the two C₁₋₆ alkyl moieties of the N,N-di-C₁₋₆ alkylcarbamoyl may be the same or different and each optionally has, at its substitutable position(s), 1 - 5 substituents selected from the group consisting of (i) a C₁₋₆ alkoxy, (ii) a phenyl, (iii) an amino, (iv) a di-C₁₋₆ alkylamino, (v) a hydroxy, (vi) a carboxyl, (vii) a C₁₋₆ alkoxycarbonyl and (viii) a heterocyclic group optionally having substituent(s) selected from substituent group C'),
      (4) an N-mono-C₆₋₁₄ aryl-carbamoyl,
      (5) an N-mono-heterocyclyl-carbamoyl (wherein the heterocycle of the N-mono-heterocyclyl-carbamoyl is a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocycle containing, besides carbon atoms, 1 to 12 hetero atoms selected from the group consisting of an oxygen atom, a sulfur atom and a nitrogen atom (e.g., a bicyclic or tricyclic fused ring wherein the 5- to 7-membered monocyclic aromatic heterocycle and a benzene ring are fused, or a bicyclic or tricyclic fused ring wherein the 5- to 7-membered monocyclic aromatic heterocycles are fused)),
      (6) an N,N-di-C₆₋₁₄ aryl-carbamoyl,
      (7) N,N-diheterocyclyl-carbamoyls (wherein the heterocycles of the N,N-diheterocyclyl-carbamoyl may be the same or different and each is a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocycle containing, besides carbon atoms, 1 to 12 hetero atoms selected from the group consisting of an oxygen atom, a sulfur atom and a nitrogen atom (e.g., a bicyclic or tricyclic fused ring wherein the 5- to 7-membered monocyclic aromatic heterocycle and a benzene ring are fused, or a bicyclic or tricyclic fused ring wherein the 5- to 7-membered monocyclic aromatic heterocycles are fused)), and
      (8) an N-C₆₋₁₄ aryl-N-heterocyclyl-carbamoyl (wherein the heterocycle of the N-C₆₋₁₄ aryl-N-heterocyclyl-carbamoyl is a 5-to 7-membered monocyclic aromatic heterocycle containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocycle containing, besides carbon atoms, 1 to 12 hetero atoms selected from the group consisting of an oxygen atom, a sulfur atom and a nitrogen atom (e.g., a bicyclic or tricyclic fused ring wherein the 5- to 7-membered monocyclic aromatic heterocycle and a benzene ring are fused, or a bicyclic or tricyclic fused ring
      wherein the 5- to 7-membered monocyclic aromatic heterocycles are fused));
   (j) a C₁₋₆ alkoxy;
   (k) a C₆₋₁₄ aryloxy;
   (l) a heterocyclyloxy (wherein the heterocycle of the heterocyclyloxy is a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocycle containing, besides carbon atoms, 1 to 12 hetero atoms selected from the group consisting of an oxygen atom, a sulfur atom and a nitrogen atom (e.g., a bicyclic or tricyclic fused ring wherein the 5- to 7-membered monocyclic aromatic heterocycle and a benzene ring are fused, or a bicyclic or tricyclic fused ring wherein the 5- to 7-membered monocyclic aromatic heterocycles are fused));
   (m) a C₁₋₆ alkyl-carbonyloxy;
   (n) a C₁₋₆ alkylthio;
   (o) a C₆₋₁₄ arylthio;
   (p) a heterocyclylthio (wherein the "heterocycle" of the heterocyclylthio is a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocycle containing, besides carbon atoms, 1 to 12 hetero atoms selected from the group consisting of an oxygen atom, a sulfur atom and a nitrogen atom (e.g., a bicyclic or tricyclic fused ring wherein the 5- to 7-membered monocyclic aromatic heterocycle and a benzene ring are fused, or a bicyclic or tricyclic fused ring wherein the 5- to 7-membered monocyclic aromatic heterocycles are fused));
   (q) a C₁₋₆ alkylsulfinyl;
   (r) a C₆₋₁₄ arylsulfinyl;
   (s) a heterocyclesulfinyl (wherein the heterocycle of the heterocyclesulfinyl is a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocycle containing, besides carbon atoms, 1 to 12 hetero atoms selected from the group consisting of an oxygen atom, a sulfur atom and a nitrogen atom (e.g., a bicyclic or tricyclic fused ring wherein the 5- to 7-membered monocyclic aromatic heterocycle and a benzene ring are fused, or a bicyclic or tricyclic fused ring wherein the 5- to 7-membered monocyclic aromatic heterocycles are fused));
   (t) a C₁₋₆ alkylsulfonyl;
   (u) a C₆₋₁₄ arylsulfonyl;
   (v) a heterocyclesulfonyl (wherein the heterocycle of the heterocyclesulfonyl is a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocycle containing, besides carbon atoms, 1 to 12 hetero atoms selected from the group consisting of an oxygen atom, a sulfur atom and a nitrogen atom (e.g., a bicyclic or tricyclic fused ring wherein the 5- to 7-membered monocyclic aromatic heterocycle and a benzene ring are fused, or a bicyclic or tricyclic fused ring wherein the 5- to 7-membered monocyclic aromatic heterocycles are fused));
   (w) a formyl;
   (x) a C₁₋₆ alkyl-carbonyl;
   (y) a C₆₋₁₄ arylcarbonyl;
   (z) a heterocyclecarbonyl (wherein the heterocycle of the heterocyclecarbonyl is a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocycle containing, besides carbon atoms, 1 to 12 hetero atoms selected from the group consisting of an oxygen atom, a sulfur atom and a nitrogen atom (e.g., a bicyclic or tricyclic fused ring wherein the 5- to 7-membered monocyclic aromatic heterocycle and a benzene ring are fused, or a bicyclic or tricyclic fused ring wherein the 5- to 7-membered monocyclic aromatic heterocycles are fused));
      (aa) a heterocyclyl-C₁₋₆ alkyl (wherein the heterocycle of the heterocyclyl-C₁₋₆ alkyl is a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocycle containing, besides carbon atoms, 1 to 12 hetero atoms selected from the group consisting of an oxygen atom, a sulfur atom and a nitrogen atom (e.g., a bicyclic or tricyclic fused ring wherein the 5- to 7-membered monocyclic aromatic heterocycle and a benzene ring are fused, or a bicyclic or tricyclic fused ring wherein the 5- to 7-membered monocyclic aromatic heterocycles are fused)),
      (bb) a C₁₋₆ alkoxy-C₁₋₆ alkyl;
      (cc) a nitro;
      (dd) an oxo;
      (ee) a C₁₋₂ alkylenedioxy; and
      (ff) R⁵O-N=CH- (R⁵ is a hydrogen atom or a linear or branched C₁₋₁₅ alkyl group, a C₃₋₈ cycloalkyl group, a C₃₋₈ cycloalkyl group fused with a C₆₋₁₄ aryl ring, a C₂₋₁₈ alkenyl group, a C₃₋₈ cycloalkenyl group, a C₂₋₁₈ alkynyl group, an aromatic monocyclic, bicyclic or tricyclic C₆₋₁₄ aryl group, a C₆₋₁₄ aryl group fused with a C₃₋₈ cycloalkane or a C₇₋₂₀ aralkyl group, each of which optionally has substituent(s) selected from substituent group C', the substituent group C' consists of a halogen; a hydroxy optionally protected by a tert-butyl(dimethyl)silyl [TBDMS]; a cyano; a carboxyl optionally protected by a trimethylsilylethyl [TMS-CH₂CH₂-]; a C₁₋₆ alkoxycarbonyl; a C₁₋₆ alkyl; a C₆₋₁₄ aryl; a C₇₋₂₀ aralkyl; a heterocyclic group; an amino; an N-mono-C₁₋₆ alkylamino; an N,N-di-C₁₋₆ alkylamino; an N-mono-C₆₋₁₄ aryl-amino; an N-mono-heterocyclyl-amino; an N,N-di-C₆₋₁₄ aryl-amino; an N,N-diheterocyclyl-amino; an N-C₆₋₁₄ aryl-N-heterocyclyl-amino; a carbamoyl; an N-mono-C₁₋₆ alkylcarbamoyl; an N,N-di-C₁₋₆ alkylcarbamoyl; an N-mono-C₆₋₁₄ aryl-carbamoyl; an N-mono-heterocyclyl-carbamoyl; an N,N-di-C₆₋₁₄ aryl-carbamoyl; an N,N-diheterocyclyl-carbamoyl; an N-C₆₋₁₄ aryl-N-heterocyclyl-carbamoyl; a C₁₋₆ alkoxy; a C₆₋₁₄ aryloxy; a heterocyclyloxy; a C₁₋₆ alkyl-carbonyloxy; a C₁₋₆ alkylthio; a C₆₋₁₄ arylthio; a heterocyclylthio ; a C₁₋₆ alkylsulfinyl; a C₆₋₁₄ arylsulfinyl; a heterocyclesulfinyl; a C₁₋₆ alkylsulfonyl; a C₆₋₁₄ arylsulfonyl; a heterocyclesulfonyl; a formyl; a C₁₋₆ alkyl-carbonyl; a C₆₋₁₄ arylcarbonyl; a heterocyclecarbonyl; a heterocyclyl-C₁₋₆ alkyl; a C₁₋₆ alkoxy-C₁₋₆ alkyl; a nitro; an oxo; and a C₁₋₂ alkylenedioxy (wherein the heterocycle is a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocycle containing, besides carbon atoms, 1 to 12 hetero atoms selected from the group consisting of an oxygen atom, a sulfur atom and a nitrogen atom (e.g., a bicyclic or tricyclic fused ring wherein the 5- to 7-membered monocyclic aromatic heterocycle and a benzene ring are fused, or a bicyclic or tricyclic fused ring wherein the 5- to 7-membered monocyclic aromatic heterocycles are fused), the heterocyclic group is a 5- to 7-membered monocyclic aromatic heterocyclic group containing, as ring-constituting atom besides carbon atom, 1 to 4 hetero atoms selected from the group consisting of an oxygen atom, a sulfur atom and a nitrogen atom, or a 8-to 16-membered bicyclic or tricyclic fused aromatic heterocyclic group containing, besides carbon atoms, 1 to 12 hetero atoms selected from the group consisting of an oxygen atom, a sulfur atom and a nitrogen atom (e.g., a bicyclic or tricyclic fused ring group wherein the 5- to 7-membered monocyclic aromatic heterocycle and a benzene ring are fused, or a bicyclic or tricyclic fused ring group wherein the 5- to 7-membered monocyclic aromatic heterocycles are fused)),
         R¹ is

   (i) a hydrogen atom or
   (ii) a linear or branched C₁₋₁₅ alkyl group, a C₃₋₈ cycloalkyl group, a C₃₋₈ cycloalkyl group fused with a C₆₋₁₄ aryl ring, a C₂₋₁₈ alkenyl group, a C₃₋₈ cycloalkenyl group, a C₂₋₁₈ alkynyl group, an aromatic monocyclic, bicyclic or tricyclic C₆₋₁₄ aryl group, a C₆₋₁₄ aryl group fused with a C₃₋₈ cycloalkane or a C₇₋₂₀ aralkyl group, each of which optionally has substituent(s) selected from substituent group B',
   W is an oxygen atom or a sulfur atom,
   X¹ and X² may be the same or different and each is
   (i) a hydrogen atom,
   (ii) a linear or branched C₁₋₁₅ alkyl group, a C₃₋₈ cycloalkyl group, a C₃₋₈ cycloalkyl group fused with a C₆₋₁₄ aryl ring, a C₂-18 alkenyl group, a C₃₋₈ cycloalkenyl group, a C₂₋₁₈ alkynyl group, an aromatic monocyclic, bicyclic or tricyclic C₆₋₁₄ aryl group, a C₆₋₁₄ aryl group fused with a C₃₋₈ cycloalkane or a C₇₋₂₀ aralkyl group, each of which optionally has substituent(s) selected from substituent group B' or
   (iii) a 5- to 7-membered monocyclic aromatic heterocyclic group containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocyclic group containing, besides carbon atoms, 1 to 12 hetero atoms selected from the group consisting of an oxygen atom, a sulfur atom and a nitrogen atom (e.g., a bicyclic or tricyclic fused ring group wherein the 5- to 7-membered monocyclic aromatic heterocyclic group and a benzene ring are fused, or a bicyclic or tricyclic fused ring group wherein the 5- to 7-membered monocyclic aromatic heterocycles are fused), each of which optionally has substituent(s) selected from substituent group B', or
   X¹ and X² optionally form, in combination, an oxygen atom, a sulfur atom or =NR²
   wherein R² is
   (i) a hydrogen atom or
   (ii) a linear or branched C₁₋₁₅ alkyl group, a C₃₋₈ cycloalkyl group, a C₃₋₈ cycloalkyl group fused with a C₆₋₁₄ aryl ring, a C₂₋₁₈ alkenyl group, a C₃₋₈ cycloalkenyl group, a C₂₋₁₈ alkynyl group, an aromatic monocyclic, bicyclic or tricyclic C₆₋₁₄ aryl group, a C₆₋₁₄ aryl group fused with a C₃₋₈ cycloalkane or a C₇₋₂₀ aralkyl group, each of which optionally has substituent(s) selected from substituent group B',
      ring B is

   (i) a ring represented by formula: 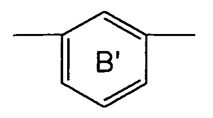 wherein ring B' is a benzene ring optionally further having substituent(s) selected from substituent group A', or
   (ii) a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom or a 8- to 16-membered bicyclic or tricyclic fused aromatic heterocycle containing, besides carbon atoms, 1 to 12 hetero atoms selected from the group consisting of an oxygen atom, a sulfur atom and a nitrogen atom (e.g., a bicyclic or tricyclic fused ring wherein the 5- to 7-membered monocyclic aromatic heterocycle and a benzene ring are fused, or a bicyclic or tricyclic fused ring wherein the 5- to 7-membered monocyclic aromatic heterocycles are fused), each of which optionally further has substituent(s) selected from substituent group A',
      Y is

   (i) a bond,
   (ii) a C₁₋₆ alkylene optionally having substituent(s) selected from substituent group B'
   (iii) a C₂₋₆ alkenylene optionally having substituent(s) selected from substituent group B'
      or
   (iv) a C₂₋₆ alkynylene optionally having substituent(s) selected from substituent group B', and
      Z is a group represented by formula: -SOₙR³ wherein n is 0, 1 or 2,
      R³ is

   (i) an amino optionally having 1 or 2 substituents selected from the group consisting of "hydrocarbon group optionally having substituent(s)" of substituent group A', "heterocyclic group optionally having substituent(s)" of substituent group A' and an acyl group,
   (ii) a 5- to 16-membered cyclic amino optionally having substituent(s) selected from the group consisting of substituent group B' and an acyl group,
   (iii) a linear or branched C₁₋₁₅ alkyl group, a C₃₋₈ cycloalkyl group, a C₃₋₈ cycloalkyl group fused with a C₆₋₁₄ aryl ring, a C₂₋₁₈ alkenyl group, a C₃₋₈ cycloalkenyl group, a C₂₋₁₈ alkynyl group, an aromatic monocyclic, bicyclic or tricyclic C₆₋₁₄ aryl group, a C₆₋₁₄ aryl group fused with a C₃₋₈ cycloalkane or a C₇₋₂₀ aralkyl group, each of which optionally has substituent(s) selected from substituent group B', or
   (iv) a 5- to 7-membered monocyclic aromatic heterocyclic group containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocyclic group containing, besides carbon atoms, 1 to 12 hetero atoms selected from the group consisting of an oxygen atom, a sulfur atom and a nitrogen atom (e.g., a bicyclic or tricyclic fused ring group wherein the 5- to 7-membered monocyclic aromatic heterocyclic group and a benzene ring are fused, or a bicyclic or tricyclic fused ring group wherein the 5- to 7-membered monocyclic aromatic heterocycles are fused), each of which optionally has substituent(s) selected from substituent group B' (provided that R³ is not a methyl group), or
      a group represented by formula: -COR⁴ wherein R⁴ is

   (i) an amino optionally having 1 or 2 substituents the group consisting of "hydrocarbon group optionally having substituent(s)" of substituent group A', "heterocyclic group optionally having substituent(s)" of substituent group A' and an acyl group,
   (ii) a 5- to 16-membered cyclic amino optionally having substituent(s) selected from the group consisting of substituent group B' and an acyl group,
   (iii) a linear or branched C₁₋₁₅ alkyl group, a C₃₋₈ cycloalkyl group, a C₃₋₈ cycloalkyl group fused with a C₆₋₁₄ aryl ring, a C₂₋₁₈ alkenyl group, a C₃₋₈ cycloalkenyl group, a C₂₋₁₈ alkynyl group, an aromatic monocyclic, bicyclic or tricyclic C₆₋₁₄ aryl group, a C₆₋₁₄ aryl group fused with a C₃₋₈ cycloalkane or a C₇₋₂₀ aralkyl group, each of which optionally has substituent(s) selected from substituent group B', or
   (iv) a 5- to 7-membered monocyclic aromatic heterocyclic group containing, as ring-constituting atom besides carbon atom, 1 to 4 hetero atoms selected from the group consisting of an oxygen atom, a sulfur atom and a nitrogen atom, or a 8- to 16-membered bicyclic or tricyclic fused aromatic heterocyclic group containing, besides carbon atoms, 1 to 12 hetero atoms selected from the group consisting of an oxygen atom, a sulfur atom and a nitrogen atom (e.g., a bicyclic or tricyclic fused ring group wherein the 5- to 7-membered monocyclic aromatic heterocyclic group and a benzene ring are fused, or a bicyclic or tricyclic fused ring group wherein the 5- to 7-membered monocyclic aromatic heterocycles are fused), each of which optionally has substituent(s) selected from substituent group B'.

### Detailed Description of the Invention

Ring A is a "5-membered aromatic heterocycle optionally having substituent(s)". "5-membered aromatic heterocycle" of "5-membered aromatic heterocycle optionally having substituent(s)" for ring A includes a 5-membered aromatic heterocycle containing, as ring-constituting atom besides carbon atoms, 1 to 4 hetero atoms selected from the group consisting of a nitrogen atom, an oxygen atom and a sulfur atom, specifically thiophene ring, furan ring, diazole ring (imidazole ring, pyrazole ring), triazole ring, thiazole ring, oxazole ring, thiadiazole ring, oxadiazole ring and the like.

Specifically, ring A includes, for example, a compound represented by ring A¹ - ring A³³ shown below. 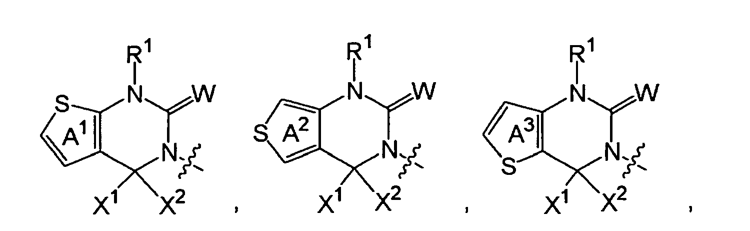 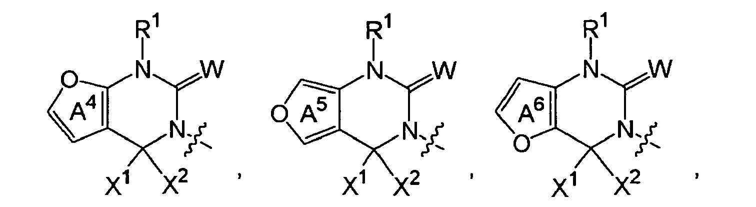 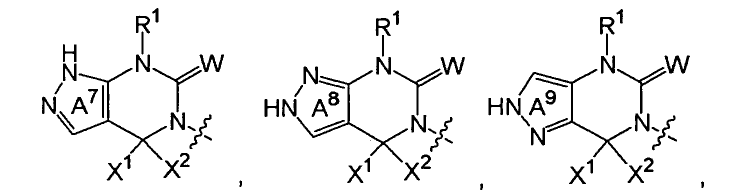 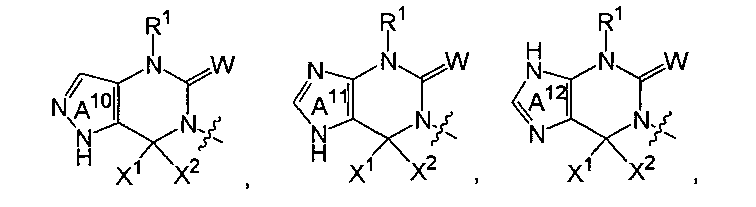 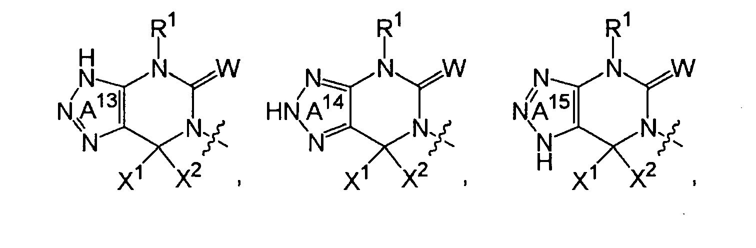 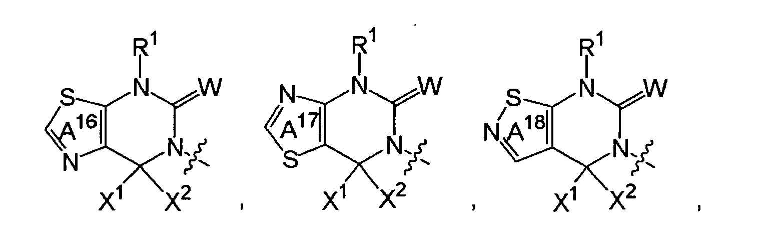 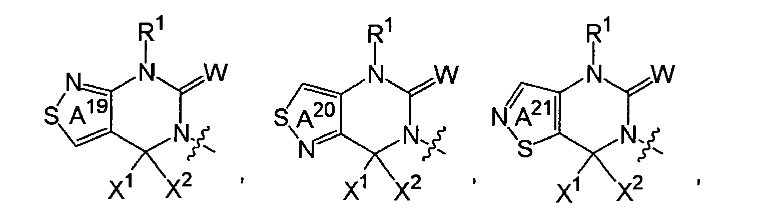 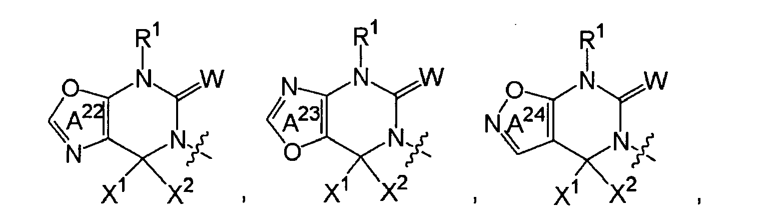 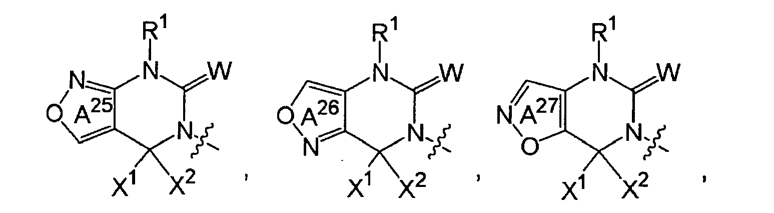 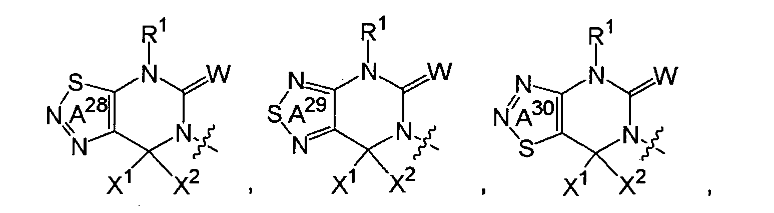 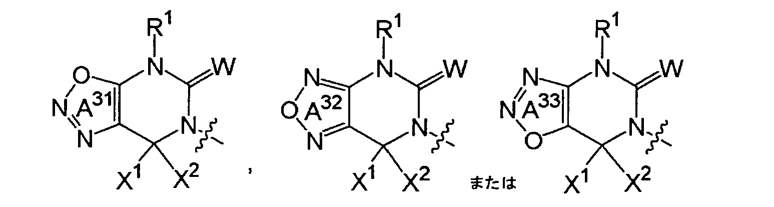 wherein ring A¹ - ring A³³ optionally have substituent(s) at the substitutable position(s) thereof, and other symbols are as defined above.

As the substituent that ring A may have, a substituent selected from the substituent group A consisting of the following (a) - (ff) can be mentioned.
(a) hydrocarbon group optionally having substituent(s) [As the "hydrocarbon group" of the above-mentioned "hydrocarbon group optionally having substituent(s)", for example, hydrocarbon group having 1 to 25, preferably 1 to 20, carbon atoms such as "alkyl group", "cycloalkyl group", "alkenyl group", "cycloalkenyl group", "alkynyl group", "aryl group", "aralkyl group" and the like, and the like can be mentioned.

As the "alkyl group", for example, a "linear or branched C₁₋₁₅ alkyl group" such as methyl, ethyl, propyl, isopropyl, butyl, isobutyl, sec-butyl, tert-butyl, pentyl, hexyl, heptyl, octyl, nonyl, decyl, undecyl, tridecyl, tetradecyl, pentadecyl and the like, and the like can be used. Preferably, a C₁₋₆ alkyl group (e.g., methyl, ethyl, propyl, isopropyl, butyl, isobutyl, sec-butyl, tert-butyl, pentyl, hexyl etc.) can be used.

As the "cycloalkyl group", for example, a "C₃₋₈ cycloalkyl group" such as cyclopropyl, cyclobutyl, cyclopentyl, cyclohexyl, cycloheptyl, cyclooctyl and the like, a C₃₋₈ cycloalkyl group fused with a C₆₋₁₄ aryl ring (e.g., benzene, naphthalene, anthracene, phenanthrene etc.) (e.g., 1,2,3,4-tetrahydronaphthalen-1-yl, 2,3-dihydro-1H-inden-1-yl, 6,7,8,9-tetrahydro-5H-benzo[7]annulen-5-yl etc.) and the like can be used.

As the "alkenyl group", for example, a "C₂₋₁₈ alkenyl group" such as vinyl, allyl, isopropenyl, 1-butenyl, 2-butenyl, 3-butenyl, 3-octenyl, 9-octadecenyl and the like, preferably a "C₂₋₆ alkenyl group" and the like can be used.

As the "cycloalkenyl group", for example, a "C₃₋₈ cycloalkenyl group" such as cyclopropenyl, cyclobutenyl, cyclopentenyl, cyclohexenyl, cycloheptenyl, cyclooctenyl and the like, and the like can be used.

As the "alkynyl group", for example, a "C₂₋₁₈ alkynyl group" such as ethynyl, propynyl, 1-butynyl, 2-butynyl, 1-pentynyl, 2-pentynyl, 3-pentynyl, 5-hexynyl and the like, preferably a "C₂₋₆ alkynyl group" and the like can be used.

As the "aryl group", for example, an aromatic monocyclic, bicyclic or tricyclic C₆₋₁₄ aryl group such as phenyl, 1-naphthyl, 2-naphthyl, phenanthryl, anthryl(anthryl) and the like; a C₆₋₁₄ aryl group fused with a C₃₋₈ cycloalkane (e.g., cyclopropane, cyclobutane, cyclopentane, cyclohexane, cycloheptane, cyclooctane etc.) (e.g., 5,6,7,8-tetrahydronaphthalen-1-yl etc.) and the like can be used.

As the "aralkyl group", for example, a C₇₋₂₀ aralkyl group such as benzyl, phenethyl, 3-phenylpropyl, 4-phenylbutyl, (1-naphthyl)methyl, 2-(1-naphthyl)ethyl, 2-(2-naphthyl)ethyl and the like [i.e., C₆₋₁₄ aryl-C₁₋₆ alkyl group] can be mentioned.

The "substituent" of the "hydrocarbon group optionally having substituent(s)" is substituent group B to be defined below. The position thereof is not particularly limited as long as it is a substitutable position, and the number of the substituent is 1 - 6, preferably 1 - 3.];
(b) heterocyclic group optionally having substituent(s) [As the "heterocycle" of the above-mentioned "heterocyclic group optionally having substituent(s)", for example, an aromatic heterocycle and a non-aromatic heterocycle and the like can be mentioned.

As the aromatic heterocycle, for example, a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom or a 8- to 16-membered bicyclic or tricyclic fused aromatic heterocycle containing, besides carbon atoms, 1 to 12 hetero atoms selected from the group consisting of an oxygen atom, a sulfur atom and a nitrogen atom can be mentioned.

As the 5-membered monocyclic aromatic heterocyclic group containing, as ring-constituting atom besides carbon atom, 1 to 4 hetero atoms selected from the group consisting of an oxygen atom, a sulfur atom and a nitrogen atom, for example, furyl (e.g., 2-furyl, 3-furyl etc.), thienyl (e.g., 2-thienyl, 3-thienyl etc.), pyrrolyl (e.g., 1-pyrrolyl, 2-pyrrolyl, 3-pyrrolyl etc.), imidazolyl (e.g., imidazol-1-yl, imidazol-2-yl, imidazol-4-yl, imidazol-5-yl etc.), pyrazolyl (e.g., 1-pyrazolyl, 3-pyrazolyl, 4-pyrazolyl, 5-pyrazolyl etc.), 1,3-oxazolyl (e.g., 1,3-oxazol-2-yl, 1,3-oxazol-4-yl, 1,3-oxazol-5-yl etc.), isoxazolyl (e.g., 3-isoxazolyl, 4-isoxazolyl, 5-isoxazolyl etc.), 1,3-thiazolyl (e.g., 1,3-thiazol-2-yl, 1,3-thiazol-4-yl, 1,3-thiazol-5-yl etc.), isothiazolyl (e.g., 3-isothiazolyl, 4-isothiazolyl, 5-isothiazolyl etc.), oxadiazolyl (e.g., 1,2,4-oxadiazol-3-yl, 1,2,4-oxadiazol-5-yl, 1,3,4-oxadiazol-2-yl, 1,2,3-oxadiazol-4-yl, 1,2,3-oxadiazol-5-yl, 1,2,5-oxadiazol-3-yl etc.), thiadiazolyl (e.g., 1,2,4-thiadiazol-3-yl, 1,2,4-thiadiazol-5-yl, 1,3,4-thiadiazol-2-yl, 1,2,3-thiadiazol-4-yl, 1,2,3-thiadiazol-5-yl, 1,2,5-thiadiazol-3-yl etc.), triazolyl [for example, 1,2,3-triazolyl (e.g., 1H-1,2,3-triazol-1-yl, 1H-1,2,3-triazol-4-yl, 1H-1,2,3-triazol-5-yl, 2H-1,2,3-triazol-2-yl, 2H-1,2,3-triazol-4-yl etc.), 1,2,4-triazolyl (e.g., 1H-1,2,4-triazol-1-yl, 1H-1,2,4-triazol-3-yl, 4H-1,2,4-triazol-3-yl, 4H-1,2,4-triazol-4-yl etc.) etc.], tetrazolyl (e.g., 1H-tetrazol-l-yl, 1H-tetrazol-5-yl, 2H-tetrazol-2-yl, 2H-tetrazol-5-yl etc.) and the like can be mentioned.

As the 6-membered monocyclic aromatic heterocyclic group containing, as ring-constituting atom besides carbon atom, 1 to 4 hetero atoms selected from the group consisting of an oxygen atom, a sulfur atom and a nitrogen atom, for example, pyridyl (e.g., 2-pyridyl, 3-pyridyl, 4-pyridyl, N-oxide-2-pyridyl, N-oxide-3-pyridyl, N-oxide-4-pyridyl etc.), pyrimidinyl (e.g., 2-pyrimidinyl, 4-pyrimidinyl, 5-pyrimidinyl, 1-oxide-2-pyrimidinyl, 1-oxide-4-pyrimidinyl, 1-oxide-5-pyrimidinyl etc.), pyridazinyl (e.g., 3-pyridazinyl, 4-pyridazinyl, 1-oxide-3-pyridazinyl, 1-oxide-4-pyridazinyl, 2-oxide-3-pyridazinyl, 2-oxide-4-pyridazinyl etc.), pyrazinyl (e.g., 2-pyrazinyl, 3-pyrazinyl etc.), triazinyl (e.g., 1,2,3-triazin-4-yl, 1,2,3-triazin-5-yl, 1,2,4-triazin-3-yl, 1,2,4-triazin-5-yl, 1,2,4-triazin-6-yl, 1,3,5-triazin-2-yl etc.) and the like can be mentioned.

As the 7-membered monocyclic aromatic heterocyclic group containing, as ring-constituting atom besides carbon atom, 1 to 4 hetero atoms selected from the group consisting of an oxygen atom, a sulfur atom and a nitrogen atom, for example, oxepinyl (e.g., 2-oxepinyl, 3-oxepinyl, 4-oxepinyl etc.), thiepinyl (e.g., 2-thiepinyl, 3-thiepinyl, 4-thiepinyl etc.), 1H-azepinyl (e.g., 1H-azepin-1-yl, 1H-azepin-2-yl, 1H-azepin-3-yl etc.), diazepinyl [for example, 1H-1,2-diazepinyl (e.g., 1H-1,2-diazepin-1-yl, 1H-1,2-diazepin-3-yl, 1H-1,2-diazepin-4-yl, 1H-1,2-diazepin-5-yl etc.), 1H-1,3-diazepinyl (e.g., 1H-1,3-diazepin-1-yl, 1H-1,3-diazepin-2-yl, 1H-1,3-diazepin-4-yl, 1H-1,3-diazepin-5-yl etc.), 1H-1,4-diazepinyl (e.g., 1H-1,4-diazepin-1-yl, 1H-1,4-diazepin-2-yl, 1H-1,4-diazepin-5-yl, 1H-1,4-diazepin-6-yl etc.) etc.], oxazepinyl [for example, 1,2-oxazepinyl (e.g., 1,2-oxazepin-3-yl, 1,2-oxazepin-4-yl, 1,2-oxazepin-5-yl, 1,2-oxazepin-6-yl, 1,2-oxazepine-7-yl etc.), 1,3-oxazepinyl (e.g., 1,3-oxazepin-2-yl, 1,3-oxazepin-4-yl, 1,3-oxazepin-5-yl, 1,3-oxazepin-6-yl, 1,3-oxazepine-7-yl etc.), 1,4-oxazepinyl (e.g., 1,4-oxazepin-2-yl, 1,4-oxazepin-3-yl, 1,4-oxazepin-5-yl, 1,4-oxazepin-6-yl, 1,4-oxazepine-7-yl etc.) etc.], thiazepinyl [for example, 1,2-thiazepinyl (e.g., 1,2-thiazepin-3-yl, 1,2-thiazepin-4-yl, 1,2-thiazepin-5-yl, 1,2-thiazepin-6-yl, 1,2-thiazepin-7-yl etc.), 1,3-thiazepinyl (e.g., 1,3-thiazepin-2-yl, 1,3-thiazepin-4-yl, 1,3-thiazepin-5-yl, 1,3-thiazepin-6-yl, 1,3-thiazepin-7-yl etc.), 1,4-thiazepinyl (e.g., 1,4-thiazepin-2-yl, 1,4-thiazepin-3-yl, 1,4-thiazepin-5-yl, 1,4-thiazepin-6-yl, 1,4-thiazepin-7-yl etc.) etc.] and the like can be mentioned.

As the 8- to 16-membered bicyclic or tricyclic fused aromatic heterocycle containing, as ring-constituting atom besides carbon atom, 1 to 12 hetero atoms selected from the group consisting of an oxygen atom, a sulfur atom and a nitrogen atom, for example, a bicyclic or tricyclic fused ring wherein the 5- to 7-membered monocyclic aromatic heterocycle and a benzene ring are fused, a bicyclic or tricyclic fused ring wherein the 5- to 7-membered monocyclic aromatic heterocycles are fused and the like can be mentioned.

For example, bicyclic or tricyclic fused ring group containing, besides carbon atoms, 1 to 12 hetero atoms selected from the group consisting of an oxygen atom, a sulfur atom and a nitrogen atom (e.g., benzofuryl, benzothienyl, benzoxazolyl, benzothiazolyl, benzothiadiazolyl, benzimidazolyl, quinolyl, isoquinolyl, quinazolinyl, quinoxalinyl, indolyl, 1H-indazolyl, 1H-pyrrolopyridyl, 1H-imidazopyridyl, 1H-imidazopyrazinyl, 1H-pyrrolo[2,3-b]pyrazinyl, tetrazolo[1,5-b]pyridazinyl, triazolo[4,5-b]pyridazinyl, cinnolinyl, phthalazinyl, indolizinyl, quinolizinyl, 1,8-naphthyridinyl, purinyl, pteridinyl, dibenzofuranyl, carbazolyl, acrydinyl, phenanthridinyl, chromanyl, benzoxazinyl, phenazinyl, phenothiazinyl, phenoxazinyl etc.) and the like can be mentioned.

Preferable examples of the aromatic heterocycle include furan, thiophene, pyrrole, imidazole, pyrazole, oxazole, isoxazole, thiazole, isothiazole, oxadiazole, thiadiazole, triazole, tetrazole, pyridine, pyrimidine, pyridazine, pyrazine, triazine, benzofuran, benzothiophene, benzoxazole, benzothiazole, benzothiadiazole, benzimidazole, quinoline, isoquinoline, quinazoline, quinoxaline, indole, 1H-indazole, 1H-pyrrolopyridine, 1H-imidazopyridine, 1H-imidazopyrazine, 1H-pyrrolo[2,3-b]pyrazine and the like. The aromatic heterocycle is preferably 5- or 6-membered aromatic heterocycle, more preferably furan, thiophene, pyrazole, oxazole, thiazole, pyridine, pyrimidine and the like.

The above-mentioned monocyclic aromatic heterocycle or fused aromatic heterocycle may be fused with the following monocyclic non-aromatic heterocycle or polycyclic non-aromatic heterocycle.

As the non-aromatic heterocycle, for example, 5- to 7-membered monocyclic non-aromatic heterocycle or 8- to 15-membered polycyclic non-aromatic heterocycle each of which contains, as ring-constituting atom besides carbon atom, 1 to 4 hetero atoms selected from the group consisting of an oxygen atom, a sulfur atom and a nitrogen atom can be mentioned.

As the monocyclic non-aromatic heterocycle, for example, pyrrolidine, pyrroline, pyrazolidine, piperidine, piperazine (e.g., 2-piperazinyl, 3-piperazinyl etc.), pyran, thiopyran, morpholine (e.g., 2-morpholinyl, 3-morpholinyl, 4-morpholinyl etc.), thiomorpholine (e.g., 2-thiomorpholinyl, 3-thiomorpholinyl, 4-thiomorpholinyl etc.), hexamethyleneimine, oxazolidine, thiazolidine, imidazolidine, imidazoline, tetrahydrofuran, azepane, tetrahydropyridine, diazepane (e.g., 1,2-diazepane, 1,3-diazepane, 1,4-diazepane etc.), oxazepane (e.g., 1,2-oxazepane, 1,3-oxazepane, 1,4-oxazepane etc.), thiazepane (e.g., 1,2-thiazepane, 1,3-thiazepane, 1,4-thiazepane etc.) and the like can be mentioned.

As the polycyclic non-aromatic heterocycle, for example, a bicyclic or tricyclic fused ring wherein the 5- to 7-membered monocyclic non-aromatic heterocycle and a cycloalkane [for example, a C₃₋₈ cycloalkane (e.g., cyclopropane, cyclobutane, cyclopentane, cyclohexane, cycloheptane, cyclooctane etc.) etc.] are fused, a bicyclic or tricyclic fused ring wherein the 5- to 7-membered monocyclic non-aromatic heterocycles are fused and the like can be mentioned.

Preferable examples of the non-aromatic heterocycle include pyrrolidine, pyrroline, pyrazolidine, piperidine, piperazine (e.g., 2-piperazinyl, 3-piperazinyl etc.), pyran, thiopyran, morpholine (e.g., 2-morpholinyl, 3-morpholinyl, 4-morpholinyl etc.), thiomorpholine (e.g., 2-thiomorpholinyl, 3-thiomorpholinyl, 4-thiomorpholinyl etc.), hexamethyleneimine, oxazolidine, thiazolidine, imidazolidine, imidazoline, tetrahydrofuran, azepane, tetrahydropyridine and the like.

The above-mentioned monocyclic non-aromatic heterocycle or polycyclic non-aromatic heterocycle may be fused with the above-mentioned monocyclic aromatic heterocycle or fused aromatic heterocycle.

The "substituent" of the "heterocyclic group optionally having substituent(s)" is substituent group B to be defined below. The position thereof is not particularly limited as long as it is a substitutable position, and the number of the substituent is 1 - 6, preferably 1 - 3.];
(c) halogen (e.g., fluorine, chlorine, bromine, iodine atom etc.);
(d) hydroxy optionally protected by a protecting group (e.g., tert-butyl(dimethyl)silyl [TBDMS] etc.);
(e) cyano;
(f) carboxyl optionally protected by a protecting group (e.g., trimethylsilylethyl [TMS-CH₂CH₂-] etc.);
(g) alkoxycarbonyl [for example, C₁₋₆ alkoxycarbonyl (e.g., methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, hexyloxycarbonyl) etc.];
(h) amino optionally having substituent(s);
[As the above-mentioned "amino optionally having substituent(s)", "amino", "monosubstituted amino", "disubstituted amino" and "cyclic amino optionally having substituent(s)" can be mentioned.

The "substituent" of "monosubstituted amino", "disubstituted amino" and "cyclic amino optionally having substituent(s)" are as defined below.

In the "disubstituted amino", the substituents may be the same or different.

The position of the substituent of "cyclic amino optionally having substituent(s)" is not particularly limited as long as it is a substitutable position, and the number of the substituent is 1 - 6, preferably 1 - 3.

As the "cyclic amino" of "cyclic amino optionally having substituent(s)", for example, a 5- to 16-membered cyclic amino such as 1-pyrrolidinyl, 1-pyrrolinyl, 1-pyrrolyl, azepan-1-yl, 1-imidazolidinyl, 1-imidazolinyl, 1-imidazolyl, 2-pyrazolidinyl, 2-pyrazolinyl, 1-pyrazolyl, 1-indolinyl, 1-indolyl, 2-isoindolinyl, 2-isoindolyl, 2,3-dihydro-1H-indol-1-yl, 1-piperidyl, 3,4-dihydro-1,5-naphthyridin-1(2H)-yl, 3,4-dihydroquinolin-1(2H)-yl, 3,4-dihydroisoquinolin-2(1H)-yl, 1-piperazinyl, octahydroquinolin-1(2H)-yl, octahydroisoquinolin-2(1H)-yl, 3,4-dihydroquinoxalin-1(2H)-yl, 1H-azepin-1-yl, 2,3,4,5,6,7-hexahydro-1H-azepin-1-yl, 2,3,4,5-tetrahydro-1H-1-benzoazepin-1-yl, 1,3,4,5-tetrahydro-2H-2-benzoazepin-2-yl, 3,4,5,6-tetrahydro-1-benzoazocin-1(2H)-yl, 2,3-dihydro-4,1-benzothiazepin-1(5H)-yl, 3,4-dihydro-1,5-benzothiazepin-5(2H)-yl, 2,3-dihydro-4,1-benzothiazepin-1(5H)-yl, 2,3-dihydro-4,1-benzooxazepin-1(5H)-yl, 1,2,4,5-tetrahydro-3H-3-benzoazepin-3-yl, 2,3,4,5-tetrahydro-1H-1,5-benzodiazepin-1-yl, 2,3,4,5-tetrahydro-1H-1,4-benzodiazepin-1-yl, 2,3-dihydro-4H-1,4-benzoxazin-4-yl, 3,4-dihydro-2H-1,4-benzoxazin-1-yl, 3,4-dihydro-1,5-benzooxazepin-5(2H)-yl, 5,6,7,8-tetrahydro-4H-thieno[3,2-b]azepin-4-yl, 4,5,6,7-tetrahydro-8H-thieno[2,3-b]azepin-8-yl, 4-morpholinyl, 4-thiomorpholinyl, 2,3-dihydro-4H-pyrido[3,2-b][1,4]oxazin-4-yl, 2,3-dihydro-4H-1,4-benzothiazin-4-yl, 5,6,7,8-tetrahydropyrazolo[4,3-b]azepin-4(1H)-yl and the like can be mentioned.];
(i) carbamoyl optionally having substituent(s)
   [As the above-mentioned "carbamoyl optionally having substituent(s)", for example, a carbamoyl optionally having 1 or 2 substituents selected from the group consisting of "hydrocarbon group optionally having substituent(s)" of substituent group A, "heterocyclic group optionally having substituent(s)" of substituent group A" and the below-mentioned acyl group and the like can be mentioned, particularly preferably, for example, the following (1) - (8) and the like can be mentioned:
   (1) carbamoyl,
   (2) N-mono-C₁₋₆ alkylcarbamoyl (e.g., methylcarbamoyl, ethylcarbamoyl, propylcarbamoyl, isopropylcarbamoyl, butylcarbamoyl, isobutylcarbamoyl, sec-butylcarbamoyl, tert-butylcarbamoyl, pentylcarbamoyl, hexylcarbamoyl etc.) [wherein the C₁₋₆ alkyl moiety of the "N-mono-C₁₋₆ alkylcarbamoyl" may have, at its substitutable position(s), 1 - 5, preferably 1 - 2, substituents selected from (i) C₁₋₆ alkoxy (e.g., methoxy, ethoxy, propoxy, isopropoxy, butoxy, isobutoxy, sec-butoxy, tert-butoxy, pentyloxy, hexyloxy etc.), (ii) phenyl, (iii) amino, (iv) di-C₁₋₆ alkylamino (e.g., dimethylamino, diethylamino, dipropylamino, diisopropylamino, dibutylamino, di(sec-butyl)amino, di(tert-butyl)amino, N-ethyl-N-methylamino, N-methyl-N-propylamino, N-methyl-N-isopropylamino, N-butyl-N-methylamino, N-(sec-butyl)-N-methylamino, N-(tert-butyl)-N-methylamino, N-ethyl-N-propylamino, N-ethyl-N-isopropylamino, N-butyl-N-ethylamino, N-(sec-butyl)-N-ethylamino, N-(tert-butyl)-N-ethylamino, N-isopropyl-N-propylamino, N-butyl-N-propylamino, N-(sec-butyl)-N-propylamino, N-(tert-butyl)-N-propylamino etc.), (v) hydroxy, (vi) carboxyl, (vii) C₁₋₆ alkoxycarbonyl (e.g., methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, hexyloxycarbonyl etc.), (viii) heterocyclic group optionally having substituent(s) (as defined for the "heterocyclic group optionally having substituent(s)" as the substituent for the above-mentioned ring A) and the like],
   (3) N,N-di-C₁₋₆ alkylcarbamoyl (e.g., dimethylcarbamoyl, diethylcarbamoyl, dipropylcarbamoyl, diisopropylcarbamoyl, dibutylcarbamoyl, diisobutylcarbamoyl, methylethylcarbamoyl etc.) [wherein the two C₁₋₆ alkyl moieties of the "N,N-di-C₁₋₆ alkylcarbamoyl" may be the same or different and each optionally has, at its substitutable position(s), 1 - 5, preferably 1 - 2, substituents selected from (i) C₁₋₆ alkoxy (e.g., methoxy, ethoxy, propoxy, isopropoxy, butoxy, isobutoxy, sec-butoxy, tert-butoxy, pentyloxy, hexyloxy etc.), (ii) phenyl, (iii) amino, (iv) di-C₁₋₆ alkylamino (e.g., dimethylamino, diethylamino, dipropylamino, diisopropylamino, dibutylamino, di(sec-butyl)amino, di(tert-butyl)amino, N-ethyl-N-methylamino, N-methyl-N-propylamino, N-methyl-N-isopropylamino, N-butyl-N-methylamino, N-(sec-butyl)-N-methylamino, N-(tert-butyl)-N-methylamino, N-ethyl-N-propylamino, N-ethyl-N-isopropylamino, N-butyl-N-ethylamino, N-(sec-butyl)-N-ethylamino, N-(tert-butyl)-N-ethylamino, N-isopropyl-N-propylamino, N-butyl-N-propylamino, N-(sec-butyl)-N-propylamino, N-(tert-butyl)-N-propylamino etc.), (v) hydroxy, (vi) carboxyl, (vii) C₁₋₆ alkoxycarbonyl (e.g., methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, hexyloxycarbonyl etc.), (viii) heterocyclic group optionally having substituent(s) (as defined for the "heterocyclic group optionally having substituent(s)" as the substituent for the above-mentioned ring A) and the like],
   (4) N-mono-C₆₋₁₄ aryl-carbamoyl (e.g., phenylcarbamoyl, 1-naphthylcarbamoyl, 2-naphthylcarbamoyl, phenanthrylcarbamoyl, anthrylcarbamoyl etc.),
   (5) N-mono-heterocyclyl-carbamoyl (wherein the "heterocycle" of the "N-mono-heterocyclyl-carbamoyl" is as defined for the "heterocycle" of the above-mentioned "heterocyclic group optionally having substituent(s)"),
   (6) N,N-di-C₆₋₁₄ aryl-carbamoyl (e.g., diphenylcarbamoyl, di(1-naphthyl)carbamoyl, di(2-naphthyl)carbamoyl, di(phenanthryl)carbamoyl, di(anthryl)carbamoyl, N-phenyl-N-(1-naphthyl)carbamoyl etc.),
   (7) N,N-diheterocyclyl-carbamoyl (wherein the "heterocycles" of the "N,N-diheterocyclyl-carbamoyl" may be the same or different and each is as defined for the "heterocycle" of the above-mentioned "heterocyclic group optionally having substituent(s)"), and
   (8) N-C₆₋₁₄ aryl-N-heterocyclyl-carbamoyl (wherein as the "C₆₋₁₄ aryl" of the "N-C₆₋₁₄ aryl-N-heterocyclyl-carbamoyl", phenyl, 1-naphthyl, 2-naphthyl, phenanthryl, anthryl and the like can be mentioned, the "heterocycle" is as defined for the "heterocycle" of the above-mentioned "heterocyclic group optionally having substituent(s)")];

(j) C₁₋₆ alkoxy (e.g., methoxy, ethoxy, propoxy, isopropoxy, butoxy, isobutoxy, sec-butoxy, tert-butoxy, pentyloxy, hexyloxy etc.);
(k) C₆₋₁₄ aryloxy (e.g., phenyloxy, 1-naphthyloxy, 2-naphthyloxy, phenanthryloxy, anthryloxy etc.);
(l) heterocyclyloxy (wherein the "heterocycle" of the "heterocyclyloxy" is as defined for the "heterocycle" of the above-mentioned "heterocyclic group optionally having substituent(s)");
(m) C₁₋₆ alkyl-carbonyloxy (e.g., methylcarbonyloxy, ethylcarbonyloxy, propylcarbonyloxy, isopropylcarbonyloxy, butylcarbonyloxy, isobutylcarbonyloxy, sec-butylcarbonyloxy, tert-butylcarbonyloxy, pentylcarbonyloxy, hexylcarbonyloxy etc.);
(n) C₁₋₆ alkylthio (e.g., methylthio, ethylthio, propylthio, isopropylthio, butylthio, isobutylthio, sec-butylthio, tert-butylthio, pentylthio, hexylthio etc.);
(o) C₆₋₁₄ arylthio (e.g., phenylthio, 1-naphthylthio, 2-naphthylthio, phenanthrylthio, anthrylthio etc.);
(p) heterocyclylthio (wherein the "heterocycle" of "heterocyclylthio" is as defined for the "heterocycle" of the above-mentioned "heterocyclic group optionally having substituent(s)");
(q) C₁₋₆ alkylsulfinyl (e.g., methylsulfinyl, ethylsulfinyl, propylsulfinyl, isopropylsulfinyl, butylsulfinyl, isobutylsulfinyl, sec-butylsulfinyl, tert-butylsulfinyl, pentylsulfinyl, hexylsulfinyl etc.);
(r) C₆₋₁₄ arylsulfinyl (e.g., phenylsulfinyl, 1-naphthylsulfinyl, 2-naphthylsulfinyl, phenanthrylsulfinyl, anthrylsulfinyl etc.);
(s) heterocyclesulfinyl (wherein the "heterocycle" of the "heterocyclesulfinyl" is as defined for the "heterocycle" of the above-mentioned "heterocyclic group optionally having substituent(s)");
(t) C₁₋₆ alkylsulfonyl (e.g., methylsulfonyl, ethylsulfonyl, propylsulfonyl, isopropylsulfonyl, butylsulfonyl, isobutylsulfonyl, sec-butylsulfonyl, tert-butylsulfonyl, pentylsulfonyl, hexylsulfonyl etc.);
(u) C₆₋₁₄ arylsulfonyl (e.g., phenylsulfonyl, 1-naphthylsulfonyl, 2-naphthylsulfonyl, phenanthrylsulfonyl, anthrylsulfonyl etc.);
(v) heterocyclesulfonyl (wherein the "heterocycle" of the "heterocyclesulfonyl" is as defined for the "heterocycle" of the above-mentioned "heterocyclic group optionally having substituent(s)");
(w) formyl;
(x) C₁₋₆ alkyl-carbonyl (e.g., acetyl, ethylcarbonyl, propylcarbonyl, isopropylcarbonyl, butylcarbonyl, isobutylcarbonyl, sec-butylcarbonyl, tert-butylcarbonyl, pentylcarbonyl, hexylcarbonyl etc.);
(y) C₆₋₁₄ arylcarbonyl (e.g., benzoyl, 1-naphthylcarbonyl, 2-naphthylcarbonyl, phenanthrylcarbonyl, anthrylcarbonyl etc.);
(z) heterocyclecarbonyl (wherein the "heterocycle" of the "heterocyclecarbonyl" is as defined for the "heterocycle" of the above-mentioned "heterocyclic group optionally having substituent(s)");
   (aa) heterocyclyl-C₁₋₆ alkyl (wherein the "heterocycle" of the "heterocyclyl-C₁₋₆ alkyl" is as defined for the "heterocycle" of the above-mentioned "heterocyclic group optionally having substituent(s)", and as the "C₁₋₆ alkyl", methyl, ethyl, propyl, isopropyl, butyl, isobutyl, sec-butyl, tert-butyl, pentyl, hexyl and the like can be mentioned);
   (bb) C₁₋₆ alkoxy-C₁₋₆ alkyl (wherein as the "C₁₋₆ alkoxy" of the "C₁₋₆ alkoxy-C₁₋₆ alkyl", methoxy, ethoxy, propoxy, isopropoxy, butoxy, isobutoxy, sec-butoxy, tert-butoxy, pentyloxy, hexyloxy and the like can be mentioned, as the "C₁₋₆ alkyl", methyl, ethyl, propyl, isopropyl, butyl, isobutyl, sec-butyl, tert-butyl, pentyl, hexyl and the like can be mentioned);
   (cc) nitro;
   (dd) oxo;
   (ee) C₁₋₂ alkylenedioxy (e.g., methylenedioxy, ethylenedioxy etc.); and
   (ff) R⁵O-N=CH- (R⁵ is a hydrogen atom or hydrocarbon group optionally having substituent(s), the "hydrocarbon group optionally having substituent(s)" for the above-mentioned R⁵ is as defined for the "hydrocarbon group optionally having substituent(s)" of the above-mentioned substituent group A).

The position of the substituent of ring A is not particularly limited as long as it is a substitutable position. The number of the substituent is 1 - 3, preferably 1 or 2. These substituents optionally have the above-defined substituent(s) at its substitutable position(s), and the number of the substituent is not particularly limited.

As the "substituent" of "hydrocarbon group optionally having substituent(s)" as the substituent for the above-mentioned ring A, the substituent selected from the substituent group B consisting of the following (a) - (ff) can be mentioned.
(a) hydrocarbon group optionally having substituent(s)
   [As the "hydrocarbon group" of the above-mentioned "hydrocarbon group optionally having substituent(s)", those similar to the "hydrocarbon group" of substituent group A can be used.
   As the "substituent" of the above-mentioned "hydrocarbon group optionally having substituent(s)", for example, halogen (e.g., fluorine, chlorine, bromine, iodine atom etc.); hydroxy optionally protected by a protecting group (e.g., tert-butyl(dimethyl)silyl[TBDMS] etc.); cyano; carboxyl optionally protected by a protecting group (e.g., trimethylsilylethyl[TMS-CH₂CH₂-] etc.); C₁₋₆ alkoxycarbonyl; C₁₋₆ alkyl; C₆₋₁₄ aryl; C₇₋₂₀ aralkyl; heterocyclic group; amino; N-mono-C₁₋₆ alkylamino; N,N-di-C₁₋₆ alkylamino; N-mono-C₆₋₁₄ aryl-amino; N-mono-heterocyclyl-amino; N,N-di-C₆₋₁₄ aryl-amino; N,N-diheterocyclyl-amino; N-C₆₋₁₄ aryl-N-heterocyclyl-amino; carbamoyl; N-mono-C₁₋₆ alkylcarbamoyl; N,N-di-C₁₋₆ alkylcarbamoyl; N-mono-C₆-₁₄ aryl-carbamoyl; N-mono-heterocyclyl-carbamoyl; N,N-di-C₆₋₁₄ aryl-carbamoyl; N,N-diheterocyclyl-carbamoyl; N-C₆₋₁₄ aryl-N-heterocyclyl-carbamoyl; C₁₋₆ alkoxy; C₆₋₁₄ aryloxy; heterocyclyloxy; C₁₋₆ alkyl-carbonyloxy; C₁₋₆ alkylthio; C₆₋₁₄ arylthio; heterocyclylthio; C₁₋₆ alkylsulfinyl; C₆₋₁₄ arylsulfinyl; heterocyclesulfinyl; C₁₋₆ alkylsulfonyl; C₆₋₁₄ arylsulfonyl; heterocyclesulfonyl; formyl; C₁₋₆ alkyl-carbonyl; C₆₋₁₄ arylcarbonyl; heterocyclecarbonyl; heterocyclyl-C₁₋₆ alkyl; C₁₋₆ alkoxy-C₁₋₆ alkyl; nitro; oxo; C₁₋₂ alkylenedioxy (hereinafter to be referred to as substituent group C. The "heterocycle" of substituent group C is as defined for the "heterocycle" of the "heterocyclic group optionally having substituent(s)" of the above-mentioned substituent group A) and the like are used. The position thereof is not particularly limited as long as it is a substitutable position, and the number of the substituent is 1 - 6, preferably 1 - 3.];
(b) a heterocyclic group optionally having substituent(s)
   [as the "heterocycle" of the above-mentioned "heterocyclic group optionally having substituent(s)", those similar to the "heterocyclic group" of the "heterocyclic group optionally having substituent(s)" of substituent group A can be used.
   As the "substituent" of "heterocyclic group optionally having substituent(s)", a substituent elected from substituent group C can be used, and the position thereof is not particularly limited as long as it is a substitutable position, and the number of the substituent is 1 - 6, preferably 1 - 3.];
(c) halogen (e.g., fluorine, chlorine, bromine, iodine atom etc.);
(d) hydroxy optionally protected by a protecting group (e.g., tert-butyl(dimethyl)silyl[TBDMS] etc.);
(e) cyano;
(f) carboxyl optionally protected by a protecting group (e.g., trimethylsilylethyl [TMS-CH₂CH₂-] etc.);
(g) alkoxycarbonyl [for example, C₁₋₆ alkoxycarbonyl (e.g., methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, hexyloxycarbonyl etc.];
(h) amino optionally having substituent(s) (e.g., amino, N-mono-C₁₋₆ alkylamino; N,N-di-C₁₋₆ alkylamino; N-mono-C₆₋₁₄ aryl-amino; N-mono-heterocyclyl-amino; N,N-di-C₆₋₁₄ aryl-amino; N,N-diheterocyclyl-amino; N-C₆₋₁₄ aryl-N-heterocyclyl-amino; 5- to 8-membered cyclic amino);
(i) carbamoyl optionally having substituent(s) (e.g., carbamoyl; N-mono-C₁₋₆ alkylcarbamoyl; N,N-di-C₁₋₆ alkylcarbamoyl; N-mono-C₆₋₁₄ aryl-carbamoyl, N-mono-heterocyclyl-carbamoyl; N,N-di-C₆₋₁₄ aryl-carbamoyl; N,N-diheterocyclyl-carbamoyl; N-C₆₋₁₄ aryl-N-heterocyclyl-carbamoyl) (wherein the C₁₋₆ alkyl moiety of the N-mono-C₁₋₆ alkylcarbamoyl may have, at its substitutable position(s), 1 - 5 substituents selected from the group consisting of (i) C₁₋₆ alkoxy, (ii) phenyl, (iii) amino, (iv) di-C₁₋₆ alkylamino, (v) hydroxy, (vi) carboxyl, (vii) C₁₋₆ alkoxycarbonyl and (viii) heterocyclic group optionally having substituent(s) selected from substituent group C, the two C₁₋₆ alkyl moieties of the N,N-di-C₁₋₆ alkylcarbamoyl may be the same or different and each optionally has, at its substitutable position(s), 1 - 5 substituents selected from the group consisting of (i) C₁₋₆ alkoxy, (ii) phenyl, (iii) amino, (iv) di-C₁₋₆ alkylamino, (v) hydroxy, (vi) carboxyl, (vii) C₁₋₆ alkoxycarbonyl and (viii) heterocyclic group optionally having substituent(s) selected from substituent group C);
(j) C₁₋₆ alkoxy (e.g., methoxy, ethoxy, propoxy, isopropoxy, butoxy, isobutoxy, sec-butoxy, tert-butoxy, pentyloxy, hexyloxy etc.);
(k) C₆₋₁₄ aryloxy (e.g., phenyloxy, 1-naphthyloxy, 2-naphthyloxy, phenanthryloxy, anthryloxy etc.);
(l) heterocyclyloxy (wherein the "heterocycle" of "heterocyclyloxy" is as defined for the "heterocycle" of the above-mentioned "heterocyclic group optionally having substituent(s)");
(m) C₁₋₆ alkyl-carbonyloxy (e.g., methylcarbonyloxy, ethylcarbonyloxy, propylcarbonyloxy, isopropylcarbonyloxy, butylcarbonyloxy, isobutylcarbonyloxy, sec-butylcarbonyloxy, tert-butylcarbonyloxy, pentylcarbonyloxy, hexylcarbonyloxy etc.);
(n) C₁₋₆ alkylthio (e.g., methylthio, ethylthio, propylthio, isopropylthio, butylthio, isobutylthio, sec-butylthio, tert-butylthio, pentylthio, hexylthio etc.);
(o) C₆₋₁₄ arylthio (e.g., phenylthio, 1-naphthylthio, 2-naphthylthio, phenanthrylthio, anthrylthio etc.);
(p) heterocyclylthio (wherein the "heterocycle" of "heterocyclylthio" is as defined for the "heterocycle" of the above-mentioned "heterocyclic group optionally having substituent(s)");
(q) C₁₋₆ alkylsulfinyl (e.g., methylsulfinyl, ethylsulfinyl, propylsulfinyl, isopropylsulfinyl, butylsulfinyl, isobutylsulfinyl, sec-butylsulfinyl, tert-butylsulfinyl, pentylsulfinyl, hexylsulfinyl etc.);
(r) C₆₋₁₄ arylsulfinyl (e.g., phenylsulfinyl, 1-naphthylsulfinyl, 2-naphthylsulfinyl, phenanthrylsulfinyl, anthrylsulfinyl etc.);
(s) heterocyclesulfinyl (wherein the "heterocycle" of the "heterocyclesulfinyl" is as defined for the "heterocycle" of the above-mentioned "heterocyclic group optionally having substituent(s)");
(t) C₁₋₆ alkylsulfonyl (e.g., methylsulfonyl, ethylsulfonyl, propylsulfonyl, isopropylsulfonyl, butylsulfonyl, isobutylsulfonyl, sec-butylsulfonyl, tert-butylsulfonyl, pentylsulfonyl, hexylsulfonyl etc.);
(u) C₆₋₁₄ arylsulfonyl (e.g., phenylsulfonyl, 1-naphthylsulfonyl, 2-naphthylsulfonyl, phenanthrylsulfonyl, anthrylsulfonyl etc.);
(v) heterocyclesulfonyl (wherein the "heterocycle" of the "heterocyclesulfonyl" is as defined for the "heterocycle" of the above-mentioned "heterocyclic group optionally having substituent(s)");
(w) formyl;
(x) C₁₋₆ alkyl-carbonyl (e.g., acetyl, ethylcarbonyl, propylcarbonyl, isopropylcarbonyl, butylcarbonyl, isobutylcarbonyl, sec-butylcarbonyl, tert-butylcarbonyl, pentylcarbonyl, hexylcarbonyl etc.);
(y) C₆₋₁₄ arylcarbonyl (e.g., benzoyl, 1-naphthylcarbonyl, 2-naphthylcarbonyl, phenanthrylcarbonyl, anthrylcarbonyl etc.);
(z) heterocyclecarbonyl (wherein the "heterocycle" of the "heterocyclecarbonyl" is as defined for the "heterocycle" of the above-mentioned "heterocyclic group optionally having substituent(s)");
   (aa) heterocyclyl-C₁₋₆ alkyl (wherein the "heterocycle" of the "heterocyclyl-C₁₋₆ alkyl" is as defined for the "heterocycle" of the above-mentioned "heterocyclic group optionally having substituent(s)", and as the "C₁₋₆ alkyl", methyl, ethyl, propyl, isopropyl, butyl, isobutyl, sec-butyl, tert-butyl, pentyl, hexyl and the like can be mentioned);
   (bb) C₁₋₆ alkoxy-C₁₋₆ alkyl (wherein as the "C₁₋₆ alkoxy" of the "C₁₋₆ alkoxy-C₁₋₆ alkyl", methoxy, ethoxy, propoxy, isopropoxy, butoxy, isobutoxy, sec-butoxy, tert-butoxy, pentyloxy, hexyloxy and the like can be mentioned, and as the "C₁₋₆ alkyl", methyl, ethyl, propyl, isopropyl, butyl, isobutyl, sec-butyl, tert-butyl, pentyl, hexyl and the like can be mentioned);
   (cc) nitro;
   (dd) oxo;
   (ee) C₁₋₂ alkylenedioxy (e.g., methylenedioxy, ethylenedioxy etc.); and
   (ff) R⁵O-N=CH- (R⁵ is a hydrogen atom or a hydrocarbon group optionally having substituent(s), and the "hydrocarbon group optionally having substituent(s)" for the above-mentioned R⁵ is as defined for the "hydrocarbon group optionally having substituent(s)" for the above-mentioned substituent group B). Preferably, it is

   (a) hydrocarbon group optionally having substituent(s) selected from substituent group C, (b) heterocyclic group optionally having substituent(s) selected from substituent group C [wherein the heterocyclic group preferably optionally has 1 to 3 substituents selected from (i) hydroxy, (ii) amino, (iii) mono-C₁₋₆ alkylamino, (iv) di-C₁-₆ alkylamino, (v) C₁₋₆ alkoxy and (vi) halogen], (c) halogen, (d) hydroxy, (e) cyano, (f) carboxyl, (g) alkoxycarbonyl, (h) amino optionally having substituent(s), (i) carbamoyl optionally having substituent(s), (j) nitro, (k) C₁₋₆ alkoxy, (l) C₁₋₆ alkyl-carbonyloxy, (m) C₁₋₆ alkylthio, (n) C₁₋₆ alkylsulfinyl, (o) C₁₋₆ alkylsulfonyl, or (p) C₁₋₂ alkylenedioxy.

As the "substituent" of the "heterocyclic group optionally having substituent(s)" as the substituent for the above-mentioned ring A, those similar to the substituent of substituent group B can be mentioned.

Preferably, it is (a) hydrocarbon group optionally having substituent(s) selected from substituent group C, (b) heterocyclic group optionally having substituent(s) selected from substituent group C [wherein the heterocyclic group, preferably, optionally has 1 to 3 substituents selected from the group consisting of (i) hydroxy, (ii) amino, (iii) mono-C₁₋₆ alkylamino, (iv) di-C₁₋₆ alkylamino, (v) C₁₋₆ alkoxy and (vi) halogen], (c) halogen, (d) hydroxy, (e) cyano, (f) carboxyl, (g) alkoxycarbonyl, (h) amino optionally having substituent(s), (i) carbamoyl optionally having substituent(s), (j) nitro, (k) C₁₋₆ alkoxy, (l) C₁₋₆ alkyl-carbonyloxy, (m) C₁₋₆ alkylthio, (n) C₁₋₆ alkylsulfinyl, (o) C₁₋₆ alkylsulfonyl, or (p) C₁₋₂ alkylenedioxy.

As the "substituent" of the "monosubstituted amino" and "disubstituted amino" as the substituent for the above-mentioned ring A, 1 or 2 substituents selected from the "hydrocarbon group optionally having substituent(s)", "heterocyclic group optionally having substituent(s)" of substituent group A, as well as the below-mentioned acyl group (preferably, C₁₋₆ alkoxycarbonyl, C₁₋₆ alkylsulfinyl, C₆₋₁₄ arylsulfinyl, heterocyclesulfinyl, C₁₋₆ alkylsulfonyl, C₆₋₁₄ arylsulfonyl, heterocyclesulfonyl, formyl, C₁₋₆ alkyl-carbonyl, C₆₋₁₄ arylcarbonyl, heterocyclecarbonyl, heterocyclyl-C₁₋₆ alkyl (wherein the "heterocycle" is as defined for the "heterocycle" of the "heterocyclic group optionally having substituent(s)" of the above-mentioned substituent group A)) and the like can be mentioned.

As the "substituent" of the "cyclic amino optionally having substituent(s)" as the substituent for the above-mentioned ring A, the "substituent" of the substituent group B, the below-mentioned acyl group and the like can be mentioned.

The "amino optionally having substituent(s)", preferably, is amino optionally having 1 substituent (i.e., monosubstitution) or 2 substituents (i.e., di-substitution) selected from the group consisting of (1) C₁₋₆ alkyl optionally having 1 to 3 substituents selected from the group consisting of (a) C₁₋₆ alkyl [wherein the C₁₋₆ alkyl optionally has 1 to 3 substituents selected from the group consisting of (i) hydroxy, (ii) amino, (iii) mono-C₁₋₆ alkylamino, (iv) di-C₁₋₆ alkylamino, (v) C₁₋₆ alkoxy, (vi) halogen and (vii) heterocyclic group (as defined for "heterocyclic group" as the substituent for the above-mentioned ring A)]; (b) C₆₋₁₄ aryl [wherein the C₆₋₁₄ aryl optionally has 1 to 3 substituents selected from the group consisting of (i) hydroxy, (ii) amino, (iii) mono-C₁₋₆ alkylamino, (iv) di-C₁₋₆ alkylamino, (v) C₁₋₆ alkoxy, (vi) halogen and (vii) heterocyclic group (as defined for the "heterocyclic group" as the substituent for the above-mentioned ring A)];(c) heterocyclic group [wherein the heterocyclic group optionally has 1 to 3 substituents selected from the group consisting of (i) hydroxy, (ii) amino, (iii) mono-C₁₋₆ alkylamino, (iv) di-C₁₋₆ alkylamino, (v) C₁₋₆ alkoxy, (vi) halogen and (vii) heterocyclic group (as defined for the "heterocyclic group" as the substituent for the above-mentioned ring A)]; (d) cyano, (e) hydroxy, (f) carboxyl, (g) nitro, (h) C₁₋₆ alkoxy, (i) C₁₋₆ alkyl-carbonyloxy, (j) C₁₋₆ alkylthio, (k) C₁₋₆ alkylsulfinyl, (l) C₁₋₆ alkylsulfonyl, (m) halogen, (n) amino, (o) mono-C₁₋₆ alkylamino, (p) di-C₁₋₆ alkylamino, (q) C₁₋₆ alkoxycarbonyl, (r) carbamoyl, (s) N-mono-C₁₋₆ alkylcarbamoyl, (t) N,N-di-C₁₋₆ alkylcarbamoyl and (u) C₁₋₂ alkylenedioxy (hereinafter to be abbreviated as substituent group D), (2) C₂₋₆ alkenyl optionally having 1 to 3 substituents selected from the aforementioned substituent group D, (3) C₂₋₆ alkynyl optionally having 1 to 3 substituents selected from the aforementioned substituent group D, (4) C₃₋₈ cycloalkyl optionally having 1 to 3 substituents selected from the aforementioned substituent group D, (5) C₆₋₁₄ aryl optionally having 1 to 3 substituents selected from the aforementioned substituent group D, (6) C₇₋₂₀ aralkyl optionally having 1 to 3 substituents selected from the aforementioned substituent group D and (7) heterocyclic group optionally having 1 to 3 substituents selected from the aforementioned substituent group D, or cyclic amino optionally having substituent(s).

Preferably, the "amino optionally having substituent(s)" is optionally protected by 1 or 2 acyls (hereinafter sometimes to be abbreviated as "acylamino", which is encompassed in amino optionally having substituent(s)).

The "acyl" of the "acylamino" is acyl group obtained by removing OH group from, for example, carboxylic acid such as R⁶COOH, R⁶OCOOH and the like, for example, sulfonic acid such as R⁶SO₃H and the like, for example, sulfinic acid such as R⁶SO₂H and the like, for example, carbamic acid such as R⁶N(R⁷)COOH and the like (in the above-mentioned formulas, R⁶ and R⁷ may be the same or different and each is hydrogen atom, hydrocarbon group optionally having substituent(s) or heterocyclic group optionally having substituent(s)) and the like, specifically, R⁶CO-, R⁶OCO-, R⁶SO₂-, R⁶SO-, R⁶N(R⁷)CO- (in each formula, R⁶ and R⁷ are as defined above) and the like.

As the "hydrocarbon group optionally having substituent(s)" for the above-mentioned R⁶ and R⁷, those similar to the "hydrocarbon group optionally having substituent(s)" of substituent group B can be mentioned.

As the "heterocyclic group optionally having substituent(s)" for the above-mentioned R⁶ and R⁷, those similar to the "heterocyclic group optionally having substituent(s)" of substituent group B can be mentioned.

The "hydrocarbon group" of the "hydrocarbon group optionally having substituent(s)" and the "heterocyclic group" of the "heterocyclic group optionally having substituent(s)" for the above-mentioned R⁶ and R⁷ preferably, optionally have 1 to 3 substituents selected from the above-mentioned substituent group D.

As the "monosubstituted amino", for example, mono-C₁₋₆ alkylamino (e.g., methylamino, ethylamino, propylamino, isopropylamino, butylamino, isobutylamino, sec-butylamino, tert-butylamino, pentylamino, hexylamino etc.), acylamino and the like are preferable.

As the "disubstituted amino", for example, di-C₁₋₆ alkylamino [The "C₁₋₆ alkyl" moieties of the "di-C₁₋₆ alkylamino" may be the same or different and each is methyl, ethyl, propyl, isopropyl, butyl, isobutyl, sec-butyl, tert-butyl, pentyl, hexyl and the like. As the "di-C₁₋₆ alkylamino", for example, dimethylamino, diethylamino, dipropylamino, diisopropylamino, dibutylamino, di(sec-butyl)amino, di(tert-butyl)amino, N-ethyl-N-methylamino, N-methyl-N-propylamino, N-methyl-N-isopropylamino, N-butyl-N-methylamino, N-(sec-butyl)-N-methylamino, N-(tert-butyl)-N-methylamino, N-ethyl-N-propylamino, N-ethyl-N-isopropylamino, N-butyl-N-ethylamino, N-(sec-butyl)-N-ethylamino, N-(tert-butyl)-N-ethylamino, N-isopropyl-N-propylamino, N-butyl-N-propylamino, N-(sec-butyl)-N-propylamino, N-(tert-butyl)-N-propylamino and the like can be mentioned.], (C₁₋₆ alkyl) (C₆₋₁₄ aryl) amino [The "C₁₋₆ alkyl" moiety of the "(C₁₋₆ alkyl) (C₆₋₁₄ aryl)amino" may be the same or different and each is methyl, ethyl, propyl, isopropyl, butyl, isobutyl, sec-butyl, tert-butyl, pentyl, hexyl and the like, and as the "C₆₋₁₄ aryl" moiety, for example, phenyl, 1-naphthyl, 2-naphthyl, phenanthryl, anthryl and the like can be mentioned], (C₁₋₆ alkyl) (acyl) amino [As the "C₁₋₆ alkyl" moiety of the "(C₁₋₆ alkyl) (acyl) amino", methyl, ethyl, propyl, isopropyl, butyl, isobutyl, sec-butyl, tert-butyl, pentyl, hexyl and the like can be mentioned, and the "acyl" moiety is as defined for the above-mentioned "acyl".], (C₁₋₆ alkyl) (heterocyclyl) amino [the "C₁₋₆ alkyl" moiety of the " (C₁₋₆ alkyl)(heterocyclyl)amino" may be the same or different and each is methyl, ethyl, propyl, isopropyl, butyl, isobutyl, sec-butyl, tert-butyl, pentyl, hexyl and the like, and the "heterocyclyl" moiety is as defined for the "heterocyclic group" of the "heterocyclic group optionally having substituent(s)" of the above-mentioned substituent group A] and the like are preferable.

When two or more substituents for ring A are present, they can be bonded and form a "heterocycle optionally having substituent(s)".

In ring A, when a "heterocycle optionally having substituent(s)" is formed, the "heterocycle optionally having substituent(s)" is as defined for the "heterocyclic group optionally having substituent(s)" of the above-mentioned substituent group A.

In ring A, when a "heterocycle optionally having substituent(s)" is formed, for example, the following can be mentioned. 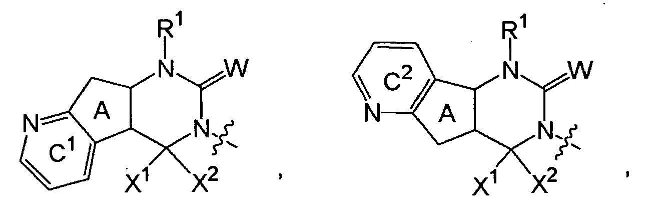 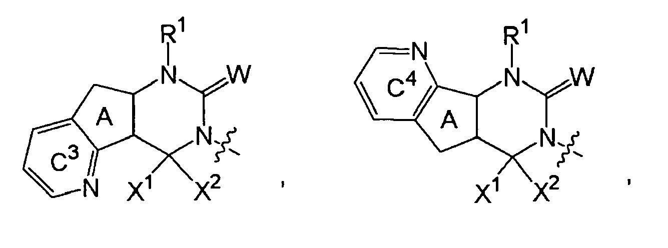 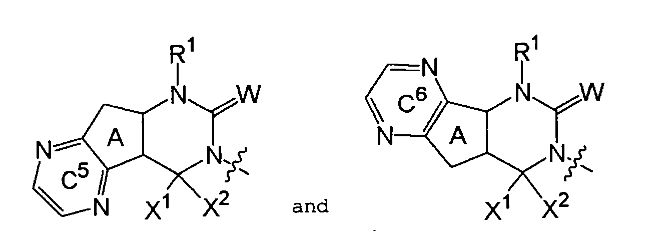 wherein ring C¹ - ring C⁶ optionally have substituent(s) at substitutable position(s) thereof, and other symbols are as defined above.

The substituent that ring C¹ - ring C⁶ optionally have is as defined for substituent group A. The number of the substituents is 1 - 3, preferably 1 or 2.

As ring A, a 5-membered aromatic heterocycle optionally substituted with 1 - 3, preferably 1 or 2, substituents selected from the group consisting of (a) hydrocarbon group optionally having substituent group B, (b) heterocyclic group optionally having substituent group B, (c) halogen, (d) hydroxy, (e) cyano, (f) carboxyl, (g) alkoxycarbonyl, (h) amino optionally having substituent(s) and (i) carbamoyl optionally having substituent(s), or optionally substituted with hydrocarbon group optionally having the above-mentioned 1 - 3, preferably 1 or 2, substituents (a) - (i) is preferable.

The "hydrocarbon group" of the "hydrocarbon group optionally having substituent(s)" of substituent group A is preferably alkyl group, cycloalkyl group, alkenyl group, cycloalkenyl group, alkynyl group, aralkyl group and aryl group other than benzene (more preferably C₁₀₋₁₄ aryl group).

As the "5-membered aromatic heterocycle" of the "5-membered aromatic heterocycle optionally having substituent(s)" represented by ring A, thiophene ring and furan ring are preferable.

R¹ is hydrogen atom or hydrocarbon group optionally having substituent(s).

The "hydrocarbon group optionally having substituent(s)" for R¹ is as defined for the "hydrocarbon group optionally having substituent(s)" of substituent group B.

As R¹, (1) hydrogen atom, or (2) C₁₋₆ alkyl optionally having 1 to 3 substituents (substituent group D) selected from the group consisting of (a) C₁₋₆ alkyl [wherein the C₁₋₆ alkyl optionally has 1 to 3 substituents selected from the group consisting of (i) hydroxy, (ii) amino, (iii) mono-C₁₋₆ alkylamino, (iv) di-C₁₋₆ alkylamino, (v) C₁₋₆ alkoxy, (vi) halogen and (vii) heterocyclic group (as defined for the "heterocyclic group" as the substituent for the above-mentioned ring A)]; (b) C₆₋₁₄ aryl [wherein the C₆₋₁₄ aryl optionally has 1 to 3 substituents selected from the group consisting of (i) hydroxy, (ii) amino, (iii) mono-C₁₋₆ alkylamino, (iv) di-C₁₋₆ alkylamino, (v) C₁₋₆ alkoxy, (vi) halogen and (vii) heterocyclic group (as defined for the "heterocyclic group" of the above-mentioned substituent group A)]; (c) heterocyclic group [wherein the heterocyclic group optionally has 1 to 3 substituents selected from the group consisting of (i) hydroxy, (ii) amino, (iii) mono-C₁₋₆ alkylamino, (iv) di-C₁₋₆ alkylamino, (v) C₁₋₆ alkoxy, (vi) halogen and (vii) heterocyclic group (as defined for the "heterocyclic group" of the above-mentioned substituent group A)];(d) cyano, (e) hydroxy, (f) carboxyl, (g) nitro, (h) C₁₋₆ alkoxy, (i) C₁₋₆ alkyl-carbonyloxy, (j) C₁₋₆ alkylthio, (k) C₁₋₆ alkylsulfinyl, (l) C₁₋₆ alkylsulfonyl, (m) halogen, (n) amino, (o) mono-C₁₋₆ alkylamino, (p) di-C₁₋₆ alkylamino, (q) C₁₋₆ alkoxycarbonyl, (r) carbamoyl, (s) N-mono-C₁₋₆ alkylcarbamoyl, (t) N,N-di-C₁₋₆ alkylcarbamoyl and (u) C₁₋₂ alkylenedioxy are preferable, particularly, hydrogen atom or C₁₋₆ alkyl, particularly hydrogen atom is preferable.

W is oxygen atom or sulfur atom, and oxygen atom is preferable.

X¹ and X² may be the same or different and each is hydrogen atom, hydrocarbon group optionally having substituent(s) or heterocyclic group optionally having substituent(s), or X¹ and X² in combination may form oxygen atom, sulfur atom or =NR² wherein R² is hydrogen atom or hydrocarbon group optionally having substituent(s).

The "hydrocarbon group optionally having substituent(s)" for X¹ or X² is as defined for the "hydrocarbon group optionally having substituent(s)" of substituent group A.

The "heterocyclic group optionally having substituent(s)" for X¹ or X² is as defined for the "heterocyclic group optionally having substituent(s)" of substituent group A.

The "hydrocarbon group optionally having substituent(s)" for R² is as defined for the "hydrocarbon group optionally having substituent(s)" of substituent group A.

The "hydrocarbon group" of the "hydrocarbon group optionally having substituent(s)" for R² includes, for example, (1) C₁₋₆ alkyl (e.g., methyl, ethyl, propyl, isopropyl, butyl, isobutyl, sec-butyl, tert-butyl, pentyl, hexyl etc.), (2) C₂₋₆ alkenyl (e.g., vinyl, allyl, 1-butenyl, 2-butenyl etc.), (3) C₂₋₆ alkynyl (e.g., ethynyl, propynyl, 1-butynyl, 2-butynyl, 5-hexynyl etc.), (4) C₃₋₈ cycloalkyl optionally fused with C₆₋₁₄ aryl ring (e.g., benzene, naphthalene, anthracene, phenanthrene etc.) (e.g., cyclopropyl, cyclobutyl, cyclopentyl, cyclohexyl, cycloheptyl, cyclooctyl, 1,2,3,4-tetrahydronaphthalen-1-yl, 2,3-dihydro-1H-inden-1-yl, 6,7,8,9-tetrahydro-5H-benzo[7]annulen-5-yl etc.), (5) C₆₋₁₄ aryl optionally fused with C₃₋₈ cycloalkane (e.g., cyclopropane, cyclobutane, cyclopentane, cyclohexane, cycloheptane, cyclooctane etc.) (e.g., phenyl, 1-naphthyl, 2-naphthyl, anthryl, phenanthryl, 5,6,7,8-tetrahydronaphthalen-1-yl etc.), (6) C₇₋₂₀ aralkyl (e.g., benzyl, phenethyl etc.) and the like.

As the "substituent" of the hydrocarbon group optionally having substituent(s) represented by R², those similar to substituent group B can be mentioned, and the number of the substituent is 1 - 5, preferably 1 - 3.

X¹ and X² in combination preferably form oxygen atom, sulfur atom or =NR² (R² is hydrogen atom or hydrocarbon group optionally having substituent(s)).

More preferably, X¹ and X² in combination preferably form oxygen atom, sulfur atom or =NR² wherein R² is (1) hydrogen atom, (2) C₁₋₆ alkyl optionally having 1 to 3 substituents selected from the group consisting of (a) C₁₋₆ alkyl [wherein the C₁₋₆ alkyl optionally has 1 to 3 substituents selected from the group consisting of (i) hydroxy, (ii) amino, (iii) mono-C₁₋₆ alkylamino, (iv) di-C₁₋₆ alkylamino, (v) C₁₋₆ alkoxy, (vi) halogen and (vii) heterocyclic group (as defined for the "heterocyclic group" as the substituent for the above-mentioned ring A)]; (b) C₆₋₁₄ aryl [wherein the C₆₋₁₄ aryl optionally has 1 to 3 substituents selected from the group consisting of (i) hydroxy, (ii) amino, (iii) mono-C₁₋₆ alkylamino, (iv) di-C₁₋₆ alkylamino, (v) C₁₋₆ alkoxy, (vi) halogen and (vii) heterocyclic group (as defined for the "heterocyclic group" of substituent group A)]; (c) heterocyclic group [wherein the heterocyclic group optionally has 1 to 3 substituents selected from the group consisting of (i) hydroxy, (ii) amino, (iii) mono-C₁₋₆ alkylamino, (iv) di-C₁₋₆ alkylamino, (v) C₁₋₆ alkoxy, (vi) halogen and (vii) heterocyclic group (as defined for the "heterocyclic group" of substituent group A)]; (d) cyano, (e) hydroxy, (f) carboxyl, (g) nitro, (h) C₁₋₆ alkoxy, (i) C₁₋₆ alkyl-carbonyloxy, (j) C₁₋₆ alkylthio, (k) C₁₋₆ alkylsulfinyl, (l) C₁₋₆ alkylsulfonyl, (m) halogen, (n) amino, (o) mono-C₁₋₆ alkylamino, (p) di-C₁₋₆ alkylamino, (q) C₁₋₆ alkoxycarbonyl, (r) carbamoyl, (s) N-mono-C₁₋₆ alkylcarbamoyl, (t) N,N-di-C₁₋₆ alkylcarbamoyl and (u) C₁₋₂ alkylenedioxy (substituent group D), (3) C₂₋₆ alkenyl optionally having 1 to 3 substituents selected from the aforementioned substituent group D, (4) C₂₋₆ alkynyl optionally having 1 to 3 substituents selected from the aforementioned substituent group D, (5) C₃₋₈ cycloalkyl optionally having 1 to 3 substituents selected from the aforementioned substituent group D, (6) C₆₋₁₄ aryl optionally having 1 to 3 substituents selected from the aforementioned substituent group D or (7) C₇₋₂₀ aralkyl optionally having 1 to 3 substituents selected from the aforementioned substituent group D. Of these, oxygen atom or =NH is preferable.

Y is a bond, C₁₋₆ alkylene optionally having substituent(s), C₂₋₆ alkenylene optionally having substituent (s) or C₂₋₆ alkynylene optionally having substituent(s).

As the "C₁₋₆ alkylene" of the "C₁₋₆ alkylene optionally having substituent(s)" for Y, for example, -CH₂-, -CH₂CH₂-,-CH₂CH₂CH₂-, -CH₂CH₂CH₂CH₂-, -CH₂CH₂CH₂CH₂CH₂-, -CH₂CH₂CH₂CH₂CH₂CH₂- and the like can be mentioned.

As the "substituent" of the "C₁₋₆ alkylene optionally having substituent(s)" for Y, the aforementioned substituent of substituent group A, preferably the substituent of substituent group B, can be used. The position thereof is not particularly limited as long as it is a substitutable position, and the number of the substituent is 1 - 6, preferably 1 - 3.

As the "C₂₋₆ alkenylene" of the "C₂₋₆ alkenylene optionally having substituent(s)" for Y, for example, -CH=CH-, -CH=CHCH₂-,-CH₂CH=CH-, -CH=CHCH₂CH₂-, -CH₂CH=CHCH₂-, -CH₂CH₂CH=CH-, -CH=CH-CH=CH-, -CH=CHCH₂CH₂CH₂-, -CH₂CH=CHCH₂CH₂-, -CH₂CH₂CH=CHCH₂-,-CH₂CH₂CH₂CH=CH-, -CH=CH-CH=CHCH₂-, -CH=CHCH₂CH=CH-, -CH₂CH=CH-CH=CH-, -CH=CHCH₂CH₂CH₂CH₂-, -CH₂CH=CHCH₂CH₂CH₂-, -CH₂CH₂CH=CHCH₂CH₂-, -CH₂CH₂CH₂CH=CHCH₂-, -CH₂CH₂CH₂CH₂CH=CH-, -CH=CH-CH=CHCH₂CH₂-,-CH=CHCH₂CH=CHCH₂-, -CH=CHCH₂CH₂CH=CH-, -CH₂CH=CH-CH=CHCH₂-,-CH₂CH=CHCH₂CH=CH-, -CH₂CH₂CH=CH-CH=CH-, -CH=CH-CH=CH-CH=CH- and the like can be mentioned. The E form and Z form of the above-mentioned "C₂₋₆ alkenylene" are not particularly limited.

The "substituent" of the "C₂₋₆ alkenylene optionally having substituent(s)" for Y is the aforementioned substituent of substituent group A, preferably substituent of substituent group B. The position thereof is not particularly limited as long as it is a substitutable position, and the number of the substituent is 1 - 6, preferably 1 - 3.

As the "C₂₋₆ alkynylene" of the "C₂₋₆ alkynylene optionally having substituent(s)" for Y, for example, -C≡C-, -C≡CCH₂-,-CH₂C≡C-, -C≡CCH₂CH₂-, -CH₂C≡CCH₂-, -CH₂CH₂C≡C-, -C≡C-C≡C-, -C≡ CCH₂CH₂CH₂-, -CH₂C≡CCH₂CH₂-, -CH₂CH₂C≡CCH₂-, -CH₂CH₂CH₂C≡C-, -C≡C-C≡CCH₂-, -C≡CCH₂C≡C-, -CH₂C≡C-C≡C-, -C≡CCH₂CH₂CH₂CH₂-, -CH2C≡ CCH₂CH₂CH₂-, -CH₂CH₂C≡CCH₂CH₂-, -CH₂CH₂CH₂C≡CCH₂-, -CH₂CH₂CH₂CH₂C≡C-, -C≡C-C≡CCH₂CH₂-, -C≡CCH₂C≡CCH₂-, -C≡CCH₂CH₂C≡C-, -CH₂C≡C-C≡ CCH₂-, -CH₂C≡CCH₂C≡C-, -CH₂CH₂C≡C-C≡C-, -C≡C-C≡C-C≡C- and the like can be mentioned.

The "substituent" of the "C₂₋₆ alkynylene optionally having substituent(s)" for Y is the aforementioned substituent of substituent group A, preferably substituent of substituent group B. The position thereof is not particularly limited as long as it is a substitutable position, and the number of the substituent is 1 - 6, preferably 1 - 3.

Y is preferably a bond.

Z is a group represented by formula: -SOₙR³ wherein n is 0, 1 or 2, preferably 2, R³ is amino optionally having substituent(s), hydrocarbon group optionally having substituent(s) or heterocyclic group optionally having substituent(s) (provided that R³ is not a methyl group), or a group represented by formula: -COR⁴ wherein R⁴ is amino optionally having substituent(s), hydrocarbon group optionally having substituent(s) or heterocyclic group optionally having substituent(s).

The "amino optionally having substituent(s)" for R³ or R⁴ is as defined for the "amino optionally having substituent(s)" defined for the above-mentioned substituent group A.

The "amino optionally having substituent(s)" for R³ or R⁴ is preferably "disubstituted amino" or "cyclic amino optionally having substituent(s)".

The "disubstituted amino" is more preferably di-C₁₋₆ alkylamino, (C₁₋₆ alkyl) (C₆₋₁₄ aryl) amino, (C₁₋₆ alkyl)(heterocyclyl)amino or the like.

As the "cyclic amino" of "cyclic amino optionally having substituent(s)", 5- to 16-membered cyclic amino such as 1-pyrrolidinyl, 1-pyrrolinyl, 1-pyrrolyl, azepan-1-yl, 1-imidazolidinyl, 1-imidazolinyl, 1-imidazolyl, 2-pyrazolidinyl, 2-pyrazolinyl, 1-pyrazolyl, 1-indolinyl, 1-indolyl, 2-isoindolinyl, 2-isoindolyl, 2,3-dihydro-1H-indol-1-yl, 1-piperidyl, 3,4-dihydro-1,5-naphthyridine-1(2H)-yl, 3,4-dihydroquinolin-1(2H)-yl, 3,4-dihydroisoquinolin-2(1H)-yl, 1-piperazinyl, octahydroquinolin-1(2H)-yl, octahydroisoquinolin-2(1H)-yl, 3,4-dihydroquinoxalin-1(2H)-yl, 1H-azepin-1-yl, 2,3,4,5,6,7-hexahydro-1H-azepin-1-yl, 2,3,4,5-tetrahydro-1H-1-benzoazepin-1-yl, 1,3,4,5-tetrahydro-2H-2-benzoazepin-2-yl, 3,4,5,6-tetrahydro-1-benzoazocin-1(2H)-yl, 2,3-dihydro-4,1-benzothiazepin-1(5H)-yl, 3,4-dihydro-1,5-benzothiazepin-5(2H)-yl, 2,3-dihydro-4,1-benzothiazepin-1(5H)-yl, 2,3-dihydro-4,1-benzooxazepin-1(5H)-yl, 1,2,4,5-tetrahydro-3H-3-benzoazepin-3-yl, 2,3,4,5-tetrahydro-1H-1,5-benzodiazepin-1-yl, 2,3,4,5-tetrahydro-1H-1,4-benzodiazepin-1-yl, 2,3-dihydro-4H-1,4-benzooxazin-4-yl, 3,4-dihydro-2H-1,4-benzooxazin-1-yl, 3,4-dihydro-1,5-benzooxazepin-5(2H)-yl, 5,6,7,8-tetrahydro-4H-thieno[3,2-b]azepin-4-yl, 4,5,6,7-tetrahydro-8H-thieno[2,3-b]azepin-8-yl, 4-morpholinyl, 4-thiomorpholinyl, 2,3-dihydro-4H-pyrido[3,2-b][1,4]oxazin-4-yl, 2,3-dihydro-4H-1,4-benzothiazin-4-yl, 5,6,7,8-tetrahydropyrazolo[4,3-b]azepin-4(1H)-yl and the like is preferable.

As the "substituent" of the "cyclic amino optionally having substituent(s)" for R³ or R⁴, the "substituent" of the above-mentioned substituent group A or acyl group, preferably the "substituent" of the substituent group B or acyl group. The position thereof is not particularly limited as long as it is a substitutable position, and the number of the substituent is 1 - 6, preferably 1 - 3.

The substituent is preferably (a) hydrocarbon group optionally having substituent(s) selected from substituent group B, (b) heterocyclic group optionally having substituent(s) selected from substituent group B [wherein the heterocyclic group, preferably, optionally has 1 to 3 substituents selected from the group consisting of (i) hydroxy, (ii) amino, (iii) mono-C₁₋₆ alkylamino, (iv) di-C₁₋₆ alkylamino, (v) C₁₋₆ alkoxy and (vi) halogen], (c) halogen, (d) hydroxy, (e) cyano, (f) carboxyl, (g) alkoxycarbonyl, (h) amino optionally having substituent(s), (i) carbamoyl optionally having substituent(s), (j) nitro, (k) C₁₋₆ alkoxy, (l) C₁₋₆ alkyl-carbonyloxy, (m) C₁₋₆ alkylthio, (n) C₁₋₆ alkylsulfinyl, (o) C₁₋₆ alkylsulfonyl or (p) C₁₋₂ alkylenedioxy.

The "hydrocarbon group optionally having substituent(s)" for R³ or R⁴ is as defined for the "hydrocarbon group optionally having substituent(s)" of the above-mentioned substituent group A.

However, the "hydrocarbon group" of the "hydrocarbon group optionally having substituent(s)" for R³ does not contain a methyl group.

For R³, a C₂₋₂₀ hydrocarbon group optionally having substituent(s) is preferable.

The "substituent" of the "hydrocarbon group optionally having substituent(s)" for R³ or R⁴ is the "substituent" of the above-mentioned substituent group B. The position thereof is not particularly limited as long as it is a substitutable position, and the number of the substituent is 1 - 6, preferably 1 - 3.

The "heterocyclic group optionally having substituent(s)" for R³ or R⁴ is as defined for the "heterocyclic group optionally having substituent(s)" as defined for the above-mentioned substituent group A.

The "substituent" of the "heterocyclic group optionally having substituent(s)" for R³ or R⁴ is as defined for the substituent of the above-mentioned substituent group B. The position thereof is not particularly limited as long as it is a substitutable position, and the number of the substituent is 1 - 6, preferably 1 - 3.

Ring B is an "aromatic ring optionally further having substituent(s)".

As the "aromatic ring" for the "aromatic ring optionally having substituent(s)", "C₆₋₁₄ aryl ring", "aromatic heterocycle" and the like can be mentioned.

The "'C₆₋₁₄ aryl ring" is, for example, benzene ring, naphthalene ring, phenanthrene ring, anthracene ring and the like, preferably benzene ring.

As the "aromatic heterocycle", for example, the above-mentioned 5- to 7-membered monocyclic aromatic heterocycle containing, as ring-constituting atom besides carbon atom, 1 to 4 hetero atoms selected from the group consisting of an oxygen atom, a sulfur atom and a nitrogen atom, or 8- to 16-membered bicyclic or tricyclic fused aromatic heterocycle containing, besides carbon atoms, 1 to 12 hetero atoms selected from the group consisting of an oxygen atom, a sulfur atom and a nitrogen atom (e.g., bicyclic or tricyclic fused ring wherein the 5- to 7-membered monocyclic aromatic heterocycle and a benzene ring are fused, or a bicyclic or tricyclic fused ring wherein the 5- to 7-membered monocyclic aromatic heterocycles are fused) and the like can be used, specifically, 5-membered aromatic heterocyclic group (e.g., thiophene ring, furan ring, pyrrole ring, oxazole ring, thiazole ring, pyrazole ring, imidazole ring, isoxazole ring, isothiazole ring, 1,2,4-oxadiazole ring, 1,3,4-oxadiazole ring, 1,2,4-thiadiazole ring, 1,3,4-thiadiazole ring, 1,2,3-triazole ring, 1,2,4-triazole ring, 1H-tetrazole ring, 2H-tetrazole ring etc.), 6-membered aromatic heterocycle (e.g., pyridine ring, pyrimidine ring, triazine ring, pyridazine ring, pyrazine ring etc.), bicyclic or tricyclic fused aromatic heterocycle (e.g., benzofuran ring, benzothiophene ring, benzoxazole ring, benzothiazole ring, benzothiadiazole ring, benzimidazole ring, quinoline ring, isoquinoline ring, quinazoline ring, quinoxaline ring, indole ring, 1H-indazole ring, 1H-pyrrolopyridine ring, 1H-imidazopyridine ring, 1H-imidazopyrazine ring, 1H-pyrrolo[2,3-b]pyrazine ring, tetrazolo[1,5-b]pyridazine ring, triazolo[4,5-b]pyridazine ring, cinnoline ring, phthalazine ring, indolizine ring, quinolizine ring, 1,8-naphthyridine ring, purine ring, pteridine ring, dibenzofuran ring, carbazole ring, acridine ring, phenanthridine ring, chromane ring, benzoxazine ring, phenazine ring, phenothiazine ring, phenoxazine ring etc.) and the like can be used.

The "aromatic heterocycle" is preferably 5- or 6-membered aromatic heterocycle, more preferably thiophene ring, furan ring, pyrazole ring, oxazole ring, thiazole ring, pyridine ring, pyrimidine ring and the like.

The "substituent" of the "aromatic ring optionally further having substituent(s)" is as defined for the above-mentioned substituent group A. The position thereof is not particularly limited as long as it is a substitutable position, and the number of the substituent is 1 - 6, preferably 1 - 3.

In compound (I), when ring B is a benzene ring represented by formula: 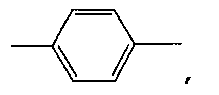 any of the following conditions (1) - (3) is satisfied.
(1) The amino optionally having substituent(s) for R³ is disubstituted amino or cyclic amino optionally having substituent(s).
(2) The amino optionally having substituent(s) for R⁴ is disubstituted amino (except dimethylamino) or cyclic amino optionally having substituent(s) (except monocyclic piperazin-1-yl optionally having substituent(s)).
(3) The hydrocarbon group optionally having substituent(s) for R⁴ does not contain a methyl group.

Ring B is preferably a ring represented by formula: 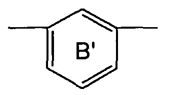 wherein ring B' is a benzene ring optionally further having substituent(s) or aromatic heterocycle optionally further having substituent(s).

The "substituent" of the "benzene ring optionally further having substituent(s)" for ring B' is as defined for the above-mentioned substituent group A. The position thereof is not particularly limited as long as it is a substitutable position, and the number of the substituent is 1 - 4, preferably 1 - 3.

A preferable mode of compound (I) is compound (I-a). Compound (I-a): a compound represented by 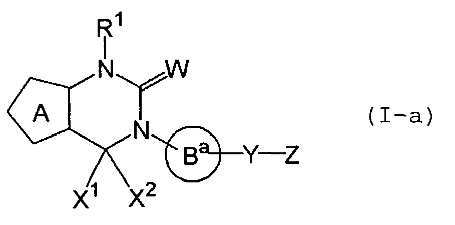 wherein
ring A is a 5-membered aromatic heterocycle optionally having substituent(s),
R¹ is a hydrogen atom or a hydrocarbon group optionally having substituent(s),
W is an oxygen atom or a sulfur atom,
X¹ and X² may be the same or different and each is a hydrogen atom, a hydrocarbon group optionally having substituent(s) or a heterocyclic group optionally having substituent(s), or X¹ and X² in combination optionally form an oxygen atom, a sulfur atom or =NR² wherein R² is a hydrogen atom or a hydrocarbon group optionally having substituent(s),
ring B^{a} is a ring represented by formula: 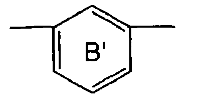 wherein ring B' is a benzene ring optionally further having substituent(s) or an aromatic heterocycle optionally further having substituent(s),
Y is a bond, a C₁₋₆ alkylene optionally having substituent(s), a C₂₋₆ alkenylene optionally having substituent (s) or a C₂₋₆ alkynylene optionally having substituent(s), and
Z a group represented by formula: -SOₙR^{3a} wherein n is 0, 1 or 2, R^{3a} is an amino optionally having substituent(s), a C₂₋₂₀ hydrocarbon group optionally having substituent(s) or a heterocyclic group optionally having substituent(s), or a group represented by formula: -COR⁴ wherein R⁴ is an amino optionally having substituent(s), a hydrocarbon group optionally having substituent(s) or a heterocyclic group optionally having substituent(s).

The "amino optionally having substituent(s)", "C₂₋₂₀ hydrocarbon group optionally having substituent(s)" and "heterocyclic group optionally having substituent(s)" for R^{3a} is as defined for each "amino optionally having substituent(s)", "C₂₋₂₀ hydrocarbon group optionally having substituent(s)" or "heterocyclic group optionally having substituent(s)" as defined for R³.

In a preferable mode of compound (I),
ring B is a benzene ring represented by the formula: 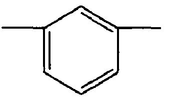 or an aromatic heterocycle, each of which optionally further has 1 to 3 substituents selected from the group consisting of (a) a C₁₋₆ alkyl [wherein the C₁₋₆ alkyl optionally has 1 to 3 substituents selected from the group consisting of (i) a hydroxy, (ii) an amino, (iii) a mono-C₁₋₆ alkylamino, (iv) a di-C₁₋₆ alkylamino, (v) a C₁₋₆ alkoxy, (vi) a halogen and (vii) a heterocyclic group (as defined for the "heterocyclic group" of the above-mentioned substituent group A)]; (b) a C₆₋₁₄ aryl [wherein the C₆₋₁₄ aryl optionally has 1 to 3 substituents selected from the group consisting of (i) a hydroxy, (ii) an amino, (iii) a mono-C₁₋₆ alkylamino, (iv) a di-C₁₋₆ alkylamino, (v) a C₁₋₆ alkoxy, (vi) a halogen and (vii) a heterocyclic group (as defined for the "heterocyclic group" of the above-mentioned substituent group A)]; (c) a heterocyclic group [wherein the heterocyclic group optionally has 1 to 3 substituents selected from the group consisting of (i) a hydroxy, (ii) an amino, (iii) a mono-C₁₋₆ alkylamino, (iv) a di-C₁₋₆ alkylamino, (v) a C₁₋₆ alkoxy, (vi) a halogen and (vii) a heterocyclic group (as defined for the "heterocyclic group" as the substituent for the above-mentioned ring A)]; (d) a cyano, (e) a hydroxy, (f) a carboxyl, (g) a nitro, (h) a C₁₋₆ alkoxy, (i) a C₁₋₆ alkyl-carbonyloxy, (j) a C₁₋₆ alkylthio, (k) a C₁₋₆ alkylsulfinyl, (l) a C₁₋₆ alkylsulfonyl, (m) a halogen, (n) an amino, (o) a mono-C₁₋₆ alkylamino, (p) a di-C₁₋₆ alkylamino, (q) a C₁₋₆ alkoxycarbonyl, (r) a carbamoyl, (s) an N-mono-C₁₋₆ alkylcarbamoyl, (t) an N,N-di-C₁₋₆ alkylcarbamoyl and (u) a C₁₋₂ alkylenedioxy (substituent group D), and
R³ and R⁴ are each an amino optionally having 1 or 2 substituents selected from the group consisting of (a) a C₁₋₆ alkyl optionally having 1 to 3 substituents selected from the aforementioned substituent group D, (b) a C₂₋₆ alkenyl optionally having 1 to 3 substituents selected from the aforementioned substituent group D, (c) a C₂₋₆ alkynyl optionally having 1 to 3 substituents selected from the aforementioned substituent group D, (d) a C₃₋₈ cycloalkyl optionally having 1 to 3 substituents selected from the aforementioned substituent group D, (e) a C₆₋₁₄ aryl optionally having 1 to 3 substituents selected from the aforementioned substituent group D, (f) a C₇₋₂₀ aralkyl optionally having 1 to 3 substituents selected from the aforementioned substituent group D and (g) a heterocyclic group optionally having 1 to 3 substituents selected from the aforementioned substituent group D or a cyclic amino optionally having substituent(s).

In another preferable mode of compound (I),
ring B is a benzene ring represented by the formula: 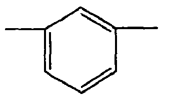 optionally further having 1 to 3 substituents selected from the group consisting of a halogen, a C₁₋₆ alkyl and a cyano, and
R³ and R⁴ are each (1) an amino optionally having 1 or 2 substituents selected from the group consisting of (a) a C₁₋₆ alkyl optionally having a cyano, (b) a C₆₋₁₄ aryl optionally having a halogen and (c) a pyridyl optionally having a halogen, (2) a 3,4-dihydroquinolin-1(2H)-yl optionally having a C₁₋₆ alkyl, a halogen or a C₁₋₆ alkoxy, (3) a 2,3-dihydro-1H-indol-1-yl optionally having a C₁₋₆ alkyl, (4) a 2,3,4,5-tetrahydro-1H-1-benzoazepin-1-yl optionally having a C₁₋₆ alkyl or (5) a 3,4-dihydro-2H-1,4-benzoxazin-1-yl.

In a preferable mode of compound (I), ring B is a thiophene ring or a furan ring optionally further having 1 or 2 substituents selected from the group consisting of a halogen, a C₁₋₆ alkyl and a cyano,

R³ and R⁴ are each (l) an amino optionally having 1 or 2 substituents selected from the group consisting of (a) a C₁₋₆ alkyl optionally having a cyano, (b) a C₆₋₁₄ aryl optionally having a halogen and (c) a pyridyl optionally having a halogen, (2) a 3,4-dihydroquinolin-1(2H)-yl optionally having a C₁₋₆ alkyl, a halogen or a C₁₋₆ alkoxy, (3) a 2,3-dihydro-1H-indol-1-yl optionally having a C₁₋₆ alkyl, (4) a 2,3,4,5-tetrahydro-1H-1-benzoazepin-1-yl optionally having a C₁₋₆ alkyl or (5) a 3,4-dihydro-2H-1,4-benzoxazin-1-yl.

As compound (I), a compound wherein R¹ is a hydrogen atom, Y is a bond, X¹ and X² in combination form an oxygen atom, a sulfur atom or =NR² wherein R² is a hydrogen atom or a hydrocarbon group optionally having substituent(s) is preferable. Particularly, a compound wherein ring A is a 5-membered aromatic heterocycle containing, as ring-constituting atom besides carbon atom, 1 to 4 hetero atoms selected from the group consisting of an oxygen atom, a sulfur atom and a nitrogen atom and optionally having substituent(s) selected from the group consisting of (i) - (xi)
(i) a C₁₋₆ alkyl group optionally having substituent(s) selected from the group consisting of (a) - (d)
   (a) a hydroxy,
   (b) a carboxyl,
   (c) a C₁₋₆ alkoxycarbonyl, and
   (d) an amino optionally mono- or di-substituted with a C₁₋₆ alkyl optionally having substituent(s) selected from the group consisting of a C₁₋₆ alkoxy and a 5- to 16-membered heterocyclic group containing, as ring-constituting atom besides carbon atom, 1 to 4 hetero atoms selected from the group consisting of an oxygen atom, a sulfur atom and a nitrogen atom,
(ii) a C₂₋₆ alkenyl group optionally having a C₁₋₆ alkoxycarbonyl,
(iii) a C₆₋₁₄ aryl group optionally having a halogen atom (preferably, phenyl group),
(iv) 5- to 16-membered heterocyclic group containing, as ring-constituting atom besides carbon atom, 1 to 4 hetero atoms selected from the group consisting of an oxygen atom, a sulfur atom and a nitrogen atom (heterocyclic group includes a 5- to 16-membered aromatic heterocyclic group containing, as ring-constituting atom besides carbon atom, 1 to 4 hetero atoms selected from the group consisting of an oxygen atom, a sulfur atom and a nitrogen atom such as oxadiazole, triazole, tetrazole, oxazole and the like, 5- to 16-membered non-aromatic heterocyclic group containing, as ring-constituting atom besides carbon atom and nitrogen atom, 1 to 4 hetero atoms selected from the group consisting of an oxygen atom and a sulfur atom such as morpholinyl and the like and the like, preferably a 5- or 6-membered heterocyclic group), which optionally has a C₁₋₆ alkyl,
(v) a cyano,
(vi) a carboxyl,
(vii) a formyl,
(viii) a C₁₋₆ alkoxycarbonyl,
(ix) HO-N=CH-,
(x) a carbamoyl optionally mono- or di-substituted with substituent(s) selected from the group consisting of (a) - (b):
   (a) a C₁₋₆ alkyl group optionally having substituent(s) selected from the group consisting of a hydroxy, a C₁₋₆ alkoxycarbonyl, a carboxyl and a 5- to 16-membered, preferably 5- or 6-membered, heterocyclic group containing, as ring-constituting atom besides carbon atom, 1 to 4 hetero atoms selected from the group consisting of an oxygen atom, a sulfur atom and a nitrogen atom, and
   (b) a 5- to 16-membered, preferably, 5- or 6-membered, heterocyclic group containing, as ring-constituting atom besides carbon atom, 1 to 4 hetero atoms selected from the group consisting of an oxygen atom, a sulfur atom and a nitrogen atom, and
(xi) a 5- to 16-membered, preferably 5- or 6-membered, heterocycle containing, as ring-constituting atom besides carbon atom, 1 to 4 hetero atoms selected from the group consisting of an oxygen atom, a sulfur atom and a nitrogen atom, which is formed by two substituents bonded to each other and optionally has a C₁₋₆ alkoxy group,

R¹ is a hydrogen atom,
W is an oxygen atom,
X¹ and X² are hydrogen atoms, or X¹ and X² form an oxygen atom in combination,
ring B is a C₆₋₁₄ aryl ring optionally further having a halogen atom, or a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom or a 8- to 16-membered bicyclic or tricyclic fused aromatic heterocycle containing, as ring-constituting atom besides carbon atom, 1 to 12 hetero atoms selected from the group consisting of an oxygen atom, a sulfur atom and a nitrogen atom, each of which optionally further having a halogen atom,
Y is a bond,
Z is

(i) a group represented by formula: -SOₙR³
   wherein n is 2, and R³ is a 5- to 16-membered cyclic amino, or
(ii) a group represented by formula: -CONR⁸(R⁹)
   wherein R⁸ is a hydrogen atom, a C₁₋₆ alkyl group or a C₃₋₈ cycloalkyl group, and R⁹ is a hydrogen atom or a C₆₋₁₄ aryl group optionally having substituent(s) selected from the group consisting of a halogen atom and a cyano, and
   when ring B is a ring represented by formula: 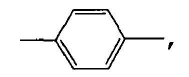
   R⁸ and R⁹ are not hydrogen atoms, is preferably used.

As compound (Ia), for example, a compound wherein ring A is a 5-membered aromatic heterocycle containing, as ring-constituting atom besides carbon atom, 1 to 4 hetero atoms selected from the group consisting of an oxygen atom, a sulfur atom and a nitrogen atom and optionally having substituent(s) selected from the group consisting of (i) - (xi),
(i) a C₁₋₆ alkyl group optionally having substituent(s) selected from the group consisting of (a) - (d),
   (a) a hydroxy,
   (b) a carboxyl,
   (c) a C₁₋₆ alkoxycarbonyl, and
   (d) an amino optionally mono- or di-substituted with a C₁₋₆ alkyl optionally having substituent(s) the group consisting of a C₁₋₆ alkoxy and a 5- to 16-membered heterocyclic group containing, as ring-constituting atom besides carbon atom, 1 to 4 hetero atoms selected from the group consisting of an oxygen atom, a sulfur atom and a nitrogen atom,
(ii) a C₂₋₆ alkenyl group optionally having a C₁₋₆ alkoxycarbonyl,
(iii) a C₆₋₁₄ aryl group optionally having a halogen atom,
(iv) a 5- to 16-membered heterocyclic group containing, as ring-constituting atom besides carbon atom, 1 to 4 hetero atoms selected from the group consisting of an oxygen atom, a sulfur atom and a nitrogen atom (the heterocyclic group includes a 5-to 16-membered aromatic heterocyclic group containing, as ring-constituting atom besides carbon atom, 1 to 4 hetero atoms selected from the group consisting of an oxygen atom, a sulfur atom and a nitrogen atom such as oxadiazole, triazole, tetrazole, oxazole and the like, a 5- to 16-membered non-aromatic heterocyclic group containing, as ring-constituting atom besides carbon atom and nitrogen atom, 1 to 4 hetero atoms selected from the group consisting of an oxygen atom and a sulfur atom such as morpholinyl and the like, and the like, preferably a 5- or 6-membered heterocyclic group), which may have a C₁₋₆ alkyl,
(v) a cyano,
(vi) a carboxyl,
(vii) a formyl,
(viii) a C₁₋₆ alkoxycarbonyl,
(ix) HO-N=CH-,
(x) a carbamoyl optionally mono- or di-substituted with substituent(s) selected from the group consisting of (a) - (b),
   (a) a C₁₋₆ alkyl group optionally having substituent(s) selected from the group consisting of a hydroxy, a C₁₋₆ alkoxycarbonyl, carboxyl and a 5- to 16-membered, preferably 5- or 6-membered, heterocyclic group containing, as ring-constituting atom besides carbon atom, 1 to 4 hetero atoms selected from the group consisting of an oxygen atom, a sulfur atom and a nitrogen atom, and
   (b) a 5- to 16-membered, preferably 5- or 6-membered, heterocyclic group containing, as ring-constituting atom besides carbon atom, 1 to 4 hetero atoms selected from the group consisting of an oxygen atom, a sulfur atom and a nitrogen atom, and
(xi) a 5- to 16-membered (preferably 5- or 6-membered) heterocycle containing, as ring-constituting atom besides carbon atom, 1 to 4 hetero atoms selected from the group consisting of an oxygen atom, a sulfur atom and a nitrogen atom, which is formed by two substituents bonded to each other and optionally has a C₁₋₆ alkoxy group,

R¹ is a hydrogen atom,
W is an oxygen atom,
X¹ and X² are hydrogen atoms, or X¹ and X² form an oxygen atom in combination,
ring B^{a} is a ring represented by the formula: 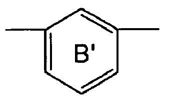 wherein ring B' is a benzene ring optionally further having a halogen atom, or a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom or a 8- to 16-membered bicyclic or tricyclic fused aromatic heterocycle containing, as ring-constituting atom besides carbon atom, 1 to 12 hetero atoms selected from the group consisting of an oxygen atom, a sulfur atom and a nitrogen atom, each of which optionally has a halogen atom,
Y is a bond, and
Z is

(i) a group represented by formula: -SOₙR³
   wherein n is 2 and R³ is a 5- to 16-membered cyclic amino, or
(ii) a group represented by formula: -CONR⁸(R⁹)
   wherein R⁸ is a hydrogen atom, a C₁₋₆ alkyl group or a C₃₋₈ cycloalkyl group, and R⁹ is a C₆₋₁₄ aryl group optionally having substituent(s) selected from the group consisting of a hydrogen atom, a halogen atom and a cyano, is preferable.

As compound (I), the following compounds are particularly preferable.
3-[3-(3,4-dihydroquinolin-1(2H)-ylsulfonyl)phenyl]thieno[3,2-d]pyrimidin-2,4(1H,3H)-dione (Example 1);
3-[2-chloro-5-(5,6,7,8-tetrahydro-4H-thieno[3,2-b]azepin-4-ylsulfonyl)-3-thienyl]-2,4-dioxo-1,2,3,4-tetrahydrothieno[3,4-d]pyrimidine-5-carboxylic acid (Example 6);
N-({3-[3-(3,4-dihydroquinolin-1(2H)-ylsulfonyl)phenyl]-2,4-dioxo-1,2,3,4-tetrahydrothieno[3,4-d]pyrimidin-5-yl}carbonyl)glycine tert-butyl ester (Example 7 (1));
3-[3-(3,4-dihydroquinolin-1(2H)-ylsulfonyl)phenyl]-5-(5-methyl-1,3,4-oxadiazol-2-yl)thieno[3,4-d]pyrimidin-2,4(1H,3H)-dione (Example 12);
3-[2-chloro-5-(3,4-dihydroquinolin-1(2H)-ylsulfonyl)phenyl]-N-methyl-2,4-dioxo-1,2,3,4-tetrahydrothieno[3,4-d]pyrimidine-5-carboxamide (Example 16);
3-[3-(3,4-dihydroquinolin-1(2H)-ylsulfonyl)phenyl]-5-(1-hydroxyethyl)thieno[3,4-d]pyrimidin-2,4(1H,3H)-dione (Example 24);
methyl 3-[4-(3,4-dihydroquinolin-1(2H)-ylsulfonyl)-2-thienyl]-2,4-dioxo-1,2,3,4-tetrahydrothieno[3,4-d]pyrimidine-5-carboxylate (Example 30);
3-[4-(3,4-dihydroquinolin-1(2H)-ylsulfonyl)-2-thienyl]-5-(hydroxymethyl)thieno[3,4-d]pyrimidin-2,4(1H,3H)-dione (Example 31) ;
3-(4-{[ethyl(phenyl)amino]carbonyl}-2-thienyl)-2,4-dioxo-1,2,3,4-tetrahydrothieno[3,4-d]pyrimidine-5-carboxylic acid (Example 33-2); and
3-[3-(3,4-dihydroquinolin-1(2H)-ylsulfonyl)phenyl]-2,4-dioxo-1,2,3,4-tetrahydrothieno[2,3-d]pyrimidine-5-carboxylic acid (Example 34(3)).

As the salt of compound (I), a physiologically acceptable acid addition salt is preferable. As such salt, for example, salts with inorganic acid (e.g., hydrochloric acid, hydrobromic acid, nitric acid, sulfuric acid, phosphoric acid etc.), or salts with organic acid (e.g., formic acid, acetic acid, trifluoroacetic acid (TFA), fumaric acid, oxalic acid, tartaric acid, maleic acid, citric acid, succinic acid, malic acid, methanesulfonic acid, benzenesulfonic acid, p-toluenesulfonic acid etc.), and the like are used. When compound (I) has an acidic group, the compound may form a physiologically acceptable salt of inorganic base (e.g., alkali metal or alkaline earth metal such as sodium, potassium, calcium, magnesium and the like, ammonia etc.) or organic base (e.g., trimethylamine, triethylamine, pyridine, picoline, ethanolamine, diethanolamine, triethanolamine, dicyclohexylamine, N,N'-dibenzylethylenediamine etc.).

Compound (I) can be produced by a method known *per se,* for example, the following production methods. The solvent and its amount to be used for the production are not particularly limited as long as the reaction mixture can be stirred. The compounds in the reaction schemes may form a salt, and as such salt, for example, those similar to the salts of compound (I) can be mentioned. In the present specification, the "equivalents" shows an equivalents relative to one equivalents of the reaction substrate.

### [Method A]

In compound (I) of the present invention, compound (I-1) wherein X¹ and X² in combination form a sulfur atom can be synthesized by, for example, the following methods and the like. 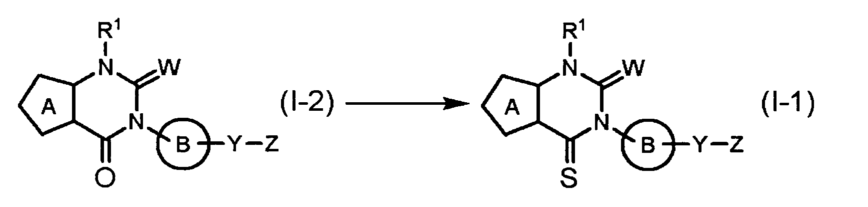 wherein the symbols in the formula are as defined above.

In this method, compound (I-1) is produced by reacting compound (I-2) with a suitable sulfide. This reaction is carried out according to a conventional method in a solvent that does not adversely influence the reaction.

As the sulfide to be used, for example, Lawesson's reagent, phosphorus pentasulfide and the like can be mentioned. The amount of sulfide to be used is preferably 1 equivalent to a large excess (preferably 1 to 10 equivalents relative to compound (1-2).

As the solvent that does not adversely influence the reaction, for example, ethers (e.g., tetrahydrofuran, dioxane, diethyl ether etc.), halogenated aliphatic hydrocarbons (e.g., dichloromethane, chloroform etc.), aliphatic hydrocarbons (e.g., hexane, pentane etc.), aromatic hydrocarbons (e.g., benzene, toluene etc.) and the like can be mentioned. These solvents may be used in a mixture at an appropriately ratio.

The reaction temperature is generally about 0°C to about 200°C. The reaction time is generally about 0.5 to about 20 hr.

### [Method B]

In compound (I) of the present invention, compound (I-3) wherein R¹ is a hydrocarbon group optionally having substituent(s) can be synthesized by, for example, the following methods and the like. 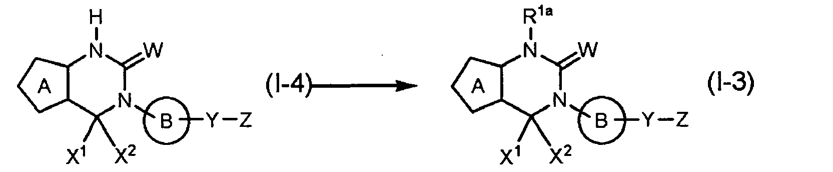 wherein R^{1a} is a hydrocarbon group optionally having substituent(s), and other symbols are as defined above.

The "hydrocarbon group optionally having substituent(s)" for R^{1a} is as defined for the aforementioned "hydrocarbon group optionally having substituent(s)" for R¹.

In this method, compound (I-3) is obtained by reacting compound (I-4) with a compound represented by the formula R^{1a}-L wherein R^{1a} is as defined above, and L is a leaving group (e.g., a halogen atom (e.g., fluorine, chlorine, bromine, iodine atom etc.), a C₁₋₆ alkylsulfonyloxy group optionally substituted by 1 to 3 halogen atoms (e.g., methanesulfonyloxy, ethanesulfonyloxy, trifluoromethanesulfonyloxy etc.), a C₆-₁₄ arylsulfonyloxy group optionally having substituent(s) (e.g., benzenesulfonyloxy, p-toluenesulfonyloxy, p-bromobenzenesulfonyloxy etc.) etc.). This reaction is carried out according to a conventional method in a solvent that does not adversely influence the reaction.

In the reaction, the compound represented by the formula R^{1a}-L is used in one equivalent to in a large excess (preferably 1 to 10 equivalents relative to compound (1-4). In this case, for example, a basic compound such as sodium hydroxide, potassium hydroxide, sodium hydride, potassium carbonate, triethylamine, diisopropylethylamine, pyridine, 4-N,N-dimethylaminopyridine (DMAP), 1,8-diazabicyclo[5.4.0]-7-undecene, 1,4-diazabicyclo[2.2.2]octane (DABCO) and the like can be used in 1 to 10 equivalents. In the reaction, an alkali metal iodide such as sodium iodide and the like may be added in one equivalent to a large excess (preferably 1 to 10 equivalents) as a reaction promoting agent.

As the solvent that does not adversely influence the reaction, for example, water, alcohols (e.g., methanol, ethanol etc.), ethers (e.g., tetrahydrofuran, dioxane, diethyl ether etc.), halogenated aliphatic hydrocarbons (e.g., methylene chloride, chloroform etc.), ketones (e.g., acetone, methyl ethyl ketone etc.), aliphatic hydrocarbons (e.g., hexane, pentane etc.), aromatic hydrocarbons (e.g., benzene, toluene etc.), aprotic polar solvents (e.g., N,N-dimethylformamide, acetonitrile, dimethyl sulfoxide etc.) and the like can be mentioned. These solvents may be used in a mixture at an appropriately ratio.

The reaction time is 10 min to 24 hr, preferably 0.5 to 6 hr. The reaction temperature is -20°C to 200°C (preferably 0°C to 150°C).

### [Method C]

In compound (I) of the present invention, compound (1-5) wherein Z is a formula

-SO_{n'}R^{3a},

wherein n' is 1 or 2, and R^{3a}' is a hydrocarbon group optionally having substituent(s) or a heterocyclic group optionally having substituent(s) (provided that R^{3a}' is not a methyl group), can be synthesized, for example, by the following methods and the like. 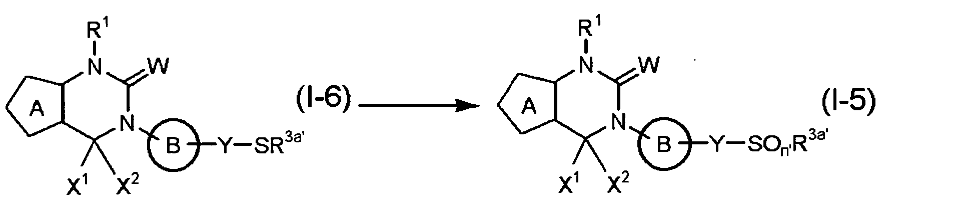 wherein the symbols in the formula are as defined above.

The "hydrocarbon group optionally having substituent(s)" and "heterocyclic group optionally having substituent(s)" for R^{3a}' are as defined for the aforementioned "hydrocarbon group optionally having substituent(s)" and "heterocyclic group optionally having substituent(s)", respectively, for R³.

In this method, compound (1-5) is produced by oxidizing compound (1-6). This oxidation reaction is carried out in the presence of an oxidant. As the oxidant, oxygen, hydrogen peroxide, organic peracids such as perbenzoic acid, m-chloroperbenzoic acid, peracetic acid and the like, perchlorates such as lithium perchlorate, silver perchlorate, tetrabutylammonium perchlorate and the like, periodates such as sodium periodate and the like, periodic acid, manganese dioxide, lead tetraacetate, permanganates such as potassium permanganate and the like, halogens such as iodine, bromine, chlorine and the like, N-bromosuccinimide, N-chlorosuccinimide, sulfuryl chloride, chloramine T and the like can be mentioned.

This reaction is generally carried out in a solvent, and a solvent that does not inhibit the reaction is appropriately selected. As such solvent, for example, alcohols (e.g., methanol, ethanol, propanol, isopropanol, butanol, tert-butanol etc.), ethers (e.g., dioxane, tetrahydrofuran, diethyl ether, tert-butyl methyl ether, diisopropyl ether, ethyleneglycol, dimethyl ether etc.), esters (e.g., ethyl formate, ethyl acetate, n-butyl acetate etc.), carboxylic acids (e.g., formic acid, acetic acid, propionic acid etc.), halogenated hydrocarbons (e.g., dichloromethane, chloroform, carbon tetrachloride, trichloroethylene, 1,2-dichloroethane, chlorobenzene etc.), hydrocarbons (e.g., n-hexane, benzene, toluene etc.), amides (e.g., formamide, N,N-dimethylformamide, N,N-dimethylacetamide etc.), ketones (e.g., acetone, methyl ethyl ketone, methylisobutylketone etc.), nitriles (e.g., acetonitrile, propionitrile etc.), sulforane, hexamethylphosphoramide, water and the like are used alone or in a mixed solvent.

This reaction may be carried out in the presence of a base. As such base, for example, inorganic bases such as alkali metal hydroxides (e.g., lithium hydroxide, sodium hydroxide, potassium hydroxide and the like,) alkaline earth metal hydroxides (e.g., magnesium hydroxide, calcium hydroxide and the like), alkali metal carbonate (e.g., sodium carbonate, potassium carbonate and the like), alkali metal hydrogencarbonates (e.g., sodium hydrogencarbonate, potassium hydrogencarbonate and the like), and the like can be used.

The oxidant is used in 0.1 to 20 equivalents, preferably about 0.4 to 10 equivalents, and the base is used in 0.1 to 20 equivalents, preferably 0.4 to 10 equivalents, relative to compound (1-6).

Where necessary, this reaction may be carried out in the presence of an acid. As such an acid, mineral acids such as hydrochloric acid, hydrobromic acid, sulfuric acid, phosphoric acid, perchloric acid and the like, sulfonic acids such as methanesulfonic acid, ethanesulfonic acid, benzenesulfonic acid, toluenesulfonic acid, camphorsulfonic acid and the like, organic acids such as formic acid, acetic acid, propionic acid, trifluoroacetic acid and the like. The amount of the acid to be used is 0.1 to 20 equivalents, preferably 0.5 to 10 equivalents, relative to compound (I-6).

The reaction temperature is about -10°C to about 250°C, preferably about -5°C to about 150°C.

While the reaction time varies depending on the kind of compound (1-6), the base or solvent, reaction temperature and the like, it is generally about 1 min - about 50 hr, preferably about 5 min - about 24 hr.

### [Method D]

In compound (I) of the present invention, compound (1-7) wherein Z is a formula

-C_{OR}4ₐ

wherein R^{4a} is an amino optionally having substituent(s), for example, can be synthesized by the following methods and the like. 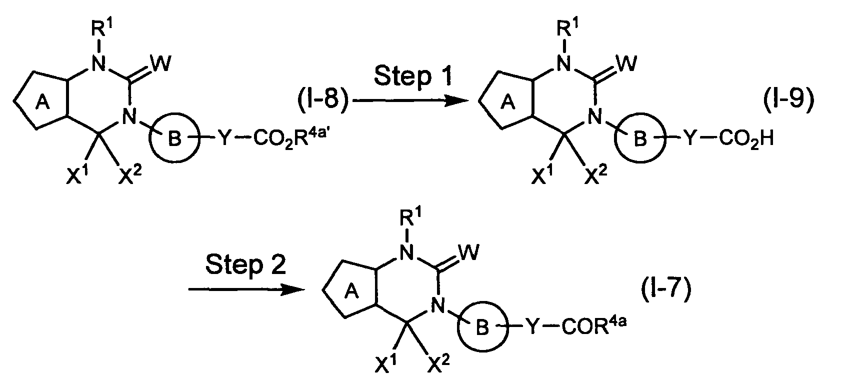 wherein R^{4a}' is a hydrocarbon group optionally having substituent(s), and other symbols are as defined above.

The "amino optionally having substituent(s)" for R^{4a} is as defined for the aforementioned "amino optionally having substituent(s)" for R⁴. The "hydrocarbon group optionally having substituent(s)" for R^{4a}' is as defined for the aforementioned "hydrocarbon group optionally having substituent(s)" for R¹.

### [Step 1]

In this Step, compound (1-9) is produced by hydrolyzing compound (I-8). This reaction is carried out according to a conventional method in a solvent that does not adversely influence the reaction.

As the acid, inorganic acids (e.g., hydrochloric acid, sulfuric acid, hydrobromic acid and the like), organic acids (acetic acid and the like) and the like can be mentioned. As the base, alkali metal carbonates (e.g., potassium carbonate, sodium carbonate and the like), alkali metal alkoxides (e.g., sodium methoxide and the like), alkali metal hydroxides (e.g., potassium hydroxide, sodium hydroxide, lithium hydroxide and the like) and the like can be mentioned. The amounts of the acid and base to be used are generally in excess relative to compound (I-8). The amount of the acid to be used is preferably about 2 to about 50 equivalents relative to compound (I-8) and the amount of the base to be used is preferably about 1.2 to about 5 equivalents relative to compound (I-8).

As the suitable solvent, alcohols (e.g., methanol, ethanol and the like), ethers (tetrahydrofuran, dioxane, diethyl ether and the like), dimethyl sulfoxide, acetone, water and the like can be mentioned. These solvents may be used in a mixture at an appropriately ratio.

The reaction temperature is generally about -20 to about 150°C, preferably about -10 to about 100°C. The reaction time is generally about 0.1 to about 20 hr.

### [Step 2]

In this Step, compound (I-7) is obtained by condensation (amidation) of compound (I-9) and a compound represented by the formula R^{4a}-H wherein the symbols in the formula are as defined above.

This reaction is carried out by a method known *per se*, for example,
(1) a method of directly condensing compound (I-9) and a compound represented by the formula R^{4a}-H using a condensation agent,
(2) a method of appropriately reacting a reactive derivative of compound (I-9) with a compound represented by the formula R^{4a}-H, and the like.

Method (1) is explained first.

As the aforementioned condensation agent, for example, carbodiimide condensation reagents such as dicyclohexylcarbodiimide, diisopropylcarbodiimide, 1-ethyl-3-(3-dimethylaminopropyl)carbodiimide and hydrochloride thereof and the like; phosphate condensation reagents such as diethyl cyanophosphate, diphenylphosphoryl azide and the like; generally-known condensation agents such as carbonyldiimidazole, 2-chloro-1,3-dimethylimidazolium tetrafluoroborate and the like, and the like can be mentioned.

Method (1) is generally performed in a solvent. As the solvent, for example, amides such as N,N-dimethylformamide, N,N-dimethylacetamide and the like; halogenated aliphatic hydrocarbons such as chloroform, dichloromethane and the like; aromatic hydrocarbons such as benzene, toluene and the like; ethers such as tetrahydrofuran, dioxane, diethyl ether and the like; ethyl acetate, water and the like can be mentioned. These solvents may be used in a mixture at an appropriately ratio.

The amount of the compound represented by the formula R^{4a}-H to be used is 0.1 to 10 molar equivalents, preferably 0.3 to 3 molar equivalents, relative to compound (I-9).

The amount of the condensation agent to be used is 0.1 to 10 molar equivalents, preferably 0.3 to 3 molar equivalents, relative to compound (I-9).

When a carbodiimide condensation reagent such as dicyclohexylcarbodiimide, diisopropylcarbodiimide, 1-ethyl-3-(3-dimethylaminopropyl)carbodiimide and hydrochloride thereof and the like is used as a condensation agent, a suitable condensation promoter (e.g., 1-hydroxy-7-azabenzotriazole, 1-hydroxybenzotriazole, N-hydroxysuccinimide, N-hydroxyphthalimide etc.) may be used as necessary. When a phosphate condensation reagent such as diethyl cyanophosphate, diphenylphosphoryl azide and the like is used as a condensation agent, an organic amine base such as triethylamine and the like may be added.

The amounts of the above-mentioned condensation promoter and organic amine base to be used are 0.1 to 10 molar equivalents, preferably 0.3 to 3 molar equivalents, relative to compound (I-9).

The reaction temperature is generally -30°C to 100°C. The reaction time is generally 0.5 to 60 hr.

Method (2) is now explained.

As the reactive derivative of compound (1-9), for example, an acid anhydride, an acid halide (e.g., an acid chloride, an acid bromide and the like), an acid imidazolide, an active ester (e.g., phenyl ester, nitro- or halogen-substituted phenyl ester (e.g., 4-nitrophenyle ster, pentafluorophenyl ester etc.), 1-hydroxy-7-azabenzotriazole ester, 1-hydroxybenzotriazole ester, N-hydroxysuccinimide ester, N-hydroxyphthalimide ester etc.), or a mixed acid anhydride (e.g., an anhydride with methyl carbonate, ethyl carbonate, isobutyl carbonate etc.) and the like can be mentioned.

For example, when an acid anhydride, an acid halide, an acid imidazolide, an active ester are used as a reactive derivative, the reaction is carried out in the presence of or in the absence of a base in a solvent that does not adversely influence the reaction.

As the base, for example, amines such as triethylamine, N-methylmorpholine, N,N-dimethylaniline and the like; alkali metal carbonates such as potassium carbonate, sodium carbonate and the like ; alkali metal hydrogencarbonates such as potassium hydrogencarbonate, sodium hydrogencarbonate and the like; alkali metal hydroxide such as potassium hydroxide, sodium hydroxide, lithium hydroxide and the like, and the like can be mentioned. The amount of the base to be used is 0.1 to 10 molar equivalents, preferably 0.3 to 3 molar equivalents, relative to compound (I-9) or a reactive derivative thereof.

As the solvent that does not adversely influence the reaction, for example, water, ethers (e.g., tetrahydrofuran, dioxane, diethyl ether etc.), halogenated aliphatic hydrocarbons (e.g., dichloromethane, chloroform etc.), ketones (e.g., acetone, methyl ethyl ketone etc.), esters (e.g., ethyl acetate etc.), aprotic polar solvents (e.g., N,N-dimethylformamide, acetonitrile, dimethyl sulfoxide etc.) and the like can be mentioned. These solvents may be used in a mixture at an appropriately ratio.

The amount of the compound represented by the formula R^{4a}-H to be used is 0.1 to 10 molar equivalents, preferably 0.3 to 3 molar equivalents, relative to compound (I-9) or a reactive derivative.

The reaction temperature is generally -30°C to 100°C. The reaction time is generally 0.5 to 20 hr.

When a mixed acid anhydride is used, compound (I-9) is reacted with a chlorocarbonate (e.g., methyl chlorocarbonate, ethyl chlorocarbonate, isobutyl chlorocarbonate etc.) in the presence of a base (e.g., amine such as triethylamine, N-methylmorpholine, N,N-dimethylaniline and the like; alkali metal carbonates such as potassium carbonate, sodium carbonate and the like; alkali metal hydrogencarbonates such as potassium hydrogencarbonate, sodium hydrogencarbonate and the like; alkali metal hydroxides such as potassium hydroxide, sodium hydroxide, lithium hydroxide and the like, etc.), and further reacted with a compound R^{4a}-H.

The chlorocarbonate is used in 0.1 to 10 equivalents, preferably 0.3 to 3 equivalents, relative to compound (I-9), and the base is used in 0.1 to 10 equivalents, preferably 0.3 to 3 equivalents, relative to compound (I-9).

The amount of the compound represented by the formula R^{4a}-Ha to be used is generally 0.1 to 10 molar equivalents, preferably 0.3 to 3 molar equivalents, relative to compound (I-9) or a mixed acid anhydride thereof.

The reaction temperature is generally -30°C to 100°C. The reaction time is generally 0.5 to 20 hr.

A compound represented by the formula R^{4a}-H to be used as a starting material compound in this method, can be produced by a method known *per se*, or can also be obtained as a commercially available product.

### [Method E]

In compound (I) of the present invention, compound (1-10) wherein Z is a formula

-COR^{4b}

wherein R^{4b} is a hydrocarbon group optionally having substituent(s) or a heterocyclic group optionally having substituent(s),
can be synthesized, for example, by the following methods and the like. 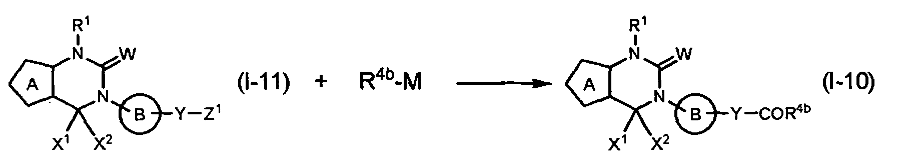 wherein Z¹ is -CO₂R^{4a,} -COX [wherein X is a halogen atom (e.g., fluorine, chlorine, bromine, iodine etc.)] or -CN, M is a metal atom such as sodium, magnesium and the like (in the case of a divalent metal, the remaining monovalent may be occupied by halogen atom (e.g., chlorine atom, bromine atom, iodine atom etc.) and the like), and other symbols are as defined above.

The "hydrocarbon group optionally having substituent(s)" and "heterocyclic group optionally having substituent(s)" for R^{4b} are each as defined for the aforementioned "hydrocarbon group optionally having substituent(s)" and "heterocyclic group optionally having substituent(s)" for R⁴.

In this method, compound (I-10) is obtained by reacting compound (I-11) with a compound represented by the formula R^{4b}-M. For the reaction, R^{4b}-M is used in 0.1 to 10 molar equivalents, preferably 0.3 to 3 molar equivalents, relative to compound (I-11). The solvent used therefor is, for example, ethers (e.g., tetrahydrofuran, dioxane, diethyl ether and the like), hydrocarbons (e.g., toluene, hexane and the like), halogenated hydrocarbons (e.g., methylene chloride, chloroform and the like) and the like.

The reaction time is 0.5 to 72 hr, preferably 1 to 24 hr. The reaction temperature is -100°C to 100°C (preferably -80°C to 50°C).

The compound represented by the formula R^{4b}-M to be used for this method can be produced by a method known *per se*, or obtained as a commercially available product.

In compound (I-11) to be used as a starting material compound in this method, a compound wherein Z¹ is -CO₂R^{4a}_{,} or -CN can be produced, for example, by the following [Method F], [Method G], [Method H] and [Method I]. In addition, in compound (I-11) to be used as a starting material compound in this method, a compound wherein Z¹ is -COX can be produced from compound (I-9) by a reaction known *per se*, for example, according to or in reference to the method described or cited in ZIKKEN KAGAKU KOZA 4th ed. (MARUZEN), vol. 22, Organic Synthesis IV, pages 115-127 and the like.

### [Method F]

Compound (I-12) [a compound wherein R¹ is a hydrogen atom, X¹ and X² in combination form an oxygen atom, and W is an oxygen atom (the compound encompasses a compound wherein R¹ is a hydrogen atom, and W is an oxygen atom in compound (I-2), or a compound wherein X¹ and X² in combination form an oxygen atom, and W is an oxygen atom in compound (I-4)) in compound (I) of the present invention, a compound wherein R¹ is a hydrogen atom, W is an oxygen atom, and X¹ and X² in combination form an oxygen atom in compound (I-8), or a compound wherein R¹ is a hydrogen atom, Z¹ is -CO₂R^{4a,} or -CN, X¹ and X² in combination form an oxygen atom, and W is an oxygen atom in compound (I-11)] can be synthesized, for example, by the following methods and the like. 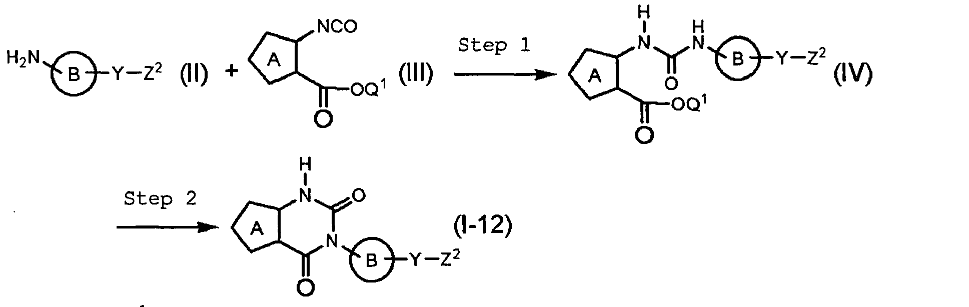 wherein Q¹ is a hydrocarbon group optionally having substituent(s), Z² is Z, -CO₂R^{4a,} or -CN, and other symbols are as defined above.

The "hydrocarbon group optionally having substituent(s)" for Q¹ is as defined for the aforementioned "hydrocarbon group optionally having substituent(s)" for R¹.

### [Step 1]

In this method, compound (IV) is obtained by condensing compound (II) and compound (III).

The reaction is carried out in the presence or absence of a base and in a solvent that does not adversely influence the reaction.

The amount of compound (III) to be used is 0.1 to 10 molar equivalents, preferably 0.3 to 3 molar equivalents, relative to compound (II).

As the base, for example, amines such as triethylamine, pyridine, N,N-dimethylaniline and the like; alkali metal carbonates such as potassium carbonate, sodium carbonate and the like; alkali metal hydrogencarbonates such as potassium hydrogencarbonate, sodium hydrogencarbonate and the like; alkali metal hydroxides such as potassium hydroxide, sodium hydroxide, lithium hydroxide and the like, and the like can be mentioned. The amount of the base to be used is 0.1 to 10 molar equivalents, preferably 0.3 to 3 molar equivalents, relative to compound (II).

As the solvent that does not adversely influence the reaction, for example, ethers (e.g., tetrahydrofuran, dioxane, diethyl ether etc.), halogenated aliphatic hydrocarbons (e.g., dichloromethane, chloroform etc.), ketones (e.g., acetone, methyl ethyl ketone etc.), esters (e.g., ethyl acetate etc.), aprotic polar solvents (e.g., N,N-dimethylformamide, acetonitrile, dimethyl sulfoxide etc.) and the like can be mentioned. These solvents may be used in a mixture at an appropriately ratio.

The reaction temperature is generally 0°C to 150°C. The reaction time is generally 0.5 to 36 hr.

Compound (III) to be used as a starting material compound in this method can be produced by a method known *per se,* or obtained as a commercially available product.

The resultant product can be used for the next step as a reaction mixture or a crude product. In can also be isolated from the reaction mixture according to a conventional method.

### [Step 2]

In this method, compound (I-12) is produced by cyclizing compound (IV). The reaction is carried out in the presence of or in the absence of a base, without solvent or in a solvent that does not adversely influence the reaction.

As the base, for example, amines such as triethylamine, pyridine, N,N-dimethylaniline and the like; alkali metal carbonates such as potassium carbonate, sodium carbonate and the like; alkali metal hydrogencarbonates such as potassium hydrogencarbonate, sodium hydrogencarbonate and the like; alkali metal hydroxides such as potassium hydroxide, sodium hydroxide, lithium hydroxide and the like, and the like can be mentioned. The amount of the base to be used is 0.1 to 10 molar equivalents, preferably 0.3 to 3 molar equivalents, relative to compound (IV).

As the solvent that does not adversely influence the reaction, for example, ethers (e.g., tetrahydrofuran, dioxane, diethyl ether etc.), halogenated aliphatic hydrocarbons (e.g., dichloromethane, chloroform etc.), ketones (e.g., acetone, methyl ethyl ketone etc.), esters (e.g., ethyl acetate etc.), aprotic polar solvents (e.g., N,N-dimethylformamide, acetonitrile, dimethyl sulfoxide etc.) and the like can be mentioned. These solvents may be used in a mixture at an appropriately ratio.

The reaction temperature is generally 0°C to 150°C. The reaction time is generally 0.5 to 36 hr.

### [Method G]

Compound (1-13) [a compound wherein R¹ is a hydrogen atom, and X¹ and X² in combination form an oxygen atom (the compound encompasses a compound wherein R¹ is a hydrogen atom in compound (1-2), or a compound wherein X¹ and X² in combination form an oxygen atom in compound (1-4)) in compound (I) of the present invention, a compound wherein R¹ is a hydrogen atom, and X¹ and X² in combination form an oxygen atom in compound (I-8), or, a compound wherein R¹ is a hydrogen atom, Z¹ is -CO₂R^{4a}, or -CN, and X¹ and X² in combination form an oxygen atom in compound (I-11)] can be synthesized, for example, by the following methods and the like. 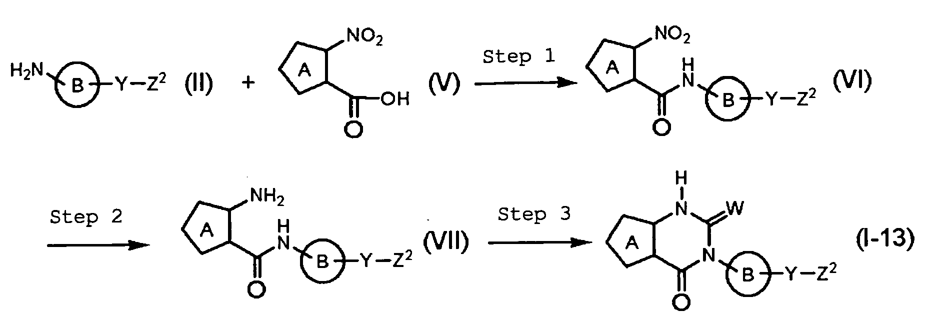 wherein the symbols in the formula are as defined above.

### [Step 1]

In this method, compound (VI) is obtained by condensing (amidating) compound (II) and compound (V).

This production method is performed, for example, under reaction conditions similar to those of production of compound (I-7) by condensation of compound (I-9) and compound R^{4a}-H in [Step 2] of the aforementioned [Method D].

Compound (V) to be used as a starting material compound in this method, and a reactive derivative of compound (V) can be produced by a method known *per se,* or obtained as a commercially available product.

### [Step 2]

In this method, compound (VII) is produced by reducing compound (VI).

The reduction reaction is carried out, for example, using a metal hydrogen complex compound (e.g., lithium aluminum hydride etc.); a metal (e.g., zinc, iron, tin etc.) in the presence of an acid (e.g., hydrochloric acid, sulfuric acid, acetic acid etc.); stannous chloride in the presence of an acid (e.g., hydrochloric acid, sulfuric acid, acetic acid etc.); or a reducing agent such as zinc and the like under neutral or alkaline conditions. The reducing agent is used in 1 equivalent to a large excess (preferably 1 to 10 equivalents), relative to compound (VI).

As the reduction reaction, moreover, a catalytic reduction under normal pressure to pressurization in the presence of a catalyst (e.g., metal such as platinum, palladium, nickel, rhodium and the like or a oxide, salts or complexes thereof, etc. These catalysts can also be carried on various carriers such as carbon etc. and used) can also be used.

The solvent to be used for the reaction can be appropriately selected according to the reduction method and, for example, water, alcohols (e.g., methanol, ethanol etc.), ethers (e.g., tetrahydrofuran, dioxane, diethyl ether etc.), halogenated aliphatic hydrocarbons (e.g., dichloromethane, chloroform etc.), aliphatic hydrocarbons (e.g., hexane, pentane etc.), aromatic hydrocarbons (e.g., benzene, toluene etc.), aprotic polar solvents (e.g., N,N-dimethylformamide, dimethyl sulfoxide, acetonitrile etc.) and the like can be used. When the acid to be used for reduction with a metal or stannous chloride is liquid, the acid can be used as a solvent. These solvents may be used in a mixture at an appropriately ratio.

The reaction time is 0.1 to 72 hr, preferably 0.1 to 24 hr. The reaction temperature is -30°C to 150°C, preferably 0°C to 80°C.

### [Step 3]

In this method, compound (I-13) is produced by reacting compound (VII) with a reagent such as phosgene, diphosgene, triphosgene, N,N'-carbonyldiimidazole, thiophosgene, 1,1'-thiocarbonyldiimidazole and the like. The amount of the reagent to be used is preferably 1 equivalent to a large excess (preferably 1 to 5 equivalents), relative to compound (VII). The reaction is carried out in the presence or absence of a base and in a solvent that does not adversely influence the reaction.

As the base, for example, amines such as triethylamine, pyridine, N,N-dimethylaniline and the like; alkali metal carbonates such as potassium carbonate, sodium carbonate and the like; alkali metal hydrogencarbonates such as potassium hydrogencarbonate, sodium hydrogencarbonate and the like; alkali metal hydroxides such as potassium hydroxide, sodium hydroxide, lithium hydroxide and the like, and the like can be mentioned. The amount of the base to be used is 0.1 to 10 molar equivalents, preferably 0.3 to 3 molar equivalent, relative to compound (VII).

As the solvent that does not adversely influence the reaction, for example, ethers (e.g., tetrahydrofuran, dioxane, diethyl ether etc.), halogenated aliphatic hydrocarbons (e.g., dichloromethane, chloroform etc.), ketones (e.g., acetone, methyl ethyl ketone etc.), esters (e.g., ethyl acetate etc.), aprotic polar solvents (e.g., N,N-dimethylformamide, acetonitrile, dimethyl sulfoxide etc.) and the like can be mentioned. These solvents may be used in a mixture at an appropriately ratio.

The reaction temperature is generally -30°C to 100°C. The reaction time is generally 0.5 to 20 hr.

### [Method H]

Compound (I-14) and (I-15) (a compound wherein R¹ is a hydrogen atom, X¹ and X² in combination form =NR², and W is an oxygen atom (the compound encompasses a compound wherein X¹ and X² in combination form =NR², and W is an oxygen atom in compound (I-4)) in compound (I) of the present invention, a compound wherein R¹ is a hydrogen atom, W is an oxygen atom, and X¹ and X² in combination form =NR² in compound (I-8), or, a compound wherein R¹ is a hydrogen atom, Z¹ is -CO₂R^{4a,} or -CN, X¹ and X² in combination form =NR², and W is an oxygen atom in compound (I-11)) can be synthesized, for example, by the following methods and the like. 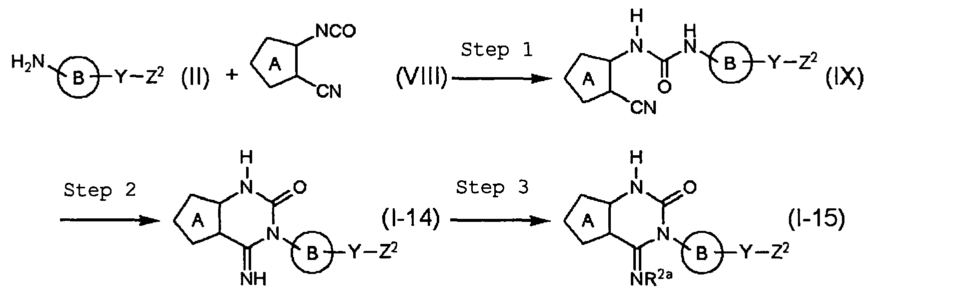 wherein R^{2a} is a hydrocarbon group optionally having substituent(s), and other symbols are as defined above.

The "hydrocarbon group optionally having substituent(s)" for R^{2a} is as defined for the aforementioned "hydrocarbon group optionally having substituent(s)" for R².

### [Step 1]

In this method, compound (IX) is obtained by condensing compound (II) and compound (VIII).

This production method is performed, for example, under reaction conditions similar to those of production of compound (I-7) by condensation of compound (I-9) and the compound represented by the formula R^{4a}-H in [Step 2] of the aforementioned [Method D].

Compound (VIII) to be used as a starting material compound in this method can be produced by a method known *per se*, or obtained as a commercially available product.

The resultant product can be used for the next step as a reaction mixture or a crude product. In can also be isolated from the reaction mixture according to a conventional method.

### [Step 2]

In this method, compound (I-14) is produced by cyclizing compound (IX).

This production method is performed, for example, under reaction conditions similar to those of production of compound (I-12) by cyclization of compound (IV) in [Step 2] of the aforementioned [Method F].

### [Step 3]

In this method, compound (1-15) is produced by reacting compound (I-14) with a compound represented by the formula R^{2a}-L wherein the symbols in the formula are as defined above.

This production method is performed, for example, under reaction conditions similar to those of production of compound (I-3) by reaction of compound (I-4) with the compound represented by the formula R^{1a}-L in the aforementioned [Method B].

### [Method I]

Compound (1-16) [a compound wherein R¹ is a hydrogen atom in compound (I) of the present invention, a compound wherein R¹ is a hydrogen atom compound in compound (I-8), or a compound wherein R¹ is a hydrogen atom, and Z¹ is -CO₂R^{4a}' or -CN in compound (I-11)] can be synthesized, for example, by the following methods and the like. 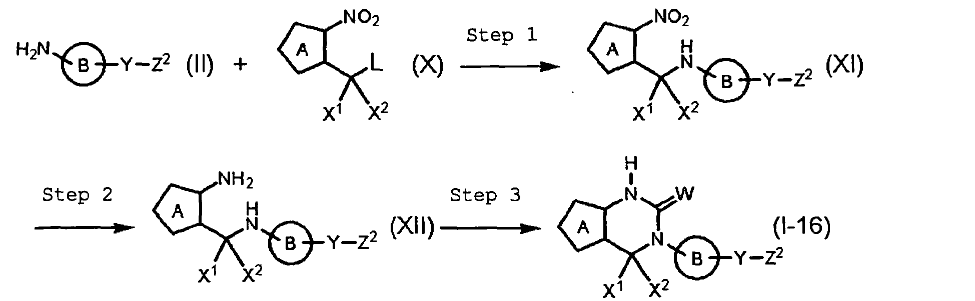 wherein the symbols in the formula are as defined above.

### [Step 1]

In this method, compound (XI) is produced by reacting compound (II) with compound (X).

This production method is performed, for example, under reaction conditions similar to those of production of compound (I-3) by reaction of compound (I-4) with the compound represented by the formula R^{1a}-L in the aforementioned [Method B].

Compound (X) to be used as a starting material compound in this method can be produced by a method known *per se,* or obtained as a commercially available product.

The resultant product can be used for the next step as a reaction mixture or a crude product. In can also be isolated from the reaction mixture according to a conventional method.

### [Step 2]

In this method, compound (XII) is produced by reducing compound (XI).

This production method is performed, for example, under reaction conditions similar to those of production of compound (VII) by reduction of compound (VI) in [Step 2] of the aforementioned [Method G].

### [Step 3]

In this method, compound (1-16) is produced by reacting compound (XII) with a reagent such as phosgene, diphosgene, triphosgene, N,N'-carbonyldiimidazole, thiophosgene, 1,1'-thiocarbonyldiimidazole and the like.

This production method is performed, for example, under reaction conditions similar to those of production of compound (I-13) by reaction of compound (VII) with a reagent such as phosgene, diphosgene, triphosgene, N,N'-carbonyldiimidazole, thiophosgene, 1,1'-thiocarbonyldiimidazole and the like in [Step 2] of the aforementioned [Method D].

Compound (II) to be used as a starting material compound in [Method F], [Method G], [Method H] and [Method I] can be produced by a method known *per se*, or obtained as a commercially available product. It can also be produced, for example, by the following Method J.

### [Method J]

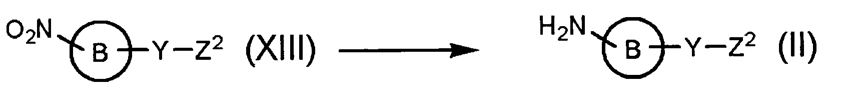

wherein the symbols in the formula are as defined above.

In this method, compound (II) is produced by reducing compound (XIII).

This production method is performed, for example, under reaction conditions similar to those of production of compound (VII) by reduction of compound (VI) in [Step 2] of the aforementioned [Method G].

Compound (XIII) to be used as a starting material compound in [Method J] can be produced by a method known *per se*, or obtained as a commercially available product. It can also be produced, for example, by the following [Method K] - [Method N].

### [Method K]

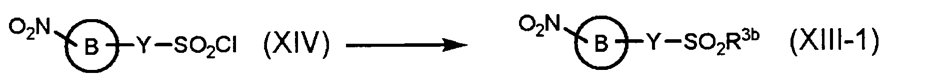

wherein R^{3b} is an amino optionally having substituent(s), and other symbols are as defined above.

The "amino optionally having substituent(s)" for R^{3b} is as defined for the aforementioned "optionally having substituent(s) amino" for R³.

In this Step, compound (XIII-1) is obtained by condensing compound (XIV) and a compound represented by the formula R^{3b}-H wherein the symbols in the formula are as defined above.

This production method is performed in the presence of or in the absence of a base and in a solvent that does not adversely influence the reaction.

As the base, for example, amines such as triethylamine, N-methylmorpholine, N,N-dimethylaniline and the like; alkali metal carbonates such as potassium carbonate, sodium carbonate and the like; alkali metal hydrogencarbonates such as potassium hydrogencarbonate, sodium hydrogencarbonate and the like; alkali metal hydroxides such as potassium hydroxide, sodium hydroxide, lithium hydroxide and the like, and the like can be mentioned. The amount of the base to be used is 0.1 to 10 molar equivalents, preferably 0.3 to 3 molar equivalents, relative to compound (XIV).

As the solvent that does not adversely influence the reaction, for example, water, ethers (e.g., tetrahydrofuran, dioxane, diethyl ether etc.), halogenated aliphatic hydrocarbons (e.g., dichloromethane, chloroform etc.), ketones (e.g., acetone, methyl ethyl ketone etc.), esters (e.g., ethyl acetate etc.), aprotic polar solvents (e.g., N,N-dimethylformamide, acetonitrile, dimethyl sulfoxide etc.) and the like can be mentioned. These solvents may be used in a mixture at an appropriately ratio.

The amount of the compound represented by the formula R^{3b}-H to be used is 0.1 to 10 molar equivalents, preferably 0.3 to 3 molar equivalents, relative to compound (XIV).

The reaction temperature is generally -30°C to 100°C. The reaction time is generally 0.5 to 20 hr.

Compound (XIV) and the compound represented by the formula R^{3b}-H to be used as starting material compounds in this method can be produced by a method known *per se*, or obtained as a commercially available product.

### [Method L]

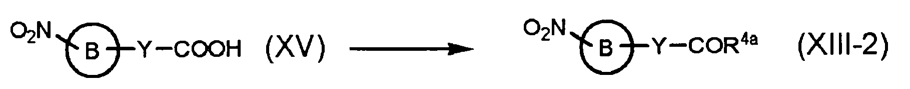

wherein the symbols in the formula are as defined above.

In this method, compound (XIII-2) is obtained by condensing compound (XV) and a compound represented by the formula R^{4a}-H wherein the symbols in the formula are as defined above.

This production method is performed, for example, under reaction conditions similar to those of production of compound (I-7) by condensation of compound (I-9) and the compound represented by the formula R^{4a}-H in [Step 2] of the aforementioned [Method D].

Compound (XV) and the compound represented by R^{4a}-H to be used as starting material compounds in this method can be produced by a method known *per se*, or obtained as a commercially available product.

### [Method M]

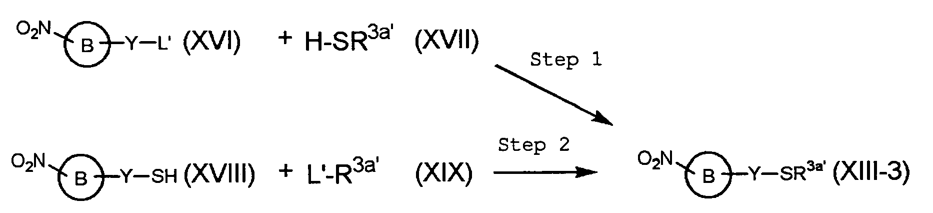

wherein, in the formula, L' is a hydroxyl group or a leaving group (e.g., a halogen atom (e.g., fluorine, chlorine, bromine, iodine etc.), a C₁₋₆ alkylsulfonyloxy group optionally substituted with 1 to 3 halogen atoms (e.g., methanesulfonyloxy, ethanesulfonyloxy, trifluoromethanesulfonyloxy etc.), a C₆₋₁₄ arylsulfonyloxy group optionally having substituent(s) (e.g., benzenesulfonyloxy, p-toluenesulfonyloxy, p-bromobenzenesulfonyloxy etc.) etc.), and other symbols are as defined above.

### [Step 1]

In this method, compound (XIII-3) is obtained by condensing compound (XVI) and compound (XVII).

### [in the case where L' in compound (XVI) is a leaving group]

This production method is performed, for example, under reaction conditions similar to those of production of compound (I-3) by reaction of compound (1-4) with the compound represented by the formula R^{1a}-L wherein the symbols in the formula are as defined above in the aforementioned [Method B]. [in the case where L' in compound (XVI) is a hydroxyl group]

This production method is performed according to a method known *per se*, for example, a method described in Synthesis, 1 page (1981), which is known as what is called the Mitsunobu reaction, or a method analogous thereto. That is, this reaction is generally carried out in the presence of an organic phosphine compound and an electrophilic agent and in a solvent that does not adversely influence the reaction.

The amount of compound (XVII) to be used is 0.1 to 10 molar equivalents, preferably 0.3 to 3 molar equivalents, relative to compound (XVI).

As the organic phosphine compound, for example, triphenylphosphine, tributylphosphine and the like can be mentioned. As the electrophilic agent, for example, diethyl azodicarboxylate, diisopropyl azodicarboxylate, azodicarbonyldipiperazine, 1,1'-(azodicarbonyl)dipiperidine and the like can be mentioned. The amount of the organic phosphine compound and electrophilic agent to be used are preferably about 1 to about 5 molar equivalents, relative to compound (XVI), respectively.

As the solvent that does not adversely influence the reaction, for example, ethers such as diethyl ether, tetrahydrofuran, dioxane and the like; halogenated hydrocarbons such as chloroform, dichloromethane and the like; aromatic hydrocarbons such as benzene, toluene, xylene and the like; amides such as N,N-dimethylformamide and the like; sulfoxides such as dimethylsulfoxide and the like, and the like can be mentioned. These solvents may be used in a mixture at an appropriately ratio.

The reaction temperature is generally about -50°C to about 150°C, preferably about -10°C to about 100°C. The reaction time is generally about 0.5 - about 20 hr.

Compound (XVI) and compound (XVII) to be used as starting material compounds in this method can be produced by a method known *per se*, or obtained as a commercially available product.

### [Step 2]

In this method, compound (XIII-3) is obtained by condensing compound (XVIII) and compound (XIX).

This production method is performed, for example, under reaction conditions similar to those of production of compound (XIII-3) by condensation of compound (XVI) and compound (XVII) in [Step 1] of the aforementioned [Method M].

Compound (XVIII) and compound (XIX) to be used as starting material compounds in this method can be produced by a method known *per se*, or obtained as a commercially available product.

### [Method N]

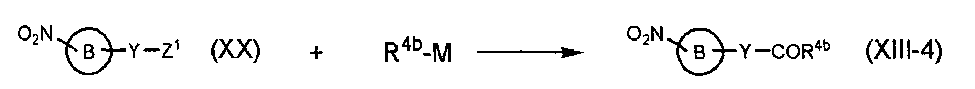

wherein the symbols in the formula are as defined above.

In this method, compound (XIII-4) is obtained by reacting compound (XX) with a compound represented by the formula R^{4b}-M.

This production method is performed, for example, under reaction conditions similar to those of production of compound (I-10) by reaction of compound (I-11) with a compound represented by the formula R^{4b}-M in the aforementioned [Method E].

Compound (XX) and the compound represented by the formula R^{4b}-M to be used as starting material compounds in this method can be produced by a method known *per se*, or obtained as a commercially available product.

Those of ordinary skill in the art will appreciate that the production method of compound (I) is not limited to the above-mentioned production methods, and also appreciate modification of the above-mentioned production methods and combinations of the above-mentioned production methods and the methods known *per se*.

Thus compound (I) can be isolated and purified by a separation means known *per se,* such as recrystallization, distillation, chromatography and the like.

When compound (I) is obtained as a free form, it can be converted to a desired salt by a method known *per se* or a modification thereof; conversely, when compound (I) is obtained as a salt, it can be converted to a free form or other desired salt by a method known *per se* or a modification thereof.

The compound (I) may be a hydrate or a non-hydrate. Examples of the hydrate include monohydrate, 1.5 hydrate, 2 hydrate and the like.

When compound (I) is obtained as a mixture of optically active forms, it can be separated into the objective (R) form or (S) form by an optical resolution means known *per se*.

The compound (I) may be used as a prodrug, and the prodrug means a compound which is converted to the compound (I) with a reaction due to an enzyme, an gastric acid, etc. under the physiological condition in the living body, that is a compound which is converted to the compound (I) with oxidation, reduction, hydrolysis, etc. according to an enzyme; a compound which is converted to the compound (I) by hydrolysis etc. due to gastric acid, etc. A prodrug for compound (I) may be a compound obtained by subjecting an amino group in compound (I) to an acylation, alkylation or phosphorylation (e.g., a compound obtained by subjecting an amino group in compound (I) to eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation and tert-butylation, etc.); a compound obtained by subjecting a hydroxy group in compound (I) to an acylation, alkylation, phosphorylation or boration (e.g., a compound obtained by subjecting an hydroxy group in compound (I) to acetylation, palmitoylation, propanoylation, pivaloylation, succinylation, fumarylation, alanylation, dimethylaminomethylcarbonylation, etc.); and the like. Any of these compounds can be produced from compound (I) by a method known *per se*.

A prodrug for compound (I) may also be one which is converted to the compound of the present invention under physiological conditions, such as those described in IYAKUHIN no KAIHATSU (Development of Pharmaceuticals), Vol. 7, Design of Molecules, p.163-198, Published by HIROKAWA SHOTEN (1990).

The compound (I) may be labeled with an isotope (e.g., ³H, ¹⁴C, ³⁵S and the like) or the like.

Compound (I) of the invention (includes Compound (I-a), a salt and a prodrug thereof. Hereinafter, may be briefly referred to as the "compound of the invention") has excellent GnRH antagonizing activity and has low toxicity. Moreover, the compound excels in oral absorption or sustained action, and is also excellent in the aspects of stability or pharmacokinetics. The compound of the invention can be safely used in prevention and/or treatment of male hormone- or female hormone-dependent diseases (sex hormone-dependent diseases), and in prevention and/or treatment of diseases attributable to excessive secretion of those hormones, since the compound can inhibit secretion of gonadotropin by means of its antagonistic activity against a GnRH receptor in a mammal (e.g., a human, a monkey, a cow, a horse, a dog, a cat, a rabbit, a rat, a mouse, etc.), thus regulating the sex hormone concentration in the blood.

For example, the compound of the invention is useful in prevention and/or treatment of sex hormone-dependent diseases such as sex hormone-dependent cancers (e.g., prostate cancer, uterine cancer, breast cancer, pituitary tumor, etc.), bone metastasis of sex hormone-dependent cancers, prostatomegaly, hysteromyoma, endometriosis, metrofibroma, precocious puberty, amenorrhea, premenstrual syndrome, dysmenorrhea, polycystic ovary syndrome, acne, baldness, and Alzheimer's disease (Alzheimer's disease, Alzheimer type senile dementia and mixed type thereof).

The compound of the invention is also useful in regulation of male and female reproduction (e.g., agent for birth control, menstrual cycle regulator, etc.).

The compound of the invention can be also used as a contraceptive for both male and female, and further as an ovulation inducing agent for female.

The compound of the invention can be used in prevention and/or treatment of infertility by using its rebound effect after cessation of medication.

The compound can be also used as a prophylactic and/or therapeutic agent for benign or malignant tumors which are sex hormone-independent and LH-RH-sensitive.

The compound of the invention can be also used as a prophylactic and/or therapeutic agent for irritable bowel syndrome and as a prophylactic agent for post-operative recurrence of sex hormone-dependent cancers (a prophylactic agent for post-operative recurrence of prostate cancer, a prophylactic agent for post-operative recurrence of breast cancer or ovarian cancer before and after menopause, etc.; particularly preferably a prophylactic agent for post-operative recurrence of breast cancer or ovarian cancer before menopause).

Moreover, the compound of the invention prevents disorder in the ovary or testicle due to the treatment using chemotherapeutic agents, and thus can be used for the preservation of the reproductive function after the treatment.

In addition, the compound of the present invention can also be used for activation of immunity.

The compound of the present invention is preferably used as an agent for the prophylaxis or treatment of prostate cancer, uterine cancer, breast cancer or hypophysis tumor or a preventive agent for post-operative recurrence of sex hormone-dependent cancer.

In addition, the compound of the invention is also useful for the regulation of animal's estrus, improvement in the table meat quality, regulation of animal growth and the like. The compound of the invention is also useful as a promoting agent for fish spawning.

Moreover, the compound of the present invention is useful for the prophylaxis or treatment of hot flash that occurs when sex hormone is reduced for the treatment of a disease such as prostate cancer, breast cancer, endometriosis and the like or during climacterium.

Furthermore, the compound of the present invention suppresses secretion of gonadal stimuli hormone by GnRH receptor antagonistic action and can be safely used for the promotion and/or assistance of external fertilization (IVF) in mammal (e.g., human, monkey, bovine, horse, dog, cat, rabbit, rat, mouse and the like).

External fertilization (in vitro fertilization; IVF) means a method comprising taking an ova, fertilizing the ova with spermatozoon in vitro, and returning the ova in the uterus at a certain stage of cleavage. The compound of the present invention can be used, for example, for recovering the ova. To recover a good ova, ova is recovered by control from outside the body rather than natural ovulation, where the compound of the present invention is used to eliminate the influence due to endogenous LH.

The compound of the present invention is highly evaluated by gynecologists because it can be administered orally, and is superior as a promoter and/or assistant agent of external fertilization as compared to peptidic GnRH antagonists administered by subcutaneous injection.

Accordingly, the compound of the present invention is also useful as a preventive of premature ovulation during external fertilization or embryo transplantation, or a preventive of ovulation due to endogenous LH during external fertilization.

The compound of the invention can be used to suppress a transient increase in the blood concentration of testosterone (flare phenomenon) that is observed upon administration of a GnRH superagonist such as leuprorelin acetate. The compound of the invention can be used in combination with a GnRH superagonist such as leuprorelin acetate, gonadorelin, buserelin, triptorelin, goserelin, nafarelin, histrelin, deslorelin, meterelin or lecirelin (preferably leuprorelin acetate).

The compound of the invention can be also effectively used in combination with at least one kind selected from steroidal or nonsteroidal antiandrogenic agents or antiestrogenic agents, chemotherapeutic agents, peptidic GnRH antagonists, α-reductase inhibitors, α-receptor inhibitors, aromatase inhibitors, 17β-hydroxysteroid dehydrogenase inhibitors, adrenal androgen production inhibitors, kinase inhibitors, hormonotherapeutic agents, cell growth factors or drugs inhibiting the action of receptors thereof, therapeutic drug for osteoporosis (e.g., bisphosphonate and the like), central pharmaceutical agent [for example, antianxiety drug, sleep-inducing drug, therapeutic agent for schizophrenia, therapeutic agent for Parkinson's disease, antidementia agent (e.g., cerebral circulation improver, cerebral metabolic activator and the like) and the like], antihypertensive agent, therapeutic agent for diabetes, antihyperlipemia agent, nutritional supplement (e.g., vitamin and the like), analgesic, digestion absorption promoter, gastrointestinal agent and the like.

Examples of the "anti-androgen" include cyproterone acetate, chlormadinone acetate, flutamide(Flutamide), bicartamide, nilutamide and the like.

Examples of the "antiestrogen" include tamoxifen, SERM (e.g., raloxifene, arzoxifene, lasofoxifene etc.), fulvestrant, ER down regulator and the like.

Examples of the "α-reductase inhibitor" include finasteride, Dutasteride, Izonsteride and the like.

Examples of the "α-receptor inhibitor" include tamsulosin, prazosin, terazosin and the like.

Examples of the "aromatase inhibitor" include anastrozole, retrozole and the like.

Examples of the "chemotherapeutic agent" include ifosfamide, Adriamycin, peplomycin, cisplatin, cyclophosphamide, 5-FU, UFT, methotrexate, mitomycin C, mitoxantrone and the like.

Examples of the "peptidic GnRH antagonist" include parenterally administered peptidic GnRH antagonists such as cetrorelix, ganirelix, abarelix and degarelix.

Examples of the "adrenal androgen production inhibitor" may be exemplified by lyase (C_{17,20}-lyase) inhibitors, and the like.

Examples of the "kinase inhibitor" include tyrosine phosphorylation enzyme inhibitor and the like.

Examples of the "hormonotherapeutic agent" include antiestrogenic agents, luteinizing hormones (e.g., MPA, etc.), androgenic agents, estrogenic agents, antiandrogenic agents and the like.

The "growth factor" may be any as long as it promotes cell proliferation, which is normally peptide having a molecular weight of not more than 20,000 that is capable of exhibiting its activity at low concentrations by binding to a receptor. Examples thereof include (1) EGF (epidermal growth factor) or substances possessing substantially the same activity as it [e.g., EGF, heregulin (HER2 ligand), and the like], (2) insulin or substances possessing substantially the same activity as it [e.g., insulin, IGF (insulin-like growth factor)-1, IGF-2, and the like], (3) FGF (fibroblast growth factor) or substances possessing substantially the same activity as it [e.g., aFGF, bFGF, KGF (keratinocyte growth factor), HGF (hepatocyte growth factor), FGF-10, and the like], (4) other cell growth factors [e.g., CSF (colony stimulating factor), EPO (erythropoietin), IL-2 (interleukin-2), NGF (nerve growth factor), PDGF (platelet-derived growth factor), TGFβ (transforming growth factor β), VEGF (vascular endothelial cell growth factor), and the like], and the like.

Examples of the "growth factor receptors" include any receptors capable of binding to the aforementioned growth factors, including EGF receptor, heregulin receptor (HER2), insulin receptor-1, insulin receptor-2, IGF receptor, FGF receptor-1, FGF receptor-2, VEGF receptor, and the like.

Examples of the aforementioned "pharmaceutical agents inhibiting the action of cell growth factor" include Herceptin (HER2 receptor antibody), Erbitux (EGF receptor antibody), Iressa or Tarceva (EGF receptor kinase inhibitor), avastin (VEGF antibody) and the like.

The drug inhibiting the action of the cell growth factor or its receptor may be exemplified by herbimycin, PD153035 (Science, 265 (5175), p.1093 (1994)), or the like.

Examples of the drug inhibiting the cell growth factor or its receptor include HER2 inhibitors. The HER2 inhibitor may be any substance inhibiting the HER2 activity (e.g., phosphorylating activity), including antibodies, low molecular weight compounds (synthetic compounds, natural compounds), antisenses, HER2 ligands, heregulin, or products resulting from partial modification or alteration of their structures. Further, the drug may be also a substance inhibiting the HER2 activity by inhibiting a HER2 receptor (e.g., HER2 receptor antibody). Examples of the low molecular weight compound having HER2 inhibiting activity include the compounds described in WO 98/03505, specifically 1-[3-[4-[2-((E)-2-phenylethenyl)-4-oxazolylmethoxy]phenyl]propyl]-1,2,4-triazole, and the like.

With respect to prostatomegaly, the compound of the invention can be used in combination with a drug such as GnRH superagonist, antiandrogenic agent, antiestrogenic agent, peptidic GnRH antagonist, α-reductase inhibitor, α-receptor inhibitor, aromatase inhibitor, 17β-hydroxysteroid dehydrogenase inhibitor, adrenal androgen production inhibitor, or phosphorylase inhibitor.

With respect to prostate cancer, the compound of the invention can be used in combination with a drug such as GnRH superagonist, antiandrogenic agent, antiestrogenic agent, chemotherapeutic agent [e.g., ifosfamide, UFT, Adriamycin, peplomycin, cisplatin, etc.], peptidic GnRH antagonist, aromatase inhibitor, 17β-hydroxysteroid dehydrogenase inhibitor, adrenal androgen production inhibitor, phosphorylase inhibitor, hormonotherapeutic agent [e.g., estrogenic agent (e.g., DSB, EMP, etc.), antiandrogenic agent (e.g., CMA, etc.), etc.], cell growth factor or a drug inhibiting the activity of receptor thereof.

With respect to breast cancer, the compound of the invention can be used in combination with a drug such as GnRH superagonist, antiestrogenic agent, chemotherapeutic agent [e.g., cyclophosphamide, 5-FU, UFT, methotrexate, Adriamycin, mitomycin C, mitoxantrone, etc.], peptidic GnRH antagonist, aromatase inhibitor, adrenal androgen production inhibitor, phosphorylase inhibitor, hormonotherapeutic agent-[e.g., antiestrogenic agent (e.g., tamoxifen, etc.), luteinizing hormone (e.g., MPA, etc.), androgenic agent, estrogenic agent, etc.], cell growth factor or a drug inhibiting the activity of receptor thereof.

The administration mode of the compound of the present invention and the concomitant drug is not particularly limited as long as the compound of the present invention and the concomitant drug are combined in administration. Examples of such administration mode include the following methods:
(1) administration of a single preparation obtained by simultaneously processing the compound of the present invention and the concomitant drug, (2) simultaneous administration of two kinds of preparations of the compound of the present invention and the concomitant drug, which have been separately produced, by the same administration route, (3) administration of two kinds of preparations of the compound of the present invention and the concomitant drug, which have been separately produced, by the same administration route in a staggered manner, (4) simultaneous administration of two kinds of preparations of the compound of the present invention and the concomitant drug, which have been separately produced, by different administration routes, (5) administration of two kinds of preparations of the compound of the present invention and the concomitant drug, which have been separately produced, by different administration routes in a staggered manner (e.g., administration in the order of the compound of the present invention and the concomitant drug, or in the reverse order) and the like.

When the compound of the invention is used as a prophylactic and/or therapeutic agent for the above-described diseases, or in the field of stockbreeding or fishery, the compound of the invention can be administered both orally or parenterally by a method known *per se*, and the compound can be orally administered after being mixed with a pharmaceutically acceptable carrier, usually as a solid preparation such as a tablet, a capsule, a granule or a powder, or can be parenterally administered as an intravenous, subcutaneous, or intramuscular injectable preparation, suppository, sublingual tablet or the like. The compound can be also administered sublingually, subcutaneously, intramuscularly and the like as a sustained release preparation such as a sublingual tablet, a microcapsule or the like. The daily dose may vary depending on the severity of symptom; the age, the gender, the body weight, the sensitivity of the subject of administration; the administration time and interval, properties, prescription or kind of the pharmaceutical preparation; the type of the active ingredient or the like. The daily dose is not particularly limited as long as the compound achieves the object of the invention. However, when the compound is used in the treatment of the above-described sex hormone-dependent cancers (e.g., prostate cancer, uterine cancer, breast cancer, pituitary tumor, etc.), prostatomegaly, hysteromyoma, endometriosis, precocious puberty or the like, the active ingredient (the compound of the invention) is administered as an oral preparation, usually in an amount of about 0.01 to 500 mg, preferably about 0.02 to 200 mg, more preferably 0.1 to 50 mg, and most preferably 0.1 to 10 mg, relative to 1 kg of body weight of a mammal, usually administered in 1 to 4 portions a day.

The dosage in the case of using the compound of the invention in the field of stockbreeding or fishery is also equivalent to the above-described range, but the active ingredient (the compound of the invention) is usually administered as an oral preparation, in an amount of about 0.01 to 500 mg, preferably about 0.1 to 200 mg, relative to 1 kg of body weight of the subject organism of administration, usually administered in 1 to 3 portions a day.

The content of Compound (I) in the pharmaceutical composition of the invention is about 0.01 to 100% by weight of the total composition.

The pharmaceutically acceptable carriers that can be used may be exemplified by various organic or inorganic carrier substances that are conventionally used as the materials for preparation, and are mixed as excipient, glidant, binding agent, disintegrant and the like in solid preparations, and as solvent, dissolution aid, suspending agent, isotonic agent, buffer, soothing agent and the like in liquid preparations. Furthermore, if necessary, preparation additives such as antiseptic agent, antioxidant, colorant and sweetener can be also used.

As preferable examples of the aforementioned excipient, lactose, sucrose, D-mannitol, starch, corn starch, crystalline cellulose, light anhydrous silicic acid and the like can be mentioned. As preferable examples of the aforementioned lubricant, for example, magnesium stearate, calcium stearate, talc, colloidal silica and the like can be mentioned. As preferable examples of the aforementioned binder, crystalline cellulose, sucrose, D-mannitol, dextrin, hydroxypropylcellulose, hydroxypropylmethylcellulose, polyvinylpyrrolidone and the like can be mentioned. As preferable examples of the aforementioned disintegrant, for example, starch, carboxymethylcellulose, carboxymethylcellulose calcium, carboxymethylstarch sodium, croscarmellose sodium and the like can be mentioned. As preferable examples of the aforementioned solvent, for example, water for injection, alcohol, propylene glycol, macrogol, sesame oil, corn oil and the like can be mentioned. As preferable examples of the aforementioned solubilizing agent s, for example, polyethylene glycol, propylene glycol, D-mannitol, benzyl benzoate, ethanol, trisaminomethane, cholesterol, triethanolamine, sodium carbonate, sodium citrate and the like can be mentioned. As preferable examples of the aforementioned suspending agent, for example, surfactants such as stearyltriethanolamine, sodium lauryl sulfate, lauryl aminopropionate, lecithin, benzalkonium chloride, benzethonium chloride, glycerol monostearate and the like; hydrophilic polymers such as polyvinyl alcohol, polyvinylpyrrolidone, carboxymethylcellulose sodium, methylcellulose, hydroxymethylcellulose, hydroxyethylcellulose, hydroxypropylcellulose and the like, and the like can be mentioned. As preferable examples of the aforementioned isotonicity agent, for example, glucose, D-sorbitol, sodium chloride, glycerin, D-mannitol and the like can be mentioned. As preferable examples of the aforementioned buffer, for example, buffers such as phosphate, acetate, carbonate, citrate and the like, and the like can be mentioned. As preferable examples of the soothing agent, for example, benzyl alcohol and the like can be mentioned. As preferable examples of the aforementioned preservative, p-oxybenzoates, chlorobutanol, benzyl alcohol, phenethyl alcohol, dehydroacetate, sorbic acid and the like can be mentioned. As preferable examples of the aforementioned antioxidant, sulfite, ascorbic acid and the like can be mentioned.

An intravenous, subcutaneous or intramuscular injectable preparation can be produced by adding a suspending agent, a dissolving aid, a stabilizer, an isotonic agent, a preservative or the like to the compound of the invention, according to a method known *per se*. Here, if necessary, the preparation can be produced as a lyophilization product by a method known *per se*. When the compound of the invention is administered to human, for example, the compound can be safely administered orally or parenterally, for example, as it is or as a pharmaceutical composition in which the compound of the invention is mixed with an appropriate pharmaceutically acceptable carrier, excipient or diluent.

The pharmaceutical composition may be exemplified by an oral preparation (e.g., powder, granule, capsule, tablet), or a parenteral preparation [e.g., injectable preparation, dip infusion, external agent (e.g., intranasal preparation, transdermal preparation, etc.), suppository (e.g., rectal suppository, vaginal suppository, etc.), etc.].

These preparations can be produced by methods known *per se* that are generally used in the processes for producing preparations.

The compound of the invention can be produced into an injectable preparation by formulating the compound into aqueous injectable preparations together with dispersants (e.g., Tween 80 (Atlas Powder Co., US), HCO60 (Nikko Chemicals Co., Ltd.), polyethylene glycol, carboxymethylcellulose, sodium alginate, etc.), preservatives (e.g., methylparaben, propylparaben, benzyl alcohol, etc.), isotonic agents (e.g., sodium chloride, mannitol, sorbitol, glucose, etc.) and the like, or into an oil-based injectable preparation by dissolving, suspending or emulsifying the compound in plant oils such as olive oil, sesame oil, cotton seed oil or corn oil, propylene glycol or the like.

To obtain a preparation for oral administration, an excipient (e.g., lactose, sucrose, starch and the like), a disintegrating agent (e.g., starch, calcium carbonate and the like), a binder (e.g., starch, gum Arabic, carboxymethylcellulose, polyvinylpyrrolidone, hydroxpropylcellulose and the like), a lubricant (e.g., talc, magnesium stearate, polyethylene glycol 6000 and the like) and the like, for example, can be added to the compound of the present invention, according to a method known *per se*, and the mixture can be compression-molded, then if desirable, the molder product can be coated by a method known *per se* for the purpose of masking of taste, enteric property or durability. As this coating agent, for example, hydroxypropylmethylcellulose, ethylcellulose, hydroxymethylcellulose, hydroxypropylcellulose, polyoxyethylene glycol, Tween 80, Pluronic F68, cellulose acetate phthalate, hydroxypropylmethylcellulose phthalate, hydroxymethylcellulose acetate succinate, Eudoragit (methacrylic acid·acrylic acid copolymer, manufactured by Rohm, DE), pigment (e.g., iron oxide red, titanium dioxide, etc.) and the like can be used. In order to use Compound (I) of the invention as enteric preparations, an intermediate phase can be provided in between an enteric phase and a drug-containing phase of the preparation by a method known *per se*, for the purpose of separation of the enteric phase and the drug-containing phase.

In order to use the compound of the invention as external preparations, the compound or a salt thereof can be formulated into solid, semi-solid or liquid externally administered preparations by methods known *per se*. For the solid preparation for example, the compound of the invention or a salt thereof is directly used or mixed with excipients (e.g., glycol, mannitol, starch, microcrystalline cellulose, etc.), thickening agents (e.g., natural gums, cellulose derivatives, acrylic acid polymers, etc.) and the like, to be produced into a pulverized composition. For the liquid preparation, an oil-based preparation or an aqueous suspension is produced, in the virtually same manner as in the preparation of injectable preparations. For the semi-solid preparation, aqueous or oil-based gel preparations, or ointments are preferable. All of these preparations may also have pH adjusting agents (e.g., carbonate, phosphate, citrate, chloride, hydroxide of sodium, etc.), antiseptic agents (e.g., paraoxybenzoic acid esters, chlorobutanol, benzalkonium chloride, etc.) and the like added therein.

Moreover, the compound of the present invention can be made into an oily or aqueous solid, semisolid or liquid suppository according to a method known *per se*. As the oily substrate to be used for the above-mentioned composition, for example, glycerides of higher fatty acids [e.g., cacao butter, Witepsols (manufactured by Dynamit Nobel, Germany), etc.], glycerides of medium chain fatty acid [e.g., Miglyols (manufactured by Dynamit Nobel, Germany), etc.], or vegetable oils (e.g., sesame oil, soybean oil, cotton seed oil and the like), and the like are listed. Further, as the aqueous substrate, for example, polyethylene glycols, propylene glycol are listed, and as the aqueous gel substrate, for example, natural gums, cellulose derivatives, vinyl polymers, acrylic acid polymers and the like are listed.

### Example

The present invention is further described in detail in with reference to Reference Examples, Examples, Preparation Examples and Experimental Examples which are not intended to restrict the invention.

¹H-NMR spectra were measured with a Varian GEMINI 200 (200 MHz) spectrometer, a MERCURY 300 (300 MHz) spectrometer or a BRUKER AVANCE 300 spectrometer (300 MHz) using tetramethylsilane as an internal standard. All of the δ values are represented in ppm.

Preparative HPLC was measured under the following conditions.

Instrument: LC-10Avp, Shimadzu Corporation.

Column: Capcell Pak C18UG 120, S-3 µm, 2.0 × 50 mm

Solvent: Solution A; 0.1% trifluoroacetic acid-containing water, Solution B; 0.1% trifluoroacetic acid-containing acetonitrile

Gradient cycle: 0.00 minute (Solution A/Solution B = 90/10), 4.00 minutes (Solution A/Solution B = 5/95), 5.50 minutes (Solution A/Solution B = 5/95), 5.51 minutes (Solution A/Solution B = 90/10), 8.00 minutes (Solution A/Solution B = 90/10)

Flow rate: 0.5 ml/min

The number indicated in the mixed solvent means a mixing ratio by volume of each solvent unless otherwise stated. Further, the eluting solvent in silica gel chromatography means a ratio by volume unless otherwise stated. "%" means weight% unless otherwise stated. Provided that, yield means mol/mol%. Other symbols used in the present text indicate the following meanings.
s: Singlet
d: Doublet
t: Triplet
q: Quartet
dd: Double doublet
dt: Double triplet
td: Triple doublet
dq: Double quartet
ddd: Double double doublet
m: Multiplet
br: Broad
Hz: Hertz
CDCl₃: deutero chloroform
DMSO-d₆: deutero dimethylsulfoxide
CD₃OD: deutero methanol
AIBN: 2,2-azobisisobutyronitrile
DMF: N,N-dimethylformamide
NBS: N-bromosuccinimide
TFA: trifluoroacetic acid
THF: tetrahydrofuran
DMAP: 4-N,N-dimethylaminopyridine
Me: Methyl
Et: Ethyl
Ph: Phenyl
TMS: Trimethylsilyl
TBDMS: tert-Butyl(dimethyl)silyl
Cbz: Benzyloxycarbonyl

Room temperature represents a temperature of about 15°C to about 30°C, but is not particularly exactly limited.

As for corn starch, magnesium stearate, lactose and distilled water for injection, which are used in Preparation Examples, products in conformity to the 14^{th} revision of the Japanese Pharmacopoeia were used.

### Reference Example 1

### 1-[(4-Chloro-3-nitrophenyl)sulfonyl]-1,2,3,4-tetrahydroquinoline

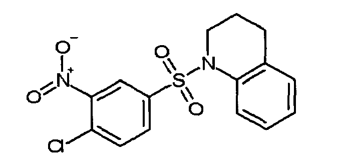

A solution of 4-chloro-3-nitrobenzenesulfonyl chloride (17.0 g) in THF (50 ml) was added to 1,2,3,4-tetrahydroquinoline (9.74 g) and sodium hydrogencarbonate (8.38 g) with stirring in THF (150 ml) and water (15 ml) at room temperature, and the mixture was further stirred overnight. The reaction mixture was diluted with ethyl acetate, washed with water, and dried over anhydrous magnesium sulfate. The solvent was evaporated under reduced pressure. The obtained crystals were washed with diisopropyl ether to give the object (15.7 g) as crystals.
¹H-NMR (CDCl₃) δ 1.66-1.78 (2H, m), 2.49 (2H, t), 3.85 (2H, t), 7.03-7.07 (1H, m), 7.14 (1H, dt), 7.23-7.27 (1H, m), 7.57 (1H, d), 7.63 (1H, dd), 7.74-7.78 (1H, m), 8.08 (1H, d).

### Reference Example 1 (1)

### 1-[(3-Nitrophenyl)sulfonyl]-1,2,3,4-tetrahydroquinoline

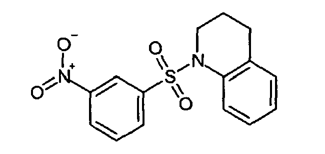

In the same manner as in Reference Example 1, the title compound was obtained using 1,2,3,4-tetrahydroquinoline and 3-nitrobenzenesulfonyl chloride.
¹H-NMR (CDCl₃) δ 1.63-1.77 (2H, m), 2.44 (2H, t), 3.87 (2H, t), 7.00-7.27 (3H, m), 7.60 (1H, t), 7.76-7.88 (2H, m), 8.37 (1H, ddd), 8.47 (1H, t).

### Reference Example 2

### 4-[(5-Chloro-4-nitro-2-thienyl)sulfonyl]-5,6,7,8-tetrahydro-4H-thieno[3,2-b]azepine

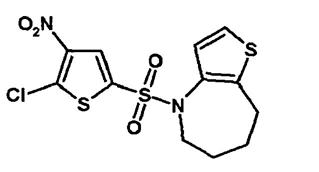

5,6,7,8-Tetrahydro-4H-thieno[3,2-b]azepin (2.09 g), triethylamine (2.09 ml) and DMAP (0.17 g) were dissolved in THF (30 ml)-acetonitrile (5 ml). A solution of 5-chloro-4-nitrothiophene-2-sulfonyl chloride (3.57 g) in THF (20 ml) was added dropwise under ice-cooling, and the mixture was stirred at 0°C for 2 hr. The reaction mixture was poured into water, and the mixture was extracted twice with ethyl acetate, and dried over anhydrous magnesium sulfate. The solvent was evaporated under reduced pressure. The obtained residue was purified by silica gel column chromatography (hexane-ethyl acetate (9:1 - 6:1)), and crystallized from diethyl ether to give the object (1.74 g) as crystals.
¹H-NMR (CDCl₃) δ 1.58-1.66 (2H, m), 1.85-1.93 (2H, m), 2.52 (2H, t), 3.77 (2H, br s), 6.99 (1H, d), 7.03 (1H, d), 7.84 (1H, s).

### Reference Example 3

### 2-Chloro-5-(3,4-dihydro-1(2H)-quinolinylsulfonyl)aniline

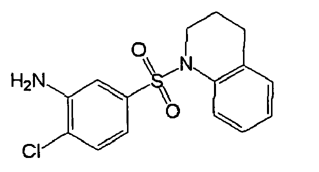

To a mixture of 1-[(4-chloro-3-nitrophenyl)sulfonyl]-1,2,3,4-tetrahydroquinoline (15.7 g), nickel(II) bromide (0.49 g), methanol (100 ml) and THF (100 ml), sodium borohydride (5.05 g) was slowly added under water-cooling, and the mixture was stirred at room temperature for 0.5 hr. The reaction mixture was poured into water and the mixture was extracted twice with ethyl acetate. The collected organic layer was dried over anhydrous magnesium sulfate, and the solvent was evaporated under reduced pressure. The obtained residue was purified by silica gel column chromatography (hexane-ethyl acetate (3:1)), and crystallized from hexane to give the object (11.9 g) as crystals.
¹H-NMR (CDCl₃) δ 1.63-1.71 (2H, m), 2.48 (2H, t), 3.76-3.80 (2H, m), 4.19 (2H, br s), 6.84 (1H, dd), 6.95 (1H, d), 7.00-7.22 (4H, m), 7.74 (1H, d).

### Reference Example 3 (1)

### 3-(3,4-Dihydro-1(2H)-quinolinylsulfonyl)aniline

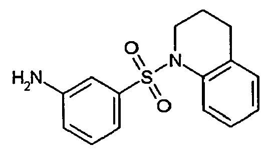

In the same manner as in Reference Example 3, the title compound was obtained using 1-[(3-nitrophenyl)sulfonyl]-1,2,3,4-tetrahydroquinoline.
¹H-NMR (CDCl₃) δ 1.67 (2H, quintet), 2.48 (2H, t), 3.79 (2H, t), 3.79 (2H, s), 6.75-7.22 (7H, m), 7.76 (1H, d).

### Reference Example 4

### N-Ethyl-5-nitro-N-phenylthiophene-3-carboxamide

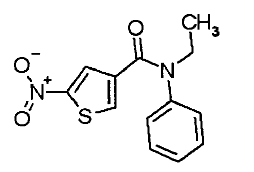

To a solution 5-nitrothiophene-3-carboxylic acid (2.5 g) in THF (40 ml), a drop of DMF was added, and then thionyl chloride (1.37 ml) was added. The mixture was stirred under nitrogen atmosphere at 60°C for 1 hr, allowed to cool to room temperature, and added dropwise to a solution N-ethylaniline (2.18 ml), pyridine (11.7 ml) and DMAP (5 mg) in THF (60 ml) under nitrogen atmosphere at 0°C. The mixture was warmed to room temperature, and stirred overnight. Water was added, and the mixture was extracted with ethyl acetate. The organic layer was washed with 1N hydrochlolic acid, saturated aqueous sodium hydrogencarbonate solution and saturated brine, and the solvent was evaporated under reduced pressure. The obtained residue was purified by silica gel column chromatography (hexane-ethyl acetate (5:1 - 1:1)) to give the object (3.9 g) as an oily substance.
¹H-NMR (CDCl₃) δ 1.22 (3H, t), 3.94 (2H, q), 7.11-7.19 (2H, m), 7.34-7.45 (4H, m), 7.52 (1H, d).

### Reference Example 5

### 5-Amino-N-ethyl-N-phenylthiaphene-3-carboxamide

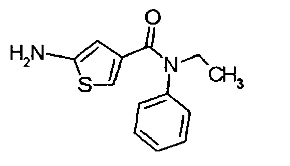

To N-ethyl-5-nitro-N-phenylthiophene-3-carboxamide (3.8 g) and ammonium chloride (3.68 g), methanol (100 ml) was added, and then zinc (13.5 g) was slowly added at 60°C. The mixture was stirred as it was for 10 min, and allowed to cool to room temperature. The insoluble material was removed by filtration. The filtrate was concentrated under reduced pressure, and purified by silica gel column chromatography (hexane-ethyl acetate (5:1 - 1:1)) to give the object (1.44 g) as an oily substance.
¹H-NMR (CDCl₃) δ 1.19 (3H, t), 3.58 (2H, br s), 3.90 (2H, q), 6.05 (1H, d), 6.29 (1H, d), 7.10-7.15 (2H, m), 7.27-7.43 (3H, m).

### Reference Example 5 (1)

### 2-chloro-5-(5,6,7,8-tetrahydro-4H-thieno[3,2-b]azepin-4-ylsulfonyl)thiophene-3-amine

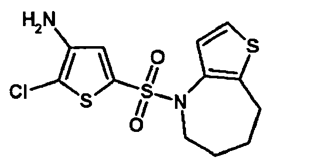

In the same manner as in Reference Example 5, the title compound was obtained using 4-[(5-chloro-4-nitro-2-thienyl)sulfonyl]-5,6,7,8-tetrahydro-4H-thieno[3,2-b]azepine.
¹H-NMR (CDCl₃) δ 1.58 (2H, ddd), 1.83-1.91 (2H, m), 2.52 (2H, t), 3.68 (2H, br s), 3.73 (2H, br s), 6.86 (1H, s), 6.93 (1H, d), 7.04 (1H, d).

### Reference Example 6

### 4-(3,4-Dihydroquinolin-1(2H)-ylsulfonyl)thiophene-2-amine

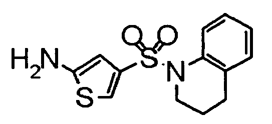

1) A half volume of a solution of commercially available 2-nitrothiophene (containg about 15 % of 3-nitrothiophene) (50.1 g) in chloroform (100 ml) was added dropwise to a solution of chlorosulfonic acid (113 g) in chloroform (100 ml) under reflux. Additional chlorosulfate (113 g) was added at once, the rest of the solution of 2-nitrothiophene in chloroform was added dropwise, and the mixture was stirred at 60°C overnight. The reaction mixture was poured into water, and the chloroform layer was separated. The aqueous layer was extracted twice with ethyl acetate. The collected organic layer was dried over anhydrous magnesium sulfate. The solvent was evaporated under reduced pressure to give an isomer mixture of nitrothiophenesulfonyl chloride (88.6 g) as an oily substance.
2) To a solution of 1,2,3,4-tetrahydroquinoline (11.7 g) in acetonitrile (50 ml), a solution of the oily substance (13.4 g) obtained in 1) in acetonitrile (50 ml) was added at room temperature, and the mixture was further stirred overnight. The reaction mixture was poured into water, and extracted twice with ethyl acetate. The collected organic layer was dried over anhydrous magnesium sulfate, and the solvent was evaporated under reduced pressure. The obtained residue was purified by silica gel column chromatography (hexane-ethyl acetate (9:1 - 3:1)) to give crystals (7.75 g).
3) To a mixture of crystals (7.06 g) obtained in 2), ammonium chloride (5.82 g) and methanol (200 ml), zinc (28.5 g) was added at 60°C in several portions, and the mixture was stirred at 60°C for 30 min. The reaction mixture was filtrated to remove the insoluble material, and the filtrate was evaporated under reduced pressure. The obtained residue was purified by silica gel column chromatography (hexane-ethyl acetate (3:1 - 2:1)) to give an isomeric mixture of the object (1.5 g) as crystals. Furthermore, the isomer was separated by NH-silica gel column chromatography (hexane-ethyl acetate (1:1 - 1:1)) to give the object (0.94 g) as crystals.
   ¹H-NMR (CDCl₃) δ 1.71-1.79 (2H, m), 2.59 (2H, t), 3.77-3.81 (2H, m), 3.84 (2H, br s), 5.96 (1H, d), 6.98 (1H, d), 7.03-7.11 (2H, m), 7.15-7.20 (1H, m), 7.75 (1H, ddd).

### Reference Example 7

### 3-Amino-N-[3-(3,4-dihydroquinolin-1(2H)-ylsulfonyl)phenyl]thiophene-2-carboxamide

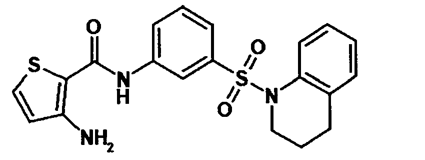

3-(3,4-dihydro-1(2H)-quinolinylsulfonyl)aniline (121 mg), methyl 3-amino-2-thiophenecarboxylate (132 mg) and potassium t-butoxide (96 mg) were mixed, and the mixture was irradiated by microwave in a sealed container using Personal Chemistry Inc. microwave reaction instrument Smith Synthesizer at 140°C for 10min. The same reaction was repeated twice, and the obtained three reaction mixtures were combined, and the mixture was separated with water-ethyl acetate, and extracted with ethyl acetate. The organic layer was washed with saturated brine, and dried over anhydrous magnesium sulfate, and the solvent was evaporated under reduced pressure. The residue was purified by silica gel column chromatography (20-55% ethyl acetate/hexane) to give the object (50.2 mg).
¹H-NMR (CDCl₃) δ 1.70 (2H, ddd), 2.47 (2H, t), 3.84 (2H, t), 5.72 (2H, br s), 6.60 (1H, d), 7.02 (1H, d), 7.08 (1H, td), 7.18 (2H, d), 7.22-7.26 (2H, m), 7.35 (1H, t), 7.70 (1H, t), 7.77 (1H, d), 7.93 (1H, ddd).

### Reference Example 8

### Methyl 2-[({[3-(3,4-dihydroquinolin-1(2H)-ylsulfonyl)phenyl]amino}carbonyl)amino]thiophene-3-carboxylate

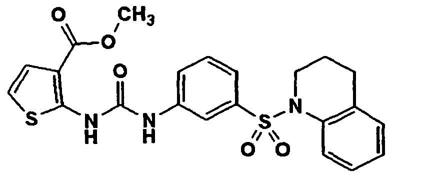

Triphosgene (47.9 mg) was dissolved in dichloromethane (2.7 ml), and a solution of methyl 2-aminothiophene-3-carboxylate (70.4 mg), triethylamine (138 mg) in dichloromethane (1.8 ml) was added dropwise with stirring under nitrogen atmosphere at -78°C. The mixture was warmed to room temperature over 30 min, and stirred at room temperature for 30 min. The reaction mixture was concentrated under reduced pressure. The residue was diluted with dichloromethane (2.7 ml) again. A solution of 3-(3,4-dihydro-1(2H)-quinolinylsulfonyl)aniline (115.6 mg) and triethylamine (66 mg) in dichloromethane (1.6 ml) was added dropwise, and the mixture was stirred at room temperature for 21 hr. The solvent was evaporated under reduced pressure, and the residue was purified by silica gel column chromatography (20-60% ethyl acetate/hexane) to give the object (92.2 mg).
¹H-NMR (DMSO-d₆) δ 1.65 (2H, ddd), 2.47 (2H, t), 3.78 (2H, t), 3.85 (3H, s), 6.93 (1H, d), 7.08 (2H, d), 7.14 (1H, d), 7.16-7.22 (2H, m), 7.47 (1H, t), 7.59 (1H, d), 7.69 (1H, dd), 7.93 (1H, t), 10.47 (1H, br s), 10.61 (1H, br).

### Reference Example 9

### Dimethyl 4-[({[3-(3,4-dihydroquinolin-1(2H)-ylsulfonyl)phenyl]amino}carbonyl)amino]thiophene-2,3-dicarboxylate

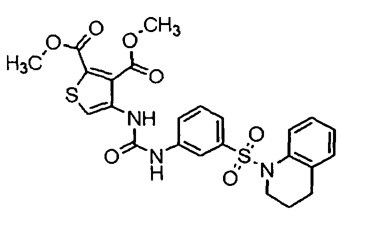

To a 0.75 N solution of dimethyl 4-aminothiophene-2,3-dicarboxylate hydrochloride in dichloromethane (1.6 ml), a 3.0 N solution of triethylamine in dichloromethane (1.6 ml) was added, and then a 0.17 N solution of triphosgene in dichloromethane (1.6 ml) was added dropwise with stirring under nitrogen atmosphere at -78°C. The cold bath was removed, and the mixture was stirred for additional 50 min. The reaction mixture was concentrated under reduced pressure, and the residue was diluted with THF (4.0 ml). A solution (1.6 ml) of 3-(3,4-dihydro-1(2H)-quinolinylsulfonyl)aniline (0.25N) and DMAP (0.375N) in THF was added dropwise, and the mixture was stirred at room temperature for 25 hr. Insoluble triethylamine hydrochloride was removed by filtration, and the solvent was evaporated under reduced pressure. The residue was purified by silica gel column chromatography (22-55% ethyl acetate/hexane) to give the object (107 mg).
MS (ESI+, m/e) 530 (M+1)

### Reference Example 9 (1) - Reference Example 9 (10)

In the same manner as in Reference Example 9, the following compounds were obtained using commercially available esters of heterocyclic carboxylic acids having amino groups; (ethyl 5-amino-1-methylpyrazole-4-carboxylate, methyl 3-amino-4-methylthiophene-2-carboxylate, ethyl 5-amino-1-(4-fluorophenyl)-1H-pyrazole-4-carboxylate, methyl 3-amino-5-(4-fluorophenyl)thiophene-2-carboxylate, methyl 4-aminothiophene-3-carboxylate, methyl 3-amino-5-(tert-butyl)thiophene-2-carboxylate, methyl 4-amino-2-(morpholin-4-yl)-1,3-thiazole-5-carboxylate, ethyl 3-amino-4-methoxythieno[2,3-b]pyridine-2-carboxylate, methyl 4-amino-1,2,5-thiadiazole-3-carboxylate or diethyl 5-amino-3-methyl-2,4-thiophenedicarboxylate) and 3-(3,4-dihydro-1(2H)-quinolinylsulfonyl)aniline.

### Reference Example 9 (1)

### Ethyl 5-[({[3-(3,4-Dihydroquinolin-1(2H)-ylsulfonyl)phenyl]amino}carbonyl)amino]-1-methyl-1H-pyrazole-4-carboxylate

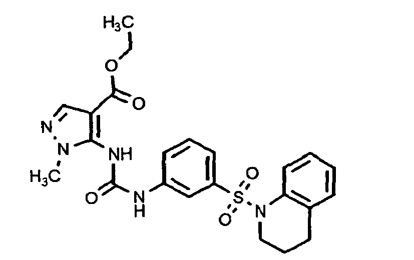

MS (ESI+, m/e) 484 (M+1)

### Reference Example 9 (2)

### Methyl 3-[({[3-(3,4-Dihydroquinolin-1(2H)-ylsulfonyl)phenyl]amino}carbonyl)amino]-4-methylthiophene-2-carboxylate

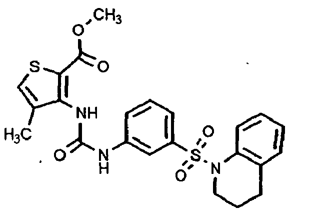

MS (ESI+, m/e) 486 (M+1)

### Reference Example 9 (3)

### Ethyl 5-[({[3-(3,4-dihydroquinolin-1(2H)-ylsulfonyl)phenyl]amino}carbonyl)amino]-1-(4-fluorophenyl)-1H-pyrazole-4-carboxylate

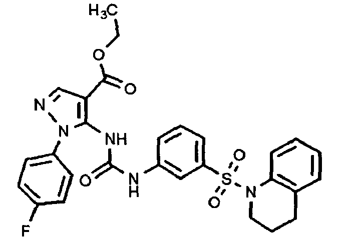

MS (ESI+, m/e) 564 (M+1)

### Reference Example 9 (4)

### Methyl 3-[({[3-(3,4-dihydroquinolin-1(2H)-ylsulfonyl)phenyl]amino}carbonyl)amino]-5-(4-fluorophenyl)thiophene-2-carboxylate

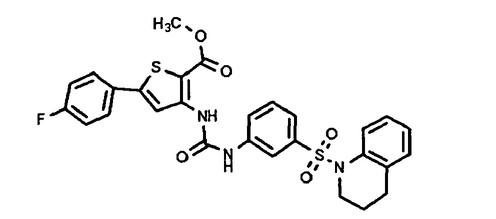

MS (ESI+, m/e) 566 (M+1)

### Reference Example 9 (5)

### Methyl 4-[({[3-(3,4-dihydroquinolin-1(2H)-ylsulfonyl)phenyl]amino}carbonyl)amino]thiophene-3-carboxylate

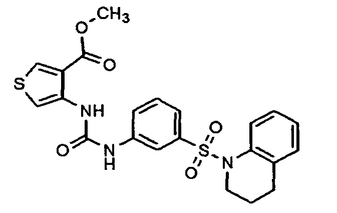

MS (ESI+, m/e) 472 (M+1)

### Reference Example 9 (6)

### Methyl 5-tert-butyl-3-[({[3-(3,4-dihydroquinolin-1(2H)-ylsulfonyl)phenyl]amino}carbonyl)amino]thiophene-2-carboxylate

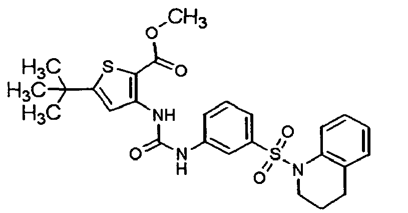

MS (ESI+, m/e) 528 (M+1)

### Reference Example 9 (7)

### Methyl 4-[({[3-(3,4-dihydroquinolin-1(2H)-ylsulfonyl)phenyl]amino}carbonyl)amino]-2-morpholin-4-yl-1,3-thiazole-5-carboxylate

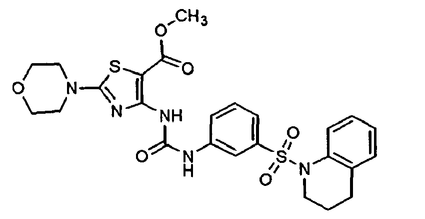

MS (ESI+, m/e) 558 (M+1)

### Reference Example 9 (8)

### Ethyl 3-[({[3-(3,4-dihydroquinolin-1(2H)-ylsulfonyl)phenyl]amino}carbonyl)amino]-4-methoxythieno[2,3-b]pyridine-2-carboxylate

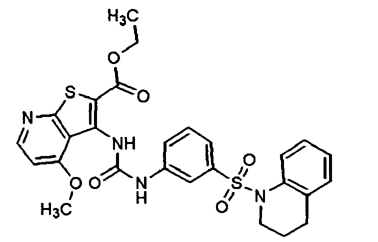

MS (ESI+, m/e) 567 (M+1)

### Reference Example 9 (9)

### Methyl 4-[({[3-(3,4-dihydroquinolin-1(2H)-ylsulfonyl)phenyl]amino}carbonyl)amino]-1,2,5-thiadiazole-3-carboxylate

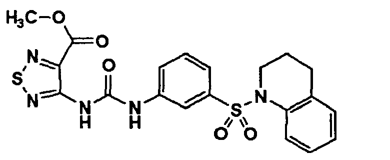

¹H-NMR (DMSO-d₆) δ 1.66 (2H, dt), 2.48 (2H, t), 3.78 (2H, t), 3.91 (3H, s), 7.06-7.10 (2H, m), 7.13-7.21 (2H, m), 7.47 (1H, t), 7.58 (1H, d), 7.69 (1H, ddd), 7.97 (1H, t), 9.82 (1H, br s), 10.17 (1H, br s).

### Reference Example 9 (10)

### Diethyl 5-[({[3-(3,4-dihydroquinolin-1(2H)-ylsulfonyl)phenyl]amino}carbonyl)amino]-3-methylthiophene-2,4-dicarboxylate

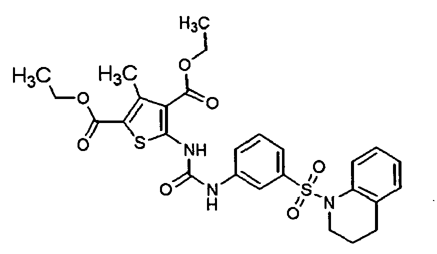

MS (ESI+, m/e) 572 (M+1)

### Reference Example 10

### tert-Butyl (2-chloro-3-nitrophenyl)carbamate

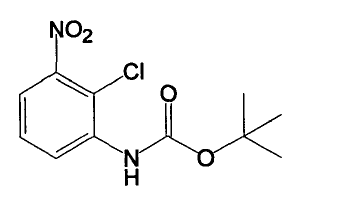

To a solution of 2-chloro-3-nitrobenzoic acid (49.5 g) in tert-butanol (700 ml), diphenylphosphoryl azide (68.8 ml) and triethylamine (49.6 ml) were added at room temperature, and the mixture was stirred at 90°C overnight. The reaction mixture was allowed to cool to room temperature, and concentrated under reduced pressure. Water was added to the obtained residue, and the mixture was extracted with ethyl acetate. The organic layer was washed with saturated brine, and dried over anhydrous magnesium sulfate, and the solvent was evaporated under reduced pressure. The residue was purified by silica gel column chromatography to give the object (66.6 g) as an oily substance. ¹H-NMR (CDCl₃) δ1.55 (9H, s), 7.17-7.30 (1H, m), 7.38 (1H, t), 7.48 (1H, dd), 8.47 (1H, dd).

### Reference Example 11

### 2-Chloro-3-nitroaniline

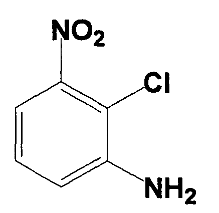

To a solution of tert-butyl (2-chloro-3-nitrophenyl)carbamate (66.0 g) in ethanol (500 ml), 48% aqueous hydrobromic acid solution (109 ml) was added, and the mixture was stirred at room temperature for 30 min. The reaction mixture was concentrated under reduced pressure, and the obtained residue was washed with ethyl acetate to give 2-chloro-3-nitroaniline hydrobromide (48.5 g) as crystals. Aqueous sodium hydrogen carbonate solution was added to 2-chloro-3-nitroaniline hydrobromide (16.0 g), and the mixture was neutralized, and extracted with ethyl acetate. The organic layer was washed with saturated brine, and dried over anhydrous sodium sulfate, and the solvent was evaporated under reduced pressure. The obtained residue was washed with hexane to give the object (10.8 g) as crystals.
¹H-NMR (CDCl₃) δ4.42 (2H, br s), 6.90 - 6.98 (1H, m), 7.13-7.20 (2H, m).

### Reference Example 12

### 1-Bromo-2-chloro-3-nitrobenzene

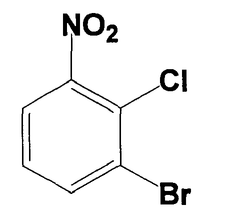

To a solution of copper (II) bromide (7.77 g) in acetonitrile (100 ml), tert-butyl nitrite (5.17 ml) was added at room temperature. Then, a solution of 2-chloro-3-nitroaniline (5.00 g) in acetonitrile (50 ml) was added dropwise at 65°C, and the mixture was further stirred for 30 min, and allowed to cool to room temperature. Aqueous sodium hydrogen carbonate solution was added, and the mixture was extracted with ethyl acetate. The organic layer was washed with aqueous thiosodium sulfate solution and saturated brine, and dried over anhydrous magnesium sulfate. The solvent was evaporated under reduced pressure. The obtained residue was purified by silica gel column chromatography to give the object (6.85 g) as an oily substance. ¹H-NMR (CDCl₃) δ7.30 (1H, t), 7.73 (1H, dd), 7.86 (1H, dd).

### Reference Example 13

### 3-Bromo-2-chloroaniline

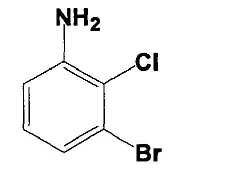

To a solution of 1-bromo-2-chloro-3-nitrobenzene (6.8 g) in ethanol (150 ml), reduced iron (8.0 g) was added. Concentrated hydrochlolic acid (36 ml) was added to the mixture, and the mixture was stirred at 60°C for 30 min, and allowed to cool to room temperature. Aqueous sodium hydrogen carbonate solution was added, and the mixture was neutralized, and extracted with ethyl acetate. The organic layer was washed with saturated brine, and dried over anhydrous magnesium sulfate. The solvent was evaporated under reduced pressure. The obtained residue was purified by silica gel column chromatography to give the object (5.8 g) as an oily substance.
¹H-NMR (CDCl₃) δ4.20 (2H, br s), 6.70 (1H, dd), 6.91 (1H, t), 7.01 (1H, dd).

### Reference Example 14

### 3-Bromo-2-chloro-N-cyclopropylaniline hydrochloride

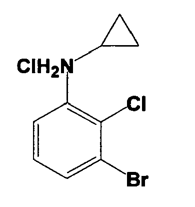

To a solution of 3-bromo-2-chloroaniline in methanol (25 ml)-acetic acid (5.4 ml), [(1-ethoxycyclopropyl)oxy]trimethylsilane was added dropwise at room temperature, and the mixture was stirred at 68°C for 3 hr. The reaction mixture was allowed to cool to room temperature, and concentrated under reduced pressure (residue A). To a suspension of sodium borohydride (1.8 g) in THF(25 ml), boron trifluoride diethyl ether complex (6.0 ml) was added dropwise under nitrogen atmosphere at 0°C, and the mixture was further stirred for 1 hr. A solution of residue A in THF (15 ml) was added dropwise to the mixture at 0°C, and the mixture was stirred at room temperature for 3 hr, and at 60°C overnight. Water was added to the reaction mixture at 0°C, and the mixture was extracted with ethyl acetate. The organic layer was washed with saturated brine, and dried over anhydrous magnesium sulfate. The solvent was evaporated under reduced pressure. The obtained residue was purified by silica gel column chromatography. 4N Hydrogen chloride-ethyl acetate (60 ml) was added, and the mixture was stirred for 10 min. The solvent was evaporated under reduced pressure, and the obtained residue was washed with ethyl acetate to give the object (3.46 g) as crystals.
¹H-NMR (DMSO-d₆) δ0.47-0.54 (2H, m), 0.70-0.78 (2H, m), 2.35-2.44 (1H, m), 6.26 (2H, br s), 6.97 (1H, dd), 7.03 (1H, dd), 7.11 (1H, t).

### Reference Example 15

### N-(3-Bromo-2-chlorophenyl)-5-chloro-N-cyclopropyl-4-nitrothiophene-2-carboxamide

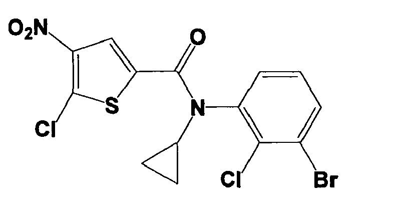

To a solution of 5-chloro-4-nitrothiophene-2-carboxylic acid (19.0 g) in THF (250 ml), DMF (0.5 ml) was added. Then, oxalyl chloride (10.38 ml) was added dropwise at 0°C, and the mixture was stirred at room temperature for 30 min. The solvent was evaporated under reduced pressure. The residue was dissolved in THF (30 ml), and the mixture was added to a solution of 3-bromo-2-chloro-N-cyclopropylaniline hydrochloride (25.9 g) in 1-methyl-2-pyrrolidone (300 ml). The reaction mixture was stirred under nitrogen atmosphere at 60°C overnight. Water was added, and the mixture was extracted with ethyl acetate. The organic layer was washed with saturated brine, and dried over anhydrous magnesium sulfate. The solvent was evaporated under reduced pressure. The obtained residue was purified by silica gel column chromatography to give the object (35.7 g) as an oily substance. ¹H-NMR (CDCl₃) δ0.58-1.06 (4H, m), 3.37 (1H, br s), 6.98 (1H, br s), 7.17-7.34 (2H, m), 7.78 (1H, d).

### Reference Example 16

### 4-Amino-N-(3-bromo-2-chlorophenyl)-5-chloro-N-cyclopropylthiophene-2-carboxamide

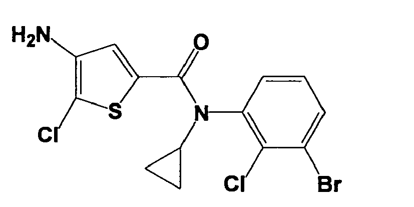

To a solution of N-(3-bromo-2-chlorophenyl)-5-chloro-N-cyclopropyl-4-nitrothiophene-2-carboxamide (35.6 g) in ethanol (400 ml), reduced iron (22.8 g), then concentrated hydrochloric acid (68 ml) were added, and the mixture was stirred at room temperature for 30 min. Aqueous sodium hydrogen carbonate solution was added to the reaction mixture, and the mixture was neutralized, and extracted with ethyl acetate. The organic layer was washed with saturated brine, and dried over anhydrous magnesium sulfate. The solvent was evaporated under reduced pressure. The obtained residue was purified by silica gel column chromatography, and the obtained crystals were washed with hexane to give the object (26.2 g) as crystals.
¹H-NMR (CDCl₃) δ0.52-0.73 (2H, m), 0.78-0.99 (2H, m), 3.28-3.37 (1H, m), 3.57 (2H, br s), 6.79 (1H, s), 7.15 (1H, dd), 7.21 (1H, t), 7.72 (1H, dd).

### Example 1

### 3-[3-(3,4-Dihydroquinolin-1(2H)-ylsulfonyl)phenyl]thieno[3,2-d]pyrimidin-2,4(1H,3H)-dione

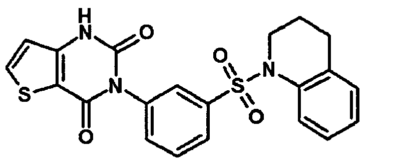

To a solution of 3-amino-N-[3-(3,4-dihydroquinolin-1(2H)-ylsulfonyl)phenyl]thiophene-2-carboxamide (122 mg) in dichloromethane (5.9 ml), triphosgene (185 mg) was added, and the mixture was heated on stirring at 35°C for 7 hr. The reaction mixture was poured into saturated aqueous sodium hydrogen carbonate, and extracted with dichloromethane. The organic layer was washed with saturated aqueous sodium hydrogen carbonate, and dried over anhydrous magnesium sulfate. The solvent was evaporated under reduced pressure. The crystals crystallized from ethyl acetate were collected by filtration, and dried under vacuum to give the object (70 mg).
¹H-NMR (CDCl₃) δ 1.64 (2H, ddd), 2.45 (2H, t), 3.76 (2H, t), 6.99 (1H, d), 7.08 (2H, d), 7.17 (1H, td), 7.50 (1H, dt), 7.57-7.66 (3H, m), 7.76 (1H, br s), 8.13 (1H, d), 12.04 (1H, br s).

### Example 2

### 3-[3-(3,4-Dihydroquinolin-1(2H)-ylsulfonyl)phenyl]thieno[2,3-d]pyrimidin-2,4(1H,3H)-dione

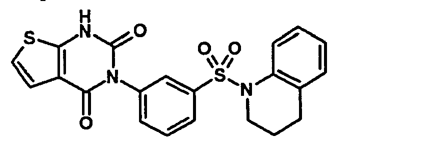

Methyl 2-[({[3-(3,4-dihydroquinolin-1(2H)-ylsulfonyl)phenyl]amino}carbonyl)amino]thiophene-3-carboxylate (77 mg) was dissolved in acetonitrile (3.3 ml). A 0.5N solution (0.84 ml) of sodium methoxide in methanol was added dropwise, and the mixture was heated on stirring at 60°C for 1.5 hr. The reaction mixture was concentrated under reduced pressure. The residue was diluted with dichloromethane, neutralized with 0.5N hydrochlolic acid, saturated aqueous sodium hydrogen carbonate, and extracted with dichloromethane. The organic layer was washed with saturated brine, and dried over anhydrous magnesium sulfate. The solvent was evaporated under reduced pressure. The crystals crystallized from ethyl acetate were collected by filtration, and dried under vacuum to give the object (54 mg).
¹H-NMR (CDCl₃) δ 1.64 (2H, ddd), 2.46 (2H, t), 3.76 (2H, t), 7.07-7.09 (2H, m), 7.14 (1H, d), 7.11-7.22 (1H, m), 7.20 (1H, d), 7.45-7.52 (1H, m), 7.56-7.63 (3H, m), 7.73-7.74 (1H, m), 12.35 (1H, br s).

### Example 3

### 3-[3-(3,4-Dihydroquinolin-1(2H)-ylsulfonyl)phenyl]-2,4-dioxo-1,2,3,4-tetrahydrothieno[3,4-d]pyrimidine-5-carboxylic acid

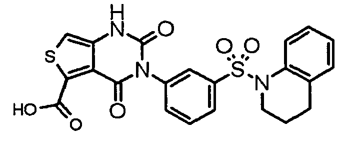

Dimethyl 4-[({[3-(3,4-dihydroquinolin-1(2H)-ylsulfonyl)phenyl]amino}carbonyl)amino]thiophene-2,3-dicarboxylate (107 mg) was dissolved in 20% acetonitrile/methanol (9.1 ml). A solution of sodium methoxide in 0.5N methanol (1.0 ml) was added dropwise, and the mixture was heated on stirring at 60°C for 75 min. The reaction mixture was concentrated under reduced pressure, and the residue was diluted with dichloromethane, neutralized with 50% aqueous ammonium chloride solution, and extracted with dichloromethane. The organic layer was washed with water, and dried over anhydrous magnesium sulfate. The solvent was evaporated under reduced pressure. The crystals crystallized from ethyl acetate was collected by filtration, and dried under vacuum to give the object (63 mg).
¹H-NMR (DMSO-d₆) δ 1.64 (2H, ddd), 2.47 (2H, t), 3.76 (2H, t), 7.08-7.09 (2H, m), 7.15-7.21 (1H, m), 7.37 (1H, s), 7.56-7.59 (2H, m), 7.63-7.70 (2H, m), 7.85 (1H, s), 11.94 (1H, br s), 14.72 (1H, br s).

### Example 3 (1) - Example 3 (9)

In the same manner as in Example 3, the following compounds were obtained using the compounds obtained in Reference Example 9 (1) - Reference Example 9 (9), respectively.

### Example 3 (1)

### 5-[3-(3,4-Dihydroquinolin-1(2H)-ylsulfonyl)phenyl]-1-methyl-1H-pyrazolo[3,4-d]pyrimidine-4,6(5H,7H)-dione

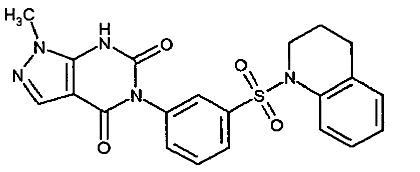

¹H-NMR (DMSO-d₆) δ 1.63 (2H, ddd), 2.46 (2H, t), 3.75 (2H, t), 3.82 (3H, s), 7.05-7.08 (2H, m), 7.15-7.20 (1H, m), 7.48-7.52 (1H, m), 7.55-7.60 (3H, m), 7.66 (1H, m), 7.90 (1H, s), 12.53 (1H, br s).

### Example 3 (2)

### 3-[3-(3,4-Dihydroquinolin-1(2H)-ylsulfonyl)phenyl]-7-methylthieno[3,2-d]pyrimidin-2,4(1H,3H)-dione

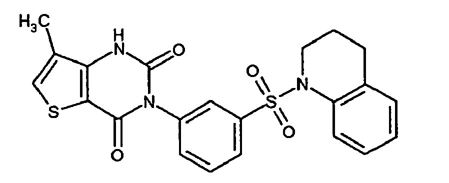

¹H-NMR (DMSO-d₆) δ 1.61-1.69 (2H, m), 2.25 (3H, s), 2.45 (2H, t), 3.76 (2H, t), 7.07-7.08 (2H, m), 7.14-7.21 (1H, m), 7.49-7.53 (1H, m), 7.57-7.65 (3H, m), 7.75-7.78 (2H, m), 11.90 (1H, br s).

### Example 3 (3)

### 5-[3-(3,4-Dihydroquinolin-1(2H)-ylsulfonyl)phenyl]-1-(4-fluorophenyl)-1H-pyrazolo[3,4-d]pyrimidine-4,6(5H,7H)-dione

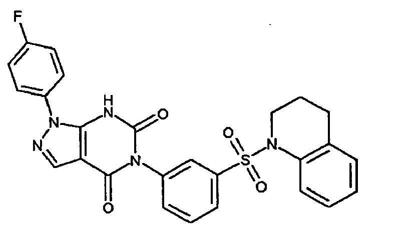

¹H-NMR (DMSO-d₆) δ 1.64 (2H, ddd), 2.46 (2H, t), 3.76 (2H, t), 7.07-7.09 (2H, m), 7.15-7.21 (1H, m), 7.40-7.46 (2H, m), 7.49-7.53 (1H, m), 7.57-7.70 (6H, m), 8.17 (1H, s), 12.54 (1H, br s).

### Example 3 (4)

### 3-[3-(3,4-Dihydroquinolin-1(2H)-ylsulfonyl)phenyl]-6-(4-fluorophenyl)thieno[3,2-d]pyrimidin-2,4(1H,3H)-dione

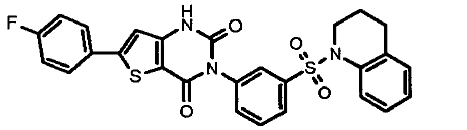

¹H-NMR (DMSO-d₆) δ 1.65 (2H, ddd), 2.46 (2H, t), 3.77 (2H, t), 7.09 (2H, d), 7.15-7.21 (1H, m), 7.28 (1H, s), 7.35 (2H, t), 7.51 (1H, dt), 7.57-7.68 (3H, m), 7.78 (1H, t), 7.86-7.91 (2H, m), 12.16 (1H, br s).

### Example 3 (5)

### 3-[3-(3,4-Dihydroquinolin-1(2H)-ylsulfonyl)phenyl]thieno[3,4-d]pyrimidin-2,4(1H,3H)-dione

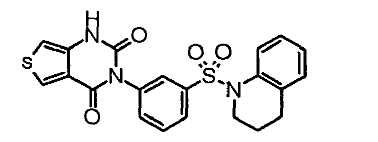

¹H-NMR (DMSO-d₆) δ 1.64 (2H, ddd), 2.45 (2H, t), 3.76 (2H, t), 6.90 (1H, d), 7.07-7.09 (2H, m), 7.18 (1H, ddd), 7.48 (1H, d), 7.57-7.61 (3H, m), 7.75 (1H, s), 8.46 (1H, d), 11.40 (1H, br s).

### Example 3 (6)

### 6-tert-Butyl-3-[3-(3,4-dihydroquinolin-1(2H)-ylsulfonyl)phenyl]thieno[3,2-d]pyrimidin-2,4(1H,3H)-dione

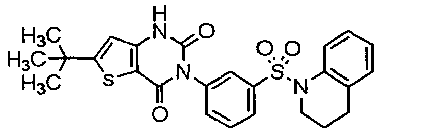

¹H-NMR (DMSO-d₆) δ 1.38 (9H, s), 1.64 (2H, ddd), 2.47 (2H, t), 3.76 (2H, t), 6.75 (1H, s), 7.07-7.09 (2H, m), 7.18 (1H, dt), 7.51 (1H, d), 7.57-7.61 (3H, m), 7.71 (1H, s), 12.01 (1H, br s).

### Example 3 (7)

### 6-[3-(3,4-Dihydroquinolin-1(2H)-ylsulfonyl)phenyl]-2-morpholin-4-yl[1,3]thiazolo[4,5-d]pyrimidine-5,7(4H,6H)-dione

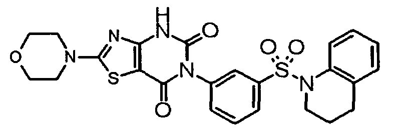

¹H-NMR (DMSO-d₆) δ 1.62 (2H, ddd), 2.45 (2H, t), 3.57-3.60 (4H, m), 3.72-3.77 (6H, m), 7.09-7.90 (2H, m), 7.18 (1H, ddd), 7.44-7.48 (1H, m), 7.56-7.59 (3H, m), 7.66-7.67 (1H, m), 12.35 (1H, br s).

### Example 3 (8)

### 3-[3-(3,4-Dihydroquinolin-1(2H)-ylsulfonyl)phenyl]-9-methoxypyrido[3',2':4,5]thieno[3,2-d]pyrimidin-2,4(1H,3H)-dione

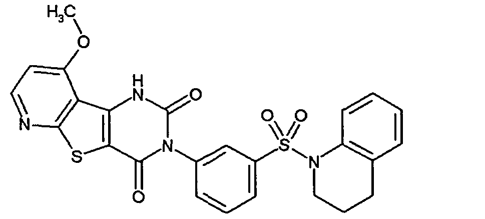

¹H-NMR (DMSO-d₆) δ 1.66 (2H, ddd), 2.48-2.50 (2H, m), 3.75 (2H, t), 3.98 (3H, s), 7.00 (1H, d), 7.07-7.09 (2H, m), 7.18 (1H, ddd), 7.40-7.48 (3H, m), 7.52-7.62 (2H, m), 8.46 (1H, d).

### Example 3 (9)

### 6-[3-(3,4-Dihydroquinolin-1(2H)-ylsulfonyl)phenyl][1,2,5] thiadiazolo[3,4-d]pyrimidine-5,7(4H,6H)-dione

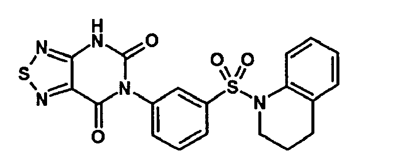

¹H-NMR (DMSO-d₆) δ 1.65 (2H, dt), 2.47 (2H, t), 3.75 (2H, t), 7.08-7.10 (2H, m), 7.17 (1H, td), 7.53 (1H, td), 7.56-7.67 (3H, m), 7.80 (1H, s), 12.80 (1H, br s).

### Example 4

### 3-[3-(3,4-Dihydroquinolin-1(2H)-ylsulfonyl)phenyl]pyrido[3',2':4,5]thieno[3,2-d]pyrimidin-2,4(1H,3H)-dione

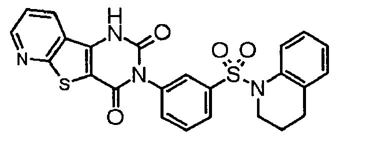

To a 0.75N solution (1.16 ml) of methyl 3-aminothieno[2,3-b]pyridine-2-carboxylate in dichloromethane, a 2.25N solution (1.12 ml) of triethylamine in dichloromethane was added, and then a 0.25N solution (1.36 ml) of triphosgene in dichloromethane was added dropwise on stirring to the mixture under nitrogen atmosphere at -78°C. The cold bath was removed, the mixture was stirred for additional 20 min, and the reaction mixture was concentrated under reduced pressure. The residue was diluted with THF (4.2 ml). A solution of 3-(3,4-dihydro-1(2H)-quinolinylsulfonyl)aniline (0.25N) and DMAP (0.375N) in THF (1.12 ml) was added dropwise, and the mixture was stirred at room temperature for 25 hr. Insoluble triethylamine hydrochloride was filtrated, and the solvent was evaporated under reduced pressure. The fraction eluted by reversed-phase preparative HPLC (Gilson Inc. UniPoint system, YMCODS column 30x75 mm, 0.1% TFA-containing acetonitrile-water (2:98 - 100:0)) was concentrated under reduced pressure, and crystallized from methanol. The obtained crystals were collected by filtration to give the object (97.5 mg).
¹H-NMR (DMSO-d₆) δ 1.66 (2H, ddd), 2.49 (2H, t), 3.78 (2H, t), 7.08-7.10 (2H, m), 7.18 (1H, ddd), 7.54 (1H, dt), 7.58-7.73 (4H, m), 7.85 (1H, t), 8.77 (1H, dd), 8.84 (1H, dd), 12.86 (1H, br s).

In the same manner as in Example 4, the following compound was obtained using methyl 7-aminothieno[2,3-b]pyrazine-6-carboxylate and 3-(3,4-dihydro-1(2H)-quinolinylsulfonyl)aniline.

### Example 4 (1)

### 3-[3-(3,4-Dihydroquinolin-1(2H)-ylsulfonyl)phenyl]pyrazino[2',3':4,5]thieno[3,2-d]pyrimidin-2,4(1H,3H)-dione

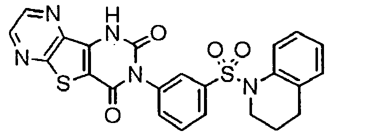

¹H-NMR (DMSO-d₆) δ 1.66 (2H, ddd), 2.48 (2H, t), 3.77 (2H, t), 7.09-7.10 (2H, m), 7.18 (1H, td), 7.51 (1H, dt), 7.58-7.72 (3H, m), 7.88 (1H, t), 8.93 (1H, d), 9.00 (1H, d), 13.03 (1H, br s).

### Example 5

### Methyl 3-[3-(3,4-dihydroquinolin-1(2H)-ylsulfonyl)phenyl]-2,4-dioxo-1,2,3,4-tetrahydrothieno[3,4-d]pyrimidine-5-carboxylate

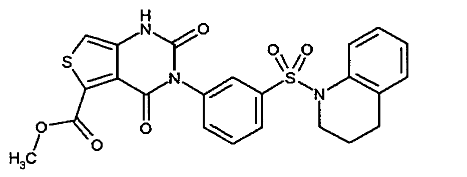

To a 0.5N solution (12.8 ml) of dimethyl 4-aminothiophene-2,3-dicarboxylate hydrochloride in dichloromethane, a 2.0N solution (14.3 ml) of triethylamine in dichloromethane was added. Then, a 0.17N solution (14.4 ml) of triphosgene in dichloromethane was added dropwise with stirring under nitrogen atmosphere at -78°C, and the mixture was stirred at the same temperature for 10 min. The cold bath was removed, the mixture was stirred for additional 50 min, and the reaction mixture was concentrated under reduced pressure. The residue was diluted with THF (32.0 ml), and a solution of 3-(3,4-dihydro-1(2H)-quinolinylsulfonyl)aniline (0.25N) and DMAP (0.375N) in THF (12.8 ml) was added dropwise, and the mixture was stirred at room temperature for 25 hr. Insoluble triethylamine hydrochloride was removed by filtration, and the solvent was evaporated under reduced pressure. The residue was purified by silica gel column chromatography (20-60% ethyl acetate/hexane) to give dimethyl 4-[({[3-(3,4-dihydroquinolin-1(2H)-ylsulfonyl)phenyl]amino}carbonyl)amino]thiophene-2,3-dicarboxylate (2.24 g). This was dissolved in 20% acetonitrile/methanol (95 ml), and a 0.5N solution of sodium methoxide in methanol (11 ml) was added dropwise, and the mixture was heated on stirring at 60°C for 30 min. The solvent was evaporated, and the residue was purified by silica gel column chromatography (0-5% methanol/ethyl acetate), and crystallized from ethyl acetate to give the object (1.43 g).
¹H-NMR (DMSO-d₆) δ 1.60-1.68 (2H, m), 2.49 (2H, t), 3.75 (2H, t), 3.82 (3H, s), 7.07-7.09 (2H, m), 7.15 (1H, s), 7.14-7.20 (1H, m), 7.49-7.62 (4H, m), 7.75-7.76 (1H, m), 11.52 (1H, s).

### Example 6

### 3-[2-Chloro-5-(5,6,7,8-tetrahydro-4H-thieno[3,2-b]azepin-4-ylsulfonyl)-3-thienyl]-2,4-dioxo-1,2,3,4-tetrahydrothieno[3,4-d]pyrimidine-5-carboxylic acid

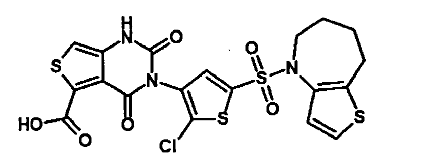

To a 0.5N solution (1.2 ml) of dimethyl 4-aminothiophene-2,3-dicarboxylate hydrochloride in dichloromethane, a 2.0N solution (1.2 ml) of triethylamine in dichloromethane was added, and a 0.17N solution (1.2 ml) of triphosgene in dichloromethane was added dropwise with stirring under nitrogen atmosphere at - 78°C. The cold bath was removed, the mixture was stirred for additional 30 min. The reaction mixture was filtrated, and the filtrate was concentrated under reduced pressure. The residue was diluted with THF (3.0 ml). A solution of 2-chloro-5-(5,6,7,8-tetrahydro-4H-thieno[3,2-b]azepin-4-ylsulfonyl)thiophene-3-amine (0.25N) and DMAP (0.375N) in THF (1.0 ml) was added dropwise, and the mixture was stirred at room temperature for 25 hr. Trisamine resin (Argonaut Inc., 4.36 mol/g, 92 mg) was added, and the mixture was stirred at room temperature for additional 1 hr. Insoluble triethylamine hydrochloride and the resin were removed by filtration, and the filtrate was washed with THF. The filtrate and the washing solution were combined, and the mixture was concentrated under reduced pressure. The residue was purified by silica gel column chromatography (20-50% ethyl acetate/hexane) to give urea intermediate (80 mg). This was dissolved in 20% acetonitrile/methanol (6.1 ml), and a 0.5N solution of sodium methoxide in methanol (0.67 ml) was added dropwise, and the mixture was heated on stirring at 65°C for 25 min. The reaction mixture was concentrated under reduced pressure, and the residue was diluted with dichloromethane, and neutralized with 50% queous ammoniuma chloride solution, and extracted with dichloromethane. The organic layer was dried over anhydrous magnesium sulfate, and the solvent was evaporated under reduced pressure. The residue was subjected to reversed-phase preparative HPLC (Gilson Inc. UniPoint system, YMC ODS column 30x75 mm), and eluted with 0.1% TFA-containing acetonitrile-water (2:98 - 100:0). The eluted fraction was concentrated under reduced pressure, and crystallized from diisopropyl ether. The crystals were collected by filtration to give the object (11 mg). ¹H-NMR (DMSO-d₆) δ 1.51 (2H, br s), 1.82 (2H, br s), 2.49-2.54 (2H, m), 3.68 (2H, br s), 6.96 (1H, d), 7.24 (1H, d), 7.36 (1H, s), 7.68 (1H, s), 12.02 (1H, s), 14.21 (1H, br s).

### Example 7

### 3-[3-(3,4-Dihydroquinolin-1(2H)-ylsulfonyl)phenyl]-N-(2-hydroxyethyl)-2,4-dioxo-1,2,3,4-tetrahydrothieno[3,4-d]pyrimidine-5-carboxamide

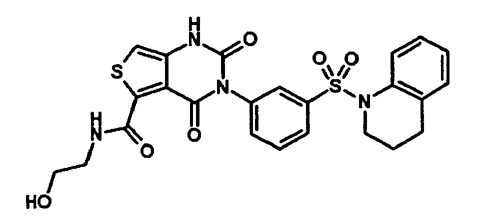

To a 0.20N solution (0.50 ml) of 3-[3-(3,4-dihydroquinolin-1(2H)-ylsulfonyl)phenyl]-2,4-dioxo-1,2,3,4-tetrahydrothieno[3,4-d]pyrimidine-5-carboxylic acid in DMF, a 0.30N solution of 1-ethyl-3-(3-dimethylaminopropyl)carbodiimide hydrochloride in dichloromethane (0.50 ml), a 0.30N solution of 1-hydroxybenzotriazole in dichloromethane (0.50 ml) and a 0.30N solution of ethanolamine in dichloromethane (0.50 ml) were added, and the mixture was shook at room temperature for 17 hr, and diluted with dichloromethane. The organic layer was washed with 5% aqueous sodium bicarbonate solution, and then water, and extracted with dichloromethane. The organic layer was dried over anhydrous magnesium sulfate, and the solvent was evaporated under reduced pressure. The residue was subjected to reversed-phase preparative HPLC (Gilson Inc. UniPoint system, YMC ODS column 30x75 mm), and eluted with 0.1% TFA-containing acetonitrile-water (2:98 - 100:0). The eluted fraction was concentrated under reduced pressure, and crystallized from diisopropyl ether. The obtained crystals were collected by filtration to give the object (46 mg).
¹H-NMR (DMSO-d₆) δ 1.65 (2H, ddd), 2.47 (2H, t), 3.33-3.39 (2H, m), 3.48 (2H, dt), 3.76 (2H, t), 4.75 (1H, t), 7.08-7.09 (2H, m), 7.17 (1H, s), 7.15-7.21 (1H, m), 7.53 (1H, dt), 7.56-7.66 (2H, m), 7.66 (1H, dt), 7.82 (1H, t), 10.49 (1H, t), 11.66 (1H, br s).

### Example 7 (1) - Example 7 (3)

In the same manner as in Example 7, the following compounds were obtained by condensing 3-[3-(3,4-dihydroquinolin-1(2H)-ylsulfonyl)phenyl]-2,4-dioxo-1,2,3,4-tetrahydrothieno[3,4-d]pyrimidine-5-carboxylic acid with the corresponding amines.

### Example 7 (1)

### N-({3-[3-(3,4-Dihydroquinolin-1(2H)-ylsulfonyl)phenyl]-2,4-dioxo-1,2,3,4-tetrahydrothieno[3,4-d]pyrimidin-5-yl}carbonyl)glycine tert-butyl ester

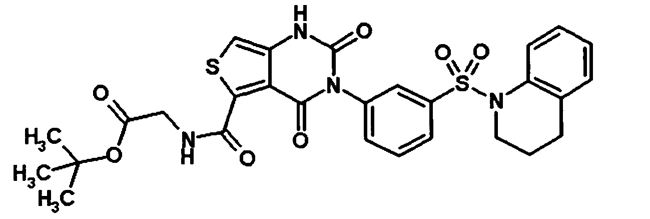

¹H-NMR (DMSO-d₆) δ 1.39 (9H, s), 1.62-1.70 (2H, m), 2.46-2.51 (2H, m), 3.77 (2H, dd), 4.04 (2H, d), 7.07-7.08 (2H, m), 7.16 (1H, td), 7.22 (1H, s), 7.54-7.70 (4H, m), 7.84 (1H, t), 10.71 (1H, t), 11.69 (1H, br s).

### Example 7 (2)

### 3-[3-(3,4-Dihydroquinolin-1(2H)-ylsulfonyl)phenyl]-2,4-dioxo-N-1H-tetrazol-5-yl-1,2,3,4-tetrahydrothieno[3,4-d]pyrimidine-5-carboxamide

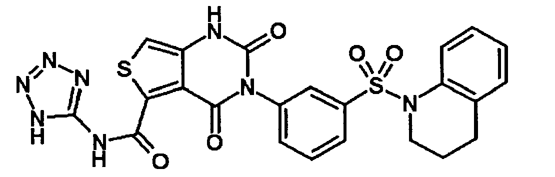

¹H-NMR (DMSO-d₆) δ 1.65 (2H, dt), 2.48 (2H, t), 3.77 (2H, t), 7.07-7.21 (4H, m), 7.40-7.46 (1H, m), 7.57-7.70 (3H, m), 7.98 (1H, s), 8.29-14.02 (3H, m).

### Example 7 (3)

### N-({3-[3-(3,4-Dihydroquinolin-1(2H)-ylsulfonyl)phenyl]-2,4-dioxo-1,2,3,4-tetrahydrothieno[3,4-d]pyrimidin-5-yl}carbonyl)-β-alanine tert-butyl ester

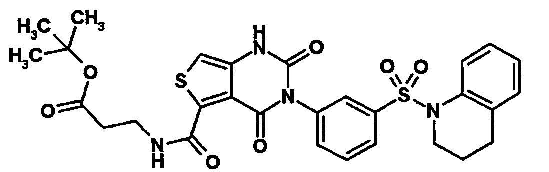

¹H-NMR (DMSO-d₆) δ 1.25 (9H, s), 1.62-1.71 (2H, m), 2.44 (2H, t), 2.45-2.51 (2H, m), 3.48 (2H, q), 3.76 (2H, dd), 7.07-7.09 (2H, m), 7.14-7.21 (1H, m), 7.17 (1H, s), 7.52-7.67 (4H, m), 7.81 (1H, s), 10.55 (1H, t), 11.66 (1H, br s).

### Example 8

### 3-[3-(3,4-Dihydroquinolin-1(2H)-ylsulfonyl)phenyl]-N-methyl-2,4-dioxo-N-(2-pyridin-2-ylethyl)-1,2,3,4-tetrahydrothieno[3,4-d]pyrimidine-5-carboxamide

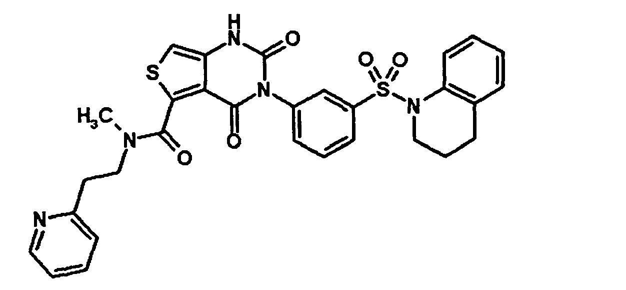

To a 0.20N solution of 3-[3-(3,4-dihydroquinolin-1(2H)-ylsulfonyl)phenyl]-2,4-dioxo-1,2,3,4-tetrahydrothieno[3,4-d]pyrimidine-5-carboxylic acid in DMF (0.50 ml), a 0.30N solution (1.0 ml) of N,N'-diisopropylcarbodiimide in dichloromethane, a 0.30N solution (1.0 ml) of 1-hydroxybenzotriazole in dichloromethane and a 0.30N solution (1.0 ml) of N-methyl-2-pyridin-2-ylethaneamine in dichloromethane were added, and the mixture was shook at room temperature for 41 hr. The reaction solution was passed through a conditioned PS-tosic acid resin (Argonaut Inc.)-packed column, and washed with methanol, then dichloromethane to remove a neutral and acidic fraction. The basic fraction containing the object was eluted with 1N ammonia/methanol. The solvent was evaporated under reduced pressure, the residue was subjected to reversed-phase preparative HPLC (Gilson Inc. UniPoint system, YMC ODS column 30x7 5mm), and eluted with 0.1% TFA-containing acetonitrile-water (2:98 - 100:0). The eluted fraction was concentrated under reduced pressure, and the residue was purified by silica gel column chromatography (0-15% methanol/ethyl acetate). The solvent was evaporated, and crystallized from diisopropyl ether. The obtained crystals were collected by filtration to give the object (51 mg).
¹H-NMR (DMSO-d₆) δ 1.03-1.22 (4H, m), 1.62 (2H, td), 2.43-2.50 (2H, m), 2.78 & 2.97 (3H, s), 3.70-3.77 (2H, m), 6.89 & 6.94 (1H, s), 7.04-7.06 (2H, m), 7.12-7.33 (3H, m), 7.50-7.61 (4H, m), 7.66-7.74 (2H, m), 8.42 & 8.51 (1H, d), 11.44 & 11.45 (1H, br s).

### Example 9

### 3-[3-(3,4-Dihydroquinolin-1(2H)-ylsulfonyl)phenyl]-2,4-dioxo-N-pyrimidin-4-yl-1,2,3,4-tetrahydrothieno[3,4-d]pyrimidine-5-carboxamide

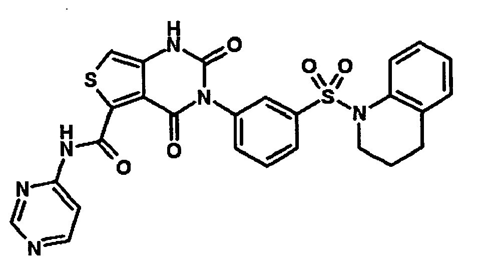

To a 0.20N solution of 3-[3-(3,4-dihydroquinolin-1(2H)-ylsulfonyl)phenyl]-2,4-dioxo-1,2,3,4-tetrahydrothieno[3,4-d]pyrimidine-5-carboxylic acid in DMF (0.50 ml), a 0.60N solution of 1-ethyl-3-(3-dimethylaminopropyl)carbodiimide hydrochloride in dichloromethane (0.50 ml), a 0.21N solution of DMAP in dichloromethane (0.50 ml) and a 1.0N solution of 4-aminopyrimidine in dichloromethane (0.50 ml) were added, and the mixture was shook at room temperature for 18 hr, and diluted with dichloromethane. The organic layer was washed with 5% aqueous sodium bicarbonate solution, then water, and extracted with dichloromethane. The organic layer was dried over anhydrous magnesium sulfate, and the solvent was evaporated under reduced pressure. The residue was subjected to reversed-phase preparative HPLC (Gilson Inc. UniPoint system, YMC ODS column 30x75 mm), and eluted with 0.1% TFA-containing acetonitrile-water (2:98 - 100:0). The eluted fraction was concentrated under reduced pressure to give the object (6.3 mg).
¹H-NMR (DMSO-d₆) δ 1.67-1.71 (2H, m), 2.53 (2H, t), 3.82 (2H, t), 7.02-7.05 (2H, m), 7.12 (1H, s), 7.44 (1H, ddd), 7.52-7.60 (3H, m), 7.64 (1H, dt), 7.77 (1H, d), 8.35 (1H, d), 8.62 (1H, s), 8.70 (1H, d), 8.94 (1H, s), 13.18 (1H, br s).

### Example 10

### 3-[3-(3,4-dihydroquinolin-1(2H)-ylsulfonyl)phenyl]-2,4-dioxo-1,2,3,4-tetrahydrothieno[3,4-d]pyrimidine-5-carboxamide

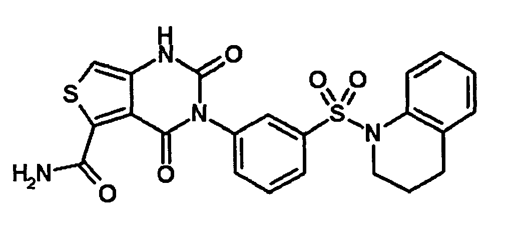

3-[3-(3,4-Dihydroquinolin-1(2H)-ylsulfonyl)phenyl]-2,4-dioxo-1,2,3,4-tetrahydrothieno[3,4-d]pyrimidine-5-carboxylic acid (306 mg) was dissolved in DMF (3.2 ml). A solution of 1-ethyl-3-(3-dimethylaminopropyl)carbodiimide hydrochloride (186 mg) in dichloromethane (3.2 ml) and a solution of ammonium 1H-1,2,3-benzotriazoe-1-olate (152 mg) in dichloromethane (3.3 ml) were added, and the mixture was stirred at room temperature for 50 hr. The reaction mixture was poured into 5% aqueous sodium bicarbonate solution, and extracted with dichloromethane. The organic layer was washed with water, and dried over anhydrous magnesium sulfate. The solvent was evaporated under reduced pressure. The residue was crystallized from methanol, and the obtained crystals were collected by filtration to give the object (186 mg).
¹H-NMR (DMSO-d₆) δ 1.58-1.67 (2H, m), 2.46 (2H, t), 3.76 (2H, t), 7.07-7.09 (2H, m), 7.11-7.20 (1H, m), 7.18 (1H, s), 7.54-7.68 (4H, m), 7.77-7.78 (1H, m), 8.09 (1H, d), 9.74 (1H, d), 11.66 (1H, br s).

### Example 11

### N-({3-[3-(3,4-Dihydroquinolin-1(2H)-ylsulfonyl)phenyl]-2,4-dioxo-1,2,3,4-tetrahydrothieno[3,4-d]pyrimidin-5-yl}carbonyl)glycine

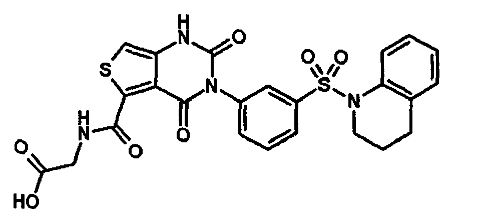

N-({3-[3-(3,4-Dihydroquinolin-1(2H)-ylsulfonyl)phenyl]-2,4-dioxo-1,2,3,4-tetrahydrothieno[3,4-d]pyrimidin-5-yl}carbonyl)glycine tert-butyl ester (20 mg) was dissolved in dichloromethane (0.5 ml). TFA (0.5 ml) was added dropwise, and the mixture was stirred at room temperature for 2 hr. The volatile component was evaporated under reduced pressure. The residue was crystallized from diisopropyl ether, and the obtained crystals were collected by filtration to give the object (19 mg).
¹H-NMR (DMSO-d₆) δ 1.61-1.70 (2H, m), 2.46 (2H, t), 3.76 (2H, t), 4.06 (2H, d), 7.07-7.08 (2H, m), 7.16 (1H, dd), 7.22 (1H, s), 7.53-7.58 (2H, m), 7.53-7.70 (2H, m), 7.83 (1H, t), 10.70 (1H, t), 11.68 (1H, s), 12.73 (1H, br s).

### Example 11(1)

### N-({3-[3-(3,4-Dihydroquinolin-1(2H)-ylsulfonyl)phenyl]-2,4-dioxo-1,2,3,4-tetrahydrothieno[3,4-d]pyrimidin-5-yl}carbonyl)-β-alanine

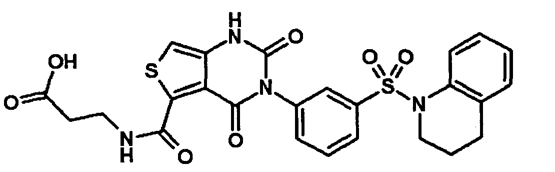

In the same manner as in Example 11, the title compound was obtained using N-({3-[3-(3,4-dihydroquinolin-1(2H)-ylsulfonyl)phenyl]-2,4-dioxo-1,2,3,4-tetrahydrothieno[3,4-d]pyrimidin-5-yl}carbonyl)-β-alanine tert-butyl ester.
¹H-NMR (DMSO-d₆) δ 1.62-1.70 (2H, m), 2.48 (2H, t), 2.47-2.51 (2H, m), 3.49 (2H, q), 3.76 (2H, t), 7.08-7.09 (2H, m), 7.15-7.21 (1H, m), 7.18 (1H, s), 7.52 (1H, dt), 7.57-7.68 (3H, m), 7.82-7.83 (1H, m), 10.48 (1H, t), 11.66 (1H, br s), 12.26 (1H, br s).

### Example 12

### 3-[3-(3,4-dihydroquinolin-1(2H)-ylsulfonyl)phenyl]-5-(5-methyl-1,3,4-oxadiazol-2-yl)thieno[3,4-d]pyrimidin-2,4(1H,3H)-dione

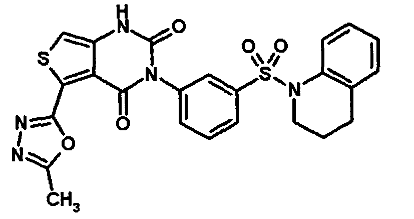

3-[3-(3,4-Dihydroquinolin-1(2H)-ylsulfonyl)phenyl]-2,4-dioxo-1,2,3,4-tetrahydrothieno[3,4-d]pyrimidine-5-carboxylic acid (102 mg) was dissolved in oxalyl chloride (0.84 ml). Acetic hydrazide (38.4 mg) was added, and the mixture was heated under reflux for 3 hr. After cooling to room temperature, the reaction mixture was poured into ice, and neutralized with ammonium hydroxide. Brine was added, and the mixture was extracted with dichloromethane and ethyl acetate. The organic layer was dried over anhydrous magnesium sulfate, and the solvent was evaporated under reduced pressure. The residue was subjected to reversed-phase preparative HPLC (Gilson Inc. UniPoint system, YMC ODS column 30x75 mm), and eluted with 0.1% TFA-containing acetonitrile-water (2:98 - 100:0). The eluted fraction was concentrated under reduced pressure, and crystallized from diisopropyl ether. The obtained crystals were collected by filtration to give the object (5.6 mg).
¹H-NMR (DMSO-d₆) δ 1.60-1.69 (2H, m), 2.46-2.50 (2H, m), 2.55 (3H, s), 3.75 (2H, t), 7.06-7.08 (2H, m), 7.17-7.19 (1H, m), 7.24 (1H, s), 7.51-7.57 (2H, m), 7.60-7.64 (2H, m), 7.76-7.77 (1H, m), 11.59 (1H, br s).

### Example 13

### 3-[3-(3,4-Dihydroquinolin-1(2H)-ylsulfonyl)phenyl]-5-(3-methyl-1,2,4-oxadiazol-5-yl)thieno[3,4-d]pyrimidin-2,4(1H,3H)-dione

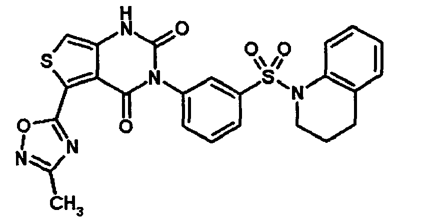

3-[3-(3,4-Dihydroquinolin-1(2H)-ylsulfonyl)phenyl]-2,4-dioxo-1,2,3,4-tetrahydrothieno[3,4-d]pyrimidine-5-carboxylic acid (49 mg) was dissolved in DMF (1.0 ml). A solution of 1-ethyl-3-(3-dimethylaminopropyl)carbodiimide hydrochloride (56 mg) and 1-hydroxybenzotriazole (42 mg) in dichloromethane (1.0 ml) and acetamidoxime (45 mg) was added, and the mixture was stirred at room temperature for 15 hr. Then, the reaction mixture was heated on stirring at 100°C for 7 hr, and the solvent was evaporated under reduced pressure. The residue was diluted with dichloromethane. The organic layer was washed with 5% aqueous sodium bicarbonate solution, ehtn water, and extracted with dichloromethane. The organic layer was dried over anhydrous magnesium sulfate, and the solvent was evaporated under reduced pressure. The residue was subjected to reversed-phase preparative HPLC (Gilson Inc. UniPoint system, YMC ODS column 30x75 mm), and eluted with 0.1% TFA-containing acetonitrile-water (2:98 - 100:0). The eluted fraction was concentrated under reduced pressure, and crystallized from diisopropyl ether. The obtained crystals were collected by filtration to give the object (8.0 mg).
¹H-NMR (DMSO-d₆) δ 1.59-1. 68 (2H, m), 2.41 (3H, s), 2.46-2.50 (2H, m), 3.75 (2H, t), 7.07-7.08 (2H, m), 7.16 (1H, td), 7.35 (1H, s), 7.51-7.65 (4H, m), 7.75-7.77 (1H, m), 11.63 (1H, s).

### Example 14

### Methyl 3-[2-chloro-5-(3,4-dihydroquinolin-1(2H)-ylsulfonyl)phenyl]-2,4-dioxo-1,2,3,4-tetrahydrothieno[3,4-d]pyrimidine-5-carboxylate

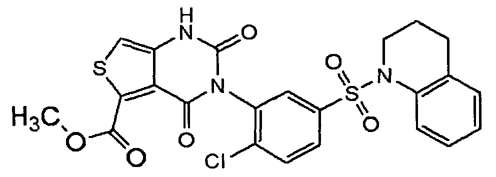

Dimethyl 4-aminothiophene-2,3-dicarboxylate hydrochloride (1.12 g) was suspended in ethyl acetate (50 ml) and aqueous sodium hydrogen carbonate solution (50 ml). The organic layer was separated, dried over anhydrous magnesium sulfate, and concentrated. The obtained residue was dissolved in THF (20 ml). Triethylamine (2.1 ml) was added, and the mixture was cooled to -78°C. A solution of triphosgene (410 mg) in THF (10 ml) was added dropwise to the reaction mixture, and the mixture was warmed to room temperature, and stirred for additional 1 hr. The reaction mixture was diluted with ethyl acetate, and the insoluble material was filtrated. The filtrate was concentrated under reduced pressure. The obtained residue was dissolved in THF (10 ml). DMAP (1.82 g) and 2-chloro-5-(3,4-dihydroquinolin-1(2H)-ylsulfonyl)phenylamine (930 mg) were added, and the mixture was stirred at room temperature for 48 hr. The reaction mixture was concentrated under reduced pressure, and the obtained residue was dissolved in methanol (20 ml). A 28% solution (1.00 g) of sodium methoxide in methanol was added, and the mixture was stirred at room temperature for 10 min. The reaction mixture was diluted with ethyl acetate and aqueous sodium hydrogen carbonate solution, and the organic layer was separated, and dried over anhydrous magnesium sulfate. The drying agent was filtrated, and the filtrate was concentrated. The obtained residue was purified by silica gel column chromatography, and recrystallized from ethyl acetate-hexane to give the object (690 mg) as crystals.
¹H-NMR (DMSO-d₆) δ 1.59-1.74 (2H, m), 2.46-2.57 (2H, m), 3.73-3.81 (2H, m), 3.83 (3H, s), 7.06-7.22 (4H, m), 7.52-7.59 (2H, m), 7.79 (1H, d), 8.03 (1H, d).

### Example 15

### 3-[2-Chloro-5-(3,4-dihydroquinolin-1(2H)-ylsulfonyl)phenyl]-2,4-dioxo-1,2,3,4-tetrahydrothieno[3,4-d]pyrimidine5-carboxylic acid

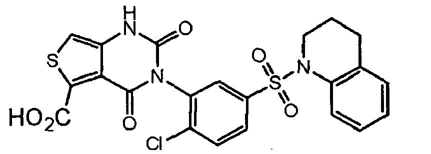

A solution of methyl 3-[2-chloro-5-(3,4-dihydroquinolin-1(2H)-ylsulfonyl)phenyl]-2,4-dioxo-1,2,3,4-tetrahydrothieno[3,4-d]pyrimidine5-carboxylate (246 mg) and lithium hydroxide monohydrate (129 mg) in aqueous ethanol (EtOH: H₂O=10 ml: 2 ml) was stirred at room temperature for 10 min, and diluted with 1N aqueous hydrochlolic acid solution. The reaction mixture was concentrated under reduced pressure. The obtained residue was washed with water, and recrystallized from ethyl acetate-methanol to give the object (170 mg) as crystals.
¹H-NMR (DMSO-d₆) δ 1.58-1.74 (2H, m), 2.42-2.50 (2H, m), 3.72-3.82 (2H, m), 7.05-7.25 (3H, m), 7.38 (1H, s), 7.52-7.65 (2H, m), 7.84 (1H, d), 8.09 (1H, d), 12.04 (1H, br s), 14.24 (1H, br s).

### Example 16

### 3-[2-Chloro-5-(3,4-dihydroquinolin-1(2H)-ylsulfonyl)phenyl]-N-methyl-2,4-dioxo-1,2,3,4-tetrahydrothieno[3,4-d]pyrimidine-5-carboxamide

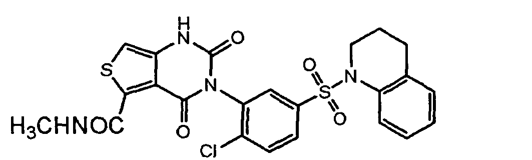

3-[2-Chloro-5-(3,4-dihydroquinolin-1(2H)-ylsulfonyl)phenyl]-2,4-dioxo-1,2,3,4-tetrahydrothieno[3,4-d]pyrimidine-5-carboxylic acid (86 mg), 1-ethyl-3-(3-dimethylaminopropyl)carbodiimide hydrochloride (45 mg), 1-hydroxybenzotriazole (36 mg), diisopropylethylamine (0.1 ml) and a 2 M solution (0.5 ml) of methylamine in THF were suspended in DMF (3 ml), and the mixture was stirred at room temperature for 16 hr. The reaction mixture was diluted with water, and the obtained solid was collected by filtration, and air-dried. The obtained residue was purified by silica gel column chromatography, and recrystallized from ethyl acetate-hexane to give the object (45 mg) as crystals.
¹H-NMR (DMSO-d₆) δ 1.62-1.75 (2H, m), 2.50-2.56 (2H, m), 2.85 (3H, d), 3.73-3.82 (2H, m), 7.03-7.25 (4H, m), 7.55 (1H, d), 7.61 (1H, dd), 7.82 (1H, d), 8.09 (1H, d), 10.05-10.17 (1H, m), 11.86 (1H, br s).

### Example 17

### 3-[3-(3,4-Dihydroquinolin-1(2H)-ylsulfonyl)phenyl]-5-(hydroxymethyl)thieno[3,4-d]pyrimidin-2,4(1H,3H)-dione

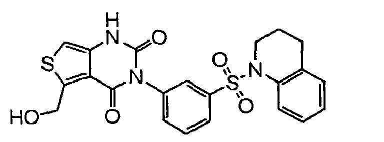

To a solution of methyl 3-[3-(3,4-dihydroquinolin-1(2H)-ylsulfonyl)phenyl]-2,4-dioxo-1,2,3,4-tetrahydrothieno[3,4-d]pyrimidine-5-carboxylate (2.00 g) in THF (200 ml) cooled to - 78°C, a solution of lithium tetrahydroborate (263 mg) in THF (40 ml) was added, and the mixture was stirred for 1 hr. After warming to room temperature, the mixture was stirred for additional 2 hr. The reaction mixture was diluted with 1N aqueous sodium hydroxide solution, and extracted with ethyl acetate. The organic layer was washed with saturated brine, and dried over anhydrous magnesium sulfate. The drying agent was filtrated, and the filtrate was concentrated. The obtained residue was recrystallized from ethyl acetate-isopropyl ether to give the object (1.70 g) as crystals.
¹H-NMR (DMSO-d₆) δ 1.57-1.69 (2H, m), 2.43-2.49 (2H, m), 3.71-3.80 (2H, m), 5.00 (2H, d), 5.96 (1H, t), 6.68 (1H, s), 7.04-7.23 (3H, m), 7.47-7.63 (4H, m), 7.65-7.70 (1H, m), 11.26 (1H, s).

### Example 18

### 3-[3-(3,4-Dihydroquinolin-1(2H)-ylsulfonyl)phenyl]-2,4-dioxo-1,2,3,4-tetrahydrothieno[3,4-d]pyrimidine-5-carbaldehyde

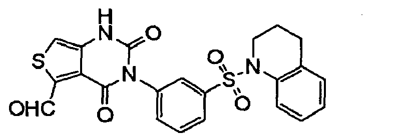

3-[3-(3,4-Dihydroquinolin-1(2H)-ylsulfonyl)phenyl]-5-(hydroxymethyl)thieno[3,4-d]pyrimidin-2,4(1H,3H)-dione (276 mg) and manganese dioxide (4.0 g) were suspended in DMF (10 ml), and the mixure was stirred at room temperature for 16 hr. The insoluble material was removed by filtration, and was washed with ethyl acetate. The obtained filtrate was washed with water. The organic layer was dried over anhydrous magnesium sulfate, and concentrated. The obtained residue was recrystallized from ethyl acetate-isopropyl ether to give the object (170 mg) as crystals.
¹H-NMR (DMSO-d₆) δ 1.59-1.71 (2H, m), 2.44-2.49 (2H, m), 3.71-3.80 (2H, m), 7.05-7.11 (2H, m), 7.12-7.23 (1H, m), 7.47 (1H, d), 7.50-7.70 (4H, m), 7.77-7.84 (1H, m), 10.55 (1H, d), 11:69 (1H, s).

### Example 19

### 3-[3-(3,4-Dihydroquinolin-1(2H)-ylsulfonyl)phenyl]-2,4-dioxo-1,2,3,4-tetrahydrothieno[3,4-d]pyrimidine-5-carbaldehyde oxime

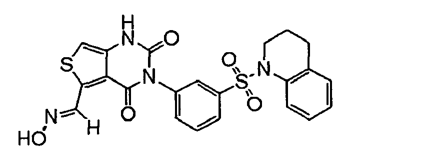

3-[3-(3,4-Dihydroquinolin-1(2H)-ylsulfonyl)phenyl]-2,4-dioxo-1,2,3,4-tetrahydrothieno[3,4-d]pyrimidine-5-carbaldehyde (120 mg) and hydroxylamine hydrochloride (30 mg) were dissolved in 1-methyl-2-pyrrolidone (5 ml), and the mixture was stirred at 110°C for 20 min. The reaction mixture was diluted with ethyl acetate and water, and the precipitate was filtrated, washed with water and isopropyl ether, and dried. The obtained solid was purified by silica gel column chromatography, and recrystallized from THF-ethyl acetate to give the object (82 mg) as crystals.
¹H-NMR (DMSO-d₆) δ 1.57-1.71 (2H, m), 2.44-2.50 (2H, m), 3.70-3.81 (2H, m), 6.79-7.12 (3H, m), 7.12-7.23 (1H, m), 7.46-7.68 (4H, m), 7.71-7.79 (1H, m), 8.83-8.91 (1H, m), 11.48 (1H, s), 11.78-13.16 (1H, m).

### Example 20

### 3-[3-(3,4-Dihydroquinolin-1(2H)-ylsulfonyl)phenyl]-2,4-dioxo-1,2,3,4-tetrahydrothieno[3,4-d]pyrimidine-5-carbonitrile

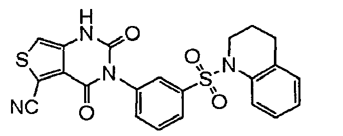

To a solution of 3-[3-(3,4-dihydroquinolin-1(2H)-ylsulfonyl)phenyl]-2,4-dioxo-1,2,3,4-tetrahydrothieno[3,4-d]pyrimidine-5-carboxamide (480 mg) in DMF (30 ml) cooled to 5°C, thionyl chloride (0.3 ml) was added, and the mixture was warmed to 100°C. After stirring at 100°C for 10 min, the reaction mixture was diluted with ethyl acetate and aqueous sodium hydrogen carbonate solution. The organic layer was separated, and dried over anhydrous magnesium sulfate. The obtained residue was purified by silica gel column chromatography, and recrystallized from THF-ethyl acetate to give the object (390 mg) as crystals.
¹H-NMR (DMSO-d₆) δ 1.58-1.71 (2H, m), 2.44-2.50 (2H, m), 3.72-3.81 (2H, m), 7.05-7.23 (3H, m), 7.37 (1H, s), 7.48-7.67 (4H, m), 7.77-7.83 (1H, m), 11.70 (1H, br s).

### Example 21

### 3-[3-(3,4-Dihydroquinolin-1(2H)-ylsulfonyl)phenyl]-5-(1H-1,2,3-triazol-4-yl)thieno[3,4-d]pyrimidin-2,4(1H,3H)-dione

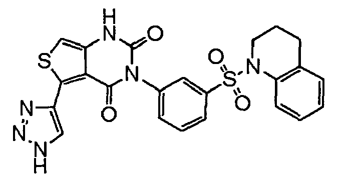

To a solution of trimethylsilyldiazomethane (0.43 ml) in THF (5 ml) cooled to 5°C, n-butyl lithium (0.54 ml) was added dropwise, and the mixture was stirred for 30 min. A suspension of 3-[3-(3,4-dihydroquinolin-1(2H)-ylsulfonyl)phenyl]-2,4-dioxo-1,2,3,4-tetrahydrothieno[3,4-d]pyrimidine-5-carbonitrile (100 mg) in THF (15 ml) was added dropwise to the reaction mixture, and the mixture was stirred at room temperature for additional 3 hr. The reaction mixture was diluted with ethyl acetate and aqueous ammonium chloride solution, and the organic layer was separated, and dried over anhydrous magnesium sulfate. The drying agent was filtrated, and the filtrate was concentrated. The obtained residue was dissolved in THF (5 ml), and 1M solution of tetrabutylammonium fluoride in THF (3 ml) was added. The reaction mixture was stirred at room temperature for 2 hr, and diluted with ethyl acetate and aqueous ammonium chloride solution. The organic layer was separated, dried over anhydrous magnesium sulfate, and concentrated. The obtained residue was purified by silica gel column chromatography, and recrystallized from ethyl acetate-hexane to give the object (53 mg) as crystals. ¹H-NMR (DMSO-d₆) δ 1.60-1.71 (2H, m), 2.45-2.50 (2H, m), 3.72-3.81 (2H, m), 7.03-7.22 (4H, m), 7.51-7.71 (4H, m), 7.76-7.82 (1H, m), 8.23 (1H, br s), 11.63 (1H, br s), 13.96 (1H, br s).

### Example 22

### 3-[3-(3,4-Dihydroquinolin-1(2H)-ylsulfonyl)phenyl]-5-(1H-tetrazol-5-yl)thieno[3,4-d]pyrimidin-2,4(1H,3H)-dione

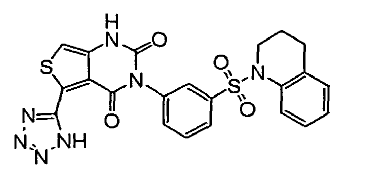

3-[3-(3,4-Dihydroquinolin-1(2H)-ylsulfonyl)phenyl]-2,4-dioxo-1,2,3,4-tetrahydrothieno[3,4-d]pyrimidine-5-carbonitrile (158 mg), sodium azide (340 mg), ammonium chloride (280 mg) were suspended in DMF (5 ml), and the mixture was stirred at 110°C for 3 hr. The reaction mixture was diluted with water, and acidified with 1N hydrochloric acid. The resulting precipitate was collected by filtration, and air-dried. The obtained residue was purified by silica gel column chromatography to give the object (8.8 mg) as an amorphous.
¹H-NMR (CDCl₃) δ 1.64-1.76 (2H, m), 2.51 (2H, t), 3.79-3.89 (2H, m), 7.01 (1H, s), 7.05-7.22 (3H, m), 7.43-7.50 (1H, m), 7.54-7.64 (2H, m), 7.67-7.73 (1H, m), 7.78 (1H, d), 9.08 (1H, br s), 15.03 (1H, br s).

### Example 23

### 3-[3-(3,4-Dihydroquinolin-1(2H)-ylsulfonyl)phenyl]-5-{[(2-methoxyethyl)(methyl)amino]methyl}thieno[3,4-d]pyrimidin-2,4(1H,3H)-dione

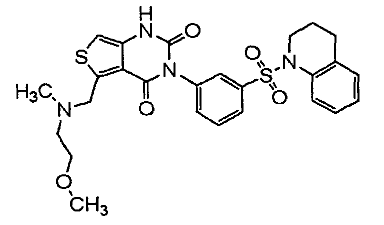

To a solution of 3-[3-(3,4-dihydroquinolin-1(2H)-ylsulfonyl)phenyl]-5-(hydroxymethyl)thieno[3,4-d]pyrimidin-2,4(1H,3H)-dione (136 mg) and triethylamine (0.08 ml) in THF (10 ml) cooled to 5°C, mesyl chloride (0.023 ml) was added, and the mixture was stirred at room temperature for 5 hr. 2-Methoxy-N-methylethaneamine (0.1 ml) was added to the reaction mixture, and the mixture was stirred at room temperature for 16 hr. The reaction mixture was diluted with ethyl acetate and aqueous sodium hydrogen carbonate solution, and the organic layer was separated, and extracted with 1N hydrochlolic acid. The aqueous layer was alkalized with 1N aqueous sodium hydroxide solution, and extracted with ethyl acetate. The organic layer was dried over anhydrous magnesium sulfate, and concentrated to give the object (17 mg) as an amorphous.
¹H-NMR (CDCl₃) δ 1.61-1.72 (2H, m), 2.43 (3H, s), 2.48 (2H, t), 2.74 (2H, t), 3.35 (3H, s), 3.54 (2H, t), 3.75-3.84 (2H, m), 4.22 (2H, s), 6.45-6.71 (1H, m), 6.99-7.04 (1H, m), 7.04-7.12 (1H, m), 7.14-7.23 (1H, m), 7.39-7.59 (4H, m), 7.78 (1H, m), 8.57 (1H, br s).

### Example 23(1)3-[3-(3,4-Dihydroquinolin-1(2H)-ylsulfonyl)phenyl]-5-{[methyl(2-pyridin-2-ylethyl)amino]methyl}thieno[3,4-d]pyrimidin-2,4(1H,3H)-dione

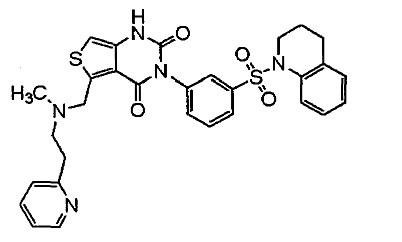

In the same manner as in Example 23, the title compound was obtained using 3-[3-(3,4-dihydroquinolin-1(2H)-ylsulfonyl)phenyl]-5-(hydroxymethyl)thieno[3,4-d]pyrimidin-2,4(1H,3H)-dione and N-methyl-2-pyridin-2-ylethaneamine.
¹H-NMR (DMSO-d₆) δ 1.55-1.68 (2H, m), 2.35 (3H, s), 2.42-2.50 (2H, m), 2.77-2.96 (4H, m), 3.70-3.79 (2H, m), 4.15 (2H, s), 6.66 (1H, s), 7.07 (2H, d), 7.12-7.23 (2H, m), 7.27 (1H, d), 7.47-7.63 (4H, m), 7.63-7.72 (2H, m), 8.42-8.47 (1H, m), 11.24 (1H, br s).

### Example 24

### 3-[3-(3,4-Dihydroquinolin-1(2H)-ylsulfonyl)phenyl]-5-(1-hydroxyethyl)thieno[3,4-d]pyrimidin-2,4(1H,3H)-dione

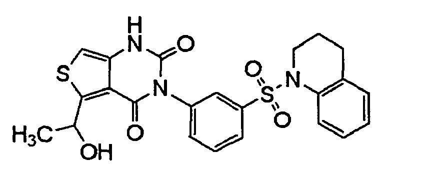

To a solution of 3-[3-(3,4-dihydroquinolin-1(2H)-ylsulfonyl)phenyl]-2,4-dioxo-1,2,3,4-tetrahydrothieno[3,4-d]pyrimidine-5-carbaldehyde (121 mg) in THF (10 ml) cooled to 5°C, a solution of methyl magnesiumbromide in diethyl ether (3.0 M, 0.51 ml) was added, and the mixture was warmed to room temperature. The reaction mixture was stirred for 1 hr, and diluted with ethyl acetate and aqueous ammonium chloride solution. The organic layer was separated, dried over anhydrous magnesium sulfate, and concentrated. The obtained residue was purified by silica gel column chromatography, and recrystallized from ethyl acetate-hexane to give the object (27 mg) as crystals. ¹H-NMR (DMSO-d₆) δ 1.38 (3H, d), 1.58-1.68 (2H, m), 2.44-2.49 (2H, m), 3.72-3.78 (2H, m), 5.48-5.57 (1H, m), 5.94 (1H, d), 6.65 (1H, s), 7.04-7.21 (3H, m), 7.50-7.66 (5H, m), 11.25 (1H, br s).

### Example 25

### 3-[3-(3,4-dihydroquinolin-1(2H)-ylsulfonyl)phenyl]-5-(1,3-oxazol-5-yl)thieno[3,4-d]pyrimidin-2,4(1H,3H)-dione

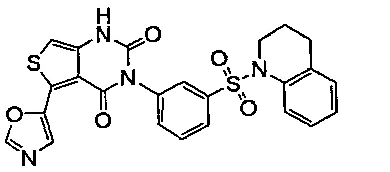

A solution of 3-[3-(3,4-dihydroquinolin-1(2H)-ylsulfonyl)phenyl]-2,4-dioxo-1,2,3,4-tetrahydrothieno[3,4-d]pyrimidine-5-carbaldehyde (100 mg) and p-toluenesulfonylmethyl isocyanate (125 mg), potassium carbonate (118 mg) in dry methanol (5 ml) was stirred at 60°C for 1 hr. The reaction mixture was diluted with ethyl acetate and 1N hydrochlolic acid. The organic layer was separated, and dried over anhydrous magnesium sulfate. The drying agent was filtrated, and the filtrate was concentrated. The obtained residue was purified by silica gel column chromatography, and recrystallized from ethyl acetate-hexane to give the object (50 mg) as crystals.
¹H-NMR (DMSO-d₆) δ 1.59-1.72 (2H, m), 2.45-2.50 (2H, m), 3.72-3.80 (2H, m), 6.98 (1H, s), 7.05-7.22 (3H, m), 7.51-7.68 (4H, m), 7.74-7.78 (1H, m), 8.10 (1H, s), 8.54 (1H, s), 11.47 (1H, br s).

### Example 26

### Methyl {3-[3-(3,4-dihydroquinolin-1(2H)-ylsulfonyl)phenyl]-2,4-dioxo-1,2,3,4-tetrahydrothieno[3,4-d]pyrimidin-5-yl}acetate

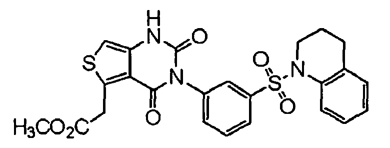

A solution of 3-[3-(3,4-dihydroquinolin-1(2H)-ylsulfonyl)phenyl]-2,4-dioxo-1,2,3,4-tetrahydrothieno[3,4-d]pyrimidine-5-carbaldehyde (445 mg) and methyl methylsulfinylmethyl sulfide (360 mg) and Triton B (0.40 ml) in THF (20 ml) was stirred at 60°C for 16 hr. Triton B (2.0 ml) was added to the reaction mixture, and the mixture was stirred for additional 16 hr. The reaction mixture was diluted with ethyl acetate and 1N hydrochlolic acid, and the organic layer was separated, and dried over anhydrous magnesium sulfate. The drying agent was filtrated, and the filtrate was concentrated. The obtained residue was purified by silica gel column chromatography, and dissolved in THF (15 ml). Copper (II) chloride (60 mg) and a 10% solution of hydrochloride in methanol (15 ml) were added, and the mixture was stirred at room temperature for 24 hr. The reaction mixture was diluted with ethyl acetate and water, and the organic layer was separated, and dried over anhydrous magnesium sulfate. The drying agent was filtrated, and the filtrate was concentrated. The obtained residue was purified by silica gel column chromatography to give the object (27 mg) as a powder.
¹H-NMR (DMSO-d₆) δ 1.56-1.68 (2H, m), 2.43-2.49 (2H, m), 3.62 (3H, s), 3.71-3.79 (2H, m), 4.33 (2H, s), 6.75 (1H, s), 7.03-7.23 (3H, m), 7.48-7.67 (5H, m), 11.34 (1H, br s).

### Example 27

### Ethyl (2E)-3-{3-[3-(3,4-dihydroquinolin-1(2H)-ylsulfonyl)phenyl]-2,4-dioxo-1,2,3,4-tetrahydrothieno[3,4-d]pyrimidin-5-yl}acrylate

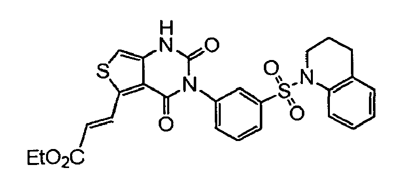

To a solution of sodium hydride(54 mg) in THF (5 ml) cooled to 5°C, diethylphosphonoethyl acetate (0.31 g) was added, and the mixture was stirred for 10 min. 3-[3-(3,4-dihydroquinolin-1(2H)-ylsulfonyl)phenyl]-2,4-dioxo-1,2,3,4-tetrahydrothieno[3,4-d]pyrimidine-5-carbaldehyde (250 mg) was added to the reaction mixture, and the mixture was stirred for additional 10 min. The reaction mixture was diluted with ethyl acetate and aqueous sodium hydrogen carbonate solution, and the organic layer was separated, and dried over anhydrous magnesium sulfate. The drying agent was filtrated, and the filtrate was concentrated. The obtained residue was purified by silica gel column chromatography, and recrystallized from ethyl acetate-hexane to give the object (220 mg) as crystals.
¹H-NMR (DMSO-d₆) δ 1.24 (3H, t), 1.58-1.71 (2H, m), 2.44-2.51 (2H, m), 3.70-3.81 (2 H, m), 4.18 (2H, q), 6.53 (1H, d), 6.99-7.23 (4H, m), 7.49-7.66 (4H, m), 7.70-7.77 (1H, m), 8.59 (1H, d), 11.50 (1H, br s).

### Example 28

### Ethyl 3-{3-[3-(3,4-dihydroquinolin-1(2H)-ylsulfonyl)phenyl]-2,4-dioxo-1,2,3,4-tetrahydrothieno[3,4-d]pyrimidin-5-yl}propanoate

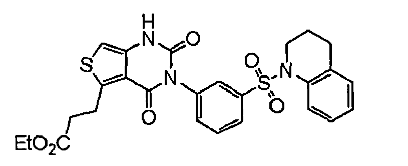

Under hydrogen atmosphere, a solution of ethyl 3-{3-[3-(3,4-dihydroquinolin-1(2H)-ylsulfonyl)phenyl]-2,4-dioxo-1,2,3,4-tetrahydrothieno[3,4-d]pyrimidin-5-yl}acrylate (180 mg) and palladium carbon (40 mg) in THF (20 ml) and methanol (20 ml) was stirred at room temperature for 30 hr. The catalyst was removed by filtration, and the filtrate was concentrated under reduced pressure. The obtained residue was recrystallized from acetate-hexane to give the object (130 mg) as crystals.
¹H-NMR (DMSO-d₆) δ 1.17 (3H, t), 1.56-1.69 (2H, m), 2.44-2.50 (2H, m), 2.69 (2H, t), 3.39 (2H, t), 3.71-3.80 (2H, m), 4.06 (2H, q), 6.64 (1H, s), 7.08 (2H, d), 7.12-7.22 (1H, m), 7.49-7.67 (5H, m), 11.29 (1H, s).

### Example 29

### 3-[3-(3,4-dihydroquinolin-1(2H)-ylsulfonyl)phenyl]-5-(1-hydroxy-1-methylethyl)thieno[3,4-d]pyrimidin-2,4(1H,3H)-dione

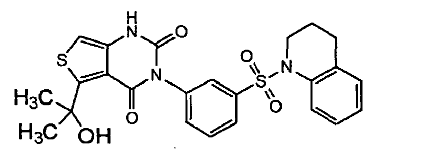

To a solution of methyl 3-[3-(3,4-dihydroquinolin-1(2H)-ylsulfonyl)phenyl]-2,4-dioxo-1,2,3,4-tetrahydrothieno[3,4-d]pyrimidine-5-carboxylate (102 mg) in THF (10 ml) cooled to 5°C, a solution of methyl magnesiumbromide in diethyl ether (3.0 M, 1.0 ml) was added, and the mixture was warmed to room temperature. The reaction mixture was stirred for 1 hr, and diluted with ethyl acetate and aqueous ammonium chloride solution. The organic layer was separated, dried over anhydrous magnesium sulfate, and concentrated. The obtained residue was purified by silica gel column chromatography, and recrystallized from ethyl acetate-hexane to give the object (57 mg) as crystals. ¹H-NMR (DMSO-d₆) δ 1.56-1.69 (8H, m), 2.42-2.50 (2H, m), 3.71-3.80 (2H, m), 6.08 (1H, s), 6.64 (1H, s), 7.02-7.25 (3H, m), 7.52-7.77 (5H, m), 11.29 (1H, br s).

### Example 30

### Methyl 3-[4-(3,4-dihydroquinolin-1(2H)-ylsulfonyl)-2-thienyl]-2,4-dioxo-1,2,3,4-tetrahydrothieno[3,4-d]pyrimidine-5-carboxylate

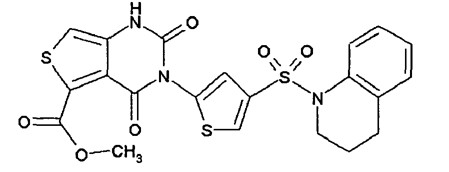

To a solution of dimethyl 4-aminothiophene-2,3-dicarboxylate hydrochloride (0.58 g) and triethylamine (0.85 ml) in dichloromethane (10 ml), a solution of triphosgene (0.23 g) in THF (5 ml) was added dropwise at -20°C, and the mixture was stirred at room temperature for 1 hr. The solvent of the reaction mixture was evaporated under reduced pressure, and the residue was diluted with THF. The obtained precipitate was removed by filtration. The filtrate was added to a solution of 4-(3,4-dihydroquinolin-1(2H)-ylsulfonyl)thiophene-2-amine (0.45 g) and DMAP (0.37 g) in THF (10 ml) at room temperature, and the mixture was further stirred overnight. The reaction mixture was poured into dilute hydrochloric acid, and extracted twice with ethyl acetate. The collected organic layer was dried over anhydrous magnesium sulfate, and the solvent was evaporated under reduced pressure to give an oily substance.

To a solution of the oily substance obtained in the above-mentioned in methanol(10 ml), a 28 % sodium methoxide-methanol solution (0.29 g) was added at room temperature, and the mixture was further stirred for 10 min. The reaction mixture was poured into dilute hydrochloric acid, and extracted twice with ethyl acetate. The collected organic layer was dried over anhydrous magnesium sulfate, and the solvent was evaporated under reduced pressure.

The obtained residue was purified by silica gel column chromatography (hexane-ethyl acetate (2:1 - 1:2)), and crystallized from ethyl acetate-diisopropyl ether to give the object (0.14 g) as crystals.
¹H-NMR (CDCl₃-DMSO-d₆) δ 1.70-1.78 (2H, m), 2.60 (2H, t), 3.79 (2H, t), 3.93 (3H, s), 6.85 (1H, d), 6.95 (1H, s), 7.02-7.10 (2H, m), 7.16 (1H, dt), 7.69 (1H, d), 7.71 (1H, d), 11.49 (1H, br s).

### Example 31

### 3-[4-(3,4-Dihydroquinolin-1(2H)-ylsulfonyl)-2-thienyl]-5-(hydroxymethyl)thieno[3,4-d]pyrimidin-2,4(1H,3H)-dione

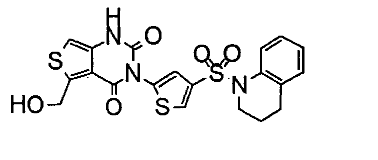

To a solution of methyl 3-[4-(3,4-dihydroquinolin-1(2H)-ylsulfonyl)-2-thienyl]-2,4-dioxo-1,2,3,4-tetrahydrothieno[3,4-d]pyrimidine-5-carboxylate (50 mg) in THF (5 ml), lithium borohydride (4 mg) was added at room temperature, and the mixture was further stirred for 1 hr. The reaction mixture was poured into diluted hydrochloric acid, and extracted twice with ethyl acetate. The collected organic layer was dried over anhydrous magnesium sulfate, and the solvent was evaporated under reduced pressure. The obtained residue was purified by silica gel column chromatography (hexane-ethyl acetate (2:1 - 1:2)), and crystallized from diethyl ether to give the object (16 mg) as a powder.
¹H-NMR (CDCl₃) δ 1.69-1.77 (2H, m), 2.58 (2H, t), 3.81 (2H, t), 3.95 (1H, t), 4.96 (2H, d), 6.48 (1H, s), 6.81 (1H, d), 7.03-7.11 (2H, m), 7.17 (1H, dt), 7.74 (1H, dd), 7.75 (1H, d), 8.86 (1H, s).

### Example 32

### Ethyl 3-[3-(3,4-dihydroquinolin-1(2H)-ylsulfonyl)phenyl]-2,4-dioxo-1,2,3,4-tetrahydrothieno[2,3-d]pyrimidine-5-carboxylate

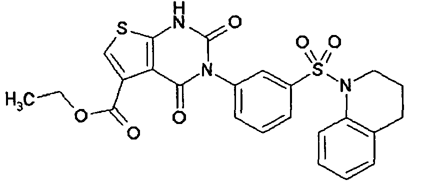

To a solution of diethyl 2-aminothiophene-3,4-dicarboxylate (100 mg), triethylamine (0.21 ml) in dichloromethane (3 ml), a solution of triphosgene (44.5 mg) in THF (1 ml) was added at 0°C, and the mixture was stirred under nitrogen atmosphere at 0°C for 1 hr. The insoluble material was removed by filtration. 3-(3,4-Dihydroquinolin-1(2H)-ylsulfonyl)aniline (118 mg) was added, and the mixture was stirred under nitrogen atmosphere at room temperature overnight. Water was added, and the mixture was extracted with ethyl acetate. The organic layer was washed with saturated brine, and dried over magnesium sulfate. The solvent was evaporated under reduced pressure. The obtained residue was purified by basic silica gel column chromatography, and dissolved in ethanol (7 ml). A 20% solution (140 mg) of sodium ethoxide in ethanol was added, and the mixture was stirred under nitrogen atmosphere at 60°C for 2 hr.

After allowing to cool to room temperature, water-saturated brine was added, and the mixture was extracted with ethyl acetate. The organic layer was washed with saturated brine, and dried over magnesium sulfate. The solvent was evaporated under reduced pressure, and the obtained residue was purified by silica gel column chromatography, and crystallized from diisopropyl ether to give the object (69.7 mg) as crystals.
¹H-NMR (DMSO-d₆) δ 1.23 (3H, t), 1.58-1.69 (2H, m), 2.43-2.54 (2H, m), 3.72-3.79 (2H, m), 4.21 (2H, q), 7.04-7.10 (2H, m), 7.12-7.22 (1H, m), 7.47-7.63 (5H, m), 7.73 (1H, s), 12.46 (1H, s).

### Example 33

### Example 33-1 Methyl 3-(4-{[ethyl(phenyl)amino]carbonyl}-2-thienyl)-2,4-dioxo-1,2,3,4-tetrahydrothieno[3,4-d]pyrimidine-5-carboxylate

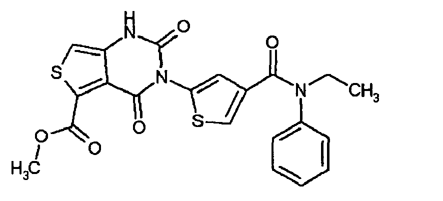

### Example 33-2

### 3-(4-{[Ethyl(phenyl)amino]carbonyl}-2-thienyl)-2,4-dioxo-1,2,3,4-tetrahydrothieno[3,4-d]pyrimidine-5-carboxylic acid

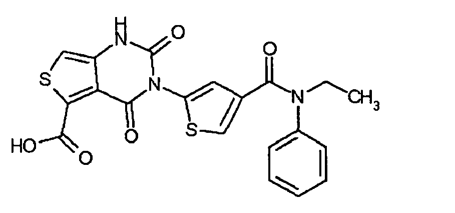

To a solution of dimethyl 2-aminothiophene-3,4-dicarboxylate (200 mg) and triethylamine (0.73 ml) in dichloromethane (10 ml), a solution of oxalyl chloride (102 mg) in THF (3 ml) was slowly added at 0°C, and the mixture was further stirred for 1 hr. The insoluble material was removed by filtration. 5-Amino-N-ethyl-N-phenylthiophene-3-carboxamide (240 mg) and DMAP (340 mg) were added to the filtrate, and the mixture was stirred under nitrogen atmosphere at room temperature overnight. Water was added, and the mixture was extracted with ethyl acetate. The organic layer was washed with saturated brine, and dried over magnesium sulfate. The solvent was evaporated under reduced pressure. Methanol (10 ml) was added to the obtained residue, and a 28% solution of sodium methoxide in methanol (538 mg) was added, and the mixture was stirred under nitrogen atmosphere at 60°C for 1 hr. After allowing to cool to room temperature, water was added, and the mixture was extracted with ethyl acetate (aqueous phase -X). The organic layer was washed with saturated brine, and dried over magnesium sulfate. The solvent was evaporated under reduced pressure. The obtained residue was purified by silica gel column chromatography, and crystallized from diisopropyl ether to give methyl 3-(4-{[ethyl(phenyl)amino]carbonyl}-2-thienyl)-2,4-dioxo-1,2,3,4-tetrahydrothieno[3,4-d]pyrimidine-5-carboxylate (11.8 mg) as crystals. The aqueous phase -X was acidified with 1N hydrochlolic acid. The organic layer was washed with saturated brine, and dried over magnesium sulfate. The solvent was evaporated under reduced pressure. The obtained residue was purified by silica gel column chromatography, and crystallized from diisopropyl ether to give 3-(4-{[ethyl(phenyl)amino]carbonyl}-2-thienyl)-2,4-dioxo-1,2,3,4-tetrahydrothieno[3,4-d]pyrimidine-5-carboxylic acid (21.4 mg) as crystals.
Methyl 3-(4-{[ethyl(phenyl)amino]carbonyl}-2-thienyl)-2,4-dioxo-1,2,3,4-tetrahydrothieno[3,4-d]pyrimidine-5-carboxylate
¹H-NMR (DMSO-d₆) δ 1.10 (3H, t), 3.77-3.88 (5H, m), 6.84 (1H, d), 7.09-7.13 (2H, m), 7.22-7.46 (5H, m), 11.51 (1H, s). 3-(4-{[Ethyl(phenyl)amino]carbonyl}-2-thienyl)-2,4-dioxo-1,2,3,4-tetrahydrothieno[3,4-d]pyrimidine-5-carboxylic acid ¹H-NMR (DMSO-d₆) δ 1.10 (3H, t), 3.82 (2H, q), 6.88 (1H, d), 7.20-7.45 (7H, m), 11.84 (1H, s), 14.51 (1H, br s).

### Example 34

### {3-[3-(3,4-Dihydroquinolin-1(2H)-ylsulfonyl)phenyl]-2,4-dioxo-1,2,3,4-tetrahydrothieno[3,4-d]pyrimidin-5-yl}acetatic acid

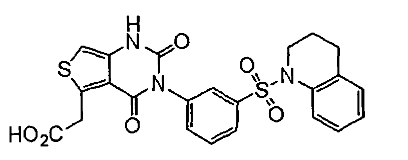

To a solution of methyl {3-[3-(3,4-dihydroquinolin-1(2H)-ylsulfonyl)phenyl]-2,4-dioxo-1,2,3,4-tetrahydrothieno[3,4-d]pyrimidin-5-yl}acetate (27 mg) in ethanol (4 ml), 1N aqueous sodium hydroxide solution (1 ml) was added, and the mixture was stirred at room temperature for 2 hr. The reaction mixture was diluted with ethyl acetate and 1N hydrochlolic acid, and the organic layer was separated, and extracted with 1N aqueous sodium hydroxide solution. The aqueous layer was acidified with 1N hydrochlolic acid, and extracted with ethyl acetate. The organic layer was dried over anhydrous magnesium sulfate. The drying agent was filtrated, and the filtrate was concentrated, and the obtained residue was recrystallized from ethyl acetate-hexane to give the object (6.3 mg) as crystals.
¹H-NMR (DMSO-de) δ 1.56-1.68 (2H, m), 2.42-2.48 (2H, m), 3.71-3.79 (2H, m), 4.23 (2H, s), 6.72 (1H, s), 7.06-7.21 (3H, m), 7.49-7.65 (5H, m), 11.29-11.34 (1H, m), 12.60 (1H, br s).

### Example 34(1) - Example 34(3)

In the same manner as in Example 34, the following compounds were obtained by hydrolysis of the corresponding esters (synthesized in Example 28, Example 30 and Example 32).

### Example 34(1)

### 3-{3-[3-(3,4-Dihydroquinolin-1(2H)-ylsulfonyl)phenyl]-2,4-dioxo-1,2,3,4-tetrahydrothieno[3,4-d]pyrimidin-5-yl}propanoic acid

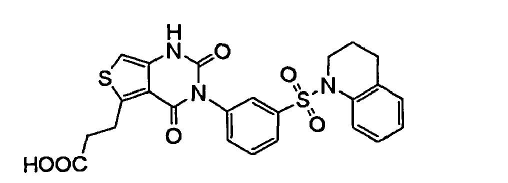

¹H-NMR (DMSO-d₆) δ 1.56-1.69 (2H, m), 2.43-2.50 (2H, m), 2.62 (2H, t), 3.25-3.45 (2H, m), 3.70-3.81 (2H, m), 6.63 (1H, s), 7.03-7.23 (3H, m), 7.49-7.68 (5H, m), 11.29 (1H, br s), 12.36 (1H, br s).

### Example 34(2)

### 3-[4-(3,4-Dihydroquinolin-1(2H)-ylsulfonyl)-2-thienyl]-2,4-dioxo-1,2,3,4-tetrahydrothieno[3,4-d]pyrimidine-5-carboxylic acid

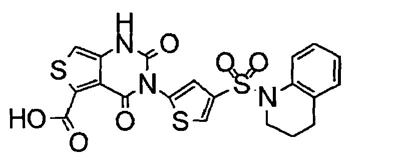

¹H-NMR (DMSO-d₆) δ 1.63-1.71 (2H, m), 2.55 (2H, t), 3.73 (2H, t), 7.00 (1H, s), 7.10 (2H, s), 7.14-7.20 (1H, m), 7.30 (1H, s), 7.55 (1H, d), 8.18 (1H, s), 11.88 (1H, s).

### Example 34 (3)

### 3-[3-(3,4-Dihydroquinolin-1(2H)-ylsulfonyl)phenyl]-2,4-dioxo-1,2,3,4-tetrahydrothieno[2,3-d]pyrimidine-5-carboxylic acid

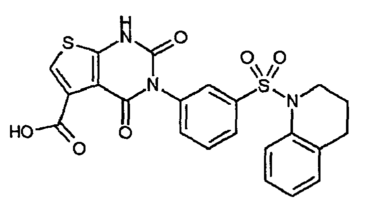

¹H-NMR (DMSO-d₆) δ 1.57-1.68 (2H, m), 2.42-2.53 (2H, m), 3.72-3.81 (2H, m), 7.05-7.22 (3H, m), 7.52-7.70 (4H, m), 7.81 (1H, s), 7.98 (1H, s), 12.94 (1H, s), 14.29 (1H, s).

### Example 35

### 3-[3-(3,4-Dihydroquinolin-1(2H)-ylsulfonyl)phenyl]-5-methyl-2,4-dioxo-1,2,3,4-tetrahydrothieno[2,3-d]pyrimidine-6-carboxylic acid

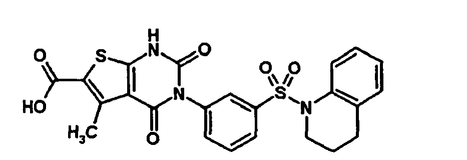

Diethyl 5-[({[3-(3,4-dihydroquinolin-1(2H)-ylsulfonyl)phenyl]amino}carbonyl)amino]-3-methylthiophene-2,4-dicarboxylate (32.5 mg) was dissolved in 20% acetonitrilemethanol (3.9 ml). A 0.5N solution of sodium methoxide in methanol (0.39 ml) was added dropwise, and the mixture was heated on stirring at 64°C for 75 min. The reaction mixture was concentrated under reduced pressure, and the residue was diluted with dichloromethane, neutralized with 50% aqueous ammonium chloride solution, and extracted with dichloromethane. The organic layer was separated from the aqueous phase, and the solvent was evaporated under reduced pressure. The crystals crystallized from ethyl acetate was collected by filtration, and dried under vacuum to give ethyl 3-[3-(3,4-dihydroquinolin-1(2H)-ylsulfonyl)phenyl]-5-methyl-2,4-dioxo-1,2,3,4-tetrahydrothieno[2,3-d]pyrimidine-6-carboxylate as a mixture (29.5 mg) with the hydrolyzed carboxylic acid derivative.

The crude product of ethyl 3-[3-(3,4-dihydroquinolin-1(2H)-ylsulfonyl)phenyl]-5-methyl-2,4-dioxo-1,2,3,4-tetrahydrothieno[2,3-d]pyrimidine-6-carboxylate obtained (29.5 mg) in the above-mentioned was dissolved in THF (0.28 ml). 1N Aqueous sodium hydroxide solution (1.1 ml) was added, and the mixture was stirred at room temperature for 5 hr, and washed with dichloromethane (0.56 ml). The aqueous layer was concentrated, neutralized with hydrochlolic acid, and extracted with dichloromethane containing a small amount of THF. The organic layer was washed with saturated brine, and separated from the aqueous layer. The solvent was evaporated under reduced pressure. The crystals crystallized from ethyl acetate was collected by filtration, and dried under vacuum to give the object (19.9 mg).
¹H-NMR (DMSO-d₆) δ 1.63 (2H, ddd), 2.47 (2H, t), 2.69 (3H, s), 3.75 (2H, t), 7.08-7.09 (2H, m), 7.18 (1H, ddd), 7.49-7.52 (1H, td), 7.56-7.62 (3H, m), 7.73 (1H, s), 12.63 (1H, br s), 13.27 (1H, br s).

### Example 36

### 3-[3-(3,4-Dihydroquinolin-1(2H)-ylsulfonyl)phenyl]-5-(hydroxymethyl)-3,4-dihydrothieno[3,4-d]pyrimidine-2(1H)-one

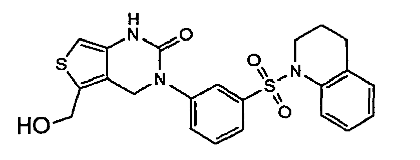

To a solution of 3-[3-(3,4-dihydroquinolin-1(2H)-ylsulfonyl)phenyl]-2,4-dioxo-1,2,3,4-tetrahydrothieno[3,4-d]pyrimidine-5-carboxylic acid (290 mg) in THF (10 ml) warmed to 45°C, 1.1 mol/l solution of borane in THF (5 ml) was added, and the mixture was stirred for 30 min. 1N Aqueous sodium hydroxide solution was added to the reaction mixture, and the mixture was extracted with ethyl acetate. The organic layer was dried over anhydrous magnesium sulfate, and concentrated. The obtained residue was purified by silica gel column chromatography, and recrystallized from ethyl acetate to give the object (125 mg) as crystals.
¹H-NMR (DMSO-d₆) δ1.57-1.69 (2H, m), 2.40-2.49 (2H, m), 3.72-3.83 (2H, m), 4.51 (2H, d), 4.65 (2H, s), 5.43 (1H, t), 6.33 (1H, s), 7.05-7.12 (2H, m), 7.12-7.25 (1H, m), 7.34-7.42 (1H, m), 7.53 (1H, t), 7.57-7.69 (3H, m), 9.78 (1H, s).

### Example 37

### Methyl 3-(5-{[(3-bromo-2-chlorophenyl)(cyclopropyl)amino]carbonyl}-2-chloro-3-thienyl)-2,4-dioxo-1,2,3,4-tetrahydrothieno[3,4-d]pyrimidine-5-carboxylate

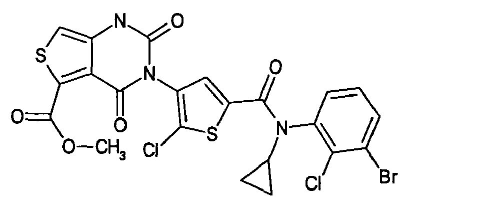

To a solution of dimethyl 4-aminothiophene-2,3-dicarboxylate hydrochloride (4.3 g), triethylamine (10.7 ml) in dichloromethane (80 ml), oxalyl chloride (1.77 g) was added at - 78°C, and the mixture was stirred at 0°C for 1 hr. The insoluble material was removed by filtration, and the filtrate was concentrated under reduced pressure. The obtained residue was dissolved in THF (100 ml). 4-Amino-N-(3-bromo-2-chlorophenyl)-5-chloro-N-cyclopropylthiophene-2-carboxamide (5.55 g) and DMAP (8.34 g) was added at 0°C. The mixture was warmed to room temperature, and stirred for 1 day. Water was added, and the mixture was extracted with ethyl acetate. The organic layer was washed with 1N hydrochlolic acid, aqueous sodium hydrogen carbonate solution and saturated brine, and dried over anhydrous magnesium sulfate. The solvent was evaporated under reduced pressure. The obtained residue was dissolved in methanol (100 ml). A 28% solution of sodium methoxide in methanol (4.28 g) waws added, and the mixture was stirred at 60°C for 1 hr. After allowing to cool to room temperature, water was added, and the mixture was extracted with ethyl acetate. The organic layer was washed with saturated brine, and dried over anhydrous magnesium sulfate. The solvent was evaporated under reduced pressure. The obtained residue was recrystallized from ethyl acetate-THF to give the object (8.66 g) as crystals.
¹H-NMR (DMSO-d₆) δ0.52-0.75 (2H, m), 0.80-1.00 (2H, m), 3.27-3.44 (1H, m), 3.83 (3H, s), 7.20 (1H, s), 7.41 (1H, t), 7.49 (1H, br s), 7.51-7.62 (1H, m), 7.81-7.92 (1H, m), 11.66 (1H, br s).

### Example 38

### Methyl 3-(2-chloro-5-{[(2-chloro-3-cyanophenyl)(cyclopropyl)amino]carbonyl}-3-thienyl)-2,4-dioxo-1,2,3,4-tetrahydrothieno[3,4-d]pyrimidine-5-carboxylate

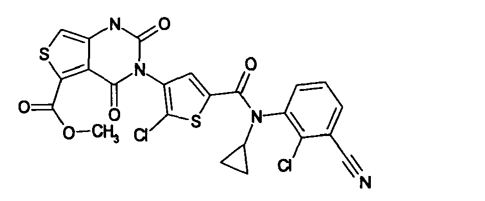

To a solution of methyl 3-(5-1[(3-bromo-2-chlorophenyl)(cyclopropyl)amino]carbonyl}-2-chloro-3-thienyl)-2,4-dioxo-1,2,3,4-tetrahydrothieno[3,4-d]pyrimidine-5-carboxylate (2.5 g) in 1-methyl-2-pyrrolidone (50 ml), tetrakis(triphenylphosphine)palladium (2.1 g), zinc cyanide (573 mg) were added, and the mixture was stirred under argon atmosphere at 100°C for 4 hr. After allowing to cool to room temperature, aqueous sodium hydrogen carbonate solution was added, and the mixture was extracted with ethyl acetate. The organic layer was washed with saturated brine, and dried over anhydrous magnesium sulfate. The solvent was evaporated under reduced pressure. The obtained residue was purified by NH-silica gel column chromatography, and recrystallized from ethyl acetate-diisopropyl ether to give the object (1.05 g) as crystals.
¹H-NMR (DMSO-d₆) δ0.54-0.70 (2H, m), 0.85-0.98 (2H, m), 3.32-3.47 (1H, m), 3.84 (3H, s), 7.20 (1H, s), 7.56 (1H, br s), 7.66 (1H, t), 7.88-7.94 (1H, m), 8.01-8.08 (1H, m), 11.66 (1H, s).

### Example 39

### 5-Chloro-N-(2-chloro-3-cyanophenyl)-N-cyclopropyl-4-[5-(hydroxymethyl)-2,4-dioxo-1,2-dihydrothieno[3,4-d]pyrimidine-3(4H)-yl]thiophene-2-carboxamide

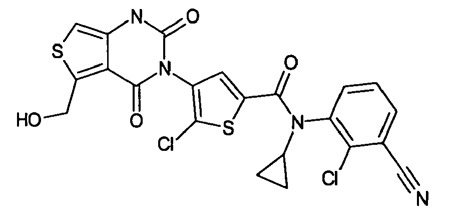

To a suspension of calcium chloride (158 mg) in ethanol (10 ml)-THF (10 ml), sodium borohydride (108 mg) was added at 0°C, and the mixture was further stirred for 1.5 hr. Methyl 3-(2-chloro-5-{[(2-chloro-3-cyanophenyl)(cyclopropyl)amino]carbonyl}-3-thienyl)-2,4-dioxo-1,2,3,4-tetrahydrothieno[3,4-d]pyrimidine-5-carboxylate (200 mg) was added, and the mixture was stirred at 0°C for 2 hr, and at room temperature overnight. Water was added to the reaction mixture, and the mixture was extracted with ethyl acetate. The organic layer was washed with saturated brine, and dried over anhydrous magnesium sulfate. The solvent was evaporated under reduced pressure. The obtained residue was purified by preparative HPLC to give the object (40.8 mg) as an amorphous.
¹H-NMR (DMSO-d₆) δ0.58-0.69 (2H, m), 0.87-0.97 (2H, m), 3.33-3.47 (1H, m), 5.00 (2H, d), 5.98 (1H, t), 6.71 (1H, s), 7.66 (1H, t), 7.85 (1H, br s), 7.91 (1H, d), 8.04 (1H, d), 11.38 (1H, br s).

### Example 40

### 3-(2-Chloro-5-{[(2-chloro-3-cyanophenyl)(cyclopropyl)amino]carbonyl}-3-thienyl)-2,4-dioxo-1,2,3,4-tetrahydrothieno[3,4-d]pyrimidine-5-carboxylic acid

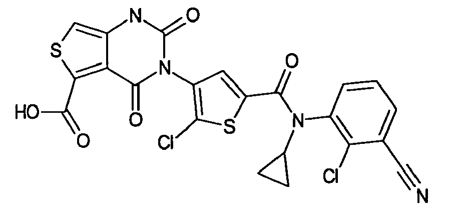

To a suspension of methyl 3-(2-chloro-5-{[(2-chloro-3-cyanophenyl)(cyclopropyl)amino]carbonyl}-3-thienyl)-2,4-dioxo-1,2,3,4-tetrahydrothieno[3,4-d]pyrimidine-5-carboxylate (400 mg) in methanol (10 ml)-THF (10 ml), 1N aqueous sodium hydroxide solution (2.1 ml) was added, and the mixture was stirred at room temperature for 1 hr. Water was added at 0°C, and 1N hydrochlolic acid (5 ml) was added, and the mixture was extracted with ethyl acetate. The organic layer was washed with saturated brine, and dried over anhydrous magnesium sulfate. The solvent was evaporated under reduced pressure to give the object (360 mg) as an amorphous.
¹H-NMR (DMSO-d₆) δ0.55-0.74 (2H, m), 0.84-0.98 (2H, m), 3.32-3.47 (1H, m), 7.35 (1H, s), 7.66 (1H, t), 7.85 (1H, br s), 7.92 (1H, d), 8.04 (1H, d), 11.99 (1H, s), 14.29 (1H, br s).

### Example 41

### 3-(2-Chloro-5-{[(2-chloro-3-cyanophenyl)(cyclopropyl)amino]carbonyl}-3-thienyl)-2,4-dioxo-1,2,3,4-tetrahydrothieno[3,4-d]pyrimidine-5-carboxamide

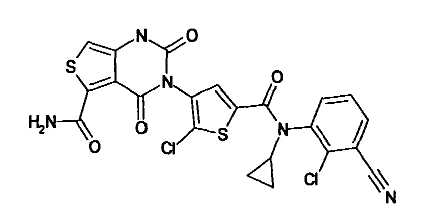

To a solution of 3-(2-chloro-5-{[(2-chloro-3-cyanophenyl)(cyclopropyl)amino]carbonyl}-3-thienyl)-2,4-dioxo-1,2,3,4-tetrahydrothieno[3,4-d]pyrimidine-5-carboxylic acid (100 mg), 1-hydroxy-1H-benztriazole(49 mg) and ammonium chloride (19.5 mg), triethylamine (0.1 ml) in DMF (10 ml), catalystic DMAP and 1-ethyl-3-(3-dimethylaminopropyl)-carbodiimide hydrochloride (70 mg) were added, and the mixture was stirred at room temperature overnight. Water was added to the reaction mixture, and the mixture was extracted with ethyl acetate. The organic layer was washed with saturated brine, and dried over anhydrous magnesium sulfate. The solvent was evaporated under reduced pressure. The obtained residue was purified by silica gel column chromatography to give the object (46.9 mg) as an amorphous.
¹H-NMR (DMSO-d₆) δ0.57-0.71 (2H, m), 0.84-0.99 (2H, m), 3.33-3.47 (1H, m), 7.21 (1H, s), 7.66 (1H, t), 7.75 (1H, br s), 7.91 (1H, d), 8.04 (1H, d), 8.11 (1H, s), 9.59 (1H, s), 11.80 (1H, s).

### Experimental Example

### (1) Production of ¹²⁵I-Leuprorelin

10 µL of a 3 × 10⁻⁴ M aqueous solution of leuprorelin and 10 µL of 0.01 mg/mL lactoperoxidase were placed in a tube, and 10 µL (37 MBq) of a Na¹²⁵I solution was added thereto. After stirring, 10 µL of 0.001% H₂O₂ was added to allow the mixture to undergo a reaction at room temperature for 20 minutes. 700 µL of a 0.05% TFA solution was added to stop the reaction, and purification by reversed phase HPLC was carried out. The conditions for HPLC are described below. ¹²⁵I-leuprorelin was eluted at a retention time of 26 to 27 minutes.

Column: TSKgel ODS-80^{™} (TM refers to a registered trademark. The same as above)

CTR (4.6 mm × 10 cm) eluent:

Solvent A (0.05% TFA)

Solvent B (40% CH₃CN-0.05% TFA)

0^{th} minute (100% Solvent A) - 3^{rd} minute (100% Solvent A) - 7^{th} minute (50% Solvent A + 50% Solvent B) - 40^{th} minute (100% Solvent B)

Elution temperature: Room temperature

Elution rate: 1 mL/min

### (2) Production of CHO (Chinese Hamster Ovary) cell membrane fraction containing human GnRH receptor

Human GnRH receptor-expressing CHO cells were cultured until the cells were reached a 90% confluent state in a T-150 flask. The cells were separated using 0.02% EDTA-PBS and were centrifuged at 500 × g for 5 minutes. A homogenate buffer for cells (10 mM NaHCO₃, 1 mM EDTA, 1 mM PMSF, 0.5 µg/mL Pepstatin, 2 µg/mL Leupeptin, pH 7.4) was added to the cell pellet, and the mixture was homogenized with a Polytron homogenizer. After centrifugation of the homogenate at 4°C and at 1000 × g for 10 minutes, the supernatant was placed in an ultracentrifuge tube and was centrifuged at 4°C and at 49,300 × g for 1 hour to obtain a precipitate of a membrane fraction. This precipitate was suspended in an assay buffer (50 mM Tris-HCl, 150 mM NaCl, 5 mM EDTA, pH 7.4) and then dispensed and stored at -80°C, so that the suspension can be thawed every time it is taken for use.

### (3) Measurement of the binding inhibitory rate of ¹²⁵I-Leuprorelin

A ¹²⁵I-leuprorelin binding assay was carried out using 200 µL of an assay buffer containing 10 µg of human GnRH receptor membrane fraction, 800 pM of ¹²⁵I-leuprorelin and 10 nM of the testing compound.

The non-specific binding radioactivity was measured in the presence of 1 µM of non-labeled leuprorelin. After leaving the mixture to stand at room temperature for 1 hour, ¹²⁵I-leuprorelin was captured on a GF/C filter (PerkinElmer, Inc.), and the filter was washed with ice-cold 50 mM Tris-HCl (pH 7.4) using a cell harvester. After drying the filter, a liquid scintillation cocktail for microplate was added, and the membrane-bound radioactivity was measured using a TopCount.

The value of (TB-SB)/(TB-NSB)×100 (SB: radioactivity upon addition of the compound, TB: maximum binding radioactivity, NSB: non-specific binding radioactivity) was calculated to determine the binding inhibitory rate in the presence of 10 nM of each testing material. The results are presented in the following.

**Table 1**

| Example No. | Binding inhibitory ratio (%) |
|---|---|
| 1 | 86 |
| 6 | 97 |
| 7 (1) | 92 |
| 12 | 72 |
| 16 | 96 |
| 24 | 95 |
| 30 | 110 |
| 31 | 100 |
| 33-2 | 85 |
| 34 (3) | 100 |
| 37 | 100 |
| 38 | 95 |
| 39 | 101 |
| 40 | 101 |
| 41 | 100 |

### Industrial Applicability

The compound of the invention has excellent antagonistic activity against gonadotropin releasing hormone. Moreover, the compound shows good oral absorbability and is excellent in the aspects of stability and pharmacokinetics. The compound also has low toxicity and is excellent in the aspect of safety. Therefore, it can be used, for example, as a prophylactic or therapeutic agent for hormone-dependent diseases. Specifically, for example, the compound is effective as a medicine such as a prophylactic or therapeutic agent for sex hormone-dependent cancers (e.g., prostate cancer, uterine cancer, breast cancer, pituitary cancer, etc.), prostatomegaly, hysteromyoma, endometriosis, metrofibroma, precocious puberty, amenorrhea, premenstrual syndrome, dysmenorrhea, polycystic ovary syndrome, acne, baldness, Alzheimer's disease and the like, as an agent for birth control (e.g., contraceptive, etc.), a therapeutic agent for infertility, a menstrual regulator, a prophylactic and/or therapeutic agent for irritable bowel syndrome, or a preventive agent for post-operative recurrence of sex hormone-dependent cancers; and is also effective for regulation of animal's estrus, improvement in table meat quality and animal growth regulation in the field of stockbreeding, and also as a promoting agent for fish spawning in the field of fishery. Moreover, the compound of the present invention is useful as a preventive of premature ovulation during external fertilization or embryo transplantation, or a preventive of ovulation due to endogenous LH during external fertilization.

This application is based on a patent application No. 2005-027806 filed in Japan, the contents of which are incorporated in full herein by this reference.

## Claims

1. A compound represented by the formula (I) 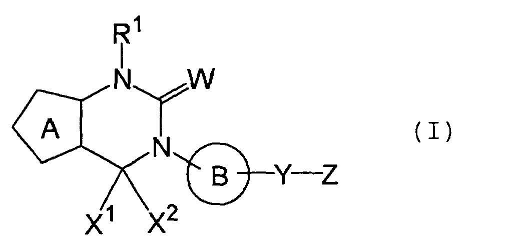 wherein
ring A is a 5-membered aromatic heterocycle optionally having substituent(s),
R¹ is a hydrogen atom or a hydrocarbon group optionally having substituent(s),
W is an oxygen atom or a sulfur atom,
X¹ and X² may be the same or different and each is a hydrogen atom, a hydrocarbon group optionally having substituent(s) or a heterocyclic group optionally having substituent(s), or, X¹ and
X² optionally form an oxygen atom, a sulfur atom or =NR² wherein R² is a hydrogen atom or a hydrocarbon group optionally having substituent(s) in combination,
ring B is an aromatic ring optionally further having substituent(s),
Y is a bond, a C₁₋₆ alkylene optionally having substituent(s), a C₂₋₆ alkenylene optionally having substituent(s) or a C₂₋₆ alkynylene optionally having substituent(s), and
Z is a group represented by the formula: -SOₙR³ wherein n is 0, 1 or 2, and R³ is an amino optionally having substituent(s), a hydrocarbon group optionally having substituent(s) or a heterocyclic group optionally having substituent(s) (provided that R³ is not a methyl group), or
a group represented by the formula: -COR⁴ wherein R⁴ is an amino optionally having substituent(s), a hydrocarbon group optionally having substituent(s) or a heterocyclic group optionally having substituent(s);
provided that when ring B is a ring represented by the formula: 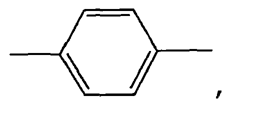
then the amino optionally having substituent(s) for R³ is a disubstituted amino or a cyclic amino optionally having substituent(s),
the amino optionally having substituent(s) for R⁴ is a disubstituted amino (except dimethylamino) or a cyclic amino optionally having substituent(s) (except monocyclic piperazin-1-yl optionally having substituent(s)), and
the hydrocarbon group optionally having substituent(s) for R⁴ is not a methyl group, or a salt thereof.

2. The compound of claim 1, wherein
ring A is a thiophene ring optionally having substituent(s) or a furan ring optionally having substituent(s).

3. The compound of claim 1, wherein
ring A is a 5-membered aromatic heterocycle optionally substituted with substituent(s) selected from the group consisting of (a) a hydrocarbon group optionally having substituent(s), (b) a heterocyclic group optionally having substituent(s), (c) a halogen, (d) a hydroxy, (e) a cyano, (f) a carboxyl, (g) an alkoxycarbonyl, (h) an amino optionally having substituent(s) and (i) a carbamoyl optionally having substituent(s), or optionally substituted with a hydrocarbon group optionally having the above-mentioned substituent(s) (a) - (i).

4. The compound of claim 3, wherein
the hydrocarbon group is an alkyl group, a cycloalkyl group, an alkenyl group, a cycloalkenyl group, an alkynyl group, an aralkyl group or a C₁₀₋₁₄ aryl group.

5. The compound of claim 1, wherein
X¹ and X² form an oxygen atom, a sulfur atom or =NR² wherein R² is a hydrogen atom or a hydrocarbon group optionally having substituent(s) in combination.

6. The compound of claim 1, wherein n is 2.

7. The compound of claim 1, wherein Y is a bond.

8. The compound of claim 1, wherein ring B is a C₆₋₁₄ aryl ring optionally further having substituent(s) or an aromatic heterocycle optionally further having substituent(s).

9. The compound of claim 1, wherein
ring B is a ring represented by the formula: 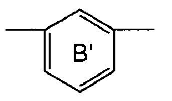 wherein ring B' is a benzene ring optionally further having substituent(s),
or an aromatic heterocycle optionally further having substituent(s).

10. The compound of claim 1, wherein
R¹ is a hydrogen atom, Y is a bond, and X¹ and X² form an oxygen atom, a sulfur atom or =NR² wherein R² is a hydrogen atom or a hydrocarbon group optionally having substituent(s) in combination.

11. The compound of claim 1, wherein
ring A is a 5-membered aromatic heterocycle containing, as ring-constituting atom besides carbon atom, 1 to 4 hetero atoms selected from the group consisting of an oxygen atom, a sulfur atom and a nitrogen atom and optionally having substituent(s) selected from the group consisting of (i) - (xi),
(i) a C₁₋₆ alkyl group optionally having substituent(s) selected from the group consisting of (a) - (d),
(a) a hydroxy,
(b) a carboxyl,
(c) a C₁₋₆ alkoxycarbonyl, and
(d) an amino optionally mono- or di-substituted with a C₁₋₆ alkyl optionally having substituent(s) selected from the group consisting of a C₁₋₆ alkoxy and a 5- to 16-membered heterocyclic group containing, as ring-constituting atom besides carbon atom, 1 to 4 hetero atoms selected from the group consisting of an oxygen atom, a sulfur atom and a nitrogen atom,
(ii) a C₂₋₆ alkenyl group optionally having a C₁₋₆ alkoxycarbonyl
(iii) a C₆₋₁₄ aryl group optionally having a halogen atom,
(iv) a 5- to 16-membered heterocyclic group containing, as ring-constituting atom besides carbon atom, 1 to 4 hetero atoms selected from the group consisting of an oxygen atom, a sulfur atom and a nitrogen atom and optionally having a C₁₋₆ alkyl,
(v) a cyano,
(vi) a carboxyl,
(vii) a formyl,
(viii) a C₁₋₆ alkoxycarbonyl,
(ix) HO-N=CH-,
(x) a carbamoyl optionally mono- or di-substituted with substituent(s) selected from the group consisting of (a) - (b),
(a) a C₁₋₆ alkyl group optionally having substituent(s) selected from the group consisting of a hydroxy, a C₁₋₆ alkoxycarbonyl, a carboxyl and a 5- to 16-membered heterocyclic group containing, as ring-constituting atom besides carbon atom, 1 to 4 hetero atoms selected from the group consisting of an oxygen atom, a sulfur atom and a nitrogen atom, and
(b) a 5- to 16-membered heterocyclic group containing, as ring-constituting atom besides carbon atom, 1 to 4 hetero atoms selected from the group consisting of an oxygen atom, a sulfur atom and a nitrogen atom, and
(xi) a 5- to 16-membered heterocycle containing, as ring-constituting atom besides carbon atom, 1 to 4 hetero atoms selected from the group consisting of an oxygen atom, a sulfur atom and a nitrogen atom, which is formed by two substituents bonded to each other and optionally has a C₁₋₆ alkoxy group,
R¹ is a hydrogen atom,
W is an oxygen atom,
X¹ and X² are hydrogen atoms, or X¹ and X² form an oxygen atom in combination,
ring B is a C₆₋₁₄ aryl ring optionally further having a halogen atom, or a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom or a 8- to 16-membered bicyclic or tricyclic fused aromatic heterocycle containing, as ring-constituting atom besides carbon atom, 1 to 12 hetero atoms selected from the group consisting of an oxygen atom, a sulfur atom and a nitrogen atom, each of which optionally further has a halogen atom,
Y is a bond, and
Z is
(i) a group represented by formula: -SOₙR³
wherein n is 2 and R³ is a 5- to 16-membered cyclic amino, or
(ii) a group represented by formula: -CONR⁸(R9)
wherein R⁸ is a hydrogen atom, a C₁₋₆ alkyl group or a C₃₋₈ cycloalkyl group, and R⁹ is a hydrogen atom or a C₆₋₁₄ aryl group optionally having substituent(s) selected from the group consisting of a halogen atom and a cyano,
when ring B is a ring represented by formula: 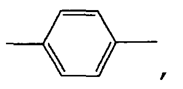 then R⁸ and R⁹ are not hydrogen atoms.

12. A compound represented by the formula (I-a): 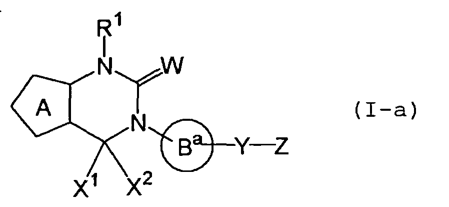 wherein
ring A is a 5-membered aromatic heterocycle optionally having substituent(s),
R¹ is a hydrogen atom or a hydrocarbon group optionally having substituent (s),
W is an oxygen atom or a sulfur atom,
X¹ and X² may be the same or different and each is a hydrogen atom, a hydrocarbon group optionally having substituent(s) or a heterocyclic group optionally having substituent(s), or X¹ and X² optionally form an oxygen atom, a sulfur atom or =NR² wherein R² is a hydrogen atom or a hydrocarbon group optionally having substituent(s) in combination,
ring B^{a} is a ring represented by formula: 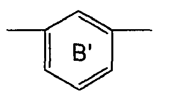 wherein ring B' is a benzene ring optionally further having substituent(s),
or an aromatic heterocycle optionally further having substituent(s),
Y is a bond, a C₁₋₆ alkylene optionally having substituent(s), a C₂₋₆ alkenylene optionally having substituent (s) or a C₂₋₆ alkynylene optionally having substituent(s), and
Z is a group represented by formula: -SOₙR^{3a} wherein n is 0, 1 or 2, and R^{3a} is an amino optionally having substituent (s), a C₂₋₂₀ hydrocarbon group optionally having substituent(s) or a heterocyclic group optionally having substituent(s), or
a group represented by formula: -COR⁴ wherein R⁴ is an amino optionally having substituent(s), a hydrocarbon group optionally having substituent(s) or a heterocyclic group optionally having substituent(s),
or a salt thereof.

13. The compound of claim 12, wherein ring A is a 5-membered aromatic heterocycle containing, as ring-constituting atom besides carbon atom, 1 to 4 hetero atoms selected from the group consisting of an oxygen atom, a sulfur atom and a nitrogen atom and optionally having substituent(s) selected from the group consisting of (i) - (xi),
(i) a C₁₋₆ alkyl group optionally having substituent(s) selected from the group consisting of (a) - (d),
(a) a hydroxy,
(b) a carboxyl,
(c) a C₁₋₆ alkoxycarbonyl, and
(d) an amino optionally mono- or di-substituted with a C₁₋₆ alkyl optionally having substituent(s) selected from the group consisting of a C₁₋₆ alkoxy and a 5- to 16-membered heterocyclic group containing, as ring-constituting atom besides carbon atom, 1 to 4 hetero atoms selected from the group consisting of an oxygen atom, a sulfur atom and a nitrogen atom,
(ii) a C₂₋₆ alkenyl group optionally having a C₁₋₆ alkoxycarbonyl,
(iii) a C₆₋₁₄ aryl group optionally having a halogen atom,
(iv) a 5- to 16-membered heterocyclic group containing, as ring-constituting atom besides carbon atom, 1 to 4 hetero atoms selected from the group consisting of an oxygen atom, a sulfur atom and a nitrogen atom and optionally having a C₁₋₆ alkyl,
(v) a cyano,
(vi) a carboxyl,
(vii) a formyl,
(viii) a C₁₋₆ alkoxycarbonyl,
(ix) HO-N=CH-,
(x) a carbamoyl optionally mono- or di-substituted with substituent(s) selected from the group consisting of (a) - (b),
(a) a C₁₋₆ alkyl group optionally having substituent(s) selected from the group consisting of a hydroxy, a C₁₋₆ alkoxycarbonyl, carboxyl and a 5- to 16-membered heterocyclic group containing, as ring-constituting atom besides carbon atom, 1 to 4 hetero atoms selected from the group consisting of an oxygen atom, a sulfur atom and a nitrogen atom, and
(b) a 5- to 16-membered heterocyclic group containing, as ring-constituting atom besides carbon atom, 1 to 4 hetero atoms selected from the group consisting of an oxygen atom, a sulfur atom and a nitrogen atom, and
(xi) a 5- to 16-membered heterocycle containing, as ring-constituting atom besides carbon atom, 1 to 4 hetero atoms selected from the group consisting of an oxygen atom, a sulfur atom and a nitrogen atom, which is formed by two substituents bonded to each other and optionally has a C₁₋₆ alkoxy group,
R¹ is a hydrogen atom,
W is an oxygen atom,
X¹ and X² are hydrogen atoms, or X¹ and X² form an oxygen atom in combination,
ring B^{a} is a ring represented by formula: 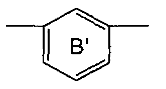 wherein ring B' is a benzene ring optionally further having a halogen atom, or a 5- to 7-membered monocyclic aromatic heterocycle containing, as ring-constituting atom besides carbon atom, 1 to 4 hetero atoms selected from the group consisting of an oxygen atom, a sulfur atom and a nitrogen atom or a 8- to 16-membered bicyclic or tricyclic fused aromatic heterocycle containing, as ring-constituting atom besides carbon atom, 1 to 12 hetero atoms selected from the group consisting of an oxygen atom, a sulfur atom and a nitrogen atom, each of which optionally further has a halogen atom,
Y is a bond, and
Z is,
(i) a group represented by formula: -SOₙR³
wherein n is 2, and R³ is a 5- to 16-membered cyclic amino, or
(ii) a group represented by formula: -CONR⁸(R⁹)
wherein R⁸ is a hydrogen atom, a C₁₋₆ alkyl group or a C₃₋₈ cycloalkyl group, and R⁹ is a hydrogen atom, or a C₆₋₁₄ aryl group optionally having substituent(s) selected from the group consisting of a halogen atom and a cyano.

14. The compound of claim 1, which is selected from the group consisting of
methyl 3-[4-(3,4-dihydroquinolin-1(2H)-ylsulfonyl)-2-thienyl]-2,4-dioxo-1,2,3,4-tetrahydrothieno[3,4-d]pyrimidine-5-carboxylate,
3-[4-(3,4-dihydroquinolin-1(2H)-ylsulfonyl)-2-thienyl]-5-(hydroxymethyl)thieno[3,4-d]pyrimidin-2,4(1H,3H)-dione, and
3-(4-{[ethyl(phenyl)amino]carbonyl}-2-thienyl)-2,4-dioxo-1,2,3,4-tetrahydrothieno[3,4-d]pyrimidine-5-carboxylic acid, and a salt thereof.

15. A prodrug of the compound of claim 1.

16. A pharmaceutical agent comprising the compound of claim 1 or a prodrug thereof.

17. A gonadotropin releasing hormone antagonist comprising the compound of claim 1 or a prodrug thereof.

18. An agent for the prophylaxis or treatment of a sex hormone-dependent disease, comprising the compound of claim 1 or a prodrug thereof.

19. An agent for the prophylaxis or treatment of prostatomegaly, hysteromyoma, endometriosis, metrofibroma, precocious puberty, amenorrhea, premenstrual syndrome, dysmenorrhea, polycystic ovary syndrome, acne, baldness, Alzheimer's disease, infertility, irritable bowel syndrome, prostate cancer, uterine cancer, breast cancer or pituitary tumor, a reproduction regulator, a contraceptive, an ovulation inducing agent, or a preventive agent for post-operative recurrence of sex hormone-dependent cancers, a preventive of premature ovulation during external fertilization or embryo transplantation, or a preventive of ovulation due to endogenous LH during external fertilization, which comprises the compound of claim 1 or a prodrug thereof.

20. A method for the prevention and/or treatment of prostatomegaly, hysteromyoma, endometriosis, metrofibroma, precocious puberty, amenorrhea, premenstrual syndrome, dysmenorrhea, polycystic ovary syndrome, acne, baldness, Alzheimer's disease, infertility or irritable bowel syndrome, prostate cancer, uterine cancer, breast cancer or pituitary tumor, a method for reproduction regulation, a method for contraception, a method for ovulation induction, a method for prevention of post-operative recurrence of sex hormone-dependent cancers, a method for prevention of premature ovulation during external fertilization or embryo transplantation, or a method for prevention of ovulation due to endogenous LH during external fertilization, which comprises administrering an effective amount of the compound of claim 1 or a prodrug thereof to a mammal.

21. Use of the compound of claim 1 or a prodrug thereof for the production of an agent for the prophylaxis or treatment of prostatomegaly, hysteromyoma, endometriosis, metrofibroma, precocious puberty, amenorrhea, premenstrual syndrome, dysmenorrhea, polycystic ovary syndrome, acne, baldness, Alzheimer's disease, infertility, irritable bowel syndrome, prostate cancer, uterine cancer, breast cancer or pituitary tumor, a reproduction regulator, a contraceptive, an ovulation inducing agent, a preventive agent for post-operative recurrence of sex hormone-dependent cancers, a preventive of premature ovulation during external fertilization or embryo transplantation, or a preventive of ovulation due to endogenous LH during external fertilization.
